(19) Europäisches Patentamt / European Patent Office / Office européen des brevets

(11) **EP 1 914 307 A2**

(12) **EUROPEAN PATENT APPLICATION**

(43) Date of publication:
   **23.04.2008 Bulletin 2008/17**

(51) Int Cl.:
   *C12N 9/54* *(2006.01)*   *C11D 3/386* *(2006.01)*

(21) Application number: **07118727.2**

(22) Date of filing: **30.01.2004**

(84) Designated Contracting States:
   **AT BE BG CH CY CZ DE DK EE ES FI FR GB GR HU IE IT LI LU MC NL PT RO SE SI SK TR**

(30) Priority: **30.01.2003 DK 200300119**
   **05.02.2003 US 445300 P**
   **07.05.2003 DK 200300689**

(62) Document number(s) of the earlier application(s) in accordance with Art. 76 EPC:
   **04706617.0 / 1 590 454**

(71) Applicant: **Novozymes A/S**
   **2880 Bagsvaerd (DK)**

(72) Inventors:
   • **Svendsen, Allan**
    **2970 Hoersholm (DK)**
   • **Draborg, Henriette**
    **3600 Frederikssund (DK)**
   • **Tindbaek, Nikolaj**
    **2100 Copenhagen OE (DK)**

Remarks:
   This application was filed on 18-10-2007 as a divisional application to the application mentioned under INID code 62.

(54) **Subtilases**

(57)   The present invention relates to methods for producing variants of a parent TY145 subtilase and of a parent BPN' subtilase and to TY145 and BPN' variants having altered properties as compared to the parent TY145/BPN' subtilase.

EP 1 914 307 A2

Description

## FIELD OF THE INVENTION

The present invention relates to variants of TY145 subtilases and BPN' subtilases and to methods of construction such variants with altered properties, such as stability (e.g. thermostability or storage stability), $Ca^{2+}$ dependency, pH dependent activity.

## BACKGROUND OF THE INVENTION

Enzymes have been used within the detergent industry as part of washing formulations for more than 30 years. Proteases are from a commercial perspective the most relevant enzyme in such formulations, but other enzymes including lipases, amylases, cellulases, hemicellulases or mixtures of enzymes are also often used.

To improve the cost and/or the performance of proteases there is an ongoing search for proteases with altered properties, such as increased activity at low temperatures, increased thermostability, increased specific activity at a given pH, altered $Ca^{2+}$ dependency, increased stability in the presence of other detergent ingredients (e.g. bleach, surfactants etc.) etc.

The search for proteases with altered properties include both discovery of naturally occurring proteases, i.e. so called wild-type proteases but also alteration of well-known proteases by e.g. genetic manipulation of the nucleic acid sequence encoding said proteases. Knowledge of the relationship between the three-dimensional structure and the function of a protein has improved the ability to evaluate which areas of a protein to alter to affect a specific characteristic of the protein.

One family of proteases, which are often used in detergents, are the subtilases. This family has previously been further grouped into 6 different sub-groups by Siezen RJ and Leunissen JAM, 1997, Protein Science, 6, 501-523. One of these sub-groups is the Subtilisin family which includes subtilases such as BPN', subtilisin 309 (SAVINASE®, NOVOZYMES A/S), subtilisin Carlsberg (ALCALASE®, NOVOZYMES A/S), subtilisin S41 (a subtilase from the psychrophilic Antarctic *Bacillus* TA41, Davail S et al. 1994, The Journal of Biological Chemistry, 269(26), 99. 17448-17453), subtilisin S39 (a subtilase from the psychrophilic Antarctic *Bacillus* TA39, Narinx E et al. 1997, Protein Engineering, 10 (11), pp. 1271-1279) and TY145 (a subtilase from Bacillus sp. TY145, NCIMB 40339 described in WO 92/17577).

However, despite the sequence homology between the subtilases belonging to the Subtilisin subgroup of subtilases, modelling of the three-dimensional structure of one subtilase on the basis of the three-dimensional structure of another subtilase may result in an incorrect three-dimensional structure because of structural differences.

The inventors of the present invention have elucidated the three-dimensional structure of the TY145 subtilase and found that there are several differences between this and the three-dimensional structure of BPN' also belonging to the Subtilisin subgroup of subtilases. This surprising difference in structure makes it advantageous to use the TY145 structure as basis for homology modelling of TY145 like subtilisins, which, in turn, will improve the ability to obtain desired changes in functionality by protein engineering.

Two studies have used protein engineering to alter functionality of TY145 like subtilisins: Miyazaki K et al. 2000, J Mol Biol, 297, pp.1015-1026 discloses enhancement of the thermostability and activity of the psychrophilic protease subtilisin S41 by methods of directed evolution.

Wintrode TL et al. 2000, Journal of Biological Chemistry, 275 (41), pp.31635-31640 discloses conversion of a mesophilic subtilisin-like protease from *Bacillus sphaericus* SSII into its psychrophilic counterpart by methods of directed evolution. Wintrode et al. constructed the three-dimensional structural model of the SSII subtilase on basis of its homology with subtilisins Carlsberg, Savinase, BPN' and Thermitase. However, according to the present invention the SSII subtilase pertain to the new group of TY145 like subtilases and thus the modelling of SSII based on the 3D structure of the BPN' like subtilases will likely give an inaccurate result.

The differences between the three-dimensional structures of TY145 and BPN' are confirmed by the recently published three-dimensional structure of the subtilase "sphericase" from B*acillus sphaericus* (PDB NO: 1 EA7, Protein Data Bank). The overall structure and many details of this subtilase are very homologous to the TY145 subtilase structure.

## BRIEF DESCRIPTION OF THE INVENTION

The inventors have modified the amino acid sequence of a subtilase to obtain variants with improved properties, based on the three-dimensional structure of the subtilases TY145 and BPN'. The variants have altered properties, such as increased activity at low temperatures, increased thermostability, increased specific activity at a given pH, altered $Ca^{2+}$ dependency, increased stability in the presence of other detergent ingredients (e.g. bleach, surfactants etc.) etc.

Accordingly, the object of the present invention is to provide a method for constructing subtilases having altered properties, in particular to provide a method for constructing subtilases having altered properties as described above.

Thus, in its broadest aspect, the present invention relates to a method for constructing a variant of a parent subtilase, wherein the variant has at least one altered property as compared to said parent subtilase, which method comprises:

i) analyzing the three-dimensional structure of the subtilase to identify, on the basis of an evaluation of structural considerations, at least one amino acid residue or at least one structural region of the subtilase, which is of relevance for altering said property;
ii) constructing a variant of the subtilase, which as compared to the parent subtilase, has been modified in the amino acid residue or structural part identified in i) so as to alter said property; and
iii) testing the resulting subtilase variant for said property.

Although it has been described in the following that modification of the parent subtilase in certain regions and/or positions is expected to confer a particular effect to the thus produced subtilase variant, it should be noted that modification of the parent subtilase in any of such regions may also give rise to any other of the above-mentioned effects. For example, any of the regions and/or positions mentioned as being of particular interest with respect to, e.g., improved thermostability, may also give rise to, e.g., higher activity at a lower pH, an altered pH optimum, or increased specific activity, such as increased peptidase activity.

Further aspects of the present invention relates to variants of a subtilase, the DNA encoding such variants and methods of preparing the variants. Still further aspects of the present invention relates to the use of the variants for various industrial purposes, in particular as an additive in detergent compositions. Other aspects of the present invention will be apparent from the below description as well as from the appended claims.

## BRIEF DESCRIPTION OF APPENDIX

APPENDIX 1 shows the structural coordinates for the solved crystal 3D structure of the TY145 subtilase.

## BRIEF DESCRIPTION OF DRAWINGS

Figure 1 shows a multiple alignment of 3D sequences of subtilases from TY145, TA39, TA41, *Bacillus sphaericus* and Savinase.

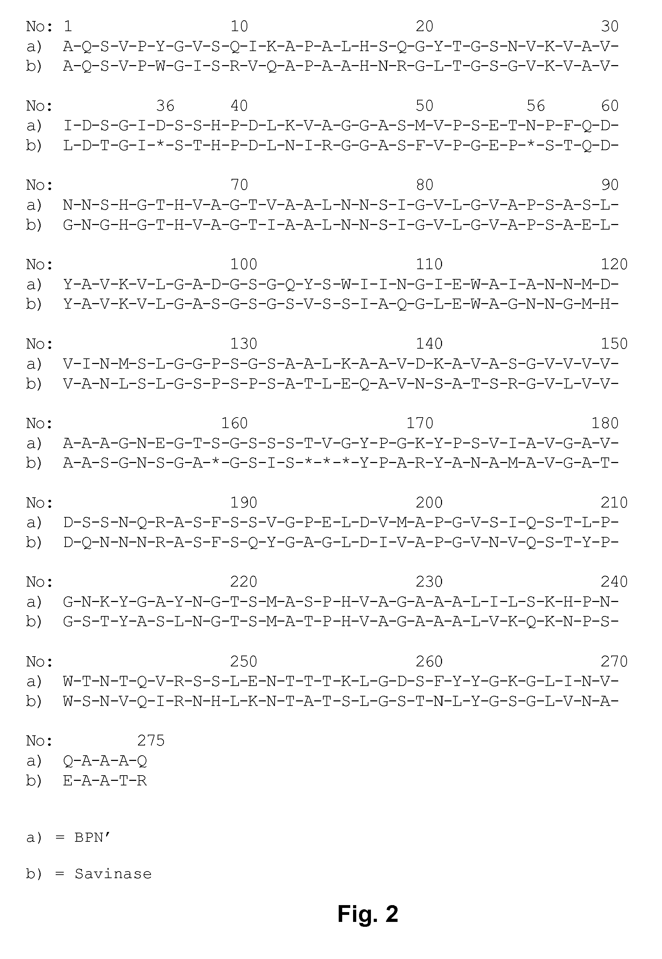

Figure 2 shows an alignment between the amino acid sequences of subtilisin BPN' and Savinase in order to define the BPN' numbering of Savinase.

Figure 3 shows a superposition of TY145 subtilase (light) and BPN' structures (dark), with spheres indicating ion-binding sites. The TY145 ion-binding sites are light and the BPN' ion-binding sites are dark.

## DEFINITIONS

Prior to discussing this invention in further detail, the following terms and conventions will first be defined.

For a detailed description of the nomenclature of amino acids and nucleic acids, we refer to WO 00/71691 page 5, hereby incorporated by reference. A description of the nomenclature of modifications introduced in a polypeptide by genetic manipulation can be found in WO 00/71691 page 7-12, hereby incorporated by reference.

The term "subtilases" refer to a sub-group of serine protease according to Siezen et al., Protein Engng. 4 (1991) 719-737 and Siezen et al. Protein Science 6 (1997) 501-523. Serine proteases or serine peptidases is a subgroup of proteases characterised by having a serine in the active site, which forms a covalent adduct with the substrate. Further the subtilases (and the serine proteases) are characterised by having two active site amino acid residues apart from the serine, namely a histidine and an aspartic acid residue.

Subtilases are defined by homology analysis of more than 170 amino acid sequences of serine proteases previously referred to as subtilisin-like proteases. The subtilases may be divided into 6 sub-divisions, i.e. the Subtilisin family, the Thermitase family, the Proteinase K family, the Lantibiotic peptidase family, the Kexin family and the Pyrolysin family.

The Subtilisin family (EC 3.4.21.62) may be further divided into 3 sub-groups, i.e. I-S1 ("true" subtilisins), I-S2 (highly alkaline proteases) and intracellular subtilisins. Definitions or grouping of enzymes may vary or change, however, in the context of the present invention the above division of subtilases into sub-division or sub-groups shall be understood as those described by Siezen et al., Protein Engng. 4 (1991) 719-737 and Siezen et al. Protein Science 6 (1997) 501-523.

The term "parent" is in the context of the present invention to be understood as a protein, which is modified to create a protein variant. The parent protein may be a naturally occurring (wild-type) polypeptide or it may be a variant thereof prepared by any suitable means. For instance, the parent protein may be a variant of a naturally occurring protein which has been modified by substitution, chemical modification, deletion or truncation of one or more amino acid residues, or by addition or insertion of one or more amino acid residues to the amino acid sequence, of a naturally-occurring polypeptide. Thus the term "parent subtilase" refers to a subtilase which is modified to create a subtilase variant.

The term "variant" is in the context of the present invention to be understood as a protein which has been modified as compared to a parent protein at one or more amino acid residues.

The term "modification(s)" or "modified" is in the context of the present invention to be understood as to include chemical modification of a protein as well as genetic manipulation of the DNA encoding a protein. The modification(s) may be replacement(s) of the amino acid side chain(s), substitution(s), deletion(s) and/or insertions in or at the amino acid(s) of interest. Thus the term "modified protein", e.g. "modified subtilase", is to be understood as a protein which contains modification(s) compared to a parent protein, e.g. subtilase.

The term "(a) TY145 subtilase" or "(a) TY145 like subtilase" should in the context of the present invention be understood as a subtilase belonging to the Subtilisin group according to Siezen et al. Protein Science 6 (1997) 501-523 and which has at least 63% homology to TY145, SEQ ID NO:1. In the context of the present invention a TY145 subtilase has three ion-binding sites.

The term "(a) BPN' subtilase" or "(a) BPN' like subtilase" should in the context of the present invention be understood as a subtilase belonging to the Subtilisin group according Siezen et al. Siezen et al. Protein Science 6 (1997) 501-523 and which has at least 61% homology to BPN' SEQ ID NO:5. Such a BPN' like subtilase is for example Savinase. In the context of the present invention a BPN' subtilase has two, three or five ion-binding sites. A BPN' like subtilase may, in the context of the present invention, belong to branch I-S of the subtilisins i.e. to branch I-S1, the "true" subtilisins or I-S2, the highly alkaline proteases (Siezen et al., Protein Engng. 4 (1991) 719-737).

"Homology" or "homologous to" is in the context of the present invention to be understood in its conventional meaning and the "homology" between two amino acid sequences should be determined by use of the "Similarity" defined by the GAP program from the University of Wisconsin Genetics Computer Group (UWGCG) package using default settings for alignment parameters, comparison matrix, gap and gap extension penalties. Default values for GAP penalties, i.e. GAP creation penalty of 3.0 and GAP extension penalty of 0.1 (Program Manual for the Wisconsin Package, Version 8, August 1994, Genetics Computer Group, 575 Science Drive, Madison, Wisconsin, USA 53711). The method is also described in S.B. Needleman and C.D. Wunsch, Journal of Molecular Biology, 48, 443-445 (1970). Identities can be extracted from the same calculation. The homology between two amino acid sequences can also be determined by "identity" or "similarity" using the GAP routine of the UWGCG package version 9.1 with default setting for alignment parameters, comparison matrix, gap and gap extension penalties can also be applied using the following parameters: gap creation penalty = 8 and gap extension penalty = 8 and all other parameters kept at their default values. The output from the routine is besides the amino acid alignment the calculation of the "Percent Identity" and the "Similarity" between the two sequences. The numbers calculated using UWGCG package version 9.1 is slightly different from the version 8.

The term "position" is in the context of the present invention to be understood as the number of an amino acid in a peptide or polypeptide when counting from the N-terminal end of said peptide/polypeptide. The position numbers used in the present invention refer to different subtilases depending on which subgroup the subtilase belongs to.

The four known subtilases belonging to the TY145 subgroup, i.e. subtilases obtained from TY145, TA39, TA41 and *Bacillus sphaericus* are numbered individually according to each of SEQ ID NO:1,2,3 and 4.

Likewise other subtilases belonging to the TY145 subgroup are numbered individually according to their own sequence. However in order to determine homologous positions in such other subtilases an alignment with each of SEQ ID's NO:1,2,3 and 4 is conducted according to the GAP procedure described above. Subsequently the homologous positions are determined with reference to the most homologous of SEQ ID's NO:1,2,3 and 4.

Alternatively subtilases belonging to the TY145 subgroup can be numbered by reference to the positions of TY145 subtilase (SEQ ID NO:1).

Subtilases belonging to the BPN' subgroup refers to the positions of Subtilisin Novo (BPN') from *B. amyloliquefaciens* (SEQ ID NO:5).

## DETAILED DESCRIPTION OF THE INVENTION

Despite the great homology of the subtilases described above the inventors of the present invention have elucidated the three-dimensional structure of TY145, SEQ ID NO:1 by X-ray crystallography and found that there are several substantial differences between the three dimensional structures of TY145 and BPN'. The inventors of the present invention have further compared the sequence homology of a representative number of subtilases belonging to the Subtilisin subgroup. This is shown in the homology matrix in Table 1 below.

Table 1

| No. | 1 | 2 | 3 | 4 | 5 | 6 | 7 | 8 | 9 | 10 | 11 | 12 | 13 | 14 | 15 |
|---|---|---|---|---|---|---|---|---|---|---|---|---|---|---|---|
| 1 | **100** | **93** | **76** | 51 | 50 | 51 | 55 | 52 | 54 | 58 | 58 | 59 | 57 | 60 | 60 |
| 2 | | **100** | 75 | 52 | 52 | 52 | 56 | 53 | 55 | 58 | 58 | 61 | 58 | 62 | 61 |
| 3 | | | **100** | 60 | 60 | 60 | 58 | 60 | 62 | 58 | 57 | 59 | 59 | 62 | 59 |
| 4 | | | | **100** | 99 | 99 | 97 | 91 | **76** | 63 | 69 | 74 | 66 | 74 | 74 |
| 5 | | | | | 100 | 99 | 97 | 90 | **76** | 69 | 74 | 66 | 74 | 74 | 56 |
| 6 | | | | | | 100 | 98 | 91 | **77** | 63 | 69 | 74 | 66 | 74 | 74 |
| 7 | | | | | | | 100 | 88 | **79** | 69 | 67 | 74 | 74 | 74 | 74 |
| 8 | | | | | | | | 100 | **77** | 66 | 71 | 74 | 67 | 74 | 74 |
| 9 | | | | | | | | | 100 | 64 | 69 | 74 | 67 | 73 | 73 |
| 10 | | | | | | | | | | 100 | 99 | 76 | 72 | 76 | 76 |
| 11 | | | | | | | | | | | 100 | 76 | 76 | 76 | 76 |
| 12 | | | | | | | | | | | | 100 | 99 | 99 | 99 |
| 13 | | | | | | | | | | | | | 100 | 99 | 99 |
| 14 | | | | | | | | | | | | | | 100 | 98 |
| 15 | | | | | | | | | | | | | | | 100 |

Legend to Table 1
TY145 like subtilases:
1: q45681; Subtilase derived from *B. subtilis* (BSTA41)
2: p28842; Psychrophilic subtilisin derived from Antarctic *Bacillus* strain (BSTA39)
3: abb77095; Subtilase derived from *Bacillus sp.* (TY145)
BPN' like subtilases, I-S1:
4: p00783; Subtilase derived from *Bacillus subtilis var. amylosacchariticus* (BSAMY)
5: p29142; Subtilase derived from *Bacillus stearothermophilus* (BSSJ)
6: p35835; Subtilase derived from *Bacillus subtilis var. natto.* (BSNAT)
7: p07518; Subtilase derived from *Bacillus pumilus (B. mesentericus)* (BPMES)
8: p00782; Subtilase derived from *Bacillus amyloliquefaciens* (BPN')
9: p00780; Subtilase derived from *Bacillus licheniformis* (BLSCAR) BPN' like subtilases, I-S2
10: p41363; Subtilase derived from *Bacillus halodurans* (BHSAH)
11: aaw62222; Subtilase derived from *Bacillus lentus* (BLS147)
12: p29600; Subtilase derived from *Bacillus lentus* (BLSAVI, BLS309)
13: p27693; Subtilase derived from *Bacillus alcalophilus* (BAALKP)
14: q99405; Subtilase derived from *Bacillus sp.* strain KSM-K16 (BSKSMK)
15: p29599; Subtilase derived from *Bacillus lentus* (BLSUBL).

On the basis of the 3D structure comparison and protein sequence the inventors of the present invention find that the subgroup of TY145 subtilases are different from BPN' subtilases based on the 3D structure comparison of the enclosed 3D structure of TY145 and the BPN' 3D structure but also indicated from the sequence homology between TY145 and BPN'.

TY145 subtilases

As described above a TY145 subtilase is in the context of the present invention to be understood as a subtilase which has at least 63% homology to SEQ ID NO:1. In particular said TY145 subtilase may have at least 65%, such as at least 70%, at least 74%, at least 80%, at least 83%, at least 90%, at least 91 %, at least 92%, at least 93%, at least 94%, at least 95%, at least 96%, at least 97%, at least 98% or at least 99% homology to TY145, i.e. to SEQ ID NO:1.
In a first embodiment of the present invention a TY145 subtilase suitable for the purpose described herein may be a subtilase homologous to the three-dimensional structure of TY145, i.e. it may be homologous to the three-dimensional structure defined by the structure coordinates in Appendix 1.

As it is well-known to a person skilled in the art that a set of structure coordinates for a protein or a portion thereof is a relative set of points that define a shape in three dimensions, it is possible that an entirely different set of coordinates could define an identical or a similar shape. Moreover, slight variations in the individual coordinates may have little or no effect on the overall shape.

These variations in coordinates may be generated because of mathematical manipulations of the structure coordinates. For example, the structure coordinates of Appendix 1 (TY145 structure) may be manipulated by crystallographic permutations of the structure coordinates, fractionalization of the structure coordinates, integer additions or subtractions to sets of the structure coordinates, inversion of the structure coordinates or any combination of the above. Alternatively, said variations may be due to differences in the primary amino acid sequence.

If such variations are within an acceptable standard error as compared to the structure coordinates of Appendix 1 said three-dimensional structure is within the context of the present invention to be understood as being homologous to the structure of Appendix 1. The standard error may typically be measured as the root mean square deviation of e.g. conserved backbone residues, where the term "root mean square deviation" (RMS) means the square root of the arithmetic mean of the squares of the deviations from the mean.

As it is also well-known to a person skilled in the art that within a group of proteins which have a homologous structure there may be variations in the three-dimensional structure in certain areas or domains of the structure, e.g. loops, which are not or at least only of a small importance to the functional domains of the structure, but which may result in a big root mean square deviation of the conserved residue backbone atoms between said structures.

Thus it is well known that a set of structure coordinates is unique to the crystallised protein. No other three dimensional structure will have the exact same set of coordinates, be it a homologous structure or even the same protein crystallised in different manner. There are natural fluctuations in the coordinates. The overall structure and the interatomic relationship can be found to be similar. The similarity can be discussed in terms of root mean square deviation of each atom of a structure from each "homologous" atom of another structure. However, only identical proteins have the exact same number of atoms. Therefore, proteins having a similarity below 100% will normally have a different number of atoms, and thus the root mean square deviation can not be calculated on all atoms, but only the ones that are considered "homologous". A precise description of the similarity based on the coordinates is thus difficult to describe and difficult to compute for homologous proteins. Regarding the present invention, similarities in 3D structure of different subtilases can be described by the content of homologous structural elements, and/or the similarity in amino acid or DNA sequence. For sequences having no deletions or insertions a RMS for the calcium atoms can be calculated.

Examples of TY145 like subtilases include the psychrophilic subtilisin protease S41 derived from the Antarctic *Bacillus* TA41, herein also called TA41 subtilase (Davail S et al., 1994, J. Biol. Chem., 269, 17448-17453), and the psychrophilic subtilisin protease S39 derived from the Antarctic *Bacillus* TA39, herein also called TA39 subtilase (Narinx E et al., 1997, Protein Engineering, 10 (11), 1271-1279). Recently a three-dimensional structure of a subtilisin homologous with the TY145 subtilisins was published in the Protein Data Bank (Accession No:1 EA7). The overall structure and many details of this *Bacillus sphaericus* "sphericase" subtilase are very homologous with the TY145 subtilase structure; however the structure of the sphericase revealed as much as five ion-binding sites. The number of ion-binding sites may vary in similar structures depending on the medium used for crystallisation. Thus it appears that the two extra ion-binding sites of *Bacillus sphaericus* "sphericase" are due to a calcium containing crystallisation medium.

Accordingly, a preferred embodiment of the present invention is a parent subtilase or a subtilase variant which is at least 63% homologous to the sequence of SEQ ID NO:1, preferably at least 65%, at least 70%, at least 74%, at least 80%, at least 83%, at least 90%, at least 91 %, at least 92%, at least 93%, at least 94%, at least 95%, at least 96%, at least 97%, at least 98% or at least 99% homologous to the sequence of SEQ ID NO:1, and optionally said subtilase further comprises the following structural characteristics:

> a) a twisted beta-sheet with 7 strands,
> b) six alpha helices,
> c) at least three ion-binding sites and

wherein the Strong ion-binding site of the BPN' like subtilases is not present, and with the exception of the TY145 subtilase, the TA39 subtilase, the TA41 subtilase, and the *Bacillus sphaericus* "sphericase".

The TY145 subtilase of the present invention is encoded by an isolated nucleic acid sequence, which nucleic acid sequence has at least 45%, at least 50%, at least 55%, at least 60%, at least 65%, at least 70%, at least 75%, at least 80%, at least 85%, at least 90%, at least 91 %, at least 92%, at least 93%, at least 94%, at least 95%, at least 96%, at least 97%, at least 98%, or at least 99% homology with the nucleic acid sequence shown in SEQ ID NO:20.

Further the isolated nucleic acid sequence encoding a TY145 subtilase of the invention hybridizes with a complementary strand of the nucleic acid sequence shown in SEQ ID NO:20 preferably under low stringency conditions, at least under medium stringency conditions, at least under medium/high stringency conditions, at least under high stringency conditions, at least under very high stringency conditions.

Suitable experimental conditions for determining hybridization at *low, medium, or high stringency between a nucleotide probe and a homologous DNA or RNA sequence involves presoaking of the filter containing the DNA fragments or RNA to hybridize in 5 x SSC (Sodium chloride/Sodium citrate, Sambrook et al. 1989) for 10 min, and prehybridization of the filter in a solution of 5 x SSC, 5 x Denhardt's solution (Sambrook et al. 1989), 0.5 % SDS and 100 $\mu$g/ml of denatured sonicated salmon sperm DNA (Sambrook et al. 1989), followed by hybridization in the same solution containing a concentration of 10ng/ml of a random-primed (Feinberg, A. P. and Vogelstein, B. (1983) Anal. Biochem. 132:6-13), $^{32}$P-dCTP-labeled (specific activity > 1 x 10$^9$ cpm/$\mu$g ) probe for 12 hours at ca. 45°C. The filter is then washed twice for 30 minutes in 2 x SSC, 0.5 % SDS at least * 55°C (low stringency), more preferably at least 60°C (medium stringency), still more preferably at least 65°C (medium/high stringency), even more preferably at least 70°C (high stringency), and even more preferably at least 75°C (very high stringency).

BPN' subtilases

As described above a BPN' subtilase is in the context of the present invention to be understood as a subtilase which has at least 61% homology to SEQ ID NO:5. In particular said BPN' subtilase may have at least 70%, such as at least 75%, at least 80%, at least 85%, at least 90%, at least 91 %, at least 92%, at least 93%, at least 94%, at least 95%, at least 96%, at least 97%, at least 98% or at least 99% homology to BPN', i.e. to SEQ ID NO:5.

In one embodiment of the present invention a BPN' subtilase suitable for the purpose described herein may be a subtilase homologous to the three-dimensional structure of BPN' as defined by the structure coordinates given in PDB Nos. 1SBT and 1GNS (Protein Data Bank), or one of the several other structures of BPN' that are accessible from the Protein Data Bank. Variations between homologous structures may occur for several reasons as described above. Thus a BPN' subtilase within the context of the present invention is to be understood as any subtilase having the structural characteristics pertaining to the BPN' subtilases as described above, and in addition such subtilases does preferably not have further structural characteristics which are not present in the BPN' subtilases as described herein. Further a BPN' subtilase of the present invention may have the necessary percentage of similarity with SEQ ID NO:5.

Examples of BPN' like subtilases include the subtilisin 309 (PDB NO:1SVN SAVINASE®, NOVOZYMES A/S) and subtilisin Carlsberg (ALCALASE®, NOVOZYMES A/S), among others.

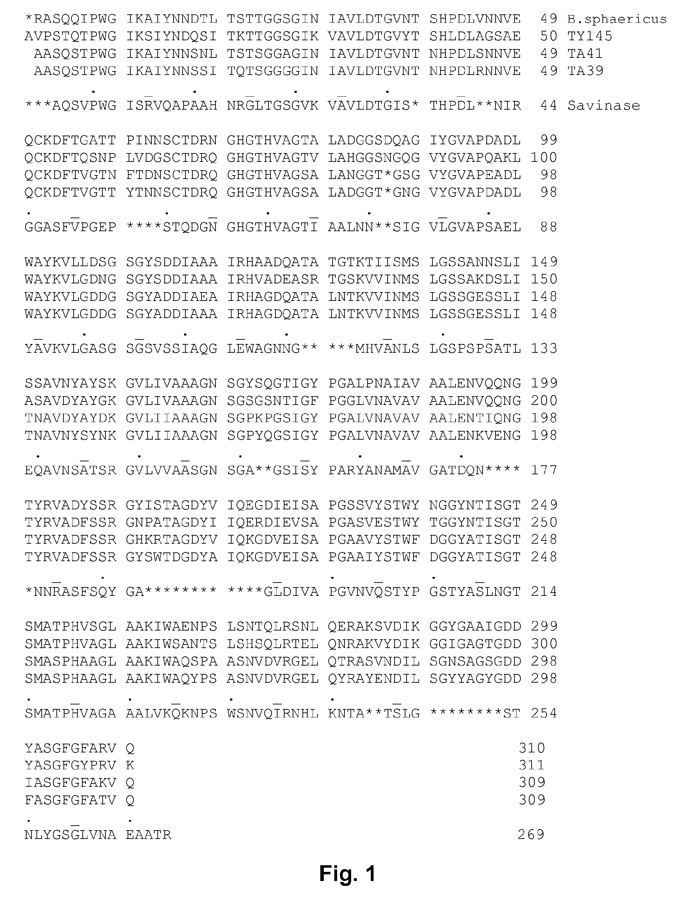

In figure 1 of R.J. Siezen and J.A.M Leunissen (Protein science, Vol. 6 (3), pp. 501-523, 1997) page 502 a structure of subtilases is described. A subtilase consists of 6-8 helices, 11 strands of which 7 are central in a twisted beta-sheet. Two ion-binding sites are mentioned, one of which is the so called "Weak" calcium-binding site. It was later discovered that for some structures (subtilisin DY PDB no. 1 BH6, 1998), this calcium-binding site was shown to be a Na (sodium) binding site when the calcium concentration in the crystallization medium was low. Thus, in the following we refer to ion-binding sites instead of calcium-binding sites.

The BPN' subtilase of the present invention is encoded by an isolated nucleic acid sequence, which nucleic acid sequence has at least 45%, at least 50%, at least 55%, at least 60%, at least 65%, at least 70%, at least 75%, at least 80%, at least 85%, at least 90%, at least 91 %, at least 92%, at least 93%, at least 94%, at least 95%, at least 96%, at least 97%, at least 98%, or at least 99% homology with the nucleic acid sequence shown in SEQ ID NO:21.

Further the isolated nucleic acid sequence encoding a BPN' subtilase of the invention hybridizes with a complementary strand of the nucleic acid sequence shown in SEQ ID NO:21 preferably under low stringency conditions, but at least under medium stringency conditions, at least under medium/high stringency conditions, at least under high stringency conditions, at least under very high stringency conditions.

**Three-dimensional structure of TY145 subtilases**

The TY145 subtilase was used to elucidate the three-dimensional structure forming the basis for the present invention.

The structure of TY145 was solved in accordance with the principle for x-ray crystallographic methods, for example, as given in X-Ray Structure Determination, Stout, G.K. and Jensen, L.H., John Wiley & Sons, Inc. NY, 1989.

The structural coordinates for the solved crystal structure of TY145 are given in standard PDB format (Protein Data Bank, Brookhaven National Laboratory, Brookhaven, CT) as set forth in Appendix 1. It is to be understood that Appendix 1 forms part of the present application. In the context of Appendix 1, the following abbreviations are used: CA refers to c-alpha (carbon atoms) or to calcium ions, (however to avoid misunderstandings we use the full names "c-alpha atoms" and "calcium" or "ion" in the present specification). Amino acid residues are given in their standard three-letter code. The attached structural coordinates contain the protease structure, and an inhibitor structure C12 as well as water molecules. The protease coordinates has a chain identification called A, whereas the C12 inhibitor is called B, the calcium ions are called C, and the water is W. In the following the positions of the mentioned residues refer to the sequence of TY145 as disclosed in SEQ ID NO:1.

The structure of TY145 shows the same "overall" fold as found in the S8 family of subtilisins. The structure comprises a twisted beta-sheet with 7 strands arranged in the following sequential order S2, S3, S1, S4, S5, S6, S7. There are six alpha helices in the structure of which number H1 contains residues 9-15, H2 contains residues 72-81, H3 contains residues 114-131, H4 contains residues 148-158, H5 contains residues 250-267and H6 contains residues 273-286.

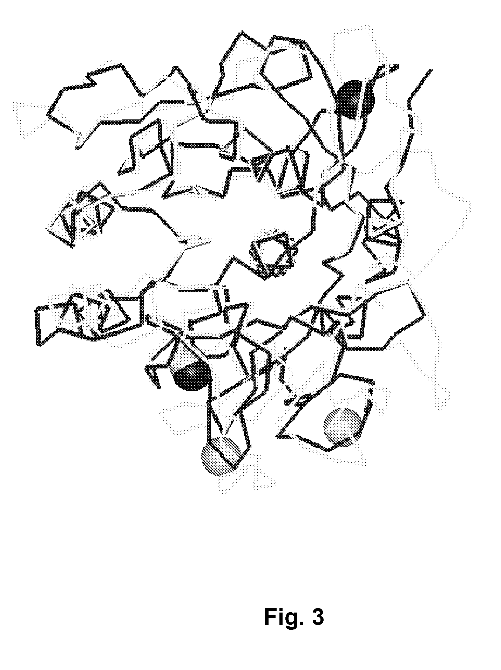

The TY145 like subtilases are shown to lack the well-known Strong ion-binding site of the BPN' subtilases. However, in addition to the Weak calcium or ion-binding site also known from the BPN' subtilases, the TY145 subtilases have two ion-binding sites which are not present in the BPN' subtilisin structures. This can be seen in the structural alignment presented in Figure 3. These additional ion-binding sites are hereinafter referred to as "Near" and "Far" according to their distance to the Weak ion-binding site. Thus in relation to the atomic coordinates disclosed in Appendix 1, the ion-binding sites of TY145 are located at:

> Weak - calcium atom named C 314,
> Near - calcium atom named C 312, and
> Far - calcium atom named C 313 in the PDB table (Appendix 1).

The position of an ion-binding site can be defined by the distance to four specific atoms in the core structure. The distance from the ion-binding site to the c-alpha atoms of the three active site residues has been chosen. Throughout the subtilases the residues Ser, His and Asp in the active site are highly conserved. In TY145 they are Asp35, His72 and Ser251. The fourth distance chosen is the distance to the c-alpha atom of the amino acid residue coming first after the active site serine residue in the sequence (herein after called "next to Ser"); in the 3D structure of TY145 it is Met252.

In a preferred embodiment of the present invention, the distance between:

> a) the Weak ion-binding site and i) Asp c-alpha atom is 17.50-19.50Å, ii) His c-alpha atom is 21-23Å, iii) Ser c-alpha atom is 13.80-15.80Å, iv) next to Ser c-alpha atom is 15.80-17.80Å,
> b) the Far ion-binding site and i) Asp c-alpha atom is 28.70-30.70Å, ii) His c-alpha atom is 28-30Å, iii) Ser c-alpha atom is 20-22Å, iv) next to Ser c-alpha atom is 19.50-21.50Å,
> c) the Near ion-binding site and i) Asp c-alpha atom is 27-29Å, ii) His c-alpha atom is 29.50-31.50Å, iii) Ser c-alpha atom is 21.40-23.40Å, iv) next to Ser c-alpha atom is 22.50-24.50Å.

Below are the specific distances between the four chosen c-alpha atoms and the three ion binding sites of the TY145 subtilase given in Å:

|  | Weak ion-binding site | Far ion-binding site | Near ion-binding site |
| --- | --- | --- | --- |
| Met252 c-alpha atom | 16.75 | 20.35 | 23.58 |
| His72 c-alpha atom | 21.98 | 29.10 | 30.43 |
| Asp35 c-alpha atom | 18.55 | 29.68 | 28.04 |
| Ser251 c-alpha atom | 14.71 | 20.96 | 22.28 |
| Weak ion-binding site | 0 | 16.62 | 9.79 |
| Far ion-binding site | 16.62 | 0 | 12.48 |
| Near ion-binding site | 9.79 | 12.48 | 0 |

However these distances may vary from one subtilase to the other, and as described above, the Weak ion binding site may also bind to a sodium ion. The present distances are given with a calcium ion in the structure. If a sodium ion was bound instead the distances would be shifted a little bit. Generally the distances can vary $\pm 0.8$Å, preferably $\pm 0.7$Å, $\pm 0.6$Å, $\pm 0.5$Å, $\pm 0.4$Å, or most preferably $\pm 0.3$Å.

Further, in the TY145 like subtilases, the peptide structure circumscribing the Weak ion-binding site is composed of the amino acid residues placed in positions 182-189 and 221-227 with the coordinating atoms being the backbone carbonyl oxygen atom of residues G182, A187, L184 and two water molecules.

The peptide structure circumscribing the Near ion-binding site is composed of residues 212-225 with the coordinating atoms being the backbone carbonyl oxygen atom of residues I220 and T215, the oxygens from the carboxylic acids of residues D225 and D218 and the amid group of residue Q222.

The peptide structure circumscribing the Far ion-binding site is composed of residues 288-306 with the coordinating atoms being the backbone carbonyl oxygen atom of residues G298, G296 and I289, the oxygens from the carboxylic acids of residues D300 and D288, and two water molecules.

In comparison with the BPN' like subtilase structures the structure of the TY145 like subtilase can be divided into a "common subtilase-like" region, an "intermediate" region and a "non-homolo-gous" region.

The active site can be found in the common subtilase-like region, which is structurally closely related to the BPN' structures. The common subtilase-like region is composed of residues 88-128 and 225-284, and contains the alpha-helix H3 and the central alpha-helix H5 in which the active site serine residue is situated in the N-terminal part. The common subtilase-like region has an RMS lower than 1.2.

Outside the common subtilase-like region the structure of the TY145 like subtilase differs from the BPN' structures to a greater extent.

The intermediate region consist of residues 24-45, 48-58, 65-66, 67-85, 134-174, 175-196, 202-212 and 287-290. The intermediate region has an RMS higher than 1.2 and lower than 1.8. The relationships between the three-dimensional structure and functionality are potentially difficult to predict in this region of the TY145 like subtilases.

The nonhomologous region consists of residues 5-15, 16-23, 86-87, 129-133, 197-201, 213-124, 285-286, 291-298 and 299-311. The nonhomologous region has a RMS higher than 1.5, which also pertains to residues 65-66 from the intermediate region. The group comprising residues 5-15 and 299-311 has an RMS between 2.1-2.2. The relationships between the three-dimensional structure and functionality are very difficult to predict in this region of the TY145 like subtilases.

The regions in areas A1-T5, N16-T24, A46-Q51, S58-C66, G84-G90, S129-K134, S129-K134, S173-S175, V196-T201, N212-R224, A284-V286, K290-D299 and V310-K311 in the TY145 structure differs significantly from the other S8 family subtilisins (including the BPN' type subtilisins) in c-alpha atom coordinates. An RMS cannot be calculated for these last residues as there are no homologous c-alpha atoms in the compared subtilases.

**Homology building of TY145 and BPN' like subtilases**

A model structure of a TY145 like subtilase or a BPN' like subtilase can be built using the Homology program or a comparable program, e.g., Modeller (both from Molecular Simulations, Inc., San Diego, CA). The principle is to align the amino acid sequence of a protein for which the 3D structure is known with the amino acid sequence of a protein for which a model 3D structure has to be constructed. The structurally conserved regions can then be built on the basis of consensus sequences. In areas lacking homology, loop structures can be inserted, or sequences can be deleted with subsequent bonding of the necessary residues using, e.g., the program Homology. Subsequent relaxing and optimization of the structure should be done using either Homology or another molecular simulation program, e.g., CHARMm from Molecular Simulations.

**Methods for designing TY145 and Subtilisin family subtilase variants**

Comparisons of the molecular dynamics of different proteins can give a hint as to which domains are important or connected to certain properties pertained by each protein.

The present invention comprises a method of producing a variant of a parent TY145 like subtilase, the variant having at least one altered property as compared to the parent TY145 like subtilase, the method comprising:

a) modelling the parent TY145 subtilase on the three-dimensional structure of a TY145 subtilase to produce a three-dimensional structure of the parent TY145 subtilase;
b) comparing the three-dimensional structure obtained in step a) to the three-dimensional structure of a TY145 subtilase;
c) identifying on the basis of the comparison in step b) at least one structural part of the parent TY145 subtilase, wherein an alteration in said structural part is predicted to result in an altered property;
d) modifying the nucleic acid sequence encoding the parent TY145 subtilase to produce a nucleic acid sequence encoding deletion or substitution of one or more amino acids at a position corresponding to said structural part, or an insertion of one or more amino acid residues in positions corresponding to said structural part;
e) expressing the modified nucleic acid sequence in a host cell to produce the variant TY145 subtilase;
f) isolating the produced subtilase;
g) purifying the isolated subtilase and
h) recovering the purified subtilase.

Further the present invention comprises a method of producing a variant of a parent Subtilisin family subtilase, such as a BPN' like subtilase, the variant having at least one altered property as compared to the parent Subtilisin family subtilase, the method comprising:

a) modelling the parent Subtilisin family subtilase on the three-dimensional structure of a Subtilisin family subtilase to produce a three-dimensional structure of the parent Subtilisin family subtilase;

b) comparing the three-dimensional structure obtained in step a) to the three-dimensional structure of a TY145 like subtilase;

c) identifying on the basis of the comparison in step b) at least one structural part of the parent Subtilisin family subtilase, wherein an alteration in said structural part is predicted to result in an altered property;

d) modifying the nucleic acid sequence encoding the parent Subtilisin family subtilase to produce a nucleic acid sequence encoding deletion or substitution of one or more amino acids at a position corresponding to said structural part, or an insertion of one or more amino acid residues in positions corresponding to said structural part;

e) expressing the modified nucleic acid sequence in a host cell to produce the variant Subtilisin family subtilase,

f) isolating the produced subtilase,

g) purifying the isolated subtilase and

h) recovering the purified subtilase.

Further the present invention comprises a method of producing a variant of a parent TY145 like subtilase, the variant having at least one altered property as compared to the parent TY145 like subtilase, the method comprising:

a) modelling the parent TY145 like subtilase on the three-dimensional structure of a TY145 like subtilase to produce a three-dimensional structure of the parent TY145 like subtilase;

b) comparing the three-dimensional structure obtained in step a) to the three-dimensional structure of a Subtilisin family subtilase;

c) identifying on the basis of the comparison in step b) at least one structural part of the parent TY145 like subtilase, wherein an alteration in said structural part is predicted to result in an altered property;

d) modifying the nucleic acid sequence encoding the parent TY145 like subtilase to produce a nucleic acid sequence encoding deletion or substitution of one or more amino acids at a position corresponding to said structural part, or an insertion of one or more amino acid residues in positions corresponding to said structural part;

e) expressing the modified nucleic acid sequence in a host cell to produce the variant TY145 like subtilase;

f) isolating the produced subtilase;

g) purifying the isolated subtilase and

h) recovering the purified subtilase.

**Stability - alteration of ion-binding sites**

As described above the TY145 subtilases has two new ion-binding sites not present in the BPN' subtilisin structures but lacks the Strong ion-binding site of the BPN' subtilases. Stability of the ion-binding site is of crucial importance for the functionality of the enzyme. Therefore alterations of the amino acid residues close to the ion-binding sites are likely to result in alterations of the stability of the enzyme.

The positions which may be modified are located:

Weak: at a distance of 10Å or less around calcium atom named C 314,
Near: at a distance of 10Å or less around calcium atom named C 312, and
Far: at a distance of 10Å or less around calcium atom named C 313 in the PDB table (Appendix 1).

Improved stability

Stabilisation of the ion-binding sites of TY145 may possibly be obtained by alterations in the positions close to the sites. Positions located at a distance of 10Å or less to the ion-binding sites of TY145 (SEQ ID NO:1) are:

Weak: 154, 155, 158, 164, 165, 166, 167, 168, 178-191 (i.e. 178, 179, 180, 181, 182, 183, 184, 185, 186, 187, 188, 189, 190, 191), 211, 220-228 (i.e. 220, 221, 222, 223, 224, 225, 226, 227, 228), 277, 281 and 305.

Near: 185, 211-227 (i.e. 211, 212, 213, 214, 215, 216, 217, 218, 219, 220, 221, 222, 223, 224, 225, 226, 227), 277, 281, 299, 300, 301, 304, 305.

Far: 193, 198, 199, 201, 202, 204, 216, 217, 219, 226, 227, 228, 229 and 284-307 (i.e. 284, 285, 286, 287, 288, 289, 290, 291, 292, 293, 294, 295, 296, 297, 298, 299, 300, 301, 302, 303, 304, 305, 306, 307).

In detergent compositions calcium chelaters contribute to removal of calcium from the subtilases with subsequent inactivation of the enzyme as the result. To decrease the inactivation due to calcium removal of e.g. calcium chelaters, variants with improved calcium stability can be constructed.

Variants with alterations close to the Near ion-binding site are 1220S,T and T215S, variants with alterations close to the Far ion-binding site are G298A,S,T and G296A,S,T, and variants with alterations close to the Weak ion-binding site are V185T and 1221 N,D,T.

TY145 with extra ion-binding site

The Strong ion-binding site from the BPN' subtilases can be transplanted into TY145 (or other subtilases in TY145 subgroup) by deletion(s) of or in the region H83-G90 (of SEQ ID NO:1) and subsequent insertion of one or more amino acid residues. A preferred variant has the whole region deleted and a subsequent insertion between A82 and V91 of the sequence LNNSIG.

Removal of ion-binding site in TY145

By removing a ion-binding site it is possible to alter the enzymes dependency of calcium or other ions in the solution. The Far and Near ion-binding sites in TY145 (or others from TY145 group) can be removed with guidance from the three-dimensional structure of BPN' and Savinase (or others in BPN' group).

Removal of the Far site can be done by deletion(s) of or in the region K290-D300 (of SEQ ID NO:1) and subsequent insertion of one or more amino acid residues. A preferred variant has the whole region deleted and a subsequent insertion between 1289 and Y301 of the sequence GDS or DST. Preferably, but not mandatory the substitution S303Y is further added.

Removal of the Near site can be done by deletion(s) of or in the region N212-R224 (of SEQ ID NO:1) and subsequent insertion of one or more amino acid residues. A preferred variant has the whole region deleted and a subsequent insertion of a proline or alanine residue between G211 and D225.

Removal of Strong ion-binding site in BPN' subtilases

The Strong ion-binding site in BPN' like subtilases can be removed. Exemplified in Savinase, the removal can be done by deletion of or in the region L75-G80 (BPN' numbering) and subsequent insertion of one or more amino acid residues. A preferred variant has the whole region deleted and a subsequent insertion of residues 84-88 from TY145. In addition the substitutions L82Y and Q2A,N can be applied.

**Alteration of thermostability**

A variant with improved stability (typically increased thermostability) may be obtained by substitution with proline, introduction of a disulfide bond, altering a hydrogen bond contact, altering charge distribution, introduction of a salt bridge, filling in an internal structural cavity with one or more amino acids with bulkier side groups (in e.g. regions which are structurally mobile), substitution of histidine residues with other amino acids, removal of a deamidation sites, or by helix capping.

Regions with increased mobility:

The following regions of TY145 have an increased mobility in the crystal structure of the enzyme, and it is presently believed that these regions can be responsible for stability or activity of TY145. Especially thermostabilisation may possibly be obtained by altering the highly mobile regions. Improvements of the enzyme can be obtained by mutation in the below regions and positions. Introducing e.g. larger residues or residues having more atoms in the side chain could increase the stability, or, e.g., introduction of residues having fewer atoms in the side chain could be important for the mobility and thus the activity profile of the enzyme. The regions can be found by analysing the B-factors taken from the coordinate file in Appendix 1, and/or from molecular dynamics calculations of the isotropic fluctuations. These can be obtained by using the program CHARMm from MSI (Molecular Simulations Inc.).

Molecular dynamics simulation at 300K of TY145 reveals the following highly mobile regions:

| | |
|---|---|
| 84-89 | (i.e. 84, 85, 86, 87, 88) |
| 108-117 | (i.e. 108, 109, 110, 111, 112, 113, 114, 115, 116, 117) |
| 141-146 | (i.e. 141, 142, 143, 144, 145, 146) |
| 150-152 | (i.e.150, 151, 152) |
| 169-171 200-201 | (i.e. 169, 170, 171) |
| 211-220 242-243 | (i.e. 211, 212, 213, 214, 215, 216, 217, 218, 219, 220) |

(continued)

268-270          (i.e. 268, 269, 270).

Also B-factors (see "in X-Ray Structure Determination, Stout, G.K. and Jensen, L.H., John Wiley & Sons, Inc. NY, 1989") from crystallographic data indicates the following more mobile regions in the TY145 structure:

| | |
|---|---|
| 1-7 | (i.e. 1, 2, 3, 4, 5, 6, 7), |
| 17-23 | (i.e. 17, 18, 19, 20, 21, 22, 23), |
| 38-50 | (i.e. 38, 39, 40, 41, 42, 43, 44, 45, 46, 47, 48, 49, 50), |
| 57-69 | (i.e. 57, 58, 59, 60, 61, 62, 63, 64, 65, 66, 67, 68, 69), |
| 84-92 | (i.e. 84, 85, 86, 87, 88, 89, 90, 91, 92), |
| 107-110 | (i.e. 107, 108, 109, 110), |
| 239-243 265-266. | (i.e. 239, 240, 241, 242, 243) and |

Preferably the regions 57-69 and 84-92.

Disulfide bonds:

A TY145 variant of the present invention with improved stability, e.g. thermostability, as compared to the parent TY145 may be obtained by introducing new inter-domain or intra-domain bonds, such as by establishing inter- or intra-domain disulfide bridges.

Thus a further aspect of the present invention relates to a method for producing a variant of a parent TY145 comprising the methods described in the paragraph "Methods of preparing TY145 like or BPN' like subtilase variants" herein.

According to the guidelines mentioned above the below mentioned amino acid residues identified in the amino acid sequence of SEQ ID NO:1 are contemplated as being suitable for cysteine replacement. With one or more of these substitutions with cysteine, disulfide bridges may possibly form in a variant of TY145. The substitutions are: G26C+A95C; A167C+T254C; R203C+G292C and V228C+A284C.

Similar residues suitable for cysteine replacement in homologous subtilases such as TA39, TA41 can be elucidated by finding the homologous positions in the alignment of Figure 1. Concerning another TY145 like sequence the homologous positions suitable for cysteine replacement can be selected by aligning said TY145 like sequence with all of the sequences of Figure 1 using the GAP analysis method as described above. The suitable residues can then be selected in accordance with the homologous positions in the most homologous of SEQ ID's NO:1,2,3 and 4 which are the sequences of the subtilases aligned in Figure 1.

## Surface charge distribution

A variant with improved stability (typically improved thermostability) as compared to the parent subtilase may be obtained by changing the surface charge distribution of the subtilase. For example, when the pH is lowered to about 5 or below histidine residues typically become positively charged and, consequently, unfavorable electrostatic interactions on the protein surface may occur. By engineering the surface charge of the subtilase one may avoid such unfavorable electrostatic interactions that in turn lead to a higher stability of the subtilase.

Therefore, a further aspect of the present invention relates to a method for constructing a variant of a parent subtilase, the method comprising:

a) identifying, on the surface of the parent subtilase, preferably a TY145 like or a BPN' like subtilase, at least one amino acid residue selected from the group consisting of Asp, Glu, Arg, Lys and His;
b) substituting, on the surface of the parent subtilase, at least one amino acid residue selected from the group consisting of Asp, Glu, Arg, Lys and His with an uncharged amino acid residue;
c) optionally repeating steps a) and b) recursively;
d) optionally, making alterations each of which is an insertion, a deletion or a substitution of an amino acid residue at one or more positions other than b);
e) preparing the variant resulting from steps a) - d);
f) testing the stability of said variant; and
g) optionally repeating steps a) - f) recursively; and
h) selecting a subtilase variant having increased stability as compared to the parent subtilase.

As it will be understood by the skilled person it may also, in some cases, be advantageous to substitute an uncharged amino acid residue with an amino acid residue bearing a charge or, alternatively, it may in some cases be advantageous to substitute an amino acid residue bearing a charge with an amino acid residue bearing a charge of opposite sign. Thus, the above-mentioned method may easily be employed by the skilled person also for these purposes. In the case of substituting an uncharged amino acid residue with an amino acid residue bearing a charge the above-mentioned method may be employed the only difference being steps a) and b) which will then read:

a) identifying, on the surface of the parent subtilase, at least one uncharged amino acid residue;
b) substituting, on the surface of the parent subtilase, at least one uncharged amino acid residue with a charged amino acid residue selected from the group consisting of Asp, Glu, Arg, Lys and His.

Also in the case of changing the sign of an amino acid residue present on the surface of the subtilase the above method may be employed. Again, compared to the above method, the only difference being steps a) and b) which, in this case, read:

a) identifying, on the surface of the parent subtilase, at least one charged amino acid residue selected from the group consisting of Asp, Glu, Arg, Lys and His;
b) substituting, on the surface of the parent subtilase, at least one charged amino acid residue identified in step a) with an amino acid residue having an opposite charge.

Thus, Asp may be substituted with Arg, Lys or His; Glu may be substituted with Arg, Lys or His; Arg may be substituted with Asp or Glu; Lys may be substituted with Asp or Glu; and His may be substituted with Asp or Glu.

In order to determine the amino acid residues of a subtilase, which are present on the surface of the enzyme, the surface accessible area are measured using the DSSP program (Kabsch and Sander, Biopolymers (1983), 22, 2577-2637). All residues having a surface accessibilty higher than 0 is regarded a surface residue.

An amino acid residue found on the surface of TY145 using the above method is D116 and it is contemplated that the substitutions D116H,K,R are of particular interest.

Similar substitutions may be introduced in equivalent positions of other TY145 like subtilases.

**Substitution with proline residues**

Improved thermostability of a subtilase can be obtained by subjecting the subtilase in question to analysis for secondary structure, identifying residues in the subtilase having dihedral angles $\phi$ (phi) and $\psi$ (psi) confined to the intervals [-90°<$\phi$<-40° and -180°<$\psi$<180°], preferably the intervals [-90°<$\phi$<-40° and 120°<$\psi$<180°] or [-90°<$\phi$<-40° and -50°<$\psi$<10°] and excluding residues located in regions in which the subtilase is characterized by possessing $\alpha$-helical or $\beta$-sheet structure.

After the dihedral angles $\phi$ (phi) and $\psi$ (psi) for the amino acids have been calculated, based on the atomic structure in the crystalline subtilases, it is possible to select position(s) which has/have dihedral phi and psi angles favourable for substitution with a proline residue. The aliphatic side chain of proline residues is bonded covalently to the nitrogen atom of the peptide group. The resulting cyclic five-membered ring consequently imposes a rigid constraint on the rotation about the N-C$_\alpha$ bond of the peptide backbone and simultaneously prevents the formation of hydrogen bonding to the backbone N-atom. For these structural reasons, proline residues are generally not compatible with $\alpha$-helical and $\beta$-sheet secondary conformations.

If a proline residue is not already at the identified position(s), the naturally occurring amino acid residue is substituted with a proline residue, preferably by site directed mutagenesis applied on a gene encoding the subtilase in question.

In the group of TY145 like subtilases proline residues can be introduced at positions 18, 115, 185, 269 and 293. Accordingly, a preferred TY145 variant has one or more of the substitutions: Q18P, D115P, V185P, T269P and 1293P.

**Alteration of activity**

Introduction of activity at low temperature in TY145 and Savinase

A comparison of the molecular dynamics at 300K of TY145 (a mesophilic-derived enzyme obtained from crystal structure) and TA41 (a psychrophilic derived enzyme obtained from modelling) was conducted.

The comparison was directed to low temperature activity and revealed a difference in dynamical behaviour of TY145 and TA41. The theory derived from the comparison is that the difference in dynamics, especially around the active site, are important for the low temperature functionality of the psychrophilic enzyme. The necessary dynamics are needed for the enzyme to have activity at low temperature and thus the activity drops if the enzymes dynamics are lowered.

The higher mobility regions in TA41 compared to TY145 measured by molecular dynamics simulation indicates important regions for the low temperature activity, of the enzyme TA41 which can be transferred to TY145.

The regions in TA41 are:

| | |
|---|---|
| 16-22 | (i.e. 16, 17, 18, 19, 20, 21, 22), |
| 40-73 | (i.e. 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73), |
| 118-131 | (i.e. 118, 119, 120, 121, 122, 123, 124, 125, 126, 127, 128, 129, 130, 131) |
| 140-161 | (i.e. 140, 141, 142, 143, 144, 145, 146, 147, 148, 149, 150, 151, 152, 153, 154, 155, 156, 157, 158, 159, 160, 161) and |
| 275-294 | (i.e. 275, 276, 277, 278, 279, 280, 281, 282, 283, 284, 285, 286, 287, 288, 289, 290, 291, 292, 293, 294). |

Regions closest to the active site and the substrate binding site are regarded as preferred in relation to making higher activity at low temperature for TY145: 40-73, and 140-161, preferably 65-73 and 140-150. The regions in TY145 should be modified to be more mobile for example by substitution with small less rigid residues, i.e. residues with smaller side chains (such as Gly, Ala, Ser, Thr or Val), into the TY145 backbone.

The other regions in TA41 are most interesting for stabilisation of the psychrophilic enzyme. These regions can easily be found in TA39 as well or in other homologous enzymes, also non psychrophilics.

The regions around the active site and the substrate binding site are the regions most likely involved in the low temperature functionality.

Below are suggestions for transferring the low temperature activity of TA41 and homologous sequences to TY145-like sequences and the BPN'-like sequences:

| TA41 | TY145 | Savinase |
|---|---|---|
| I31 | V31I,A,L | V28I,A,L |
| V38 | V38A, L | I35V,A, L |
| S79 | T79S | T71S |
| A80 | V80A,G,V | I72A,G,V |
| L81 | L81 G | A73L,G |
| V187 | V188A | M175V,A |
| T253 | T254S,A | T224S,A |

The numbering is according to SEQ ID NO:3, 1 and 5 respectively. Savinase is numbered according to BPN'.

Preferred Savinase variants are V28I, I35V, T71S, I72A, A73L, M175V and T224S.

Examples of core variants of TY145 are: V31I, V80A, T79S.

The alterations of the TY145-like sequences and the BPN'-like sequences can be single mutations or combinations of the suggested mutations.

**Substrate bindings site**

The substrate binding site is identified by the residues in contact with a substrate model, such as the C12 inhibitor. The 3D structure coordinates of the TY145 subtilase with C12 bound in the active site can be found in Appendix 1. Without being limited to any theory, it is presently believed that binding between a substrate and an enzyme is supported by favorable interactions found within a sphere 10 Å from the substrate molecule, in particular within a sphere of 6 Å from the substrate molecule. Examples of such favorable bonds are hydrogen bonds, strong electrostatic interaction and/or hydrophobic interactions.

The following residues of the TY145 subtilase (SEQ ID NO: 1), are within a distance of 6Å from the C12 inhibitor and thus believed to be involved in interactions with said substrate: 35, 36, 70, 72, 106, 109, 110, 111, 112, 113, 114, 117, 139, 140, 141, 142, 143, 144, 145, 147, 150, 167, 168, 169, 170, 171, 172, 173, 174, 177, 180, 207, 239, 247, 148, 149, 150, 151 and 252.

**Stabilization by modification of Asn-Gly pairs**

It is known that at alkaline pH, the side chain of Asn may interact with the NH group of a sequential neighbouring amino acid to form an isoAsp residue where the backbone goes through the Asp side chain. This will leave the backbone more vulnerable to proteolysis. The deamidation is much more likely to occur if the residue that follows is a Gly. Changing the Asn in front of the Gly or the Gly will prevent this from happening and thus improve the stability, especially as concerns thermo- and storage stability.

The invention consequently further relates to a subtilase, in which either or both residues of any of the Asn-Gly sequence appearing in the amino acid sequence of the parent RP-II protease is/are deleted or substituted with a residue of a different amino acid.

The Asn and/or Gly residue may, for instance, be substituted with a residue of an amino acid selected from the group consisting of A, Q, S, P, T and Y.

Asn-Gly sequences can be found in the following positions:

B. sphaericus: 198-199, 240-241
TY145: 87-88, 109-110, 199-200
TA41: 83-84, 198-199
TA39: 88-89, 198-199

The present invention in this respect thus relates to modifications, such as deletions and substitutions in one or more of these positions in accordance with the principles given above.

**Modification of Tyrosine residues**

In relation to wash performance it has been found that the modification of certain tyrosine residues to phenylalanine provides an improved wash performance. Without being bound by any specific theory, it is believed that titration of these Tyr residues in the alkaline wash liquor has negative effects that are alleviated by replacing the Tyr residues with other residues, especially Phe or Trp, particularly Phe.

Tyrosines can be found in the following positions:

B. sphaericus: 14, 91, 102, 112, 155, 157, 172, 179, 201, 206, 211, 218, 235, 239, 243, 292, 300,
TY145: 15, 39, 92, 103, 113, 156, 158, 202, 219, 240, 244, 287, 301, 307,
TA41: 15, 91, 102, 112, 155, 157, 179, 201, 218, 235, 243,
TA39: 15, 61, 91, 102, 112, 155, 157, 173, 179, 201, 211, 218, 235, 243, 267, 281, 284, 292, 293,296

The present invention in this respect thus relates to modifications, such as deletions and substitutions in one or more of these positions in accordance with the principles given above.

**Modification of methionine residues**

In order to improve the oxidation stability of proteins it has been found that the substitution or even deletion of methionine residues is beneficial, Especially modification of the methionine residue normally found next to the active serine residue may provide a significant improvement of the oxidation stability. Modifications to Ser or Ala are the most preferred substitutions for this Met.

Methionines can be found in the following positions:

B. sphaericus: 138, 251,
TY145: 139,252,
TA41: 1, 138,251,
TA39: 1, 138, 251,

The present invention in this respect thus relates to modifications, such as deletions and substitutions in one or more of these positions in accordance with the principles given above.

**Combined modifications**

The present invention also encompasses any of the above mentioned subtilase variants in combination with any other modification to the amino acid sequence thereof. Especially combinations with other modifications known in the art to provide improved properties to the enzyme are envisaged.

Such combinations comprise the positions: 222 (improves oxidation stability), 218 (improves thermal stability), substitutions in the $Ca^{2+}$-binding sites stabilizing the enzyme, e.g. position 76, and many other apparent from the prior art (all positions according to BPN' numbering).

In further embodiments a subtilase variant described herein may advantageously be combined with one or more modification(s) in any of the positions:

27, 36, 56, 76, 87, 95, 96, 97, 98, 99, 100, 101, 102, 103, 104, 120, 123, 159, 167, 170, 206, 218, 222, 224, 232, 235, 236, 245, 248, 252 and 274 (BPN' numbering).

Specifically, the following BLSAVI, BLSUBL, BSKSMK, and BAALKP modifications are considered appropriate for combination:

K27R, *36D, S56P, N76D, S87N, G97N, S101G, S103A, V104A, V104I, V104N, V104Y, H120D, N123S, G159D, Y167A, R170S, R170L, Q206E, N218S, M222S, M222A, T224S, A232V, K235L, Q236H, Q245R, N248D, N252K and T274A (BPN' numbering).

Furthermore variants comprising any of the modifications S101G+V104N, S87N+S101G+V104N, K27R+V104Y+N123S+T274A, N76D+S103A+V104I or N76D+V104A, or other combinations of the modifications K27R, N76D, S101G, S103A, V104N, V104Y, V104I, V104A, N123S, G159D, A232V, Q236H, Q245R, N248D, N252K, T274A in combination with any one or more of the modification(s) mentioned above exhibit improved properties.

A particular interesting variant is a variant, which, in addition to modifications according to the invention, contains the following substitutions:

S101G+S103A+V104I+G159D+A232V+Q236H+Q245R+N248D+N252K.

Moreover, subtilase variants of the main aspect(s) of the invention are preferably combined with one or more modification(s) in any of the positions 129, 131 and 194, preferably as 129K, 131H and 194P modifications, and most preferably as P129K, P131H and A194P modifications. Any of those modification(s) are expected to provide a higher expression level of the subtilase variant in the production thereof.

**Methods of preparing TY145 like or BPN' like subtilase variants**

The subtilase variants, i.e. the TY145 and BPN' variants of the present invention may be produced by any known method within the art and the present invention also relates to nucleic acid encoding a subtilase variant of the present invention, a DNA construct comprising said nucleic acid and a host cell comprising said nucleic acid sequence.

In general natural occurring proteins may be produced by culturing the organism expressing the protein and subsequently purifying the protein or it may be produced by cloning a nucleic acid, e.g. genomic DNA or cDNA, encoding the protein into an expression vector, introducing said expression vector into a host cell, culturing the host cell and purifying the expressed protein.

Typically protein variants may be produced by site-directed mutagenesis of a parent protein, introduction into expression vector, host cell etc. The parent protein may be cloned from a strain producing the polypeptide or from an expression library, i.e. it may be isolated from genomic DNA or prepared from cDNA, or a combination thereof.

In general standard procedures for cloning of genes and/or introducing mutations (random and/or site directed) into said genes may be used in order to obtain a parent subtilase, or subtilase or subtilase variant of the invention. For further description of suitable techniques reference is made to Molecular cloning: A laboratory manual (Sambrook et al. (1989), Cold Spring Harbor lab., Cold Spring Harbor, NY; Ausubel, F. M. et al. (eds.)); Current protocols in Molecular Biology (John Wiley and Sons, 1995; Harwood, C. R., and Cutting, S. M. (eds.)); Molecular Biological Methods for Bacillus (John Wiley and Sons, 1990); DNA Cloning: A Practical Approach, Volumes I and II (D.N. Glover ed. 1985); Oligonucleotide Synthesis (M.J. Gait ed. 1984); Nucleic Acid Hybridization (B.D. Hames & S.J. Higgins eds (1985)); Transcription And Translation (B.D. Hames & S.J. Higgins, eds. (1984)); Animal Cell Culture (R.I. Freshney, ed. (1986)); Immobilized Cells And Enzymes (IRL Press, (1986)); A Practical Guide To Molecular Cloning (B. Perbal, (1984)) and WO 96/34946.

Further, variants could be constructed by:

Random Mutagenesis

Random mutagenesis is suitably performed either as localized or region-specific random mutagenesis in at least three parts of the gene translating to the amino acid sequence shown in question, or within the whole gene.

The random mutagenesis of a DNA sequence encoding a parent subtilase may be conveniently performed by use of any method known in the art.

In relation to the above, a further aspect of the present invention relates to a method for generating a variant of a parent subtilase, wherein the variant exhibits an altered property, such as increased thermostability, increased stability at low pH and at low calcium concentration, relative to the parent subtilase, the method comprising:

> (a) subjecting a DNA sequence encoding the parent subtilase to random mutagenesis,
> (b) expressing the mutated DNA sequence obtained in step (a) in a host cell, and
> (c) screening for host cells expressing a subtilase variant which has an altered property relative to the parent subtilase.

Step (a) of the above method of the invention is preferably performed using doped primers.

For instance, the random mutagenesis may be performed by use of a suitable physical or chemical mutagenizing agent, by use of a suitable oligonucleotide, or by subjecting the DNA sequence to PCR generated mutagenesis. Furthermore, the random mutagenesis may be performed by use of any combination of these mutagenizing agents. The mutagenizing agent may, e.g., be one which induces transitions, transversions, inversions, scrambling, deletions, and/or insertions.

Examples of a physical or chemical mutagenizing agent suitable for the present purpose include ultraviolet (UV) irradiation, hydroxylamine, N-methyl-N'-nitro-N-nitrosoguanidine (MNNG), O-methyl hydroxylamine, nitrous acid, ethyl methane sulphonate (EMS), sodium bisulphite, formic acid, and nucleotide analogues. When such agents are used, the mutagenesis is typically performed by incubating the DNA sequence encoding the parent enzyme to be mutagenized in the presence of the mutagenizing agent of choice under suitable conditions for the mutagenesis to take place, and selecting for mutated DNA having the desired properties.

When the mutagenesis is performed by the use of an oligonucleotide, the oligonucleotide may be doped or spiked with the three non-parent nucleotides during the synthesis of the oligonucleotide at the positions that are to be changed. The doping or spiking may be done so that codons for unwanted amino acids are avoided. The doped or spiked oligonucleotide can be incorporated into the DNA encoding the subtilase enzyme by any published technique, using, e.g., PCR, LCR or any DNA polymerase and ligase as deemed appropriate.

Preferably, the doping is carried out using "constant random doping", in which the percentage of wild-type and modification in each position is predefined. Furthermore, the doping may be directed toward a preference for the introduction of certain nucleotides, and thereby a preference for the introduction of one or more specific amino acid residues. The doping may be made, e.g., so as to allow for the introduction of 90% wild type and 10% modifications in each position. An additional consideration in the choice of a doping scheme is based on genetic as well as protein-structural constraints. The doping scheme may be made by using the DOPE program which, *inter alia,* ensures that introduction of stop codons is avoided (L.J. Jensen et al. Nucleic Acid Research, 26, 697-702 (1998).

When PCR-generated mutagenesis is used, either a chemically treated or non-treated gene encoding a parent subtilase enzyme is subjected to PCR under conditions that increase the misincorporation of nucleotides (Deshler 1992; Leung et al., Technique, 1, 1989, pp. 11-15).

A mutator strain of *E. coli* (Fowler et al., Molec. Gen. Genet., 133, 1974, 179-191), *S. cereviseae* or any other microbial organism may be used for the random mutagenesis of the DNA encoding the subtilase by, e.g., transforming a plasmid containing the parent enzyme into the mutator strain, growing the mutator strain with the plasmid and isolating the mutated plasmid from the mutator strain. The mutated plasmid may be subsequently transformed into the expression organism.

The DNA sequence to be mutagenized may conveniently be present in a genomic or cDNA library prepared from an organism expressing the parent subtilase. Alternatively, the DNA sequence may be present on a suitable vector such as a plasmid or a bacteriophage, which as such may be incubated with or otherwise exposed to the mutagenising agent. The DNA to be mutagenized may also be present in a host cell either by being integrated in the genome of said cell or by being present on a vector harbored in the cell. Finally, the DNA to be mutagenized may be in isolated form. It will be understood that the DNA sequence to be subjected to random mutagenesis is preferably a cDNA or a genomic DNA sequence.

In some cases it may be convenient to amplify the mutated DNA sequence prior to performing the expression step b) or the screening step c). Such amplification may be performed in accordance with methods known in the art, the presently preferred method being PCR-generated amplification using oligonucleotide primers prepared on the basis of the DNA or amino acid sequence of the parent enzyme.

Subsequent to the incubation with or exposure to the mutagenising agent, the mutated DNA is expressed by culturing a suitable host cell carrying the DNA sequence under conditions allowing expression to take place. The host cell used for this purpose may be one which has been transformed with the mutated DNA sequence, optionally present on a vector, or one which was carried the DNA sequence encoding the parent enzyme during the mutagenesis treatment.

Examples of suitable host cells are the following: gram positive bacteria such as *Bacillus subtilis, Bacillus licheniformis, Bacillus lentus, Bacillus brevis, Bacillus stearothermophilus, Bacillus alkalophilus, Bacillus amyloliquefaciens, Bacillus coagulans, Bacillus circulans, Bacillus lautus, Bacillus megaterium, Bacillus thuringiensis, Streptomyces lividans* or *Streptomyces murinus;* and gram negative bacteria such as *E. coli.*

The mutated DNA sequence may further comprise a DNA sequence encoding functions permitting expression of the mutated DNA sequence.

Localised random mutagenesis

The random mutagenesis may be advantageously localised to a part of the parent subtilase in question. This may, e.g., be advantageous when certain regions of the enzyme have been identified to be of particular importance for a given property of the enzyme, and when modified are expected to result in a variant having improved properties. Such regions may normally be identified when the tertiary structure of the parent enzyme has been elucidated and related to the function of the enzyme.

The localised or region-specific, random mutagenesis is conveniently performed by use of PCR generated mutagenesis techniques as described above or any other suitable technique known in the art. Alternatively, the DNA sequence encoding the part of the DNA sequence to be modified may be isolated, e.g., by insertion into a suitable vector, and said part may be subsequently subjected to mutagenesis by use of any of the mutagenesis methods discussed above.

General method for random mutagenesis by use of the DOPE program

The random mutagenesis may be carried out by the following steps:

1. Select regions of interest for modification in the parent enzyme
2. Decide on mutation sites and non-mutated sites in the selected region
3. Decide on which kind of mutations should be carried out, e.g. with respect to the de sired stability and/or performance of the variant to be constructed
4. Select structurally reasonable mutations
5. Adjust the residues selected by step 3 with regard to step 4.
6. Analyse by use of a suitable dope algorithm the nucleotide distribution.
7. If necessary, adjust the wanted residues to genetic code realism, e.g. taking into account constraints resulting from the genetic code, e.g. in order to avoid introduction of stop codons; the skilled person will be aware that some codon combinations cannot be used in practice and will need to be adapted
8. Make primers
9. Perform random mutagenesis by use of the primers
10. Select resulting subtilase variants by screening for the desired improved properties.

Suitable dope algorithms for use in step 6 are well known in the art. One such algorithm is described by Tomandl, D. et al., 1997, Journal of Computer-Aided Molecular Design 11:29-38. Another algorithm is DOPE (Jensen, LJ, Andersen, KV, Svendsen, A, and Kretzschmar, T (1998) Nucleic Acids Research 26:697-702).

Expression vectors

A recombinant expression vector comprising a nucleic acid sequence encoding a subtilase variant of the invention may be any vector that may conveniently be subjected to recombinant DNA procedures and which may bring about the expression of the nucleic acid sequence.

The choice of vector will often depend on the host cell into which it is to be introduced. Examples of a suitable vector include a linear or closed circular plasmid or a virus. The vector may be an autonomously replicating vector, i.e., a vector which exists as an extra-chromosomal entity, the replication of which is independent of chromosomal replication, e.g., a plasmid, an extra-chromosomal element, a mini chromosome, or an artificial chromosome. The vector may contain any means for assuring self-replication. Examples of bacterial origins of replication are the origins of replication of plasmids pBR322, pUC19, pACYC177, pACYC184, pUB110, pE194, pTA1060, and pAMβ1. Examples of origin of replications for use in a yeast host cell are the 2 micron origin of replication, the combination of CEN6 and ARS4, and the combination of CEN3 and ARS1. The origin of replication may be one having a mutation which makes it function as temperature-sensitive in the host cell (see, e.g., Ehrlich, 1978, Proceedings of the National Academy of Sciences USA 75:1433).

Alternatively, the vector may be one which, when introduced into the host cell, is integrated into the genome and replicated together with the chromosome(s) into which it has been integrated. Vectors which are integrated into the genome of the host cell may contain any nucleic acid sequence enabling integration into the genome, in particular it may contain nucleic acid sequences facilitating integration into the genome by homologous or non-homologous recombination. The vector system may be a single vector, e.g. plasmid or virus, or two or more vectors, e.g. plasmids or virus', which together contain the total DNA to be introduced into the genome of the host cell, or a transposon.

The vector may in particular be an expression vector in which the DNA sequence encoding the subtilase variant of the invention is operably linked to additional segments or control sequences required for transcription of the DNA. The term, "operably linked" indicates that the segments are arranged so that they function in concert for their intended purposes, e.g. transcription initiates in a promoter and proceeds through the DNA sequence encoding the subtilase variant. Additional segments or control sequences include a promoter, a leader, a polyadenylation sequence, a propeptide sequence, a signal sequence and a transcription terminator. At a minimum the control sequences include a promoter and transcriptional and translational stop signals.

The promoter may be any DNA sequence that shows transcriptional activity in the host cell of choice and may be derived from genes encoding proteins either homologous or heterologous to the host cell.

Examples of suitable promoters for use in bacterial host cells include the promoter of the *Bacillus subtilis* levansucrase gene (sacB), the *Bacillus stearothermophilus* maltogenic amylase gene (amyM), the *Bacillus licheniformis* alpha-amylase gene (amyL), the *Bacillus amyloliquefaciens* alpha-amylase gene (amyQ), the *Bacillus subtilis* alkaline protease gene, or the Ba*cillus pumilus* xylosidase gene, the *Bacillus amyloliquefaciens* BAN amylase gene, the *Bacillus licheniformis* penicillinase gene (penP), the *Bacillus subtilis* xylA and xylB genes, and the prokaryotic beta-lactamase gene (Villa-Kamaroff et al., 1978, Proceedings of the National Academy of Sciences USA 75:3727-3731). Other examples include the phage Lambda $P_R$ or $P_L$ promoters or the E. coli lac, trp or tac promoters or the Streptomyces coelicolor agarase gene (dagA). Further promoters are described in "Useful proteins from recombinant bacteria" in Scientific American, 1980, 242:74-94; and in Sambrook et al., 1989, supra.

Examples of suitable promoters for use in a filamentous fungal host cell are promoters obtained from the genes encoding *Aspergillus oryzae* TAKA amylase, *Rhizomucor miehei* aspartic proteinase, *Aspergillus niger* neutral alpha-amylase, *Aspergillus niger* acid stable alpha-amylase, *Aspergillus niger* or *Aspergillus awamori* glucoamylase (glaA), *Rhizomucor miehei* lipase, *Aspergillus oryzae* alkaline protease, *Aspergillus oryzae* triose phosphate isomerase, *Aspergillus nidulans* acetamidase, *Fusarium oxysporum* trypsin-like protease (as described in U.S. Patent No. 4,288,627, which is incorporated herein by reference), and hybrids thereof. Particularly preferred promoters for use in filamentous fungal host cells are the TAKA amylase, NA2-tpi (a hybrid of the promoters from the genes encoding *Aspergillus niger* neutral (-amylase and *Aspergillus oryzae* triose phosphate isomerase), and glaA promoters. Further suitable promoters for use in filamentous fungus host cells are the ADH3 promoter (McKnight et al., The EMBO J. 4 (1985), 2093 - 2099) or the tpiA promoter.

Examples of suitable promoters for use in yeast host cells include promoters from yeast glycolytic genes (Hitzeman et al., J. Biol. Chem. 255 (1980), 12073 - 12080; Alber and Kawasaki, J. Mol. Appl. Gen. 1 (1982), 419 - 434) or alcohol dehydrogenase genes (Young et al., in Genetic Engineering of Microorganisms for Chemicals (Hollaender et al, eds.), Plenum Press, New York, 1982), or the TP11 (US 4,599,311) or ADH2-4c (Russell et al., Nature 304 (1983), 652 - 654) promoters.

Further useful promoters are obtained from the *Saccharomyces cerevisiae* enolase (ENO-1) gene, the *Saccharomyces cerevisiae* galactokinase gene (GAL1), the *Saccharomyces cerevisiae* alcohol dehydrogenase/glyceraldehyde-3-phosphate dehydrogenase genes (ADH2/GAP), and the *Saccharomyces cerevisiae* 3-phosphoglycerate kinase gene. Other useful promoters for yeast host cells are described by Romanos et al., 1992, Yeast 8:423-488. In a mammalian host cell, useful promoters include viral promoters such as those from Simian Virus 40 (SV40), Rous sarcoma virus (RSV), adenovirus, and bovine papilloma virus (BPV).

Examples of suitable promoters for use in mammalian cells are the SV40 promoter (Subramani et al., Mol. Cell Biol. 1 (1981), 854 -864), the MT-1 (metallothionein gene) promoter (Palmiter et al., Science 222 (1983), 809 - 814) or the adenovirus 2 major late promoter.

An example of a suitable promoter for use in insect cells is the polyhedrin promoter (US 4,745,051; Vasuvedan et al., FEBS Lett. 311, (1992) 7 - 11), the P10 promoter (J.M. Vlak et al., J. Gen. Virology 69, 1988, pp. 765-776), the Autographa californica polyhedrosis virus basic protein promoter (EP 397 485), the baculovirus immediate early gene 1 promoter (US 5,155,037; US 5,162,222), or the baculovirus 39K delayed-early gene promoter (US 5,155,037; US 5,162,222).

The DNA sequence encoding a subtilase variant of the invention may also, if necessary, be operably connected to a suitable terminator.

The recombinant vector of the invention may further comprise a DNA sequence enabling the vector to replicate in the host cell in question.

The vector may also comprise a selectable marker, e.g. a gene the product of which complements a defect in the host cell, or a gene encoding resistance to e.g. antibiotics like ampicillin, kanamycin, chloramphenicol, erythromycin, tetracycline, spectinomycine, neomycin, hygromycin, methotrexate, or resistance to heavy metals, virus or herbicides, or which provides for prototrophy or auxotrophs. Examples of bacterial selectable markers are the dal genes from *Bacillus subtilis* or *Bacillus licheniformis,* resistance. A frequently used mammalian marker is the dihydrofolate reductase gene (DHFR). Suitable markers for yeast host cells are ADE2, HIS3, LEU2, LYS2, MET3, TRP1, and URA3. A selectable marker for use in a filamentous fungal host cell may be selected from the group including, but not limited to, amdS (acetamidase), argB (ornithine carbamoyltransferase), bar (phosphinothricin acetyl-transferase), hygB (hygromycin phosphotransferase), niaD (nitrate reductase), pyrG (orotidine-5'-phosphate decarboxylase), sC (sulfate adenyltransferase), trpC (anthranilate synthase), and glufosinate resistance markers, as well as equivalents from other species. Particularly, for use in an *Aspergillus* cell are the amdS and pyrG markers of *Aspergillus nidulans* or *Aspergillus oryzae* and the bar marker of *Streptomyces hygroscopicus.* Furthermore, selection may be accomplished by co-transformation, e.g., as described in WO 91/17243, where the selectable marker is on a separate vector.

To direct a subtilase variant of the present invention into the secretory pathway of the host cells, a secretory signal sequence (also known as a leader sequence, prepro sequence or pre sequence) may be provided in the recombinant vector. The secretory signal sequence is joined to the DNA sequence encoding the enzyme in the correct reading frame. Secretory signal sequences are commonly positioned 5' to the DNA sequence encoding the enzyme. The secretory signal sequence may be that normally associated with the enzyme or may be from a gene encoding another secreted protein.

The procedures used to ligate the DNA sequences coding for the present enzyme, the promoter and optionally the terminator and/or secretory signal sequence, respectively, or to assemble these sequences by suitable PCR amplification schemes, and to insert them into suitable vectors containing the information necessary for replication or integration, are well known to persons skilled in the art (cf., for instance, Sambrook et al.).

More than one copy of a nucleic acid sequence encoding an enzyme of the present invention may be inserted into the host cell to amplify expression of the nucleic acid sequence. Stable amplification of the nucleic acid sequence can be obtained by integrating at least one additional copy of the sequence into the host cell genome using methods well known in the art and selecting for transformants.

The nucleic acid constructs of the present invention may also comprise one or more nucleic acid sequences which encode one or more factors that are advantageous in the expression of the polypeptide, e.g., an activator (e.g., a trans-acting factor), a chaperone, and a processing protease. Any factor that is functional in the host cell of choice may be used in the present invention. The nucleic acids encoding one or more of these factors are not necessarily in tandem with the nucleic acid sequence encoding the polypeptide.

Host cells

The DNA sequence encoding a subtilase variant of the present invention may be either homologous or heterologous to the host cell into which it is introduced. If homologous to the host cell, i.e. produced by the host cell in nature, it will typically be operably connected to another promoter sequence or, if applicable, another secretory signal sequence and/or terminator sequence than in its natural environment. The term "homologous" is intended to include a DNA sequence encoding an enzyme native to the host organism in question. The term "heterologous" is intended to include a DNA sequence not expressed by the host cell in nature. Thus, the DNA sequence may be from another organism, or it may be a synthetic sequence.

The host cell into which the DNA construct or the recombinant vector of the invention is introduced may be any cell that is capable of producing the present subtilase variants, such as prokaryotes, e.g. bacteria or eukaryotes, such as fungal cells, e.g. yeasts or filamentous fungi, insect cells, plant cells or mammalian cells.

Examples of bacterial host cells which, on cultivation, are capable of producing the subtilase variants of the invention are gram-positive bacteria such as strains of *Bacillus,* e.g. strains of *B. subtilis, B. licheniformis, B. lentus, B. brevis, B. stearothermophilus, B. alkalophilus, B. amyloliquefaciens, B. coagulans, B. circulans, B. lautus, B. megaterium* or *B. thuringiensis,* or strains of *Streptomyces,* such as *S. lividans* or *S. murinus,* or gram-negative bacteria such as *Escherichia coli* or *Pseudomonas sp.*

The transformation of the bacteria may be effected by protoplast transformation, electroporation, conjugation, or by using competent cells in a manner known per se (cf. Sambrook et al., supra).

When expressing the subtilase variant in bacteria such as *E. coli,* the enzyme may be retained in the cytoplasm, typically as insoluble granules (known as inclusion bodies), or it may be directed to the periplasmic space by a bacterial secretion sequence. In the former case, the cells are lysed and the granules are recovered and denatured after which the enzyme is refolded by diluting the denaturing agent. In the latter case, the enzyme may be recovered from the periplasmic space by disrupting the cells, e.g. by sonication or osmotic shock, to release the contents of the periplasmic space and recovering the enzyme.

When expressing the subtilase variant in gram-positive bacteria such as *Bacillus* or *Streptomyces* strains, the enzyme may be retained in the cytoplasm, or it may be directed to the extracellular medium by a bacterial secretion sequence. In the latter case, the enzyme may be recovered from the medium as described below.

Examples of host yeast cells include cells of a species of *Candida, Kluyveromyces, Saccharomyces, Schizosaccharomyces, Pichia, Hansehula,* or *Yarrowia.* In a particular embodiment, the yeast host cell is a *Saccharomyces carlsbergensis, Saccharomyces cerevisiae, Saccharomyces diastaticus, Saccharomyces douglasii, Saccharomyces kluyveri, Saccharomyces norbensis* or *Saccharomyces oviformis* cell. Other useful yeast host cells are a *Kluyveromyces lactis, Kluyveromyces fragilis, Hansehula polymorpha, Pichia pastoris, Yarrowia lipolytica, Schizosaccharomyces pombe, Ustilgo maylis, Candida maltose, Pichia guillermondii* and *Pichia methanolio* cell (cf. Gleeson et al., J. Gen. Microbiol. 132, 1986, pp. 3459-3465; US 4,882,279 and US 4,879,231). Since the classification of yeast may change in the future, for the purposes of this invention, yeast shall be defined as described in Biology and Activities of Yeast (Skinner, F.A., Passmore, S.M., and Davenport, R.R., eds, Soc. App. Bacteriol. Symposium Series No. 9, 1980. The biology of yeast and manipulation of yeast genetics are well known in the art (see, e.g., Biochemistry and Genetics of Yeast, Bacil, M., Horecker, B.J., and Stopani, A.O.M., editors, 2nd edition, 1987; The Yeasts, Rose, A.H., and Harrison, J.S., editors, 2nd edition, 1987; and The Molecular Biology of the Yeast Saccharomyces, Strathern et al., editors, 1981). Yeast may be transformed using the procedures described by Becker and Guarente, In Abelson, J.N. and Simon, M.I., editors, Guide to Yeast Genetics and Molecular Biology, Methods in Enzymology, Volume 194, pp 182-187, Academic Press, Inc., New York; Ito et al., 1983, Journal of Bacteriology 153:163; and Hinnen et al., 1978, Proceedings of the National Academy of Sciences USA 75:1920.

Examples of filamentous fungal cells include filamentous forms of the subdivision Eumycota and Oomycota (as defined by Hawksworth et al., 1995, supra), in particular it may of the a cell of a species of *Acremonium,* such as *A. chrysogenum, Aspergillus,* such as *A. awamori, A. foetidus, A. japonicus, A. niger, A. nidulans* or *A. oryzae, Fusarium,* such as *F. bactridioides, F. cerealis, F. crookwellense, F. culmorum, F. graminearum, F. graminum, F. heterosporum, F. negundi, F. reticulatum, F. roseum, F. sambucinum, F. sarcochroum, F. sulphureum, F. trichothecioides* or *F. oxysporum, Humicola,* such as *H. insolens* or *H. lanuginose, Mucor,* such as *M. miehei, Myceliophthora,* such as *M. thermophilum, Neurospora,* such as *N. crassa, Penicillium,* such as *P. purpurogenum, Thielavia,* such as *T. terrestris, Tolypocladium,* or *Trichoderma,* such as *T. harzianum, T. koningii, T. longibrachiatum, T. reesei* or *T. viride,* or a teleomorph or synonym thereof. The use of *Aspergillus spp.* for the expression of proteins is described in, e.g., EP 272 277, EP 230 023.

Examples of insect cells include a *Lepidoptera* cell line, such as *Spodoptera frugiperda* cells or *Trichoplusia ni* cells (cf. US 5,077,214). Culture conditions may suitably be as described in WO 89/01029 or WO 89/01028.Transformation of insect cells and production of heterologous polypeptides therein may be performed as described in US 4,745,051; US 4, 775, 624; US 4,879,236; US 5,155,037; US 5,162,222; EP 397,485).

Examples of mammalian cells include Chinese hamster ovary (CHO) cells, HeLa cells, baby hamster kidney (BHK) cells, COS cells, or any number of other immortalized cell lines available, e.g., from the American Type Culture Collection. Methods of transfecting mammalian cells and expressing DNA sequences introduced in the cells are described in e.g. Kaufman and Sharp, J. Mol. Biol. 159 (1982), 601 - 621; Southern and Berg, J. Mol. Appl. Genet. 1 (1982), 327 - 341; Loyter et al., Proc. Natl. Acad. Sci. USA 79 (1982), 422 - 426; Wigler et al., Cell 14 (1978), 725; Corsaro and Pearson, Somatic Cell Genetics 7 (1981), 603, Ausubel et al., Current Protocols in Molecular Biology, John Wiley and Sons, Inc., N.Y., 1987, Hawley-Nelson et al., Focus 15 (1993), 73; Ciccarone et al., Focus 15 (1993), 80; Graham and van der Eb, Virology 52 (1973), 456; and Neumann et al., EMBO J. 1 (1982), 841 - 845. Mammalian cells may be transfected by direct uptake using the calcium phosphate precipitation method of Graham and Van der Eb (1978, Virology 52:546).

Methods for expression and isolation of proteins

To express an enzyme of the present invention the above mentioned host cells transformed or transfected with a vector comprising a nucleic acid sequence encoding an enzyme of the present invention are typically cultured in a suitable nutrient medium under conditions permitting the production of the desired molecules, after which these are recovered from the cells, or the culture broth.

The medium used to culture the host cells may be any conventional medium suitable for growing the host cells, such as minimal or complex media containing appropriate supplements. Suitable media are available from commercial suppliers or may be prepared according to published recipes (e.g. in catalogues of the American Type Culture Collection). The media may be prepared using procedures known in the art (see, e.g., references for bacteria and yeast; Bennett, J.W. and LaSure, L., editors, More Gene Manipulations in Fungi, Academic Press, CA, 1991).

If the enzymes of the present invention are secreted into the nutrient medium, they may be recovered directly from the medium. If they are not secreted, they may be recovered from cell lysates. The enzymes of the present invention may be recovered from the culture medium by conventional procedures including separating the host cells from the medium by centrifugation or filtration, precipitating the proteinaceous components of the supernatant or filtrate by means of a salt, e.g. ammonium sulphate, purification by a variety of chromatographic procedures, e.g. ion exchange chromatography, gelfiltration chromatography, affinity chromatography, or the like, dependent on the enzyme in question.

The enzymes of the invention may be detected using methods known in the art that are specific for these proteins. These detection methods include use of specific antibodies, formation of a product, or disappearance of a substrate. For example, an enzyme assay may be used to determine the activity of the molecule. Procedures for determining various kinds of activity are known in the art.

The enzymes of the present invention may be purified by a variety of procedures known in the art including, but not limited to, chromatography (e.g., ion exchange, affinity, hydrophobic, chromatofocusing, and size exclusion), electrophoretic procedures (e.g., preparative isoelectric focusing (IEF), differential solubility (e.g., ammonium sulfate precipitation), or extraction (see, e.g., Protein Purification, J-C Janson and Lars Ryden, editors, VCH Publishers, New York, 1989).

When an expression vector comprising a DNA sequence encoding an enzyme of the present invention is transformed/transfected into a heterologous host cell it is possible to enable heterologous recombinant production of the enzyme. An advantage of using a heterologous host cell is that it is possible to make a highly purified enzyme composition, characterized in being free from homologous impurities, which are often present when a protein or peptide is expressed in a homologous host cell. In this context homologous impurities mean any impurity (e.g. other polypeptides than the enzyme of the invention) which originates from the homologous cell where the enzyme of the invention is originally obtained from.

## DETERGENT APPLICATIONS

The enzyme of the invention may be added to and thus become a component of a detergent composition.

The detergent composition of the invention may for example be formulated as a hand or machine laundry detergent composition including a laundry additive composition suitable for pre-treatment of stained fabrics and a rinse added fabric softener composition, or be formulated as a detergent composition for use in general household hard surface cleaning operations, or be formulated for hand or machine dishwashing operations.

In a specific aspect, the invention provides a detergent additive comprising the enzyme of the invention. The detergent additive as well as the detergent composition may comprise one or more other enzymes such as a protease, a lipase, a cutinase, an amylase, a carbohydrase, a cellulase, a pectinase, a mannanase, an arabinase, a galactanase, a xylanase, an oxidase, e.g., a laccase, and/or a peroxidase.

In general the properties of the chosen enzyme(s) should be compatible with the selected detergent, (i.e. pH-optimum, compatibility with other enzymatic and non-enzymatic ingredients, etc.), and the enzyme(s) should be present in effective amounts.

Proteases: Suitable proteases include those of animal, vegetable or microbial origin. Microbial origin is preferred. Chemically modified or protein engineered mutants are included. The protease may be a serine protease or a metallo protease, preferably an alkaline microbial protease or a trypsin-like protease. Examples of alkaline proteases are subtilisins, especially those derived from *Bacillus,* e.g., subtilisin Novo, subtilisin Carlsberg, subtilisin 309, subtilisin 147 and subtilisin 168 (described in WO 89/06279). Examples of trypsin-like proteases are trypsin (e.g. of porcine or bovine origin) and the *Fusarium* protease described in WO 89/06270 and WO 94/25583.

Examples of useful proteases are the variants described in WO 92/19729, WO 98/20115, WO 98/20116, and WO 98/34946, especially the variants with substitutions in one or more of the following positions: 27, 36, 57, 76, 87, 97, 101, 104, 120, 123, 167, 170, 194, 206, 218, 222, 224, 235 and 274.

Preferred commercially available protease enzymes include Alcalase®, Savinase®, Primase®, Duralase®, Esperase®, Ovozyme® and Kannase® (Novozymes A/S), Maxatase™, Maxacal™, Maxapem™, Properase™, Purafect™, Purafect OxP™, FN2™ FN3™ and FN4™ (Genencor International Inc.).

Lipases: Suitable lipases include those of bacterial or fungal origin. Chemically modified or protein engineered mutants are included. Examples of useful lipases include lipases from *Humicola* (synonym *Thermomyces),* e.g. from *H. lanuginosa (T. lanuginosus)* as described in EP 258 068 and EP 305 216 or from *H. insolens* as described in WO 96/13580, a *Pseudomonas* lipase, e.g. from *P. alcaligenes* or *P. pseudoalcaligenes* (EP 218 272), *P. cepacia* (EP 331 376), *P. stutzeri* (GB 1,372,034), *P. fluorescens, Pseudomonas sp.* strain SD 705 (WO 95/06720 and WO 96/27002), *P. wisconsinensis* (WO 96/12012), a *Bacillus* lipase, e.g. from *B. subtilis* (Dartois et al. (1993), Biochemica et Biophysica Acta, 1131, 253-360), *B. stearothermophilus* (JP 64/744992) or *B. pumilus* (WO 91/16422).

Other examples are lipase variants such as those described in WO 92/05249, WO 94/01541, EP 407 225, EP 260 105, WO 95/35381, WO 96/00292, WO 95/30744, WO 94/25578, WO 95/14783, WO 95/22615, WO 97/04079 and WO 97/07202.

Preferred commercially available lipase enzymes include Lipex®, Lipolase® and Lipolase Ultra® (Novozymes A/S).

Amylases: Suitable amylases (α and/or β) include those of bacterial or fungal origin. Chemically modified or protein engineered mutants are included. Amylases include, for example, α-amylases obtained from *Bacillus,* e.g. a special strain of *B. licheniformis,* described in more detail in GB 1,296,839.

Examples of useful amylases are the variants described in WO 94/02597, WO 94/18314, WO 96/23873, and WO 97/43424, especially the variants with substitutions in one or more of the following positions: 15, 23, 105, 106, 124, 128, 133, 154, 156, 181, 188, 190, 197, 202, 208, 209, 243, 264, 304, 305, 391, 408, and 444.

Commercially available amylases are Duramyl™, Termamyl™, Fungamyl™ and BAN™ (Novozymes A/S), Rapidase™ and Purastar™ (from Genencor International Inc.).

Cellulases: Suitable cellulases include those of bacterial or fungal origin. Chemically modified or protein engineered mutants are included. Suitable cellulases include cellulases from the genera *Bacillus, Pseudomonas, Humicola, Fusarium, Thielavia, Acremonium,* e.g. the fungal cellulases produced from *Humicola insolens, Myceliophthora thermophila* and *Fusarium oxysporum* disclosed in US 4,435,307, US 5,648,263, US 5,691,178, US 5,776,757 and WO 89/09259.

Especially suitable cellulases are the alkaline or neutral cellulases having colour care benefits. Examples of such cellulases are cellulases described in EP 0 495 257, EP 0 531 372, WO 96/11262, WO 96/29397, WO 98/08940. Other examples are cellulase variants such as those described in WO 94/07998, EP 0 531 315, US 5,457,046, US 5,686,593, US 5,763,254, WO 95/24471, WO 98/12307 and PCT/DK98/00299.

Commercially available cellulases include Celluzyme® and Carezyme® (Novozymes A/S), Clazinase™, and Puradax HA™ (Genencor International Inc.), and KAC-500(B)™ (Kao Corporation).

Peroxidases/Oxidases: Suitable peroxidases/oxidases include those of plant, bacterial or fungal origin. Chemically modified or protein engineered mutants are included. Examples of useful peroxidases include peroxidases from *Coprinus,* e.g. from *C. cinereus,* and variants thereof as those described in WO 93/24618, WO 95/10602, and WO 98/15257.

Commercially available peroxidases include Guardzyme® (Novozymes A/S).

The detergent enzyme(s) may be included in a detergent composition by adding separate additives containing one or more enzymes, or by adding a combined additive comprising all of these enzymes. A detergent additive of the invention, i.e. a separate additive or a combined additive, can be formulated e.g. as a granulate, a liquid, a slurry, etc. Preferred detergent additive formulations are granulates, in particular non-dusting granulates, liquids, in particular stabilized liquids, or slurries.

Non-dusting granulates may be produced, e.g., as disclosed in US 4,106,991 and 4,661,452 and may optionally be coated by methods known in the art. Examples of waxy coating materials are poly(ethylene oxide) products (polyethyleneglycol, PEG) with mean molar weights of 1000 to 20000; ethoxylated nonylphenols having from 16 to 50 ethylene oxide units; ethoxylated fatty alcohols in which the alcohol contains from 12 to 20 carbon atoms and in which there are 15 to 80 ethylene oxide units; fatty alcohols; fatty acids; and mono- and di- and triglycerides of fatty acids. Examples of film-forming coating materials suitable for application by fluid bed techniques are given in GB 1483591. Liquid enzyme preparations may, for instance, be stabilized by adding a polyol such as propylene glycol, a sugar or sugar alcohol, lactic acid or boric acid according to established methods. Protected enzymes may be prepared according to the method disclosed in EP 238,216.

The detergent composition of the invention may be in any convenient form, e.g., a bar, a tablet, a powder, a granule, a paste or a liquid. A liquid detergent may be aqueous, typically containing up to 70 % water and 0-30 % organic solvent, or non-aqueous.

The detergent composition comprises one or more surfactants, which may be non-ionic including semi-polar and/or anionic and/or cationic and/or zwitterionic. The surfactants are typically present at a level of from 0.1 % to 60% by weight.

When included therein the detergent will usually contain from about 1% to about 40% of an anionic surfactant such as linear alkylbenzenesulfonate, alpha-olefinsulfonate, alkyl sulfate (fatty alcohol sulfate), alcohol ethoxysulfate, secondary alkanesulfonate, alpha-sulfo fatty acid methyl ester, alkyl- or alkenylsuccinic acid or soap.

When included therein the detergent will usually contain from about 0.2% to about 40% of a non-ionic surfactant such as alcohol ethoxylate, nonylphenol ethoxylate, alkylpolyglycoside, alkyldimethylamineoxide, ethoxylated fatty acid monoethanolamide, fatty acid monoethanolamide, polyhydroxy alkyl fatty acid amide, or N-acyl N-alkyl derivatives of glucosamine ("glucamides").

The detergent may contain 0-65 % of a detergent builder or complexing agent such as zeolite, diphosphate, triphosphate, phosphonate, carbonate, citrate, nitrilotriacetic acid, ethylenediaminetetraacetic acid, diethylenetriaminepentaacetic acid, alkyl- or alkenylsuccinic acid, soluble silicates or layered silicates (e.g. SKS-6 from Hoechst).

The detergent may comprise one or more polymers. Examples are carboxymethylcellulose, poly(vinylpyrrolidone), poly (ethylene glycol), poly(vinyl alcohol), poly(vinylpyridine-N-oxide), poly(vinylimidazole), polycarboxylates such as polyacrylates, maleic/acrylic acid copolymers and lauryl methacrylate/acrylic acid copolymers.

The detergent may contain a bleaching system which may comprise a $H_2O_2$ source such as perborate or percarbonate which may be combined with a peracid-forming bleach activator such as tetraacetylethylenediamine or nonanoyloxybenzenesulfonate. Alternatively, the bleaching system may comprise peroxyacids of e.g. the amide, imide, or sulfone type.

The enzyme(s) of the detergent composition of the invention may be stabilized using conventional stabilizing agents, e.g., a polyol such as propylene glycol or glycerol, a sugar or sugar alcohol, lactic acid, boric acid, or a boric acid derivative, e.g., an aromatic borate ester, or a phenyl boronic acid derivative such as 4-formylphenyl boronic acid, and the composition may be formulated as described in e.g. WO 92/19709 and WO 92/19708.

The detergent may also contain other conventional detergent ingredients such as e.g. fabric conditioners including clays, foam boosters, suds suppressors, anti-corrosion agents, soil-suspending agents, anti-soil redeposition agents, dyes, bactericides, optical brighteners, hydrotropes, tarnish inhibitors, or perfumes.

It is at present contemplated that in the detergent compositions any enzyme, in particular the enzyme of the invention, may be added in an amount corresponding to 0.01-200 mg of enzyme protein per liter of wash liqour, preferably 0.05-50 mg of enzyme protein per liter of wash liqour, in particular 0.1-10 mg of enzyme protein per liter of wash liqour.

The enzyme of the invention may additionally be incorporated in the detergent formulations disclosed in WO 97/07202 which is hereby incorporated as reference.

## MATERIALS AND METHODS

### Textiles

Standard textile pieces are obtained from EMPA St. Gallen, Lerchfeldstrasse 5, CH-9014 St. Gallen, Switzerland. Especially type EMPA 116 (cotton textile stained with blood, milk and ink) and EMPA 117 (polyester/cotton textile stained with blood, milk and ink).

### Method for producing a subtilase variant

The present invention provides a method of producing an isolated enzyme according to the invention, wherein a suitable host cell, which has been transformed with a DNA sequence encoding the enzyme, is cultured under conditions permitting the production of the enzyme, and the resulting enzyme is recovered from the culture.

When an expression vector comprising a DNA sequence encoding the enzyme is transformed into a heterologous host cell it is possible to enable heterologous recombinant production of the enzyme of the invention. Thereby it is possible to make a highly purified subtilase composition, characterized in being free from homologous impurities.

The medium used to culture the transformed host cells may be any conventional medium suitable for growing the host cells in question. The expressed subtilase may conveniently be secreted into the culture medium and may be recovered there-from by well-known procedures including separating the cells from the medium by centrifugation or filtration, precipitating proteinaceous components of the medium by means of a salt such as ammonium sulfate, followed by chromatographic procedures such as ion exchange chromatography, affinity chromatography, or the like.

## EXAMPLE 1

Construction of library of Savinase variants

A library, based on Savinase positions V28, I35, T71, I72, A73, M175 and T224 (BPN' numbering) have been synthesized. The library contains exclusively TY145-suggested alterations and covers the introduced mutations V28I, A,L; I35V,A,L; T71S; I72A,G,V; A73L,G; M175V,A; T224S,A introduced in oligopeptides, some of which are doped. Doping of nucleotide bases from a desired doping of individual amino acid residues, which is used for the example below, can be calculated as described at page 18 herein.

In the attached sequence listing, the doped nucleotides below have been given the nucleotide symbols recommended by the WIPO Standard ST25.

The constructed oligopeptide primers are listed below. The primers are named after which positions are subject to modifications, thus 28-35-CN may have alterations in positions 28 and 35, 71-72-73-NC may have alterations in positions 71, 72 and 73, and so forth.

28-35-CN, SEQ ID NO:7
5'-TAG ATC TGG ATG AGT GGA (50%T/50%A)(80%A/10%G/10%C)(75%T/25%C) CCC TGT ATC GAG GAC AGC (75%A/25%T)(90%A/10%G)(80%C/10%T/10%G) TTT TAC ACC AGA ACC TGT-3'

28-35-NC, SEQ ID NO:8
5'-TCC ACT CAT CCA GAT CTA-3'

(I) 71-72-73-CN, SEQ ID NO:9
5'-AAT CGA ATT GTT TAA AGC AGC (65%T/35%A)(80%A/10%C/10%G)(75%T/25%C) (90%C/10%T)G(90%T/10%A) CCC GGC CAC ATG CGT GCC-3'

(II) 71-72-73-CN, SEQ ID NO:10
5'-AAT CGA ATT GTT TAA AGC AAG (65%T/35%A)(80%A/10%C/10%G)(75%T/25%C) (90%C/10%T)G(90%T/10%A) CCC GGC CAC ATG CGT GCC-3'

(III) 71-72-73-CN, SEQ ID NO:11
5'-AAT CGA ATT GTT TAA AGC GCC (65%T/35%A)(80%A/10%C/10%G)(75%T/25%C) (90%C/10%T)G(90%T/10%A) CCC GGC CAC ATG CGT GCC-3'

71-72-73-NC, SEQ ID NO:12
5' GCT TTA AAC AAT TCG ATT 3'

139, SEQ ID NO:13
5'-GAT TAA CGC GTT GCC GCT TCT GCG-3'

(I) 175-CN (90%), SEQ ID NO:14
5'-ATC AGT AGC TCC GAC TGC CA(90%T/10%C) TGC GTT CGC ATA GCG CGC-3'

(II) 175-CN (10%), SEQ ID NO:15
5'-ATC AGT AGC TCC GAC TGC CGC TGC GTT CGC ATA GCG CGC-3'

175-NC, SEQ ID NO:16
5'-GCA GTC GGA GCT ACT GAT-3'

224-CN, SEQ ID NO:17
5'-CGC ACC TGC AAC ATG AGG CG(80%T/10%C/10%A) AGC CAT CGA TGT ACC GTT-3'

224-NC, SEQ ID NO:18
5'-CCT CAT GTT GCA GGT GCG-3'

317, SEQ ID NO:19
5'-TGG CGC AAT CGG TAC CAT GGG G-3'

The Savinase gene is used as template for five individual PCR reactions under standard PCR conditions (Sambrook et al., Molecular Cloning: A Laboratory Manual, 2nd Ed., Cold Spring Harbor, 1989) where the oligos are combined as follows:

317 with 28-35-CN, 28-35-NC with 71-72-73-CN (a mixture of 80% (I) 71-72-73-CN, 10% (II) 71-72-73-CN and 10% (III) 71-72-73-CN), 71-72-73-NC with 175-CN (a mixture of 90% (I)175-CN and 10% (II)175-CN), 175-NC with 224-CN, 224-NC with 139, giving PCR products of 125 bp, 126 bp, 312 bp, 165 bp and 158 bp respectively.

The library is assembled by an additional PCR reaction where the five PCR products are mixed in equal molar amounts. Thereby the library contains a large number of different Savinase variants altered in one or more of the mentioned positions. The PCR reaction was assembled using a PTC-200 DNA Engine (MJ Research, Watertown, MA) and the following cycling parameters: 1 cycle of 2 min at 94 °C followed by 25 cycles of 30 sec at 94 °C, 30 sec at 55 °C and 1 min at 68 °C, and 1 cycle of 2 min at 68 °C. The library was cloned by PCR multimerization (Shafikhani et al. 1997) into Savinase expression vector psx222 and transformed into a *B. subtilis* host for expression. Subsequently Savinase variants can be isolated from the library, purified and characterized.

Likewise, properties from a BPN' like subtilase could be transferred to TY145 like subtilase by applying a similar procedure.

## EXAMPLE 2

Transfer of regions from TY145 to BPN' subtilases

The below mentioned highly mobile regions in TY145 have been selected for transfer from TY145 to Savinase. The Savinase regions (BPN' numbering) are deleted and the TY145 regions (SEQ ID NO:1) are inserted instead. In addition regions can be selected for transfer between the psycrophiles TA41/TA39 and BPN' type protease like Savinase, or from TA39/TA41 to TY145 type non-psychrophilic subtilases.

|  | SEGMENT | |
| --- | --- | --- |
| TY145 | SAKDSLIASAVD, positions 144-155 (SEQ ID NO: 22) | |
| Savinase | PSPSATLEQAVN, positions 129-140 (SEQ ID NO: 23) | |

|  | SEGMENT II | |
| --- | --- | --- |
| TY145 | AGNSGSGSNTIGFPGGLV, positions 168-185 (SEQ ID NO: 24) | |
| Savinase | SGNSGAGSISYPARYA, positions 153-172 (SEQ ID NO: 25) | |

|  | SEGMENT IV | |
| --- | --- | --- |
| TY145 | ASVESTWYTGGYNTIS, positions 233-248 (SEQ ID NO: 26) | |
| Savinase | VNVQSTYPGSTYASLN, positions 203-218 (SEQ ID NO: 27) | |

Savinase variants modified by receiving respectively segments II (Hybrid II), IV (Hybrid IV) or I+II (Hybrid I+II) from TY145 were observed to exhibit subtilase activity as determined by the formation of clearing zones on skim milk powder plates.

## EXAMPLE 3

Transfer of regions from S39 and S41 to BPN' subtilases

The below mentioned highly mobile regions in the TA39 subtilase S39 and the TA41 subtilase S41, determined by the previous described homology building programs, have been selected for transfer to Savinase. The Savinase regions (BPN' numbering) are deleted and the S39 regions or S41 regions are inserted instead. Below, the S39 and S41 regions are numbered according to Figure 1. In addition regions can be selected for transfer between the psychrophiles TA41/TA39 and TY145 type non-psychrophilic subtilases. Savinase variant V104S is used as acceptor for the S39 segment II.

| SEGMENT I | |
| --- | --- |
| S39 | MSLGSSG, positions 137-143 (SEQ ID NO: 28) |
| Savinase2 | LSLGSPS, positions 124-130 (SEQ ID NO: 29) |

| SEGMENT II | |
| --- | --- |
| S39 | MSLGSSGESSLI, positions 137-148 (SEQ ID NO: 30) |
| Savinase variant V104S | LSLGSPSPSATL, positions 124-135 (SEQ ID NO: 31) |

(continued)

SEGMENT III
S39                         NNSSITQT, positions 15-22 (SEQ ID NO: 32)
Savinase                    VQAPAAHN, positions 11-18 (SEQ ID NO: 33)


SEGMENT IV
S39                         TVGTTYTN, positions 55-62 (SEQ ID NO: 34)
Savinase2                   VPG*EPST, positions 51*-58 (SEQ ID NO:35)


SEGMENT V
S39                         RQ, positions 68-69
Savinase                    GN, positions 61-62


SEGMENT VI
S39                         SGESSLI, positions 142-148 (SEQ ID NO: 36)
Savinase                    PSPSATL, positions 129-135 (SEQ ID NO: 37)


SEGMENT VII
S39                         WFDGGYATI, positions 237-245 (SEQ ID NO: 38)
Savinase                    YPGSTYASL, positions 209-217 (SEQ ID NO: 39)

Savinase variants modified by receiving respectively segments I or II from S39 were observed to have subtilase activity against the substrate suc-AAPF-pNA (Suc-Ala-Ala-Pro-Phe-pNA). The subtilase activity was determined in a temperature profile assay where specific activities i.e. micromole substrate per minute per mg enzyme against before mentioned substrates, were determined at every 5 degrees Celsius. The measurements were done in a Tris-base buffer pH 9.

To measure subtilase activity in suc-AAPF-pNA: 100 uL 1.56 mM Suc-Ala-Ala-Pro-Phe-pNA in 0.1 M Tris was added to 100 uL Tris-base, pH 9.0 buffer and 20 uL enzyme. The development of the degradation product pNA (paranitrophenol) was measured as initial velocities at 405 nm on an Elisa Reader for 1 minute.

The Savinase variant with segment I substituted had less specific activity against suc-AAPF-pNA compared to Savinase, whereas the Savinase variant with segment II substituted had more than 2 times higher specific activity against suc-AAPF-pNA than Savinase. In an AMSA-test (performed like described in Example 5 herein) the wash performance was shown to be preserved in Savinase variant with segment II compared to Savinase.

Further, four Savinase variants were constructed with the following combinations of segments from S39: Segments III, V and VII; Segments III and V; Segments III, V, VI and Segments III and IV. All four Savinase variants showed subtilase activity on skim milk plates.

Segments from the S41 subtilase suggested for transfer to Savinase are:

SEGMENT VIII
S41         TVGTNFTD, positions 55-62 (SEQ ID NO: 40)
Savinase    VPG*EPST, positions 51-58 (SEQ ID NO: 41)


SEGMENT IX
S41         NGGTGS, positions 82-87 (SEQ ID NO: 42)
Savinase    ALNNSI, positions 74-79 (SEQ ID NO: 43)


SEGMENT X
S41         DDGSGYA, positions 106-112 (SEQ ID NO: 44)
Savinase    ASGSGSV, positions 98-104 (SEQ ID NO: 45)

(continued)

SEGMENT XI

S41          WAQSPAA, positions 263-269 (SEQ ID NO: 46)

Savinase   KQKNPSW, positions 235-241 (SEQ ID NO: 47)

Four Savinase variants were constructed with the following segments from S41:

Segment X; Segments IX and X; Segments VIII and X; and Segments X and XI. All four Savinase variants showed subtilase activity on skim milk plates.

AMSA wash tests were performed on variants with Segment X and Segments X and XI like described in Example 5 herein.

The assay was conducted under the experimental conditions specified below:

| | |
|---|---|
| Detergent base | Omo Acao |
| Detergent dosage | 2.5 g/l |
| Test solution volume | 160 micro l |
| pH | 10-10.5 adjusted with NaHCO$_3$ |
| Wash time | 14 minutes |
| Temperature | 15°C |
| Water hardness | 9°dH |
| Enzyme concentration in test solution | 5 nM, 10 nM and 30 nM |
| Test material | EMPA 117 |

The wash performance score (described in Example 5 herein) of the Savinase variants with Segment X and Segments X and XI was S (1) indicating an improved wash performance compared to Savinase.

**EXAMPLE 4**

Purification and assessment of enzyme concentration

After fermentation, purification of subtilisin variants is accomplished using Hydrophobic Charge Induction Chromatography (HCIC) and subsequent vacuum filtration.

To capture the enzyme, the HCIC uses a cellulose matrix to which 4-Mercapto-Ethyl-Pyridine (4-MEP) is bound.

Beads of the cellulose matrix sized 80-100 μm are mixed with a media containing yeast and the transformed *B. subtilis* capable of secreting the subtilisin variants and incubated at pH 9.5 in Unifilter® microplates.

As 4-MEP is hydrophobic at pH > 7 and the subtilisin variants are hydrophobic at pH 9.5 a hydrophobic association is made between the secreted enzyme and the 4-MEP on the beads. After incubation the media and cell debris is removed by vacuum filtration while the beads and enzyme are kept on the filter.

To elute the enzyme from the beads the pH is now lowered by washing the filter with an elution buffer (pH 5). Hereby the enzymes part from the beads and can be retrieved from the buffer.

The concentration of the purified subtilisin enzyme variants is assessed by active site titration (AST).

The purified enzyme is incubated with the high affinity inhibitor CI-2A at different concentrations to inhibit a varying amount of the active sites. The protease and inhibitor binds to each other at a 1:1 ratio and accordingly the enzyme concentration can be directly related to the concentration of inhibitor, at which all protease is inactive. To measure the residual protease activity, a substrate suc-AAPF-pNA (0.6 mM Suc-Ala-Ala-Pro-Phe-pNA in Tris/HCl buffer) is added after the incubation with inhibitor and during the following 4 minutes the development of the degradation product pNA (paranitrophenol) is measured periodically at 405 nm on an Elisa Reader.

## EXAMPLE 5

Wash performance of detergent compositions comprising modified enzymes

Wash performance of detergent compositions comprising enzyme hybrids or enzyme variants of the present is tested at low washing temperature.

The Savinase variant Hybrid IV of Example 2 was tested for washing performance in two different assays; a microlitre scale assay (AMSA) and a millilitre scale assay (Mini wash).

## AMSA

The enzyme variants of the present application were tested using the Automatic Mechanical Stress Assay (AMSA). With the AMSA test the wash performance of a large quantity of small volume enzyme-detergent solutions can be examined. The AMSA plate has a number of slots for test solutions and a lid firmly squeezing the textile swatch to be washed against all the slot openings. During the washing time, the plate, test solutions, textile and lid are vigorously shaken to bring the test solution in contact with the textile and apply mechanical stress. For further description see WO 02/42740 especially the paragraph "Special method embodiments" at page 23-24.

The assay was conducted under the experimental conditions specified below:

| Detergent base | Omo Acao |
| --- | --- |
| Detergent dosage | 1.5 g/l |
| Test solution volume | 160 micro l |
| pH | 10-10.5 adjusted with NaHCO$_3$ |
| Wash time | 12 minutes |
| Temperature | 20°C |
| Water hardness | 9°dH |
| Enzyme concentration in test solution | 5 nM, 10 nM and 30 nM |
| Test material | EMPA 117 |

After washing the textile pieces were flushed in tap water and air-dried.

The performance of the enzyme variant is measured as the brightness of the colour of the textile samples washed with that specific enzyme variant. Brightness can also be expressed as the intensity of the light reflected from the textile sample when luminated with white light. When the textile is stained the intensity of the reflected light is lower, than that of a clean textile. Therefore the intensity of the reflected light can be used to measure wash performance of an enzyme variant.

Colour measurements are made with a professional flatbed scanner (*PFU DL2400pro*), which is used to capture an image of the washed textile samples. The scans are made with a resolution of 200 dpi and with an output colour dept of 24 bits. In order to get accurate results, the scanner is frequently calibrated with a *Kodak reflective IT8 target*.

To extract a value for the light intensity from the scanned images, a special designed software application is used (*Novozymes Color Vector Analyzer*). The program retrieves the 24 bit pixel values from the image and converts them into values for red, green and blue (RGB). The intensity value (Int) is calculated by adding the RGB values together as vectors and then taking the length of the resulting vector:

**Error! Objects cannot be created from editing field codes..**

The wash performance (P) of the variants was calculated in accordance with the below formula:

$$P = Int(v) - Int(r)$$

where

Int(v) is the light intensity value of textile surface washed with enzyme variant and

Int(r) is the light intensity value of textile surface washed with the reference enzyme subtilisin 309 (BLSAVI).

The result of the AMSA wash of Hybrid IV was a Performance Score of S (2) in accordance with the definition:

Performance Scores (S) are summing up the performances (P) of the tested enzyme variants as:

S (2) which indicates that the variant performs better than the reference at all three concentrations (5, 10 and 30 nM) and
S (1) which indicates that the variant performs better than the reference at one or two concentrations.

**Mini wash assay**

The millilitre scale wash performance assay was conducted under the following conditions:

| Detergent base | Omo Acao detergent powder |
|---|---|
| Detergent dose | 1.5 g/l |
| pH | "as is" in the current detergent solution and is not adjusted. |
| Wash time | 14 min. |
| Temperature | 20°C |
| Water hardness | 9°dH, adjusted by adding $CaCl_2*2H_2O$; $MgCl_2*6H_2O$; $NaHCO_3$ ($Ca^{2+}$:$Mg^{2+}$:$HCO^{3-}$ = 2:1:6) to milli-Q water. |
| Enzymes | Hybrid IV, Savinase |
| Enzyme conc. | 5 nM, 10 nM |
| Test system | 125 ml glass beakers. Textile dipped in test solution. Continuously up and down, 50 times per minute |
| Textile/volume | 1 textile piece (13 x 3 cm) in 50 ml test solution |
| Test material | EMPA 117 textile swatches |

After wash the measurement of remission from the test material was done at 460 nm using a Zeiss MCS 521 VIS spectrophotometer. The measurements were done according to the manufacturer's protocol.
As shown in Table 1 the textile washed with the Savinase variant Hybrid IV at 20°C in Omo Acao has a higher remission than the textile washed with the parent. This result indicates that this variant has better wash performance at low temperature than the parent Savinase.

Table 1. Wash performance results of the subtilase variant in Omo Acao for a dosage of 5 nM and 10 nM enzyme.

| Enzyme | Remission, 5 nM enzyme | Remission, 10 nM enzyme |
|---|---|---|
| Blank (no enzyme) | 12,0 | 12,3 |
| Savinase | 15,8 | 17,4 |
| Hybrid IV | 17,0 | 18,3 |

As it can be concluded from Table 1 the modified subtilases of the invention exhibits an improvement in wash performance.

**EXAMPLE 6**

Wash performance of detergent compositions comprising enzyme variants of the present invention was tested at low washing temperature using the Automatic Mechanical Stress Assay (AMSA) as described in Example 5 herein.

Table 2. AMSA wash results of subtilase variants.

| Enzyme variant | Performance score |
|---|---|
| V28I+*98aD+T224S | 2 |

(continued)

| Enzyme variant | Performance score |
|---|---|
| *98aS+P131F+M175V+T224A | 1 |

**APPENDIX 1**

```
REMARK  3 REFINEMENT.
REMARK  3   PROGRAM     : REFMAC 5.0
REMARK  3   AUTHORS     : MURSHUDOV,VAGIN,DODSON
REMARK  3
REMARK  3   REFINEMENT TARGET : MAXIMUM LIKELIHOOD
REMARK  3
REMARK  3 DATA USED IN REFINEMENT.
REMARK  3   RESOLUTION RANGE HIGH (ANGSTROMS) :  1.80
REMARK  3   RESOLUTION RANGE LOW  (ANGSTROMS) : 56.80
REMARK  3   DATA CUTOFF          (SIGMA(F)) : NONE
REMARK  3   COMPLETENESS FOR RANGE       (%) :  99.88
REMARK  3   NUMBER OF REFLECTIONS            :  38045
REMARK  3
REMARK  3 FIT TO DATA USED IN REFINEMENT.
REMARK  3   CROSS-VALIDATION METHOD        : THROUGHOUT
REMARK  3   FREE R VALUE TEST SET SELECTION  : RANDOM
REMARK  3   R VALUE     (WORKING + TEST SET) : 0.15648
REMARK  3   R VALUE          (WORKING SET) : 0.15487
REMARK  3   FREE R VALUE                 : 0.18707
REMARK  3   FREE R VALUE TEST SET SIZE  (%) : 5.0
REMARK  3   FREE R VALUE TEST SET COUNT   : 2009
REMARK  3
REMARK  3 FIT IN THE HIGHEST RESOLUTION BIN.
REMARK  3   TOTAL NUMBER OF BINS USED       :     20
REMARK  3   BIN RESOLUTION RANGE HIGH       :   1.796
REMARK  3   BIN RESOLUTION RANGE LOW        :   1.842
REMARK  3   REFLECTION IN BIN    (WORKING SET) :    2738
REMARK  3   BIN R VALUE          (WORKING SET) :   0.191
REMARK  3   BIN FREE R VALUE SET COUNT      :     138
REMARK  3   BIN FREE R VALUE            :   0.234
REMARK  3
REMARK  3 NUMBER OF NON-HYDROGEN ATOMS USED IN REFINEMENT.
REMARK  3   ALL ATOMS              :    3156
REMARK  3
REMARK  3 B VALUES.
REMARK  3   FROM WILSON PLOT         (A**2) : NULL
REMARK  3   MEAN B VALUE      (OVERALL, A**2) : 14.804
REMARK  3   OVERALL ANISOTROPIC B VALUE.
REMARK  3    B11 (A**2) :    0.28
REMARK  3    B22 (A**2) :   -0.86
REMARK  3    B33 (A**2) :    0.58
REMARK  3    B12 (A**2) :    0.00
REMARK  3    B13 (A**2) :    0.00
REMARK  3    B23 (A**2) :    0.00
REMARK  3
REMARK  3 ESTIMATED OVERALL COORDINATE ERROR.
REMARK  3   ESU BASED ON R VALUE                (A):  0.100
REMARK  3   ESU BASED ON FREE R VALUE           (A):  0.098
REMARK  3   ESU BASED ON MAXIMUM LIKELIHOOD        (A):  0.093
REMARK  3   ESU FOR B VALUES BASED ON MAXIMUM LIKELIHOOD (A**2):  2.910
REMARK  3
REMARK  3 CORRELATION COEFFICIENTS.
REMARK  3   CORRELATION COEFFICIENT FO-FC    :  0.963
REMARK  3   CORRELATION COEFFICIENT FO-FC FREE :  0.952
REMARK  3
REMARK  3 RMS DEVIATIONS FROM IDEAL VALUES      COUNT   RMS   WEIGHT
REMARK  3   BOND LENGTHS REFINED ATOMS       (A): 2798 ; 0.021 ; 0.021
REMARK  3   BOND LENGTHS OTHERS             (A): 2500 ; 0.001 ; 0.020
REMARK  3   BOND ANGLES REFINED ATOMS   (DEGREES): 3805 ; 1.859 ; 1.943
REMARK  3   BOND ANGLES OTHERS          (DEGREES): 5821 ; 0.854 ; 3.000
```

```
REMARK   3   TORSION ANGLES, PERIOD 1    (DEGREES):   372 ; 5.125 ; 3.000
REMARK   3   TORSION ANGLES, PERIOD 3    (DEGREES):   462 ;16.877 ;15.000
REMARK   3   CHIRAL-CENTER RESTRAINTS       (A**3):   437 ; 0.119 ; 0.200
REMARK   3   GENERAL PLANES REFINED ATOMS     (A): 3201 ; 0.009 ; 0.020
REMARK   3   GENERAL PLANES OTHERS            (A):  535 ; 0.004 ; 0.020
REMARK   3   NON-BONDED CONTACTS REFINED ATOMS (A):  610 ; 0.228 ; 0.300
REMARK   3   NON-BONDED CONTACTS OTHERS       (A): 2548 ; 0.203 ; 0.300
REMARK   3   H-BOND (X...Y) REFINED ATOMS     (A):  374 ; 0.184 ; 0.500
REMARK   3   H-BOND (X...Y) OTHERS            (A):    3 ; 0.279 ; 0.500
REMARK   3   POTENTIAL METAL-ION REFINED ATOMS (A):   16 ; 0.119 ; 0.500
REMARK   3   SYMMETRY VDW REFINED ATOMS       (A):    7 ; 0.127 ; 0.300
REMARK   3   SYMMETRY VDW OTHERS              (A):   27 ; 0.152 ; 0.300
REMARK   3   SYMMETRY H-BOND REFINED ATOMS    (A):   37 ; 0.278 ; 0.500
REMARK   3
REMARK   3 ISOTROPIC THERMAL FACTOR RESTRAINTS.    COUNT   RMS    WEIGHT
REMARK   3   MAIN-CHAIN BOND REFINED ATOMS  (A**2): 1840 ; 1.131 ; 1.500
REMARK   3   MAIN-CHAIN ANGLE REFINED ATOMS (A**2): 2941 ; 1.781 ; 2.000
REMARK   3   SIDE-CHAIN BOND REFINED ATOMS  (A**2):  958 ; 2.873 ; 3.000
REMARK   3   SIDE-CHAIN ANGLE REFINED ATOMS (A**2):  864 ; 4.300 ; 4.500
REMARK   3
REMARK   3 NCS RESTRAINTS STATISTICS
REMARK   3   NUMBER OF NCS GROUPS : NULL
REMARK   3
REMARK   3
REMARK   3 TLS DETAILS
REMARK   3   NUMBER OF TLS GROUPS  : NULL
REMARK   3
REMARK   3
REMARK   3 BULK SOLVENT MODELLING.
REMARK   3   METHOD USED : BABINET MODEL WITH MASK
REMARK   3   PARAMETERS FOR MASK CALCULATION
REMARK   3   VDW PROBE RADIUS   :   1.40
REMARK   3   ION PROBE RADIUS   :   0.80
REMARK   3   SHRINKAGE RADIUS   :   0.80
REMARK   3
REMARK   3 OTHER REFINEMENT REMARKS:
REMARK   3 HYDROGENS HAVE BEEN ADDED IN THE RIDING POSITIONS
REMARK   3
CISPEP   1 GLY A  172    SER A  173                  0.00
CISPEP   2 PHE A  180    PRO A  181                  0.00
SSBOND   1 CYS A   52    CYS A   66
CRYST1   58.753   66.838  107.082  90.00  90.00  90.00 P 21 21 21
SCALE1     0.017020  0.000000  0.000000       0.00000
SCALE2     0.000000  0.014962  0.000000       0.00000
SCALE3     0.000000  0.000000  0.009339       0.00000
ATOM      1  N   ALA A   1      2.336 20.870  1.027 1.00 27.48       N
ATOM      3  CA  ALA A   1      1.951 20.940  2.465 1.00 29.42       C
ATOM      5  CB  ALA A   1      2.391 19.637  3.197 1.00 29.45       C
ATOM      9  C   ALA A   1      2.665 22.149  3.096 1.00 28.87       C
ATOM     10  O   ALA A   1      3.696 22.577  2.627 1.00 30.49       O
ATOM     13  N   VAL A   2      2.014 22.747  4.052 1.00 30.31       N
ATOM     15  CA  VAL A   2      2.658 23.754  4.877 1.00 30.69       C
ATOM     17  CB  VAL A   2      2.068 25.139  4.604 1.00 30.94       C
ATOM     19  CG1 VAL A   2      2.611 25.702  3.252 1.00 32.62       C
ATOM     23  CG2 VAL A   2      0.577 25.086  4.667 1.00 30.84       C
ATOM     27  C   VAL A   2      2.494 23.346  6.347 1.00 29.48       C
ATOM     28  O   VAL A   2      1.580 22.610  6.743 1.00 29.33       O
ATOM     29  N   PRO A   3      3.412 23.788  7.186 1.00 28.10       N
ATOM     30  CA  PRO A   3      3.298 23.380  8.581 1.00 27.90       C
ATOM     32  CB  PRO A   3      4.645 23.830  9.185 1.00 26.48       C
ATOM     35  CG  PRO A   3      5.116 24.998  8.340 1.00 26.57       C
ATOM     38  CD  PRO A   3      4.530 24.697  6.933 1.00 27.60       C
ATOM     41  C   PRO A   3      2.129 24.112  9.216 1.00 27.01       C
```

```
ATOM   42   O    PRO A   3    1.602  25.037   8.600  1.00  28.48        O
ATOM   43   N    SER A   4    1.767  23.774  10.434  1.00  26.37        N
ATOM   45   CA   SER A   4    0.718  24.505  11.159  1.00  25.29        C
ATOM   47   CB   SER A   4    0.279  23.780  12.444  1.00  26.52        C
ATOM   50   OG   SER A   4    1.173  23.913  13.554  1.00  25.41        O
ATOM   52   C    SER A   4    1.105  25.956  11.445  1.00  26.09        C
ATOM   53   O    SER A   4    0.212  26.810  11.603  1.00  25.32        O
ATOM   54   N    THR A   5    2.415  26.233  11.497  1.00  24.12        N
ATOM   56   CA   THR A   5    3.023  27.567  11.741  1.00  22.81        C
ATOM   58   CB   THR A   5    3.009  28.007  13.237  1.00  24.21        C
ATOM   60   OG1  THR A   5    3.793  29.191  13.386  1.00  23.29        O
ATOM   62   CG2  THR A   5    3.727  26.999  14.151  1.00  22.71        C
ATOM   66   C    THR A   5    4.413  27.436  11.219  1.00  23.10        C
ATOM   67   O    THR A   5    5.012  26.322  11.267  1.00  21.48        O
ATOM   68   N    GLN A   6    4.959  28.518  10.692  1.00  21.30        N
ATOM   70   CA   GLN A   6    6.260  28.438  10.102  1.00  21.50        C
ATOM   72   CB   GLN A   6    6.512  29.634   9.209  1.00  22.77        C
ATOM   75   CG   GLN A   6    5.626  29.570   7.926  1.00  23.15        C
ATOM   78   CD   GLN A   6    5.999  30.579   6.911  1.00  28.40        C
ATOM   79   OE1  GLN A   6    5.356  31.619   6.822  1.00  30.34        O
ATOM   80   NE2  GLN A   6    7.016  30.300   6.133  1.00  24.59        N
ATOM   83   C    GLN A   6    7.295  28.389  11.185  1.00  20.24        C
ATOM   84   O    GLN A   6    8.438  28.017  10.927  1.00  18.46        O
ATOM   85   N    THR A   7    6.870  28.777  12.378  1.00  18.84        N
ATOM   87   CA   THR A   7    7.752  28.808  13.565  1.00  19.27        C
ATOM   89   CB   THR A   7    8.135  30.238  13.914  1.00  19.55        C
ATOM   91   OG1  THR A   7    6.958  31.041  14.091  1.00  23.00        O
ATOM   93   CG2  THR A   7    8.910  30.878  12.842  1.00  19.61        C
ATOM   97   C    THR A   7    7.111  28.128  14.755  1.00  18.28        C
ATOM   98   O    THR A   7    6.436  28.735  15.547  1.00  18.57        O
ATOM   99   N    PRO A   8    7.288  26.803  14.834  1.00  17.34        N
ATOM  100   CA   PRO A   8    6.795  26.000  15.922  1.00  17.79        C
ATOM  102   CB   PRO A   8    7.459  24.615  15.659  1.00  17.18        C
ATOM  105   CG   PRO A   8    7.556  24.570  14.138  1.00  18.32        C
ATOM  108   CD   PRO A   8    7.961  25.984  13.814  1.00  16.59        C
ATOM  111   C    PRO A   8    7.162  26.584  17.273  1.00  16.99        C
ATOM  112   O    PRO A   8    8.105  27.339  17.369  1.00  18.02        O
ATOM  113   N    TRP A   9    6.426  26.203  18.280  1.00  18.21        N
ATOM  115   CA   TRP A   9    6.613  26.750  19.611  1.00  17.56        C
ATOM  117   CB   TRP A   9    5.723  26.059  20.603  1.00  17.84        C
ATOM  120   CG   TRP A   9    6.129  24.806  21.197  1.00  14.36        C
ATOM  121   CD1  TRP A   9    5.772  23.569  20.796  1.00  15.37        C
ATOM  123   NE1  TRP A   9    6.278  22.630  21.658  1.00  15.66        N
ATOM  125   CE2  TRP A   9    7.033  23.276  22.609  1.00  16.83        C
ATOM  126   CD2  TRP A   9    6.952  24.642  22.345  1.00  14.81        C
ATOM  127   CE3  TRP A   9    7.642  25.531  23.186  1.00  14.62        C
ATOM  129   CZ3  TRP A   9    8.362  24.982  24.301  1.00  11.92        C
ATOM  131   CH2  TRP A   9    8.393  23.626  24.526  1.00  15.97        C
ATOM  133   CZ2  TRP A   9    7.757  22.750  23.677  1.00  14.67        C
ATOM  135   C    TRP A   9    8.073  26.760  20.083  1.00  18.17        C
ATOM  136   O    TRP A   9    8.531  27.737  20.662  1.00  15.91        O
ATOM  137   N    GLY A  10    8.780  25.675  19.859  1.00  16.36        N
ATOM  139   CA   GLY A  10   10.180  25.618  20.307  1.00  15.48        C
ATOM  142   C    GLY A  10   11.108  26.619  19.663  1.00  15.80        C
ATOM  143   O    GLY A  10   12.089  27.060  20.254  1.00  14.71        O
ATOM  144   N    ILE A  11   10.801  26.939  18.410  1.00  15.18        N
ATOM  146   CA   ILE A  11   11.599  27.866  17.642  1.00  15.64        C
ATOM  148   CB   ILE A  11   11.303  27.768  16.151  1.00  14.65        C
ATOM  150   CG1  ILE A  11   11.479  26.328  15.653  1.00  15.91        C
ATOM  153   CD1  ILE A  11   12.945  25.811  15.725  1.00  16.96        C
ATOM  157   CG2  ILE A  11   12.204  28.704  15.385  1.00  16.09        C
ATOM  161   C    ILE A  11   11.291  29.225  18.193  1.00  15.73        C
ATOM  162   O    ILE A  11   12.197  29.995  18.438  1.00  15.02        O
```

```
ATOM    163  N    LYS A  12     10.005  29.552  18.352  1.00 16.62           N
ATOM    165  CA   LYS A  12      9.649  30.832  18.949  1.00 16.28           C
ATOM    167  CB   LYS A  12      8.147  30.926  19.078  1.00 17.21           C
ATOM    170  CG   LYS A  12      7.419  31.148  17.707  1.00 20.12           C
ATOM    173  CD   LYS A  12      5.874  31.303  17.997  1.00 20.50           C
ATOM    176  CE   LYS A  12      5.137  31.855  16.795  1.00 28.93           C
ATOM    179  NZ   LYS A  12      4.564  30.819  15.858  1.00 19.42           N
ATOM    183  C    LYS A  12     10.242  30.956  20.345  1.00 15.82           C
ATOM    184  O    LYS A  12     10.790  32.014  20.745  1.00 15.87           O
ATOM    185  N    SER A  13     10.172  29.865  21.075  1.00 13.09           N
ATOM    187  CA   SER A  13     10.655  29.893  22.450  1.00 13.64           C
ATOM    189  CB   SER A  13     10.284  28.586  23.149  1.00 12.46           C
ATOM    192  OG   SER A  13     10.790  28.548  24.491  1.00 14.12           O
ATOM    194  C    SER A  13     12.167  30.131  22.519  1.00 12.81           C
ATOM    195  O    SER A  13     12.650  30.959  23.323  1.00 11.51           O
ATOM    196  N    ILE A  14     12.931  29.371  21.752  1.00 12.90           N
ATOM    198  CA   ILE A  14     14.391  29.521  21.839  1.00 13.05           C
ATOM    200  CB   ILE A  14     15.108  28.318  21.206  1.00 12.73           C
ATOM    202  CG1  ILE A  14     16.498  28.183  21.810  1.00 13.73           C
ATOM    205  CD1  ILE A  14     17.265  26.959  21.415  1.00 17.32           C
ATOM    209  CG2  ILE A  14     15.161  28.394  19.753  1.00 14.13           C
ATOM    213  C    ILE A  14     14.869  30.861  21.299  1.00 14.41           C
ATOM    214  O    ILE A  14     15.907  31.367  21.680  1.00 15.23           O
ATOM    215  N    TYR A  15     14.094  31.423  20.389  1.00 15.25           N
ATOM    217  CA   TYR A  15     14.392  32.753  19.877  1.00 16.87           C
ATOM    219  CB   TYR A  15     13.742  32.965  18.490  1.00 15.13           C
ATOM    222  CG   TYR A  15     14.683  32.629  17.348  1.00 16.40           C
ATOM    223  CD1  TYR A  15     14.956  31.303  17.008  1.00 14.01           C
ATOM    225  CE1  TYR A  15     15.834  30.998  16.024  1.00 16.36           C
ATOM    227  CZ   TYR A  15     16.453  31.993  15.321  1.00 16.82           C
ATOM    228  OH   TYR A  15     17.364  31.738  14.329  1.00 15.20           O
ATOM    230  CE2  TYR A  15     16.182  33.303  15.621  1.00 17.27           C
ATOM    232  CD2  TYR A  15     15.320  33.610  16.628  1.00 16.64           C
ATOM    234  C    TYR A  15     13.925  33.826  20.856  1.00 16.27           C
ATOM    235  O    TYR A  15     14.311  35.008  20.744  1.00 17.84           O
ATOM    236  N    ASN A  16     13.075  33.432  21.780  1.00 17.21           N
ATOM    238  CA   ASN A  16     12.534  34.334  22.811  1.00 17.53           C
ATOM    240  CB   ASN A  16     13.628  34.860  23.743  1.00 16.83           C
ATOM    243  CG   ASN A  16     13.098  35.265  25.103  1.00 18.27           C
ATOM    244  OD1  ASN A  16     11.901  35.461  25.288  1.00 21.90           O
ATOM    245  ND2  ASN A  16     13.987  35.324  26.075  1.00 17.31           N
ATOM    248  C    ASN A  16     11.788  35.480  22.114  1.00 19.04           C
ATOM    249  O    ASN A  16     11.940  36.642  22.463  1.00 18.19           O
ATOM    250  N    ASP A  17     10.972  35.107  21.135  1.00 18.56           N
ATOM    252  CA   ASP A  17     10.176  36.069  20.372  1.00 19.69           C
ATOM    254  CB   ASP A  17     11.019  36.634  19.287  1.00 19.45           C
ATOM    257  CG   ASP A  17     10.362  37.812  18.579  1.00 20.65           C
ATOM    258  OD1  ASP A  17      9.160  38.017  18.745  1.00 24.61           O
ATOM    259  OD2  ASP A  17     11.034  38.547  17.849  1.00 19.73           O
ATOM    260  C    ASP A  17      8.937  35.412  19.778  1.00 19.80           C
ATOM    261  O    ASP A  17      9.032  34.693  18.834  1.00 22.05           O
ATOM    262  N    GLN A  18      7.791  35.647  20.369  1.00 20.51           N
ATOM    264  CA   GLN A  18      6.593  34.957  19.960  1.00 21.49           C
ATOM    266  CB   GLN A  18      5.549  35.062  21.054  1.00 22.51           C
ATOM    269  CG   GLN A  18      5.917  34.348  22.318  1.00 24.55           C
ATOM    272  CD   GLN A  18      6.243  32.907  22.041  1.00 29.84           C
ATOM    273  OE1  GLN A  18      7.347  32.450  22.314  1.00 31.44           O
ATOM    274  NE2  GLN A  18      5.301  32.204  21.457  1.00 28.00           N
ATOM    277  C    GLN A  18      6.076  35.511  18.658  1.00 21.92           C
ATOM    278  O    GLN A  18      5.213  34.911  18.021  1.00 22.98           O
ATOM    279  N    SER A  19      6.697  36.572  18.185  1.00 22.89           N
ATOM    281  CA   SER A  19      6.231  37.202  16.951  1.00 23.92           C
ATOM    283  CB   SER A  19      6.338  38.715  17.096  1.00 23.69           C
```

```
ATOM    286  OG  SER A  19      7.627  39.225  16.746  1.00 25.66           O
ATOM    288  C   SER A  19      6.947  36.728  15.670  1.00 24.30           C
ATOM    289  O   SER A  19      6.454  36.972  14.566  1.00 25.69           O
ATOM    290  N   ILE A  20      8.079  36.029  15.764  1.00 23.62           N
ATOM    292  CA  ILE A  20      8.791  35.673  14.529  1.00 23.92           C
ATOM    294  CB  ILE A  20     10.104  34.965  14.836  1.00 24.53           C
ATOM    296  CG1 ILE A  20      9.858  33.755  15.727  1.00 22.48           C
ATOM    299  CD1 ILE A  20     11.041  32.826  15.641  1.00 23.83           C
ATOM    303  CG2 ILE A  20     11.127  35.902  15.477  1.00 27.13           C
ATOM    307  C   ILE A  20      8.011  34.784  13.573  1.00 23.40           C
ATOM    308  O   ILE A  20      7.241  33.913  13.999  1.00 24.30           O
ATOM    309  N   THR A  21      8.295  34.963  12.296  1.00 23.97           N
ATOM    311  CA  THR A  21      7.686  34.184  11.215  1.00 24.95           C
ATOM    313  CB  THR A  21      6.856  35.107  10.283  1.00 24.97           C
ATOM    315  OG1 THR A  21      7.690  36.186   9.842  1.00 27.61           O
ATOM    317  CG2 THR A  21      5.771  35.794  11.039  1.00 28.02           C
ATOM    321  C   THR A  21      8.799  33.570  10.392  1.00 23.92           C
ATOM    322  O   THR A  21      8.544  32.865   9.419  1.00 23.90           O
ATOM    323  N   LYS A  22     10.044  33.862  10.735  1.00 23.93           N
ATOM    325  CA  LYS A  22     11.148  33.239  10.041  1.00 24.18           C
ATOM    327  CB  LYS A  22     11.386  33.880   8.675  1.00 25.22           C
ATOM    330  CG  LYS A  22     11.830  35.314   8.724  1.00 28.95           C
ATOM    333  CD  LYS A  22     12.397  35.808   7.348  1.00 32.55           C
ATOM    336  CE  LYS A  22     13.701  35.129   6.988  0.10 31.56           C
ATOM    339  NZ  LYS A  22     14.274  35.671   5.722  0.10 31.66           N
ATOM    343  C   LYS A  22     12.406  33.333  10.903  1.00 23.63           C
ATOM    344  O   LYS A  22     12.471  34.145  11.823  1.00 24.17           O
ATOM    345  N   THR A  23     13.395  32.491  10.628  1.00 21.41           N
ATOM    347  CA  THR A  23     14.661  32.526  11.342  1.00 20.21           C
ATOM    349  CB  THR A  23     14.914  31.202  12.030  1.00 20.28           C
ATOM    351  OG1 THR A  23     14.859  30.158  11.034  1.00 19.28           O
ATOM    353  CG2 THR A  23     13.846  30.915  13.074  1.00 21.44           C
ATOM    357  C   THR A  23     15.785  32.791  10.384  1.00 20.28           C
ATOM    358  O   THR A  23     15.565  32.740   9.182  1.00 19.80           O
ATOM    359  N   THR A  24     16.996  33.027  10.908  1.00 19.65           N
ATOM    361  CA  THR A  24     18.201  33.246  10.130  1.00 19.91           C
ATOM    363  CB  THR A  24     18.532  34.784   9.840  1.00 21.93           C
ATOM    365  OG1 THR A  24     18.685  35.435  11.102  1.00 25.69           O
ATOM    367  CG2 THR A  24     17.402  35.514   9.229  1.00 25.02           C
ATOM    371  C   THR A  24     19.407  32.743  10.928  1.00 19.55           C
ATOM    372  O   THR A  24     19.372  32.551  12.149  1.00 19.70           O
ATOM    373  N   GLY A  25     20.473  32.497  10.225  1.00 17.41           N
ATOM    375  CA  GLY A  25     21.716  32.187  10.893  1.00 17.64           C
ATOM    378  C   GLY A  25     22.286  30.799  10.641  1.00 16.16           C
ATOM    379  O   GLY A  25     21.583  29.936  10.124  1.00 15.92           O
ATOM    380  N   GLY A  26     23.548  30.620  11.068  1.00 15.49           N
ATOM    382  CA  GLY A  26     24.296  29.380  10.938  1.00 15.44           C
ATOM    385  C   GLY A  26     25.166  29.220   9.692  1.00 15.61           C
ATOM    386  O   GLY A  26     25.782  28.199   9.493  1.00 15.36           O
ATOM    387  N   SER A  27     25.264  30.249   8.861  1.00 17.72           N
ATOM    389  CA  SER A  27     26.108  30.182   7.691  1.00 18.45           C
ATOM    391  CB  SER A  27     25.990  31.500   6.837  1.00 20.08           C
ATOM    394  OG  SER A  27     26.686  32.496   7.490  1.00 26.34           O
ATOM    396  C   SER A  27     27.534  29.838   8.029  1.00 17.45           C
ATOM    397  O   SER A  27     28.166  30.341   8.969  1.00 17.59           O
ATOM    398  N   GLY A  28     28.071  28.913   7.241  1.00 16.01           N
ATOM    400  CA  GLY A  28     29.421  28.494   7.385  1.00 16.46           C
ATOM    403  C   GLY A  28     29.615  27.360   8.377  1.00 15.36           C
ATOM    404  O   GLY A  28     30.739  26.917   8.527  1.00 16.82           O
ATOM    405  N   ILE A  29     28.587  26.931   9.076  1.00 14.25           N
ATOM    407  CA  ILE A  29     28.782  25.832  10.044  1.00 13.92           C
ATOM    409  CB  ILE A  29     28.057  26.170  11.335  1.00 14.10           C
ATOM    411  CG1 ILE A  29     28.482  27.563  11.856  1.00 13.61           C
```

```
ATOM    414  CD1 ILE A   29      29.986  27.710  12.081  1.00 15.86          C
ATOM    418  CG2 ILE A   29      28.269  25.076  12.417  1.00 14.40          C
ATOM    422  C   ILE A   29      28.143  24.586   9.459  1.00 14.30          C
ATOM    423  O   ILE A   29      27.190  24.708   8.690  1.00 14.55          O
ATOM    424  N   LYS A   30      28.614  23.402   9.853  1.00 13.73          N
ATOM    426  CA  LYS A   30      28.008  22.173   9.422  1.00 13.73          C
ATOM    428  CB  LYS A   30      29.019  21.204   8.860  1.00 14.64          C
ATOM    431  CG  LYS A   30      30.072  21.822   7.951  1.00 15.03          C
ATOM    434  CD  LYS A   30      29.438  22.566   6.745  1.00 16.42          C
ATOM    437  CE  LYS A   30      30.497  23.364   5.987  1.00 19.44          C
ATOM    440  NZ  LYS A   30      29.865  24.009   4.752  1.00 17.01          N
ATOM    444  C   LYS A   30      27.354  21.482  10.655  1.00 13.57          C
ATOM    445  O   LYS A   30      27.978  21.473  11.716  1.00 14.48          O
ATOM    446  N   VAL A   31      26.163  20.941  10.498  1.00 13.11          N
ATOM    448  CA  VAL A   31      25.572  20.118  11.583  1.00 13.65          C
ATOM    450  CB  VAL A   31      24.249  20.639  12.061  1.00 13.16          C
ATOM    452  CG1 VAL A   31      23.726  19.743  13.173  1.00 15.78          C
ATOM    456  CG2 VAL A   31      24.401  22.058  12.573  1.00 12.55          C
ATOM    460  C   VAL A   31      25.470  18.708  11.047  1.00 14.30          C
ATOM    461  O   VAL A   31      24.828  18.458  10.003  1.00 14.74          O
ATOM    462  N   ALA A   32      26.129  17.789  11.737  1.00 14.62          N
ATOM    464  CA  ALA A   32      26.092  16.387  11.366  1.00 13.46          C
ATOM    466  CB  ALA A   32      27.419  15.728  11.606  1.00 11.29          C
ATOM    470  C   ALA A   32      24.972  15.696  12.149  1.00 13.08          C
ATOM    471  O   ALA A   32      25.056  15.556  13.393  1.00 12.55          O
ATOM    472  N   VAL A   33      23.916  15.340  11.435  1.00 10.76          N
ATOM    474  CA  VAL A   33      22.778  14.654  12.038  1.00 11.75          C
ATOM    476  CB  VAL A   33      21.468  15.152  11.453  1.00 11.00          C
ATOM    478  CG1 VAL A   33      20.317  14.313  11.935  1.00 13.80          C
ATOM    482  CG2 VAL A   33      21.268  16.618  11.738  1.00 12.19          C
ATOM    486  C   VAL A   33      22.959  13.155  11.847  1.00 12.72          C
ATOM    487  O   VAL A   33      22.830  12.623  10.715  1.00 11.99          O
ATOM    488  N   LEU A   34      23.290  12.466  12.932  1.00 12.34          N
ATOM    490  CA  LEU A   34      23.599  11.022  12.949  1.00 11.72          C
ATOM    492  CB  LEU A   34      24.811  10.744  13.878  1.00 11.57          C
ATOM    495  CG  LEU A   34      26.190  10.819  13.262  1.00 11.59          C
ATOM    497  CD1 LEU A   34      26.515  12.228  12.621  1.00 10.38          C
ATOM    501  CD2 LEU A   34      27.275  10.414  14.265  1.00 12.17          C
ATOM    505  C   LEU A   34      22.303  10.366  13.416  1.00 12.33          C
ATOM    506  O   LEU A   34      21.964  10.409  14.581  1.00 11.53          O
ATOM    507  N   ASP A   35      21.571   9.765  12.490  1.00 11.34          N
ATOM    509  CA  ASP A   35      20.224   9.416  12.794  1.00 11.07          C
ATOM    511  CB  ASP A   35      19.397  10.677  12.779  1.00 12.91          C
ATOM    514  CG  ASP A   35      18.231  10.611  13.697  1.00 11.93          C
ATOM    515  OD1 ASP A   35      17.334   9.791  13.472  1.00 12.81          O
ATOM    516  OD2 ASP A   35      18.166  11.390  14.700  1.00 15.59          O
ATOM    517  C   ASP A   35      19.687   8.393  11.816  1.00 12.34          C
ATOM    518  O   ASP A   35      20.470   7.623  11.250  1.00 12.33          O
ATOM    519  N   THR A   36      18.376   8.385  11.604  1.00 12.33          N
ATOM    521  CA  THR A   36      17.783   7.395  10.702  1.00 13.86          C
ATOM    523  CB  THR A   36      16.312   7.198  10.972  1.00 13.36          C
ATOM    525  OG1 THR A   36      15.603   8.446  10.753  1.00 14.42          O
ATOM    527  CG2 THR A   36      16.058   6.781  12.383  1.00 11.98          C
ATOM    531  C   THR A   36      17.933   7.710   9.199  1.00 14.93          C
ATOM    532  O   THR A   36      17.341   7.023   8.379  1.00 15.02          O
ATOM    533  N   GLY A   37      18.699   8.735   8.885  1.00 15.27          N
ATOM    535  CA  GLY A   37      18.838   9.282   7.530  1.00 15.54          C
ATOM    538  C   GLY A   37      18.041  10.594   7.487  1.00 16.06          C
ATOM    539  O   GLY A   37      17.413  10.973   8.482  1.00 14.08          O
ATOM    540  N   VAL A   38      18.065  11.292   6.337  1.00 16.27          N
ATOM    542  CA  VAL A   38      17.315  12.547   6.177  1.00 15.20          C
ATOM    544  CB  VAL A   38      18.214  13.784   6.447  1.00 15.83          C
ATOM    546  CG1 VAL A   38      17.501  15.075   6.182  1.00 16.87          C
```

```
ATOM    550  CG2 VAL A  38       18.774  13.766   7.839  1.00 17.47           C
ATOM    554  C   VAL A  38       16.863  12.628   4.695  1.00 16.04           C
ATOM    555  O   VAL A  38       17.622  12.277   3.793  1.00 14.66           O
ATOM    556  N   TYR A  39       15.623  13.008   4.532  1.00 18.21           N
ATOM    558  CA  TYR A  39       15.046  13.247   3.214  1.00 19.90           C
ATOM    560  CB  TYR A  39       13.564  13.150   3.366  1.00 18.60           C
ATOM    563  CG  TYR A  39       12.795  13.480   2.082  1.00 23.59           C
ATOM    564  CD1 TYR A  39       13.278  13.110   0.833  1.00 27.19           C
ATOM    566  CE1 TYR A  39       12.555  13.413  -0.309  1.00 30.38           C
ATOM    568  CZ  TYR A  39       11.391  14.129  -0.226  1.00 31.18           C
ATOM    569  OH  TYR A  39       10.734  14.412  -1.434  1.00 30.34           O
ATOM    571  CE2 TYR A  39       10.912  14.528   1.005  1.00 29.74           C
ATOM    573  CD2 TYR A  39       11.623  14.208   2.144  1.00 23.70           C
ATOM    575  C   TYR A  39       15.495  14.623   2.786  1.00 19.47           C
ATOM    576  O   TYR A  39       14.795  15.631   2.992  1.00 22.39           O
ATOM    577  N   THR A  40       16.675  14.659   2.240  1.00 22.23           N
ATOM    579  CA  THR A  40       17.366  15.869   1.904  1.00 23.84           C
ATOM    581  CB  THR A  40       18.797  15.520   1.499  1.00 25.17           C
ATOM    583  OG1 THR A  40       18.841  14.473   0.518  1.00 27.20           O
ATOM    585  CG2 THR A  40       19.633  14.890   2.687  1.00 23.66           C
ATOM    589  C   THR A  40       16.650  16.659   0.804  1.00 25.31           C
ATOM    590  O   THR A  40       17.008  17.803   0.566  1.00 25.93           O
ATOM    591  N   SER A  41       15.671  16.051   0.147  1.00 25.63           N
ATOM    593  CA  SER A  41       14.953  16.703  -0.942  1.00 26.05           C
ATOM    595  CB  SER A  41       14.662  15.676  -2.047  1.00 25.60           C
ATOM    598  OG  SER A  41       15.836  15.411  -2.759  1.00 26.14           O
ATOM    600  C   SER A  41       13.669  17.317  -0.445  1.00 26.09           C
ATOM    601  O   SER A  41       12.896  17.889  -1.232  1.00 26.66           O
ATOM    602  N   HIS A  42       13.366  17.179   0.857  1.00 22.90           N
ATOM    604  CA  HIS A  42       12.245  17.917   1.419  1.00 21.28           C
ATOM    606  CB  HIS A  42       12.224  17.792   2.927  1.00 21.28           C
ATOM    609  CG  HIS A  42       10.988  18.267   3.562  1.00 18.79           C
ATOM    610  ND1 HIS A  42       10.616  19.591   3.556  1.00 17.72           N
ATOM    612  CE1 HIS A  42        9.482  19.706   4.197  1.00 14.42           C
ATOM    614  NE2 HIS A  42        9.124  18.516   4.654  1.00 18.07           N
ATOM    616  CD2 HIS A  42       10.028  17.601   4.230  1.00 15.85           C
ATOM    618  C   HIS A  42       12.427  19.379   1.036  1.00 20.43           C
ATOM    619  O   HIS A  42       13.543  19.890   1.077  1.00 19.85           O
ATOM    620  N   LEU A  43       11.326  20.044   0.686  1.00 21.20           N
ATOM    622  CA  LEU A  43       11.380  21.409   0.210  1.00 21.25           C
ATOM    624  CB  LEU A  43       10.030  21.945  -0.087  1.00 22.55           C
ATOM    627  CG  LEU A  43        9.448  21.512  -1.433  1.00 25.81           C
ATOM    629  CD1 LEU A  43        8.021  21.976  -1.464  1.00 27.58           C
ATOM    633  CD2 LEU A  43       10.234  22.108  -2.559  1.00 27.86           C
ATOM    637  C   LEU A  43       12.023  22.311   1.227  1.00 21.05           C
ATOM    638  O   LEU A  43       12.699  23.255   0.879  1.00 18.87           O
ATOM    639  N   ASP A  44       11.847  22.042   2.500  1.00 21.71           N
ATOM    641  CA  ASP A  44       12.514  22.885   3.487  1.00 20.87           C
ATOM    643  CB  ASP A  44       11.642  22.917   4.719  1.00 21.17           C
ATOM    646  CG  ASP A  44       10.262  23.417   4.441  1.00 23.00           C
ATOM    647  OD1 ASP A  44       10.060  24.154   3.406  1.00 21.93           O
ATOM    648  OD2 ASP A  44        9.325  23.156   5.206  1.00 16.28           O
ATOM    649  C   ASP A  44       13.962  22.528   3.812  1.00 21.02           C
ATOM    650  O   ASP A  44       14.593  23.214   4.604  1.00 18.05           O
ATOM    651  N   LEU A  45       14.488  21.431   3.252  1.00 18.30           N
ATOM    653  CA  LEU A  45       15.868  21.070   3.445  1.00 19.90           C
ATOM    655  CB  LEU A  45       15.922  19.624   4.024  1.00 18.80           C
ATOM    658  CG  LEU A  45       15.174  19.394   5.300  1.00 18.65           C
ATOM    660  CD1 LEU A  45       15.424  17.925   5.756  1.00 18.05           C
ATOM    664  CD2 LEU A  45       15.714  20.351   6.357  1.00 20.33           C
ATOM    668  C   LEU A  45       16.750  21.110   2.197  1.00 20.37           C
ATOM    669  O   LEU A  45       17.960  20.890   2.251  1.00 22.13           O
ATOM    670  N   ALA A  46       16.104  21.400   1.079  1.00 22.51           N
```

```
ATOM    672   CA   ALA  A   46      16.728  21.327   -0.210  1.00  21.13           C
ATOM    674   CB   ALA  A   46      15.738  21.695   -1.365  1.00  21.15           C
ATOM    678   C    ALA  A   46      17.880  22.199   -0.244  1.00  20.28           C
ATOM    679   O    ALA  A   46      17.806  23.344    0.177  1.00  22.66           O
ATOM    680   N    GLY  A   47      18.959  21.639   -0.759  1.00  20.67           N
ATOM    682   CA   GLY  A   47      20.217  22.320   -0.968  1.00  21.84           C
ATOM    685   C    GLY  A   47      21.042  22.419    0.291  1.00  21.53           C
ATOM    686   O    GLY  A   47      22.226  22.881    0.280  1.00  24.34           O
ATOM    687   N    SER  A   48      20.522  21.910    1.392  1.00  22.52           N
ATOM    689   CA   SER  A   48      21.310  21.980    2.610  1.00  21.98           C
ATOM    691   CB  ASER  A   48      20.384  22.083    3.833  0.50  22.54           C
ATOM    692   CB  BSER  A   48      20.408  22.073    3.814  0.50  22.26           C
ATOM    697   OG  ASER  A   48      19.449  21.001    3.944  0.50  23.88           O
ATOM    698   OG  BSER  A   48      19.660  23.258    3.738  0.50  21.10           O
ATOM    701   C    SER  A   48      22.295  20.852    2.826  1.00  21.47           C
ATOM    702   O    SER  A   48      23.317  21.066    3.466  1.00  21.64           O
ATOM    703   N    ALA  A   49      22.035  19.670    2.285  1.00  20.96           N
ATOM    705   CA   ALA  A   49      22.951  18.532    2.483  1.00  22.38           C
ATOM    707   CB   ALA  A   49      22.192  17.247    2.068  1.00  22.64           C
ATOM    711   C    ALA  A   49      24.233  18.644    1.718  1.00  23.21           C
ATOM    712   O    ALA  A   49      24.228  18.551    0.500  1.00  26.19           O
ATOM    713   N    GLU  A   50      25.328  18.866    2.426  1.00  20.40           N
ATOM    715   CA   GLU  A   50      26.598  18.826    1.753  1.00  21.76           C
ATOM    717   CB   GLU  A   50      27.625  19.892    2.250  1.00  22.32           C
ATOM    720   CG   GLU  A   50      27.374  21.244    1.591  1.00  26.62           C
ATOM    723   CD   GLU  A   50      28.046  22.421    2.279  1.00  30.59           C
ATOM    724   OE1  GLU  A   50      28.886  22.227    3.181  1.00  24.43           O
ATOM    725   OE2  GLU  A   50      27.683  23.561    1.918  1.00  35.88           O
ATOM    726   C    GLU  A   50      27.208  17.435    1.866  1.00  20.83           C
ATOM    727   O    GLU  A   50      28.257  17.188    1.220  1.00  21.81           O
ATOM    728   N    GLN  A   51      26.761  16.586    2.783  1.00  19.08           N
ATOM    730   CA   GLN  A   51      27.273  15.186    2.847  1.00  18.38           C
ATOM    732   CB   GLN  A   51      28.416  14.936    3.863  1.00  18.98           C
ATOM    735   CG   GLN  A   51      29.720  15.707    3.698  1.00  16.56           C
ATOM    738   CD   GLN  A   51      30.864  15.082    4.418  1.00  18.35           C
ATOM    739   OE1  GLN  A   51      30.728  14.001    4.993  1.00  16.59           O
ATOM    740   NE2  GLN  A   51      32.021  15.746    4.421  1.00  18.88           N
ATOM    743   C    GLN  A   51      26.048  14.303    3.122  1.00  18.15           C
ATOM    744   O    GLN  A   51      25.088  14.739    3.753  1.00  17.95           O
ATOM    745   N    CYS  A   52      26.032  13.097    2.549  1.00  19.15           N
ATOM    747   CA   CYS  A   52      24.933  12.197    2.657  1.00  19.58           C
ATOM    749   CB   CYS  A   52      23.994  12.383    1.463  1.00  20.58           C
ATOM    752   SG   CYS  A   52      22.757  11.113    1.313  1.00  23.23           S
ATOM    753   C    CYS  A   52      25.609  10.827    2.666  1.00  19.62           C
ATOM    754   O    CYS  A   52      26.112  10.376    1.630  1.00  17.75           O
ATOM    755   N    LYS  A   53      25.706  10.188    3.841  1.00  18.28           N
ATOM    757   CA   LYS  A   53      26.435   8.938    3.934  1.00  17.30           C
ATOM    759   CB   LYS  A   53      27.835   9.165    4.542  1.00  17.03           C
ATOM    762   CG   LYS  A   53      28.733  10.042    3.720  1.00  16.05           C
ATOM    765   CD   LYS  A   53      30.097  10.281    4.325  1.00  17.76           C
ATOM    768   CE   LYS  A   53      31.031  11.033    3.333  1.00  17.09           C
ATOM    771   NZ   LYS  A   53      32.138  11.733    3.893  1.00  19.33           N
ATOM    775   C    LYS  A   53      25.698   7.913    4.801  1.00  18.20           C
ATOM    776   O    LYS  A   53      24.966   8.299    5.712  1.00  15.09           O
ATOM    777   N    ASP  A   54      25.905   6.619    4.518  1.00  15.90           N
ATOM    779   CA   ASP  A   54      25.186   5.563    5.218  1.00  17.38           C
ATOM    781   CB   ASP  A   54      24.244   4.911    4.223  1.00  17.91           C
ATOM    784   CG   ASP  A   54      23.222   3.960    4.825  1.00  20.51           C
ATOM    785   OD1  ASP  A   54      23.261   3.554    6.029  1.00  15.70           O
ATOM    786   OD2  ASP  A   54      22.292   3.563    4.088  1.00  19.49           O
ATOM    787   C    ASP  A   54      26.131   4.552    5.807  1.00  17.88           C
ATOM    788   O    ASP  A   54      26.969   3.968    5.093  1.00  17.08           O
ATOM    789   N    PHE  A   55      25.998   4.356    7.135  1.00  15.74           N
```

```
ATOM   791  CA  PHE A  55    26.865   3.464   7.867  1.00 15.03          C
ATOM   793  CB  PHE A  55    27.359   4.168   9.131  1.00 13.99          C
ATOM   796  CG  PHE A  55    28.268   5.336   8.844  1.00 15.45          C
ATOM   797  CD1 PHE A  55    27.753   6.544   8.432  1.00 15.76          C
ATOM   799  CE1 PHE A  55    28.616   7.657   8.155  1.00 14.10          C
ATOM   801  CZ  PHE A  55    29.907   7.536   8.256  1.00 13.98          C
ATOM   803  CE2 PHE A  55    30.431   6.303   8.656  1.00 15.61          C
ATOM   805  CD2 PHE A  55    29.594   5.232   8.950  1.00 14.62          C
ATOM   807  C   PHE A  55    26.160   2.191   8.265  1.00 15.07          C
ATOM   808  O   PHE A  55    26.732   1.387   9.025  1.00 15.44          O
ATOM   809  N   THR A  56    24.962   1.994   7.769  1.00 15.31          N
ATOM   811  CA  THR A  56    24.149   0.858   8.159  1.00 16.77          C
ATOM   813  CB  THR A  56    22.724   1.253   8.463  1.00 17.17          C
ATOM   815  OG1 THR A  56    22.006   1.623   7.272  1.00 15.48          O
ATOM   817  CG2 THR A  56    22.628   2.535   9.443  1.00 13.67          C
ATOM   821  C   THR A  56    24.134  -0.328   7.166  1.00 20.28          C
ATOM   822  O   THR A  56    23.451  -1.319   7.407  1.00 21.69          O
ATOM   823  N   GLN A  57    24.852  -0.239   6.069  1.00 22.81          N
ATOM   825  CA  GLN A  57    24.736  -1.314   5.061  1.00 25.52          C
ATOM   827  CB  GLN A  57    24.681  -0.724   3.646  1.00 25.67          C
ATOM   830  CG  GLN A  57    23.521   0.217   3.502  1.00 27.65          C
ATOM   833  CD  GLN A  57    23.366   0.800   2.117  1.00 36.31          C
ATOM   834  OE1 GLN A  57    23.871   0.240   1.156  1.00 35.97          O
ATOM   835  NE2 GLN A  57    22.686   1.938   2.016  1.00 30.80          N
ATOM   838  C   GLN A  57    25.848  -2.331   5.196  1.00 28.75          C
ATOM   839  O   GLN A  57    26.735  -2.182   6.034  1.00 28.60          O
ATOM   840  N   SER A  58    25.792  -3.388   4.363  1.00 32.27          N
ATOM   842  CA  SER A  58    26.798  -4.440   4.371  1.00 34.96          C
ATOM   844  CB  SER A  58    26.488  -5.494   3.291  1.00 35.23          C
ATOM   847  OG  SER A  58    25.088  -5.548   3.041  1.00 37.60          O
ATOM   849  C   SER A  58    28.149  -3.762   4.140  1.00 35.56          C
ATOM   850  O   SER A  58    29.096  -3.989   4.843  1.00 36.58          O
ATOM   851  N   ASN A  59    28.224  -2.889   3.147  1.00 37.91          N
ATOM   853  CA  ASN A  59    29.409  -2.054   3.003  1.00 38.41          C
ATOM   855  CB  ASN A  59    29.288  -1.232   1.739  1.00 39.92          C
ATOM   858  CG  ASN A  59    30.172  -1.727   0.636  1.00 44.62          C
ATOM   859  OD1 ASN A  59    31.413  -1.752   0.759  1.00 52.42          O
ATOM   860  ND2 ASN A  59    29.547  -2.121  -0.468  1.00 50.97          N
ATOM   863  C   ASN A  59    29.421  -1.061   4.156  1.00 37.64          C
ATOM   864  O   ASN A  59    28.436  -0.360   4.338  1.00 37.26          O
ATOM   865  N   PRO A  60    30.474  -1.028   4.961  1.00 37.50          N
ATOM   866  CA  PRO A  60    30.591  -0.066   6.064  1.00 36.81          C
ATOM   868  CB  PRO A  60    32.016  -0.315   6.585  1.00 37.80          C
ATOM   871  CG  PRO A  60    32.661  -1.116   5.519  1.00 38.85          C
ATOM   874  CD  PRO A  60    31.589  -1.986   4.997  1.00 38.04          C
ATOM   877  C   PRO A  60    30.421   1.431   5.770  1.00 35.74          C
ATOM   878  O   PRO A  60    30.266   2.188   6.749  1.00 34.22          O
ATOM   879  N   LEU A  61    30.478   1.876   4.517  1.00 33.77          N
ATOM   881  CA  LEU A  61    30.183   3.278   4.258  1.00 33.86          C
ATOM   883  CB  LEU A  61    31.403   4.170   4.541  1.00 34.63          C
ATOM   886  CG  LEU A  61    31.122   5.691   4.652  1.00 38.34          C
ATOM   888  CD1 LEU A  61    32.418   6.454   4.765  1.00 41.14          C
ATOM   892  CD2 LEU A  61    30.383   6.236   3.501  1.00 40.32          C
ATOM   896  C   LEU A  61    29.681   3.440   2.838  1.00 31.90          C
ATOM   897  O   LEU A  61    30.371   3.109   1.887  1.00 32.30          O
ATOM   898  N   VAL A  62    28.452   3.862   2.682  1.00 29.42          N
ATOM   900  CA  VAL A  62    27.944   4.129   1.363  1.00 29.40          C
ATOM   902  CB  VAL A  62    26.721   3.365   1.091  1.00 28.97          C
ATOM   904  CG1 VAL A  62    26.082   3.877  -0.187  1.00 30.81          C
ATOM   908  CG2 VAL A  62    27.060   1.874   1.015  1.00 31.48          C
ATOM   912  C   VAL A  62    27.768   5.625   1.255  1.00 27.76          C
ATOM   913  O   VAL A  62    27.015   6.233   1.970  1.00 26.96          O
ATOM   914  N   ASP A  63    28.646   6.224   0.470  1.00 28.18          N
```

| ATOM | 916 | CA | ASP | A | 63 | 28.600 | 7.643 | 0.235 | 1.00 | 26.20 | C |
|------|-----|-----|-----|---|----|--------|-------|-------|------|-------|---|
| ATOM | 918 | CB | ASP | A | 63 | 29.993 | 8.062 | -0.215 | 1.00 | 26.90 | C |
| ATOM | 921 | CG | ASP | A | 63 | 30.222 | 9.517 | -0.099 | 1.00 | 25.49 | C |
| ATOM | 922 | OD1 | ASP | A | 63 | 29.290 | 10.281 | -0.017 | 1.00 | 26.02 | O |
| ATOM | 923 | OD2 | ASP | A | 63 | 31.318 | 10.031 | -0.150 | 1.00 | 28.08 | O |
| ATOM | 924 | C | ASP | A | 63 | 27.571 | 7.929 | -0.826 | 1.00 | 27.79 | C |
| ATOM | 925 | O | ASP | A | 63 | 27.455 | 7.199 | -1.812 | 1.00 | 27.28 | O |
| ATOM | 926 | N | GLY | A | 64 | 26.753 | 8.936 | -0.581 | 1.00 | 26.48 | N |
| ATOM | 928 | CA | GLY | A | 64 | 25.703 | 9.316 | -1.502 | 1.00 | 25.76 | C |
| ATOM | 931 | C | GLY | A | 64 | 24.357 | 8.742 | -1.234 | 1.00 | 25.62 | C |
| ATOM | 932 | O | GLY | A | 64 | 23.474 | 8.881 | -2.053 | 1.00 | 28.14 | O |
| ATOM | 933 | N | SER | A | 65 | 24.184 | 8.096 | -0.080 | 1.00 | 22.83 | N |
| ATOM | 935 | CA | SER | A | 65 | 22.953 | 7.499 | 0.304 | 1.00 | 22.85 | C |
| ATOM | 937 | CB | SER | A | 65 | 23.003 | 6.005 | 0.117 | 1.00 | 23.23 | C |
| ATOM | 940 | OG | SER | A | 65 | 21.699 | 5.584 | 0.027 | 1.00 | 29.80 | O |
| ATOM | 942 | C | SER | A | 65 | 22.705 | 7.773 | 1.749 | 1.00 | 21.08 | C |
| ATOM | 943 | O | SER | A | 65 | 23.671 | 7.638 | 2.504 | 1.00 | 19.41 | O |
| ATOM | 944 | N | CYS | A | 66 | 21.521 | 8.181 | 2.140 | 1.00 | 20.00 | N |
| ATOM | 946 | CA | CYS | A | 66 | 21.278 | 8.539 | 3.546 | 1.00 | 20.11 | C |
| ATOM | 948 | CB | CYS | A | 66 | 22.034 | 9.822 | 3.885 | 1.00 | 19.63 | C |
| ATOM | 951 | SG | CYS | A | 66 | 21.484 | 11.254 | 2.900 | 1.00 | 19.95 | S |
| ATOM | 952 | C | CYS | A | 66 | 19.803 | 8.601 | 3.712 | 1.00 | 18.81 | C |
| ATOM | 953 | O | CYS | A | 66 | 19.168 | 9.468 | 4.308 | 1.00 | 17.95 | O |
| ATOM | 954 | N | THR | A | 67 | 19.180 | 7.568 | 3.214 | 1.00 | 19.39 | N |
| ATOM | 956 | CA | THR | A | 67 | 17.768 | 7.596 | 3.075 | 1.00 | 19.72 | C |
| ATOM | 958 | CB | THR | A | 67 | 17.481 | 6.628 | 1.924 | 1.00 | 20.77 | C |
| ATOM | 960 | OG1 | THR | A | 67 | 18.082 | 7.189 | 0.735 | 1.00 | 26.00 | O |
| ATOM | 962 | CG2 | THR | A | 67 | 16.113 | 6.443 | 1.665 | 1.00 | 23.79 | C |
| ATOM | 966 | C | THR | A | 67 | 16.941 | 7.325 | 4.315 | 1.00 | 18.14 | C |
| ATOM | 967 | O | THR | A | 67 | 17.066 | 6.297 | 4.990 | 1.00 | 16.62 | O |
| ATOM | 968 | N | ASP | A | 68 | 16.070 | 8.278 | 4.623 | 1.00 | 17.54 | N |
| ATOM | 970 | CA | ASP | A | 68 | 15.191 | 8.149 | 5.786 | 1.00 | 17.68 | C |
| ATOM | 972 | CB | ASP | A | 68 | 14.877 | 9.530 | 6.360 | 1.00 | 16.82 | C |
| ATOM | 975 | CG | ASP | A | 68 | 14.131 | 9.480 | 7.697 | 1.00 | 17.49 | C |
| ATOM | 976 | OD1 | ASP | A | 68 | 13.988 | 8.380 | 8.314 | 1.00 | 14.56 | O |
| ATOM | 977 | OD2 | ASP | A | 68 | 13.610 | 10.516 | 8.221 | 1.00 | 16.31 | O |
| ATOM | 978 | C | ASP | A | 68 | 13.909 | 7.425 | 5.423 | 1.00 | 19.14 | C |
| ATOM | 979 | O | ASP | A | 68 | 13.100 | 7.936 | 4.626 | 1.00 | 19.83 | O |
| ATOM | 980 | N | ARG | A | 69 | 13.688 | 6.262 | 6.023 | 1.00 | 19.38 | N |
| ATOM | 982 | CA | ARG | A | 69 | 12.427 | 5.549 | 5.858 | 1.00 | 20.20 | C |
| ATOM | 984 | CB | ARG | A | 69 | 12.665 | 4.106 | 5.357 | 1.00 | 20.57 | C |
| ATOM | 987 | CG | ARG | A | 69 | 13.461 | 4.061 | 4.081 | 1.00 | 23.84 | C |
| ATOM | 990 | CD | ARG | A | 69 | 13.499 | 2.688 | 3.401 | 1.00 | 28.11 | C |
| ATOM | 993 | NE | ARG | A | 69 | 14.384 | 2.688 | 2.239 | 1.00 | 31.97 | N |
| ATOM | 995 | CZ | ARG | A | 69 | 15.683 | 2.433 | 2.284 | 1.00 | 34.34 | C |
| ATOM | 996 | NH1 | ARG | A | 69 | 16.288 | 2.155 | 3.437 | 1.00 | 33.57 | N |
| ATOM | 999 | NH2 | ARG | A | 69 | 16.416 | 2.464 | 1.173 | 1.00 | 37.78 | N |
| ATOM | 1002 | C | ARG | A | 69 | 11.615 | 5.543 | 7.120 | 1.00 | 20.20 | C |
| ATOM | 1003 | O | ARG | A | 69 | 10.605 | 4.861 | 7.222 | 1.00 | 19.58 | O |
| ATOM | 1004 | N | GLN | A | 70 | 12.022 | 6.341 | 8.120 | 1.00 | 18.56 | N |
| ATOM | 1006 | CA | GLN | A | 70 | 11.359 | 6.330 | 9.404 | 1.00 | 18.67 | C |
| ATOM | 1008 | CB | GLN | A | 70 | 12.459 | 6.087 | 10.480 | 1.00 | 17.38 | C |
| ATOM | 1011 | CG | GLN | A | 70 | 11.887 | 5.512 | 11.734 | 1.00 | 24.45 | C |
| ATOM | 1014 | CD | GLN | A | 70 | 11.094 | 6.496 | 12.618 | 1.00 | 29.69 | C |
| ATOM | 1015 | OE1 | GLN | A | 70 | 11.259 | 7.719 | 12.568 | 1.00 | 28.81 | O |
| ATOM | 1016 | NE2 | GLN | A | 70 | 10.180 | 5.934 | 13.390 | 1.00 | 36.98 | N |
| ATOM | 1019 | C | GLN | A | 70 | 10.678 | 7.677 | 9.729 | 1.00 | 16.99 | C |
| ATOM | 1020 | O | GLN | A | 70 | 9.502 | 7.745 | 10.177 | 1.00 | 18.53 | O |
| ATOM | 1021 | N | GLY | A | 71 | 11.448 | 8.740 | 9.546 | 1.00 | 17.00 | N |
| ATOM | 1023 | CA | GLY | A | 71 | 10.936 | 10.086 | 9.792 | 1.00 | 16.91 | C |
| ATOM | 1026 | C | GLY | A | 71 | 11.766 | 10.862 | 10.826 | 1.00 | 16.73 | C |
| ATOM | 1027 | O | GLY | A | 71 | 12.023 | 12.040 | 10.683 | 1.00 | 16.08 | O |
| ATOM | 1028 | N | HIS | A | 72 | 12.190 | 10.148 | 11.848 | 1.00 | 15.53 | N |

```
ATOM   1030  CA   HIS A   72      12.902  10.764  12.965  1.00 14.82          C
ATOM   1032  CB   HIS A   72      13.305   9.625  13.926  1.00 14.91          C
ATOM   1035  CG   HIS A   72      13.996  10.088  15.170  1.00 11.42          C
ATOM   1036  ND1  HIS A   72      15.356  10.264  15.228  1.00 11.65          N
ATOM   1038  CE1  HIS A   72      15.690  10.620  16.456  1.00 15.57          C
ATOM   1040  NE2  HIS A   72      14.603  10.660  17.194  1.00 12.24          N
ATOM   1042  CD2  HIS A   72      13.527  10.309  16.414  1.00 15.18          C
ATOM   1044  C    HIS A   72      14.077  11.632  12.515  1.00 14.31          C
ATOM   1045  O    HIS A   72      14.157  12.811  12.906  1.00 14.46          O
ATOM   1046  N    GLY A   73      14.993  11.101  11.686  1.00 13.23          N
ATOM   1048  CA   GLY A   73      16.140  11.851  11.227  1.00 13.67          C
ATOM   1051  C    GLY A   73      15.743  13.097  10.452  1.00 14.47          C
ATOM   1052  O    GLY A   73      16.388  14.147  10.556  1.00 14.58          O
ATOM   1053  N    THR A   74      14.691  12.976   9.638  1.00 14.43          N
ATOM   1055  CA   THR A   74      14.223  14.163   8.902  1.00 14.75          C
ATOM   1057  CB   THR A   74      13.166  13.722   7.889  1.00 15.14          C
ATOM   1059  OG1  THR A   74      13.832  12.851   6.979  1.00 14.14          O
ATOM   1061  CG2  THR A   74      12.703  14.949   7.052  1.00 17.07          C
ATOM   1065  C    THR A   74      13.672  15.256   9.779  1.00 13.89          C
ATOM   1066  O    THR A   74      13.964  16.449   9.549  1.00 14.36          O
ATOM   1067  N    HIS A   75      12.985  14.834  10.823  1.00 13.97          N
ATOM   1069  CA   HIS A   75      12.345  15.653  11.803  1.00 13.53          C
ATOM   1071  CB   HIS A   75      11.464  14.793  12.693  1.00 14.00          C
ATOM   1074  CG   HIS A   75      10.525  15.543  13.566  1.00 13.88          C
ATOM   1075  ND1  HIS A   75      10.923  16.209  14.706  1.00 13.19          N
ATOM   1077  CE1  HIS A   75       9.888  16.830  15.235  1.00 15.22          C
ATOM   1079  NE2  HIS A   75       8.826  16.616  14.465  1.00 15.33          N
ATOM   1081  CD2  HIS A   75       9.203  15.822  13.415  1.00 14.61          C
ATOM   1083  C    HIS A   75      13.464  16.423  12.565  1.00 14.20          C
ATOM   1084  O    HIS A   75      13.447  17.650  12.685  1.00 11.71          O
ATOM   1085  N    VAL A   76      14.436  15.685  13.031  1.00 13.61          N
ATOM   1087  CA   VAL A   76      15.543  16.273  13.761  1.00 13.90          C
ATOM   1089  CB   VAL A   76      16.471  15.117  14.276  1.00 12.59          C
ATOM   1091  CG1  VAL A   76      17.771  15.657  14.716  1.00 13.85          C
ATOM   1095  CG2  VAL A   76      15.788  14.354  15.381  1.00 13.36          C
ATOM   1099  C    VAL A   76      16.280  17.319  12.925  1.00 13.76          C
ATOM   1100  O    VAL A   76      16.549  18.419  13.362  1.00 13.81          O
ATOM   1101  N    ALA A   77      16.598  16.976  11.693  1.00 13.31          N
ATOM   1103  CA   ALA A   77      17.316  17.850  10.844  1.00 13.43          C
ATOM   1105  CB   ALA A   77      17.586  17.164   9.553  1.00 12.82          C
ATOM   1109  C    ALA A   77      16.538  19.154  10.631  1.00 13.42          C
ATOM   1110  O    ALA A   77      17.137  20.256  10.595  1.00 16.51          O
ATOM   1111  N    GLY A   78      15.223  19.047  10.501  1.00 13.22          N
ATOM   1113  CA   GLY A   78      14.413  20.237  10.270  1.00 14.59          C
ATOM   1116  C    GLY A   78      14.431  21.221  11.448  1.00 14.40          C
ATOM   1117  O    GLY A   78      14.427  22.440  11.294  1.00 14.85          O
ATOM   1118  N    THR A   79      14.537  20.673  12.643  1.00 12.64          N
ATOM   1120  CA   THR A   79      14.546  21.535  13.817  1.00 11.83          C
ATOM   1122  CB   THR A   79      14.350  20.656  15.063  1.00 11.71          C
ATOM   1124  OG1  THR A   79      12.990  20.162  15.166  1.00 12.60          O
ATOM   1126  CG2  THR A   79      14.569  21.491  16.347  1.00 11.10          C
ATOM   1130  C    THR A   79      15.842  22.248  13.795  1.00 12.20          C
ATOM   1131  O    THR A   79      15.917  23.440  14.122  1.00 12.14          O
ATOM   1132  N    VAL A   80      16.917  21.568  13.358  1.00 11.44          N
ATOM   1134  CA   VAL A   80      18.195  22.225  13.293  1.00 11.80          C
ATOM   1136  CB   VAL A   80      19.299  21.273  12.865  1.00 11.91          C
ATOM   1138  CG1  VAL A   80      20.637  21.963  12.687  1.00 13.34          C
ATOM   1142  CG2  VAL A   80      19.520  20.158  13.884  1.00 12.77          C
ATOM   1146  C    VAL A   80      18.216  23.369  12.266  1.00 13.39          C
ATOM   1147  O    VAL A   80      18.646  24.514  12.553  1.00 12.65          O
ATOM   1148  N    LEU A   81      17.751  23.054  11.069  1.00 13.69          N
ATOM   1150  CA   LEU A   81      18.057  23.946   9.965  1.00 14.13          C
ATOM   1152  CB   LEU A   81      19.454  23.675   9.439  1.00 14.13          C
```

```
ATOM   1155  CG  LEU A  81    19.893 22.189   9.225 1.00 11.54           C
ATOM   1157  CD1 LEU A  81    19.058 21.552   8.105 1.00 15.65           C
ATOM   1161  CD2 LEU A  81    21.308 22.059   8.854 1.00 14.83           C
ATOM   1165  C   LEU A  81    17.043 24.065   8.827 1.00 15.14           C
ATOM   1166  O   LEU A  81    17.442 24.518   7.766 1.00 17.76           O
ATOM   1167  N   ALA A  82    15.791 23.694   9.035 1.00 15.38           N
ATOM   1169  CA  ALA A  82    14.830 23.894   7.920 1.00 16.54           C
ATOM   1171  CB  ALA A  82    13.485 23.412   8.253 1.00 16.56           C
ATOM   1175  C   ALA A  82    14.807 25.381   7.616 1.00 17.73           C
ATOM   1176  O   ALA A  82    14.873 26.246   8.522 1.00 16.11           O
ATOM   1177  N   HIS A  83    14.637 25.678   6.321 1.00 18.61           N
ATOM   1179  CA  HIS A  83    14.802 27.048   5.845 1.00 17.82           C
ATOM   1181  CB  HIS A  83    16.057 27.116   4.996 1.00 18.66           C
ATOM   1184  CG  HIS A  83    16.040 26.187   3.831 1.00 19.55           C
ATOM   1185  ND1 HIS A  83    14.935 26.066   3.023 1.00 23.39           N
ATOM   1187  CE1 HIS A  83    15.196 25.205   2.056 1.00 24.12           C
ATOM   1189  NE2 HIS A  83    16.395 24.706   2.259 1.00 24.21           N
ATOM   1191  CD2 HIS A  83    16.960 25.326   3.349 1.00 22.73           C
ATOM   1193  C   HIS A  83    13.606 27.689   5.119 1.00 19.24           C
ATOM   1194  O   HIS A  83    13.802 28.694   4.468 1.00 20.21           O
ATOM   1195  N   GLY A  84    12.433 27.158   5.342 1.00 19.88           N
ATOM   1197  CA  GLY A  84    11.151 27.653   4.874 1.00 23.01           C
ATOM   1200  C   GLY A  84    10.891 27.373   3.388 1.00 23.83           C
ATOM   1201  O   GLY A  84     9.816 27.693   2.873 1.00 25.88           O
ATOM   1202  N   GLY A  85    11.891 26.852   2.716 1.00 25.14           N
ATOM   1204  CA  GLY A  85    11.754 26.452   1.333 1.00 28.31           C
ATOM   1207  C   GLY A  85    11.845 27.607   0.361 1.00 30.82           C
ATOM   1208  O   GLY A  85    11.704 28.777   0.750 1.00 32.32           O
ATOM   1209  N   SER A  86    12.066 27.253  -0.910 1.00 33.23           N
ATOM   1211  CA  SER A  86    12.332 28.220  -1.982 1.00 35.37           C
ATOM   1213  CB  SER A  86    12.287 27.551  -3.374 1.00 35.51           C
ATOM   1216  OG  SER A  86    11.022 26.920  -3.531 1.00 36.16           O
ATOM   1218  C   SER A  86    11.323 29.323  -1.984 1.00 35.85           C
ATOM   1219  O   SER A  86    11.673 30.481  -2.155 1.00 37.71           O
ATOM   1220  N   ASN A  87    10.066 28.993  -1.784 1.00 36.18           N
ATOM   1222  CA  ASN A  87     9.060 30.024  -1.844 1.00 37.05           C
ATOM   1224  CB  ASN A  87     7.842 29.472  -2.589 1.00 37.33           C
ATOM   1227  CG  ASN A  87     6.943 28.626  -1.702 1.00 40.53           C
ATOM   1228  OD1 ASN A  87     7.323 28.240  -0.581 1.00 40.83           O
ATOM   1229  ND2 ASN A  87     5.732 28.329  -2.205 1.00 39.22           N
ATOM   1232  C   ASN A  87     8.678 30.600  -0.469 1.00 35.97           C
ATOM   1233  O   ASN A  87     7.564 31.143  -0.295 1.00 36.81           O
ATOM   1234  N   GLY A  88     9.554 30.402   0.526 1.00 34.13           N
ATOM   1236  CA  GLY A  88     9.307 30.979   1.841 1.00 32.36           C
ATOM   1239  C   GLY A  88     8.149 30.556   2.701 1.00 30.27           C
ATOM   1240  O   GLY A  88     7.882 31.189   3.728 1.00 30.72           O
ATOM   1241  N   GLN A  89     7.375 29.545   2.305 1.00 28.00           N
ATOM   1243  CA  GLN A  89     6.212 29.190   3.117 1.00 27.11           C
ATOM   1245  CB  GLN A  89     4.898 29.091   2.269 1.00 28.51           C
ATOM   1248  CG  GLN A  89     3.596 28.969   3.114 0.10 26.48           C
ATOM   1251  CD  GLN A  89     2.269 28.881   2.318 0.10 25.84           C
ATOM   1252  OE1 GLN A  89     2.243 28.873   1.085 0.10 20.77           O
ATOM   1253  NE2 GLN A  89     1.164 28.811   3.052 0.10 23.86           N
ATOM   1256  C   GLN A  89     6.384 27.908   3.974 1.00 26.64           C
ATOM   1257  O   GLN A  89     5.463 27.490   4.638 1.00 25.73           O
ATOM   1258  N   GLY A  90     7.572 27.312   3.967 1.00 26.19           N
ATOM   1260  CA  GLY A  90     7.781 26.104   4.760 1.00 24.89           C
ATOM   1263  C   GLY A  90     8.133 26.372   6.223 1.00 25.02           C
ATOM   1264  O   GLY A  90     7.940 27.492   6.751 1.00 25.06           O
ATOM   1265  N   VAL A  91     8.598 25.330   6.888 1.00 22.83           N
ATOM   1267  CA  VAL A  91     8.942 25.462   8.304 1.00 20.77           C
ATOM   1269  CB  VAL A  91     8.681 24.116   9.045 1.00 19.91           C
ATOM   1271  CG1 VAL A  91     9.781 23.160   8.797 1.00 21.25           C
```

```
ATOM   1275  CG2 VAL A  91      8.463  24.309  10.528  1.00 21.36           C
ATOM   1279  C   VAL A  91     10.344  25.938   8.411  1.00 18.60           C
ATOM   1280  O   VAL A  91     11.184  25.738   7.532  1.00 19.96           O
ATOM   1281  N   TYR A  92     10.632  26.589   9.547  1.00 18.11           N
ATOM   1283  CA  TYR A  92     11.941  27.076   9.868  1.00 17.39           C
ATOM   1285  CB  TYR A  92     11.880  28.546  10.256  1.00 16.87           C
ATOM   1288  CG  TYR A  92     11.827  29.420   9.027  1.00 17.86           C
ATOM   1289  CD1 TYR A  92     12.989  29.758   8.379  1.00 16.53           C
ATOM   1291  CE1 TYR A  92     12.993  30.516   7.233  1.00 20.55           C
ATOM   1293  CZ  TYR A  92     11.793  30.963   6.717  1.00 23.54           C
ATOM   1294  OH  TYR A  92     11.862  31.737   5.549  1.00 25.77           O
ATOM   1296  CE2 TYR A  92     10.619  30.672   7.325  1.00 19.89           C
ATOM   1298  CD2 TYR A  92     10.626  29.840   8.507  1.00 20.04           C
ATOM   1300  C   TYR A  92     12.542  26.375  11.099  1.00 14.56           C
ATOM   1301  O   TYR A  92     11.856  26.154  12.042  1.00 14.48           O
ATOM   1302  N   GLY A  93     13.824  26.133  11.058  1.00 13.49           N
ATOM   1304  CA  GLY A  93     14.547  25.546  12.182  1.00 15.74           C
ATOM   1307  C   GLY A  93     15.350  26.635  12.819  1.00 14.86           C
ATOM   1308  O   GLY A  93     15.203  27.819  12.473  1.00 17.02           O
ATOM   1309  N   VAL A  94     16.231  26.278  13.759  1.00 14.32           N
ATOM   1311  CA  VAL A  94     16.981  27.306  14.421  1.00 14.34           C
ATOM   1313  CB  VAL A  94     17.654  26.753  15.712  1.00 13.59           C
ATOM   1315  CG1 VAL A  94     18.263  27.876  16.515  1.00 13.33           C
ATOM   1319  CG2 VAL A  94     16.633  26.043  16.538  1.00 13.90           C
ATOM   1323  C   VAL A  94     18.010  28.055  13.577  1.00 14.53           C
ATOM   1324  O   VAL A  94     18.196  29.258  13.779  1.00 16.71           O
ATOM   1325  N   ALA A  95     18.724  27.356  12.692  1.00 14.24           N
ATOM   1327  CA  ALA A  95     19.859  27.839  11.990  1.00 14.00           C
ATOM   1329  CB  ALA A  95     21.100  27.227  12.574  1.00 14.43           C
ATOM   1333  C   ALA A  95     19.757  27.491  10.498  1.00 15.52           C
ATOM   1334  O   ALA A  95     20.476  26.644   9.954  1.00 13.66           O
ATOM   1335  N   PRO A  96     18.847  28.184   9.840  1.00 15.59           N
ATOM   1336  CA  PRO A  96     18.487  27.876   8.443  1.00 16.93           C
ATOM   1338  CB  PRO A  96     17.330  28.851   8.170  1.00 17.23           C
ATOM   1341  CG  PRO A  96     17.628  29.986   9.086  1.00 16.22           C
ATOM   1344  CD  PRO A  96     18.078  29.307  10.383  1.00 13.71           C
ATOM   1347  C   PRO A  96     19.598  28.049   7.403  1.00 17.82           C
ATOM   1348  O   PRO A  96     19.478  27.477   6.306  1.00 16.77           O
ATOM   1349  N   GLN A  97     20.664  28.772   7.719  1.00 17.73           N
ATOM   1351  CA  GLN A  97     21.812  28.891   6.826  1.00 18.02           C
ATOM   1353  CB  GLN A  97     22.374  30.341   6.726  1.00 18.03           C
ATOM   1356  CG  GLN A  97     21.509  31.218   5.783  1.00 22.97           C
ATOM   1359  CD  GLN A  97     20.220  31.715   6.401  1.00 23.17           C
ATOM   1360  OE1 GLN A  97     20.303  32.467   7.345  1.00 27.41           O
ATOM   1361  NE2 GLN A  97     19.016  31.311   5.865  1.00 26.08           N
ATOM   1364  C   GLN A  97     22.901  27.903   7.080  1.00 17.35           C
ATOM   1365  O   GLN A  97     23.900  27.913   6.351  1.00 17.54           O
ATOM   1366  N   ALA A  98     22.763  27.057   8.125  1.00 15.56           N
ATOM   1368  CA  ALA A  98     23.794  26.040   8.361  1.00 16.07           C
ATOM   1370  CB  ALA A  98     23.615  25.387   9.738  1.00 15.36           C
ATOM   1374  C   ALA A  98     23.657  24.997   7.256  1.00 15.80           C
ATOM   1375  O   ALA A  98     22.610  24.906   6.610  1.00 18.34           O
ATOM   1376  N   LYS A  99     24.683  24.195   7.082  1.00 16.19           N
ATOM   1378  CA  LYS A  99     24.670  23.108   6.118  1.00 15.45           C
ATOM   1380  CB  LYS A  99     25.882  23.152   5.268  1.00 16.64           C
ATOM   1383  CG  LYS A  99     25.789  24.264   4.222  1.00 17.86           C
ATOM   1386  CD  LYS A  99     24.616  24.101   3.322  1.00 22.94           C
ATOM   1389  CE  LYS A  99     24.844  25.062   2.185  1.00 28.36           C
ATOM   1392  NZ  LYS A  99     23.614  25.181   1.383  1.00 28.94           N
ATOM   1396  C   LYS A  99     24.604  21.759   6.887  1.00 15.54           C
ATOM   1397  O   LYS A  99     25.012  21.684   8.019  1.00 15.10           O
ATOM   1398  N   LEU A 100     24.136  20.742   6.185  1.00 14.73           N
ATOM   1400  CA  LEU A 100     23.801  19.460   6.791  1.00 15.28           C
```

44

```
ATOM   1402   CB   LEU A 100     22.361  19.131   6.489  1.00 14.80         C
ATOM   1405   CG   LEU A 100     21.852  17.724   6.719  1.00 16.65         C
ATOM   1407   CD1  LEU A 100     21.751  17.484   8.242  1.00 17.43         C
ATOM   1411   CD2  LEU A 100     20.500  17.473   6.155  1.00 17.01         C
ATOM   1415   C    LEU A 100     24.743  18.373   6.336  1.00 15.64         C
ATOM   1416   O    LEU A 100     25.114  18.290   5.154  1.00 17.60         O
ATOM   1417   N    TRP A 101     25.206  17.566   7.298  1.00 15.09         N
ATOM   1419   CA   TRP A 101     25.895  16.350   6.942  1.00 15.10         C
ATOM   1421   CB   TRP A 101     27.265  16.234   7.534  1.00 14.66         C
ATOM   1424   CG   TRP A 101     28.408  17.171   7.076  1.00 13.68         C
ATOM   1425   CD1  TRP A 101     28.342  18.164   6.137  1.00 14.24         C
ATOM   1427   NE1  TRP A 101     29.575  18.741   5.956  1.00 14.73         N
ATOM   1429   CE2  TRP A 101     30.465  18.110   6.770  1.00 14.20         C
ATOM   1430   CD2  TRP A 101     29.751  17.123   7.498  1.00 15.01         C
ATOM   1431   CE3  TRP A 101     30.470  16.329   8.413  1.00 15.44         C
ATOM   1433   CZ3  TRP A 101     31.791  16.598   8.605  1.00 14.31         C
ATOM   1435   CH2  TRP A 101     32.451  17.587   7.845  1.00 15.17         C
ATOM   1437   CZ2  TRP A 101     31.780  18.363   6.977  1.00 14.09         C
ATOM   1439   C    TRP A 101     24.932  15.267   7.451  1.00 16.46         C
ATOM   1440   O    TRP A 101     24.830  15.022   8.675  1.00 14.81         O
ATOM   1441   N    ALA A 102     24.250  14.579   6.534  1.00 14.78         N
ATOM   1443   CA   ALA A 102     23.255  13.610   6.910  1.00 15.54         C
ATOM   1445   CB   ALA A 102     22.086  13.639   5.973  1.00 16.18         C
ATOM   1449   C    ALA A 102     23.897  12.221   6.941  1.00 15.83         C
ATOM   1450   O    ALA A 102     24.187  11.661   5.898  1.00 15.17         O
ATOM   1451   N    TYR A 103     24.148  11.692   8.140  1.00 14.35         N
ATOM   1453   CA   TYR A 103     24.797  10.400   8.290  1.00 14.35         C
ATOM   1455   CB   TYR A 103     25.985  10.493   9.225  1.00 13.92         C
ATOM   1458   CG   TYR A 103     27.247  11.147   8.697  1.00 13.29         C
ATOM   1459   CD1  TYR A 103     27.275  11.938   7.550  1.00 14.70         C
ATOM   1461   CE1  TYR A 103     28.455  12.512   7.113  1.00 14.99         C
ATOM   1463   CZ   TYR A 103     29.587  12.335   7.783  1.00 13.42         C
ATOM   1464   OH   TYR A 103     30.820  12.886   7.417  1.00 17.55         O
ATOM   1466   CE2  TYR A 103     29.608  11.561   8.961  1.00 11.55         C
ATOM   1468   CD2  TYR A 103     28.445  10.996   9.399  1.00 12.45         C
ATOM   1470   C    TYR A 103     23.813   9.419   8.860  1.00 13.70         C
ATOM   1471   O    TYR A 103     23.336   9.583   9.966  1.00 13.01         O
ATOM   1472   N    LYS A 104     23.490   8.383   8.101  1.00 13.67         N
ATOM   1474   CA   LYS A 104     22.524   7.385   8.564  1.00 12.46         C
ATOM   1476   CB   LYS A 104     21.773   6.738   7.407  1.00 14.90         C
ATOM   1479   CG   LYS A 104     20.789   5.718   7.815  1.00 15.07         C
ATOM   1482   CD   LYS A 104     19.991   5.144   6.616  1.00 14.19         C
ATOM   1485   CE   LYS A 104     18.751   4.402   7.036  1.00 17.35         C
ATOM   1488   NZ   LYS A 104     18.027   3.784   5.831  1.00 15.18         N
ATOM   1492   C    LYS A 104     23.249   6.327   9.362  1.00 14.29         C
ATOM   1493   O    LYS A 104     24.138   5.652   8.836  1.00 14.08         O
ATOM   1494   N    VAL A 105     22.893   6.215  10.645  1.00 12.75         N
ATOM   1496   CA   VAL A 105     23.513   5.287  11.592  1.00 13.70         C
ATOM   1498   CB   VAL A 105     24.301   6.043  12.684  1.00 13.12         C
ATOM   1500   CG1  VAL A 105     25.244   6.961  12.010  1.00 14.29         C
ATOM   1504   CG2  VAL A 105     23.388   6.804  13.578  1.00 12.47         C
ATOM   1508   C    VAL A 105     22.491   4.405  12.292  1.00 13.35         C
ATOM   1509   O    VAL A 105     22.851   3.501  13.036  1.00 14.40         O
ATOM   1510   N    LEU A 106     21.218   4.733  12.140  1.00 13.80         N
ATOM   1512   CA   LEU A 106     20.133   3.912  12.678  1.00 15.31         C
ATOM   1514   CB   LEU A 106     19.165   4.715  13.533  1.00 14.78         C
ATOM   1517   CG   LEU A 106     19.820   5.395  14.752  1.00 14.84         C
ATOM   1519   CD1  LEU A 106     18.745   6.216  15.434  1.00 13.44         C
ATOM   1523   CD2  LEU A 106     20.365   4.281  15.645  1.00 13.80         C
ATOM   1527   C    LEU A 106     19.328   3.395  11.488  1.00 16.71         C
ATOM   1528   O    LEU A 106     19.217   4.083  10.457  1.00 14.73         O
ATOM   1529   N    GLY A 107     18.812   2.184  11.617  1.00 18.94         N
ATOM   1531   CA   GLY A 107     17.950   1.629  10.581  1.00 20.89         C
```

```
ATOM   1534  C    GLY A 107      16.534   2.176  10.597  1.00 22.18          C
ATOM   1535  O    GLY A 107      16.136   3.087  11.335  1.00 21.65          O
ATOM   1536  N    ASP A 108      15.714   1.570   9.755  1.00 24.79          N
ATOM   1538  CA   ASP A 108      14.419   2.139   9.442  1.00 25.79          C
ATOM   1540  CB   ASP A 108      13.946   1.584   8.117  1.00 26.09          C
ATOM   1543  CG   ASP A 108      14.971   1.774   7.022  1.00 28.51          C
ATOM   1544  OD1  ASP A 108      15.795   2.721   7.082  1.00 27.80          O
ATOM   1545  OD2  ASP A 108      15.020   1.038   6.025  1.00 30.77          O
ATOM   1546  C    ASP A 108      13.331   1.997  10.489  1.00 26.41          C
ATOM   1547  O    ASP A 108      12.229   2.535  10.294  1.00 26.78          O
ATOM   1548  N    ASN A 109      13.629   1.262  11.566  1.00 26.19          N
ATOM   1550  CA   ASN A 109      12.751   1.172  12.719  1.00 25.53          C
ATOM   1552  CB   ASN A 109      12.399  -0.264  13.022  1.00 26.84          C
ATOM   1555  CG   ASN A 109      11.599  -0.920  11.863  1.00 30.82          C
ATOM   1556  OD1  ASN A 109      10.606  -0.338  11.353  1.00 35.24          O
ATOM   1557  ND2  ASN A 109      12.038  -2.093  11.429  1.00 36.51          N
ATOM   1560  C    ASN A 109      13.340   1.943  13.933  1.00 24.23          C
ATOM   1561  O    ASN A 109      12.941   1.773  15.071  1.00 23.68          O
ATOM   1562  N    GLY A 110      14.325   2.774  13.652  1.00 22.01          N
ATOM   1564  CA   GLY A 110      14.823   3.684  14.685  1.00 20.58          C
ATOM   1567  C    GLY A 110      15.783   3.001  15.656  1.00 18.79          C
ATOM   1568  O    GLY A 110      15.989   3.492  16.797  1.00 18.63          O
ATOM   1569  N    SER A 111      16.373   1.885  15.208  1.00 16.52          N
ATOM   1571  CA   SER A 111      17.377   1.225  16.057  1.00 17.48          C
ATOM   1573  CB   SER A 111      16.805   0.002  16.752  1.00 18.93          C
ATOM   1576  OG   SER A 111      16.663  -1.046  15.856  1.00 20.57          O
ATOM   1578  C    SER A 111      18.625   0.916  15.250  1.00 15.84          C
ATOM   1579  O    SER A 111      18.585   0.814  14.022  1.00 15.08          O
ATOM   1580  N    GLY A 112      19.767   0.761  15.913  1.00 15.46          N
ATOM   1582  CA   GLY A 112      20.991   0.537  15.198  1.00 14.74          C
ATOM   1585  C    GLY A 112      22.080  -0.033  16.063  1.00 13.99          C
ATOM   1586  O    GLY A 112      21.852  -0.365  17.219  1.00 12.44          O
ATOM   1587  N    TYR A 113      23.229  -0.171  15.431  1.00 13.65          N
ATOM   1589  CA   TYR A 113      24.372  -0.836  16.000  1.00 14.69          C
ATOM   1591  CB   TYR A 113      24.992  -1.754  14.982  1.00 14.83          C
ATOM   1594  CG   TYR A 113      24.139  -2.928  14.627  1.00 16.70          C
ATOM   1595  CD1  TYR A 113      24.217  -4.098  15.339  1.00 18.38          C
ATOM   1597  CE1  TYR A 113      23.392  -5.213  14.997  1.00 18.28          C
ATOM   1599  CZ   TYR A 113      22.516  -5.121  13.926  1.00 19.64          C
ATOM   1600  OH   TYR A 113      21.730  -6.214  13.573  1.00 20.11          O
ATOM   1602  CE2  TYR A 113      22.465  -3.958  13.189  1.00 19.66          C
ATOM   1604  CD2  TYR A 113      23.269  -2.871  13.557  1.00 15.59          C
ATOM   1606  C    TYR A 113      25.441   0.094  16.471  1.00 13.86          C
ATOM   1607  O    TYR A 113      25.825   1.019  15.758  1.00 14.60          O
ATOM   1608  N    SER A 114      25.880  -0.113  17.706  1.00 13.30          N
ATOM   1610  CA   SER A 114      26.958   0.655  18.290  1.00 13.35          C
ATOM   1612  CB   SER A 114      27.507  -0.213  19.432  1.00 15.78          C
ATOM   1615  OG   SER A 114      28.722   0.270  19.995  1.00 14.15          O
ATOM   1617  C    SER A 114      28.125   0.916  17.339  1.00 14.05          C
ATOM   1618  O    SER A 114      28.615   2.023  17.226  1.00 13.89          O
ATOM   1619  N    ASP A 115      28.581  -0.139  16.665  1.00 13.29          N
ATOM   1621  CA   ASP A 115      29.724   0.014  15.754  1.00 14.72          C
ATOM   1623  CB  AASP A 115      30.159  -1.299  15.125  0.50 16.85          C
ATOM   1624  CB  BASP A 115      29.949  -1.288  14.969  0.50 15.71          C
ATOM   1629  CG  AASP A 115      29.100  -1.900  14.308  0.50 20.25          C
ATOM   1630  CG  BASP A 115      30.701  -2.324  15.752  0.50 16.91          C
ATOM   1631  OD1AASP A 115      28.555  -1.177  13.460  0.50 32.11          O
ATOM   1632  OD1BASP A 115      31.693  -1.951  16.379  0.50 24.07          O
ATOM   1633  OD2AASP A 115      28.717  -3.057  14.477  0.50 33.25          O
ATOM   1634  OD2BASP A 115      30.401  -3.501  15.764  0.50 20.69          O
ATOM   1635  C    ASP A 115      29.511   1.034  14.682  1.00 13.31          C
ATOM   1636  O    ASP A 115      30.488   1.671  14.268  1.00 13.00          O
ATOM   1637  N    ASP A 116      28.292   1.098  14.156  1.00 12.95          N
```

```
        ATOM   1639  CA   ASP A 116     27.982   1.996  13.046  1.00 13.15           C
        ATOM   1641  CB   ASP A 116     26.618   1.685  12.446  1.00 14.36           C
        ATOM   1644  CG   ASP A 116     26.506   0.299  11.819  1.00 14.39           C
        ATOM   1645  OD1  ASP A 116     27.509  -0.378  11.592  1.00 13.15           O
        ATOM   1646  OD2  ASP A 116     25.408  -0.179  11.584  1.00 13.28           O
        ATOM   1647  C    ASP A 116     28.001   3.437  13.581  1.00 13.01           C
        ATOM   1648  O    ASP A 116     28.529   4.354  12.937  1.00 13.99           O
        ATOM   1649  N    ILE A 117     27.409   3.644  14.748  1.00 13.05           N
        ATOM   1651  CA   ILE A 117     27.404   4.952  15.339  1.00 12.04           C
        ATOM   1653  CB   ILE A 117     26.518   4.961  16.572  1.00 12.89           C
        ATOM   1655  CG1  ILE A 117     25.034   4.744  16.168  1.00 17.41           C
        ATOM   1658  CD1  ILE A 117     24.279   3.948  17.085  1.00 21.55           C
        ATOM   1662  CG2  ILE A 117     26.715   6.288  17.378  1.00 15.14           C
        ATOM   1666  C    ILE A 117     28.813   5.403  15.623  1.00 11.86           C
        ATOM   1667  O    ILE A 117     29.195   6.548  15.321  1.00 12.61           O
        ATOM   1668  N    ALA A 118     29.609   4.532  16.227  1.00 10.45           N
        ATOM   1670  CA   ALA A 118     30.981   4.891  16.519  1.00 11.56           C
        ATOM   1672  CB   ALA A 118     31.649   3.800  17.353  1.00 11.97           C
        ATOM   1676  C    ALA A 118     31.786   5.248  15.273  1.00 11.35           C
        ATOM   1677  O    ALA A 118     32.511   6.241  15.232  1.00 10.56           O
        ATOM   1678  N    ALA A 119     31.597   4.459  14.253  1.00 11.09           N
        ATOM   1680  CA   ALA A 119     32.298   4.693  13.010  1.00 10.91           C
        ATOM   1682  CB   ALA A 119     32.030   3.600  12.104  1.00 11.91           C
        ATOM   1686  C    ALA A 119     31.875   6.029  12.430  1.00 10.50           C
        ATOM   1687  O    ALA A 119     32.721   6.808  11.942  1.00 12.46           O
        ATOM   1688  N    ALA A 120     30.589   6.342  12.539  1.00 11.95           N
        ATOM   1690  CA   ALA A 120     30.079   7.579  12.001  1.00 11.18           C
        ATOM   1692  CB   ALA A 120     28.626   7.575  12.034  1.00 11.63           C
        ATOM   1696  C    ALA A 120     30.643   8.813  12.743  1.00 11.63           C
        ATOM   1697  O    ALA A 120     31.033   9.799  12.104  1.00 11.85           O
        ATOM   1698  N    ILE A 121     30.708   8.753  14.070  1.00 10.33           N
        ATOM   1700  CA   ILE A 121     31.291   9.848  14.892  1.00 10.58           C
        ATOM   1702  CB   ILE A 121     31.215   9.481  16.379  1.00 11.12           C
        ATOM   1704  CG1  ILE A 121     29.768   9.320  16.750  1.00 11.01           C
        ATOM   1707  CD1  ILE A 121     29.566   8.668  18.128  1.00 11.36           C
        ATOM   1711  CG2  ILE A 121     31.860  10.543  17.229  1.00 12.83           C
        ATOM   1715  C    ILE A 121     32.749  10.129  14.510  1.00 11.68           C
        ATOM   1716  O    ILE A 121     33.158  11.259  14.287  1.00 12.00           O
        ATOM   1717  N    ARG A 122     33.536   9.055  14.448  1.00 11.70           N
        ATOM   1719  CA   ARG A 122     34.929   9.171  14.046  1.00 13.86           C
        ATOM   1721  CB   ARG A 122     35.603   7.810  14.114  1.00 14.57           C
        ATOM   1724  CG   ARG A 122     35.715   7.320  15.531  1.00 14.77           C
        ATOM   1727  CD   ARG A 122     36.384   5.975  15.679  1.00 18.79           C
        ATOM   1730  NE   ARG A 122     36.784   5.757  17.048  1.00 21.92           N
        ATOM   1732  CZ   ARG A 122     37.945   6.112  17.577  1.00 22.62           C
        ATOM   1733  NH1  ARG A 122     38.894   6.640  16.838  1.00 20.73           N
        ATOM   1736  NH2  ARG A 122     38.178   5.850  18.857  1.00 29.11           N
        ATOM   1739  C    ARG A 122     35.088   9.760  12.636  1.00 14.45           C
        ATOM   1740  O    ARG A 122     35.992  10.563  12.389  1.00 13.33           O
        ATOM   1741  N    HIS A 123     34.198   9.348  11.743  1.00 12.96           N
        ATOM   1743  CA   HIS A 123     34.231   9.828  10.385  1.00 14.40           C
        ATOM   1745  CB   HIS A 123     33.324   9.001   9.522  1.00 14.46           C
        ATOM   1748  CG   HIS A 123     33.390   9.347   8.065  1.00 17.57           C
        ATOM   1749  ND1  HIS A 123     34.358   8.843   7.224  1.00 26.34           N
        ATOM   1751  CE1  HIS A 123     34.183   9.333   6.005  1.00 26.65           C
        ATOM   1753  NE2  HIS A 123     33.120  10.115   6.015  1.00 23.02           N
        ATOM   1755  CD2  HIS A 123     32.596  10.125   7.299  1.00 22.31           C
        ATOM   1757  C    HIS A 123     33.913  11.345  10.332  1.00 15.58           C
        ATOM   1758  O    HIS A 123     34.587  12.095   9.658  1.00 14.13           O
        ATOM   1759  N    VAL A 124     32.914  11.801  11.081  1.00 13.58           N
        ATOM   1761  CA   VAL A 124     32.701  13.233  11.195  1.00 13.94           C
        ATOM   1763  CB   VAL A 124     31.583  13.598  12.235  1.00 13.67           C
        ATOM   1765  CG1  VAL A 124     31.476  15.111  12.408  1.00 11.98           C
```

```
ATOM   1769  CG2 VAL A 124     30.258  13.019  11.847  1.00  14.52           C
ATOM   1773  C   VAL A 124     33.980  13.971  11.580  1.00  12.11           C
ATOM   1774  O   VAL A 124     34.323  14.973  10.938  1.00  13.19           O
ATOM   1775  N   ALA A 125     34.672  13.495  12.604  1.00  12.61           N
ATOM   1777  CA  ALA A 125     35.875  14.123  13.121  1.00  12.03           C
ATOM   1779  CB  ALA A 125     36.351  13.398  14.322  1.00  11.98           C
ATOM   1783  C   ALA A 125     36.972  14.158  12.062  1.00  13.52           C
ATOM   1784  O   ALA A 125     37.610  15.186  11.838  1.00  13.20           O
ATOM   1785  N   ASP A 126     37.081  13.059  11.312  1.00  13.79           N
ATOM   1787  CA  ASP A 126     38.087  12.980  10.268  1.00  15.94           C
ATOM   1789  CB  ASP A 126     38.180  11.566   9.743  1.00  16.11           C
ATOM   1792  CG  ASP A 126     38.895  10.635  10.677  1.00  16.70           C
ATOM   1793  OD1 ASP A 126     39.620  11.075  11.580  1.00  17.85           O
ATOM   1794  OD2 ASP A 126     38.795   9.393  10.586  1.00  17.09           O
ATOM   1795  C   ASP A 126     37.736  13.933   9.133  1.00  16.71           C
ATOM   1796  O   ASP A 126     38.604  14.612   8.602  1.00  17.22           O
ATOM   1797  N   GLU A 127     36.465  14.027   8.798  1.00  16.48           N
ATOM   1799  CA  GLU A 127     36.033  14.934   7.759  1.00  17.08           C
ATOM   1801  CB  GLU A 127     34.580  14.723   7.386  1.00  17.14           C
ATOM   1804  CG  GLU A 127     34.319  13.431   6.618  1.00  18.23           C
ATOM   1807  CD  GLU A 127     34.875  13.485   5.205  1.00  23.17           C
ATOM   1808  OE1 GLU A 127     34.333  14.250   4.412  1.00  22.57           O
ATOM   1809  OE2 GLU A 127     35.887  12.809   4.967  1.00  25.80           O
ATOM   1810  C   GLU A 127     36.256  16.370   8.204  1.00  18.24           C
ATOM   1811  O   GLU A 127     36.634  17.255   7.393  1.00  17.10           O
ATOM   1812  N   ALA A 128     35.969  16.626   9.465  1.00  16.20           N
ATOM   1814  CA  ALA A 128     36.165  17.979  10.000  1.00  17.01           C
ATOM   1816  CB  ALA A 128     35.582  18.100  11.469  1.00  18.11           C
ATOM   1820  C   ALA A 128     37.607  18.403   9.959  1.00  17.06           C
ATOM   1821  O   ALA A 128     37.923  19.537   9.561  1.00  18.46           O
ATOM   1822  N   SER A 129     38.496  17.519  10.360  1.00  17.73           N
ATOM   1824  CA  SER A 129     39.896  17.869  10.334  1.00  19.04           C
ATOM   1826  CB  SER A 129     40.735  16.796  10.996  1.00  19.88           C
ATOM   1829  OG  SER A 129     40.289  15.493  10.649  1.00  28.54           O
ATOM   1831  C   SER A 129     40.367  18.063   8.907  1.00  18.31           C
ATOM   1832  O   SER A 129     41.158  18.968   8.641  1.00  19.92           O
ATOM   1833  N   ARG A 130     39.927  17.209   8.003  1.00  17.50           N
ATOM   1835  CA  ARG A 130     40.418  17.258   6.611  1.00  17.79           C
ATOM   1837  CB  ARG A 130     39.938  16.052   5.802  1.00  17.78           C
ATOM   1840  CG  ARG A 130     40.573  15.989   4.406  1.00  17.70           C
ATOM   1843  CD  ARG A 130     40.048  14.864   3.632  1.00  18.82           C
ATOM   1846  NE  ARG A 130     38.768  15.271   3.064  1.00  25.15           N
ATOM   1848  CZ  ARG A 130     37.641  14.760   3.393  1.00  25.02           C
ATOM   1849  NH1 ARG A 130     37.620  13.808   4.337  1.00  26.77           N
ATOM   1852  NH2 ARG A 130     36.524  15.209   2.775  1.00  23.08           N
ATOM   1855  C   ARG A 130     39.990  18.543   5.958  1.00  19.20           C
ATOM   1856  O   ARG A 130     40.816  19.255   5.352  1.00  20.50           O
ATOM   1857  N   THR A 131     38.740  18.915   6.155  1.00  18.72           N
ATOM   1859  CA  THR A 131     38.127  20.089   5.461  1.00  19.43           C
ATOM   1861  CB  THR A 131     36.673  19.866   5.244  1.00  20.43           C
ATOM   1863  OG1 THR A 131     35.973  19.754   6.517  1.00  18.62           O
ATOM   1865  CG2 THR A 131     36.421  18.548   4.449  1.00  22.94           C
ATOM   1869  C   THR A 131     38.262  21.415   6.203  1.00  20.40           C
ATOM   1870  O   THR A 131     37.906  22.461   5.657  1.00  20.95           O
ATOM   1871  N   GLY A 132     38.758  21.356   7.431  1.00  18.57           N
ATOM   1873  CA  GLY A 132     38.841  22.513   8.289  1.00  19.51           C
ATOM   1876  C   GLY A 132     37.464  23.129   8.581  1.00  20.63           C
ATOM   1877  O   GLY A 132     37.313  24.336   8.829  1.00  23.12           O
ATOM   1878  N   SER A 133     36.442  22.287   8.646  1.00  18.55           N
ATOM   1880  CA  SER A 133     35.094  22.754   8.904  1.00  18.11           C
ATOM   1882  CB  SER A 133     34.080  21.819   8.260  1.00  18.42           C
ATOM   1885  OG  SER A 133     34.242  21.666   6.844  1.00  21.03           O
ATOM   1887  C   SER A 133     34.768  22.836  10.427  1.00  16.26           C
```

```
ATOM   1888  O    SER A 133      35.348  22.145  11.284  1.00 15.54        O
ATOM   1889  N    LYS A 134      33.798  23.687  10.720  1.00 16.27        N
ATOM   1891  CA   LYS A 134      33.275  23.830  12.096  1.00 15.54        C
ATOM   1893  CB   LYS A 134      32.921  25.274  12.419  1.00 15.50        C
ATOM   1896  CG   LYS A 134      34.154  26.176  12.525  1.00 18.18        C
ATOM   1899  CD   LYS A 134      33.819  27.647  12.502  1.00 25.94        C
ATOM   1902  CE   LYS A 134      35.064  28.567  12.191  1.00 30.57        C
ATOM   1905  NZ   LYS A 134      36.391  28.051  12.564  1.00 34.14        N
ATOM   1909  C    LYS A 134      32.032  22.951  12.094  1.00 13.21        C
ATOM   1910  O    LYS A 134      31.121  23.177  11.349  1.00 14.18        O
ATOM   1911  N    VAL A 135      32.015  21.919  12.921  1.00 12.06        N
ATOM   1913  CA   VAL A 135      30.964  20.957  12.863  1.00 12.02        C
ATOM   1915  CB   VAL A 135      31.487  19.632  12.321  1.00 13.28        C
ATOM   1917  CG1  VAL A 135      30.363  18.596  12.114  1.00 14.27        C
ATOM   1921  CG2  VAL A 135      32.322  19.867  11.006  1.00 14.42        C
ATOM   1925  C    VAL A 135      30.383  20.673  14.241  1.00 11.74        C
ATOM   1926  O    VAL A 135      31.097  20.566  15.220  1.00 11.76        O
ATOM   1927  N    VAL A 136      29.071  20.604  14.286  1.00 11.28        N
ATOM   1929  CA   VAL A 136      28.389  20.206  15.478  1.00 10.73        C
ATOM   1931  CB   VAL A 136      27.285  21.177  15.819  1.00 11.25        C
ATOM   1933  CG1  VAL A 136      26.576  20.755  17.127  1.00 13.78        C
ATOM   1937  CG2  VAL A 136      27.897  22.594  16.013  1.00 12.65        C
ATOM   1941  C    VAL A 136      27.702  18.852  15.159  1.00 11.22        C
ATOM   1942  O    VAL A 136      26.973  18.747  14.178  1.00 11.53        O
ATOM   1943  N    ILE A 137      27.928  17.850  15.993  1.00  9.65        N
ATOM   1945  CA   ILE A 137      27.255  16.573  15.854  1.00 10.61        C
ATOM   1947  CB   ILE A 137      28.113  15.459  16.381  1.00  9.23        C
ATOM   1949  CG1  ILE A 137      29.215  15.121  15.395  1.00 11.83        C
ATOM   1952  CD1  ILE A 137      30.266  14.104  15.930  1.00 11.56        C
ATOM   1956  CG2  ILE A 137      27.238  14.230  16.611  1.00 10.38        C
ATOM   1960  C    ILE A 137      25.993  16.610  16.690  1.00 10.44        C
ATOM   1961  O    ILE A 137      26.031  17.014  17.869  1.00 11.92        O
ATOM   1962  N    ASN A 138      24.899  16.203  16.096  1.00  9.00        N
ATOM   1964  CA   ASN A 138      23.654  15.942  16.764  1.00 10.52        C
ATOM   1966  CB   ASN A 138      22.494  16.601  15.996  1.00  8.55        C
ATOM   1969  CG   ASN A 138      21.146  16.503  16.715  1.00 12.38        C
ATOM   1970  OD1  ASN A 138      20.648  17.515  17.236  1.00 10.74        O
ATOM   1971  ND2  ASN A 138      20.519  15.297  16.722  1.00  9.55        N
ATOM   1974  C    ASN A 138      23.376  14.477  16.861  1.00 10.74        C
ATOM   1975  O    ASN A 138      23.256  13.799  15.833  1.00  9.80        O
ATOM   1976  N    MET A 139      23.208  13.987  18.091  1.00  9.89        N
ATOM   1978  CA   MET A 139      22.830  12.585  18.304  1.00 10.28        C
ATOM   1980  CB   MET A 139      23.975  11.764  18.906  1.00  9.62        C
ATOM   1983  CG   MET A 139      24.984  11.345  17.895  1.00  8.67        C
ATOM   1986  SD   MET A 139      26.240  10.206  18.525  1.00 11.34        S
ATOM   1987  CE   MET A 139      27.161  11.324  19.556  1.00 13.56        C
ATOM   1991  C    MET A 139      21.581  12.474  19.162  1.00 10.20        C
ATOM   1992  O    MET A 139      21.587  12.497  20.413  1.00  9.22        O
ATOM   1993  N    SER A 140      20.467  12.424  18.458  1.00 10.57        N
ATOM   1995  CA   SER A 140      19.166  12.207  19.083  1.00 10.10        C
ATOM   1997  CB   SER A 140      18.082  12.817  18.201  1.00 10.22        C
ATOM   2000  OG   SER A 140      18.142  14.229  18.264  1.00 11.42        O
ATOM   2002  C    SER A 140      18.959  10.705  19.255  1.00 11.04        C
ATOM   2003  O    SER A 140      18.006  10.119  18.716  1.00 10.43        O
ATOM   2004  N    LEU A 141      19.844  10.063  20.011  1.00 10.62        N
ATOM   2006  CA   LEU A 141      19.890   8.627  20.116  1.00 10.50        C
ATOM   2008  CB   LEU A 141      20.446   8.014  18.833  1.00 10.74        C
ATOM   2011  CG   LEU A 141      21.881   8.483  18.439  1.00 11.49        C
ATOM   2013  CD1  LEU A 141      22.939   7.897  19.316  1.00 11.20        C
ATOM   2017  CD2  LEU A 141      22.119   8.057  17.001  1.00 12.54        C
ATOM   2021  C    LEU A 141      20.748   8.223  21.297  1.00  9.69        C
ATOM   2022  O    LEU A 141      21.402   9.060  21.892  1.00  9.38        O
ATOM   2023  N    GLY A 142      20.640   6.975  21.689  1.00 11.48        N
```

| ATOM | 2025 | CA  | GLY A 142 | 21.450 | 6.472 | 22.789 | 1.00 | 10.69 | C |
|------|------|-----|-----------|--------|-------|--------|------|-------|---|
| ATOM | 2028 | C   | GLY A 142 | 21.046 | 5.146 | 23.335 | 1.00 | 9.74  | C |
| ATOM | 2029 | O   | GLY A 142 | 20.283 | 4.413 | 22.660 | 1.00 | 12.20 | O |
| ATOM | 2030 | N   | SER A 143 | 21.517 | 4.850 | 24.562 | 1.00 | 11.68 | N |
| ATOM | 2032 | CA  | SER A 143 | 21.200 | 3.610 | 25.308 | 1.00 | 11.10 | C |
| ATOM | 2034 | CB  | SER A 143 | 22.187 | 2.489 | 25.050 | 1.00 | 11.52 | C |
| ATOM | 2037 | OG  | SER A 143 | 23.517 | 2.918 | 25.316 | 1.00 | 11.14 | O |
| ATOM | 2039 | C   | SER A 143 | 21.272 | 3.995 | 26.777 | 1.00 | 12.09 | C |
| ATOM | 2040 | O   | SER A 143 | 21.941 | 4.966 | 27.161 | 1.00 | 11.62 | O |
| ATOM | 2041 | N   | SER A 144 | 20.523 | 3.292 | 27.587 | 1.00 | 11.90 | N |
| ATOM | 2043 | CA  | SER A 144 | 20.527 | 3.552 | 28.992 | 1.00 | 11.92 | C |
| ATOM | 2045 | CB  | SER A 144 | 19.513 | 2.623 | 29.653 | 1.00 | 13.61 | C |
| ATOM | 2048 | OG  | SER A 144 | 19.521 | 2.848 | 31.017 | 1.00 | 18.36 | O |
| ATOM | 2050 | C   | SER A 144 | 21.903 | 3.362 | 29.593 | 1.00 | 13.78 | C |
| ATOM | 2051 | O   | SER A 144 | 22.341 | 4.161 | 30.401 | 1.00 | 14.05 | O |
| ATOM | 2052 | N   | ALA A 145 | 22.551 | 2.278 | 29.189 | 1.00 | 12.68 | N |
| ATOM | 2054 | CA  | ALA A 145 | 23.892 | 1.972 | 29.678 | 1.00 | 11.54 | C |
| ATOM | 2056 | CB  | ALA A 145 | 24.135 | 0.503 | 29.488 | 1.00 | 12.43 | C |
| ATOM | 2060 | C   | ALA A 145 | 24.956 | 2.745 | 28.886 | 1.00 | 12.26 | C |
| ATOM | 2061 | O   | ALA A 145 | 24.814 | 3.001 | 27.712 | 1.00 | 12.18 | O |
| ATOM | 2062 | N   | LYS A 146 | 26.067 | 3.066 | 29.521 | 1.00 | 10.71 | N |
| ATOM | 2064 | CA  | LYS A 146 | 27.184 | 3.640 | 28.760 | 1.00 | 11.08 | C |
| ATOM | 2066 | CB  | LYS A 146 | 28.219 | 4.095 | 29.754 | 1.00 | 10.62 | C |
| ATOM | 2069 | CG  | LYS A 146 | 29.563 | 4.535 | 29.197 | 1.00 | 13.37 | C |
| ATOM | 2072 | CD  | LYS A 146 | 30.506 | 5.206 | 30.245 | 1.00 | 14.55 | C |
| ATOM | 2075 | CE  | LYS A 146 | 31.796 | 5.638 | 29.643 | 1.00 | 17.59 | C |
| ATOM | 2078 | NZ  | LYS A 146 | 32.732 | 6.238 | 30.665 | 1.00 | 17.20 | N |
| ATOM | 2082 | C   | LYS A 146 | 27.767 | 2.559 | 27.834 | 1.00 | 8.93  | C |
| ATOM | 2083 | O   | LYS A 146 | 27.978 | 1.383 | 28.241 | 1.00 | 11.42 | O |
| ATOM | 2084 | N   | ASP A 147 | 28.075 | 2.956 | 26.621 | 1.00 | 10.15 | N |
| ATOM | 2086 | CA  | ASP A 147 | 28.694 | 2.129 | 25.575 | 1.00 | 10.08 | C |
| ATOM | 2088 | CB  | ASP A 147 | 27.843 | 2.189 | 24.296 | 1.00 | 10.84 | C |
| ATOM | 2091 | CG  | ASP A 147 | 28.460 | 1.509 | 23.117 | 1.00 | 11.61 | C |
| ATOM | 2092 | OD1 | ASP A 147 | 29.701 | 1.630 | 22.895 | 1.00 | 11.69 | O |
| ATOM | 2093 | OD2 | ASP A 147 | 27.753 | 0.879 | 22.305 | 1.00 | 10.96 | O |
| ATOM | 2094 | C   | ASP A 147 | 30.057 | 2.784 | 25.353 | 1.00 | 10.79 | C |
| ATOM | 2095 | O   | ASP A 147 | 30.163 | 3.902 | 24.859 | 1.00 | 9.34  | O |
| ATOM | 2096 | N   | SER A 148 | 31.104 | 2.055 | 25.717 | 1.00 | 9.95  | N |
| ATOM | 2098 | CA  | SER A 148 | 32.450 | 2.588 | 25.641 | 1.00 | 11.43 | C |
| ATOM | 2100 | CB  | SER A 148 | 33.431 | 1.838 | 26.533 | 1.00 | 11.86 | C |
| ATOM | 2103 | OG  | SER A 148 | 33.164 | 2.051 | 27.904 | 1.00 | 13.07 | O |
| ATOM | 2105 | C   | SER A 148 | 32.999 | 2.765 | 24.221 | 1.00 | 12.17 | C |
| ATOM | 2106 | O   | SER A 148 | 33.958 | 3.537 | 24.021 | 1.00 | 10.72 | O |
| ATOM | 2107 | N   | LEU A 149 | 32.471 | 1.997 | 23.269 | 1.00 | 9.59  | N |
| ATOM | 2109 | CA  | LEU A 149 | 32.936 | 2.120 | 21.909 | 1.00 | 10.95 | C |
| ATOM | 2111 | CB  | LEU A 149 | 32.374 | 1.029 | 21.030 | 1.00 | 11.13 | C |
| ATOM | 2114 | CG  | LEU A 149 | 32.863 | 1.087 | 19.569 | 1.00 | 13.82 | C |
| ATOM | 2116 | CD1 | LEU A 149 | 34.404 | 0.961 | 19.536 | 1.00 | 14.72 | C |
| ATOM | 2120 | CD2 | LEU A 149 | 32.162 | 0.036 | 18.699 | 1.00 | 15.05 | C |
| ATOM | 2124 | C   | LEU A 149 | 32.474 | 3.511 | 21.419 | 1.00 | 10.85 | C |
| ATOM | 2125 | O   | LEU A 149 | 33.201 | 4.268 | 20.807 | 1.00 | 10.58 | O |
| ATOM | 2126 | N   | ILE A 150 | 31.223 | 3.823 | 21.649 | 1.00 | 9.24  | N |
| ATOM | 2128 | CA  | ILE A 150 | 30.671 | 5.127 | 21.255 | 1.00 | 9.06  | C |
| ATOM | 2130 | CB  | ILE A 150 | 29.145 | 5.136 | 21.471 | 1.00 | 8.95  | C |
| ATOM | 2132 | CG1 | ILE A 150 | 28.499 | 4.376 | 20.337 | 1.00 | 9.94  | C |
| ATOM | 2135 | CD1 | ILE A 150 | 27.038 | 4.154 | 20.508 | 1.00 | 11.53 | C |
| ATOM | 2139 | CG2 | ILE A 150 | 28.601 | 6.553 | 21.538 | 1.00 | 11.45 | C |
| ATOM | 2143 | C   | ILE A 150 | 31.433 | 6.226 | 22.052 | 1.00 | 7.79  | C |
| ATOM | 2144 | O   | ILE A 150 | 31.793 | 7.297 | 21.500 | 1.00 | 8.15  | O |
| ATOM | 2145 | N   | ALA A 151 | 31.724 | 5.956 | 23.300 | 1.00 | 8.34  | N |
| ATOM | 2147 | CA  | ALA A 151 | 32.396 | 6.930 | 24.152 | 1.00 | 9.35  | C |
| ATOM | 2149 | CB  | ALA A 151 | 32.479 | 6.402 | 25.577 | 1.00 | 10.22 | C |
| ATOM | 2153 | C   | ALA A 151 | 33.796 | 7.240 | 23.629 | 1.00 | 9.49  | C |

```
ATOM   2154  O    ALA A 151     34.215   8.422  23.583  1.00  9.45        O
ATOM   2155  N    SER A 152     34.508   6.177  23.181  1.00 10.40        N
ATOM   2157  CA   SER A 152     35.823   6.371  22.613  1.00 11.13        C
ATOM   2159  CB   SER A 152     36.466   5.047  22.278  1.00 10.46        C
ATOM   2162  OG   SER A 152     37.628   5.216  21.460  1.00 13.86        O
ATOM   2164  C    SER A 152     35.737   7.285  21.349  1.00 11.17        C
ATOM   2165  O    SER A 152     36.585   8.207  21.144  1.00 11.47        O
ATOM   2166  N    ALA A 153     34.688   7.103  20.578  1.00 11.26        N
ATOM   2168  CA   ALA A 153     34.476   7.917  19.358  1.00 11.23        C
ATOM   2170  CB   ALA A 153     33.413   7.295  18.527  1.00 12.05        C
ATOM   2174  C    ALA A 153     34.143   9.349  19.699  1.00 11.53        C
ATOM   2175  O    ALA A 153     34.699  10.314  19.103  1.00 11.12        O
ATOM   2176  N    VAL A 154     33.285   9.529  20.695  1.00  9.67        N
ATOM   2178  CA   VAL A 154     32.941  10.878  21.104  1.00  9.77        C
ATOM   2180  CB   VAL A 154     31.908  10.810  22.223  1.00 10.26        C
ATOM   2182  CG1  VAL A 154     31.833  12.136  22.988  1.00 12.19        C
ATOM   2186  CG2  VAL A 154     30.583  10.402  21.661  1.00 11.04        C
ATOM   2190  C    VAL A 154     34.229  11.606  21.565  1.00 11.02        C
ATOM   2191  O    VAL A 154     34.449  12.779  21.212  1.00 11.61        O
ATOM   2192  N    ASP A 155     35.069  10.954  22.367  1.00 10.47        N
ATOM   2194  CA   ASP A 155     36.309  11.603  22.838  1.00 12.38        C
ATOM   2196  CB   ASP A 155     37.040  10.744  23.859  1.00 13.32        C
ATOM   2199  CG   ASP A 155     36.328  10.668  25.183  1.00 19.66        C
ATOM   2200  OD1  ASP A 155     35.449  11.508  25.455  1.00 21.98        O
ATOM   2201  OD2  ASP A 155     36.604   9.813  26.030  1.00 21.73        O
ATOM   2202  C    ASP A 155     37.242  11.932  21.674  1.00 13.46        C
ATOM   2203  O    ASP A 155     37.928  12.926  21.695  1.00 12.65        O
ATOM   2204  N    TYR A 156     37.308  11.034  20.694  1.00 12.30        N
ATOM   2206  CA   TYR A 156     38.119  11.263  19.510  1.00 11.91        C
ATOM   2208  CB   TYR A 156     37.992  10.073  18.607  1.00 12.85        C
ATOM   2211  CG   TYR A 156     38.753  10.140  17.309  1.00 13.57        C
ATOM   2212  CD1  TYR A 156     40.154   9.965  17.286  1.00 16.30        C
ATOM   2214  CE1  TYR A 156     40.822   9.989  16.093  1.00 15.71        C
ATOM   2216  CZ   TYR A 156     40.136  10.172  14.899  1.00 20.85        C
ATOM   2217  OH   TYR A 156     40.795  10.142  13.677  1.00 19.47        O
ATOM   2219  CE2  TYR A 156     38.771  10.352  14.883  1.00 14.75        C
ATOM   2221  CD2  TYR A 156     38.096  10.349  16.111  1.00 14.20        C
ATOM   2223  C    TYR A 156     37.653  12.497  18.764  1.00 11.70        C
ATOM   2224  O    TYR A 156     38.463  13.343  18.408  1.00 12.40        O
ATOM   2225  N    ALA A 157     36.332  12.649  18.630  1.00 11.59        N
ATOM   2227  CA   ALA A 157     35.773  13.776  17.895  1.00 10.92        C
ATOM   2229  CB   ALA A 157     34.319  13.493  17.547  1.00 11.98        C
ATOM   2233  C    ALA A 157     35.926  15.058  18.670  1.00 11.69        C
ATOM   2234  O    ALA A 157     36.214  16.117  18.072  1.00 11.27        O
ATOM   2235  N    TYR A 158     35.740  14.996  19.983  1.00 11.31        N
ATOM   2237  CA   TYR A 158     35.855  16.210  20.809  1.00 12.56        C
ATOM   2239  CB   TYR A 158     35.410  15.940  22.243  1.00 12.28        C
ATOM   2242  CG   TYR A 158     35.147  17.188  23.090  1.00  9.96        C
ATOM   2243  CD1  TYR A 158     34.015  17.937  22.878  1.00 11.56        C
ATOM   2245  CE1  TYR A 158     33.754  19.051  23.629  1.00 13.20        C
ATOM   2247  CZ   TYR A 158     34.635  19.477  24.580  1.00 13.62        C
ATOM   2248  OH   TYR A 158     34.370  20.612  25.295  1.00 12.22        O
ATOM   2250  CE2  TYR A 158     35.813  18.809  24.787  1.00 13.48        C
ATOM   2252  CD2  TYR A 158     36.078  17.647  24.028  1.00 12.56        C
ATOM   2254  C    TYR A 158     37.308  16.655  20.783  1.00 12.48        C
ATOM   2255  O    TYR A 158     37.591  17.853  20.822  1.00 12.68        O
ATOM   2256  N    GLY A 159     38.207  15.683  20.642  1.00 12.38        N
ATOM   2258  CA   GLY A 159     39.628  15.978  20.651  1.00 13.17        C
ATOM   2261  C    GLY A 159     40.055  16.611  19.371  1.00 13.46        C
ATOM   2262  O    GLY A 159     41.161  17.165  19.297  1.00 14.28        O
ATOM   2263  N    LYS A 160     39.238  16.495  18.350  1.00 12.48        N
ATOM   2265  CA   LYS A 160     39.486  17.099  17.035  1.00 14.66        C
ATOM   2267  CB   LYS A 160     39.324  16.046  15.953  1.00 15.36        C
```

```
ATOM   2270   CG   LYS A 160      40.421  14.964  15.992  1.00 20.16        C
ATOM   2273   CD   LYS A 160      40.057  13.848  15.058  1.00 25.00        C
ATOM   2276   CE   LYS A 160      41.183  13.408  14.161  1.00 31.38        C
ATOM   2279   NZ   LYS A 160      41.602  14.404  13.204  1.00 30.54        N
ATOM   2283   C    LYS A 160      38.603  18.344  16.761  1.00 14.03        C
ATOM   2284   O    LYS A 160      38.469  18.786  15.621  1.00 12.37        O
ATOM   2285   N    GLY A 161      38.076  18.954  17.829  1.00 13.97        N
ATOM   2287   CA   GLY A 161      37.363  20.230  17.751  1.00 13.11        C
ATOM   2290   C    GLY A 161      35.928  20.180  17.281  1.00 12.99        C
ATOM   2291   O    GLY A 161      35.434  21.185  16.743  1.00 14.25        O
ATOM   2292   N    VAL A 162      35.269  19.020  17.395  1.00 11.44        N
ATOM   2294   CA   VAL A 162      33.858  18.848  16.972  1.00 10.32        C
ATOM   2296   CB   VAL A 162      33.621  17.492  16.309  1.00 11.03        C
ATOM   2298   CG1  VAL A 162      32.146  17.268  15.950  1.00 11.59        C
ATOM   2302   CG2  VAL A 162      34.438  17.378  15.034  1.00 13.60        C
ATOM   2306   C    VAL A 162      32.991  18.918  18.219  1.00 10.43        C
ATOM   2307   O    VAL A 162      33.306  18.259  19.222  1.00 11.54        O
ATOM   2308   N    LEU A 163      31.965  19.748  18.217  1.00  9.11        N
ATOM   2310   CA   LEU A 163      31.075  19.817  19.382  1.00 10.40        C
ATOM   2312   CB   LEU A 163      30.278  21.105  19.344  1.00 11.53        C
ATOM   2315   CG   LEU A 163      29.336  21.334  20.515  1.00 10.22        C
ATOM   2317   CD1  LEU A 163      30.163  21.561  21.748  1.00 12.89        C
ATOM   2321   CD2  LEU A 163      28.486  22.497  20.248  1.00 13.67        C
ATOM   2325   C    LEU A 163      30.118  18.647  19.257  1.00 10.89        C
ATOM   2326   O    LEU A 163      29.620  18.367  18.176  1.00 13.34        O
ATOM   2327   N    ILE A 164      29.832  17.975  20.347  1.00 11.01        N
ATOM   2329   CA   ILE A 164      28.860  16.890  20.383  1.00  9.92        C
ATOM   2331   CB   ILE A 164      29.500  15.573  20.908  1.00 10.24        C
ATOM   2333   CG1  ILE A 164      30.616  15.070  19.976  1.00 13.39        C
ATOM   2336   CD1  ILE A 164      31.893  15.389  20.491  1.00 16.69        C
ATOM   2340   CG2  ILE A 164      28.496  14.458  20.907  1.00 12.88        C
ATOM   2344   C    ILE A 164      27.673  17.259  21.275  1.00 10.29        C
ATOM   2345   O    ILE A 164      27.851  17.505  22.479  1.00 10.17        O
ATOM   2346   N    VAL A 165      26.489  17.191  20.694  1.00  9.19        N
ATOM   2348   CA   VAL A 165      25.257  17.479  21.391  1.00  9.03        C
ATOM   2350   CB   VAL A 165      24.576  18.708  20.753  1.00 10.21        C
ATOM   2352   CG1  VAL A 165      23.300  19.053  21.542  1.00  9.33        C
ATOM   2356   CG2  VAL A 165      25.483  19.888  20.715  1.00 11.01        C
ATOM   2360   C    VAL A 165      24.360  16.245  21.311  1.00  8.47        C
ATOM   2361   O    VAL A 165      24.193  15.681  20.222  1.00 10.40        O
ATOM   2362   N    ALA A 166      23.833  15.747  22.452  1.00  9.28        N
ATOM   2364   CA   ALA A 166      23.163  14.469  22.484  1.00  8.57        C
ATOM   2366   CB   ALA A 166      24.104  13.327  22.794  1.00  9.70        C
ATOM   2370   C    ALA A 166      22.011  14.454  23.489  1.00  9.40        C
ATOM   2371   O    ALA A 166      22.028  15.186  24.476  1.00  9.37        O
ATOM   2372   N    ALA A 167      21.000  13.646  23.186  1.00 10.21        N
ATOM   2374   CA   ALA A 167      19.794  13.637  23.965  1.00 10.10        C
ATOM   2376   CB   ALA A 167      18.747  12.726  23.251  1.00 11.66        C
ATOM   2380   C    ALA A 167      20.086  13.087  25.329  1.00 10.38        C
ATOM   2381   O    ALA A 167      20.787  12.038  25.431  1.00  8.93        O
ATOM   2382   N    ALA A 168      19.424  13.572  26.360  1.00 10.36        N
ATOM   2384   CA   ALA A 168      19.623  13.036  27.685  1.00 10.56        C
ATOM   2386   CB   ALA A 168      19.014  13.978  28.698  1.00 11.45        C
ATOM   2390   C    ALA A 168      19.026  11.631  27.894  1.00 10.08        C
ATOM   2391   O    ALA A 168      19.441  10.860  28.771  1.00 10.97        O
ATOM   2392   N    GLY A 169      18.020  11.315  27.108  1.00  9.80        N
ATOM   2394   CA   GLY A 169      17.216  10.125  27.318  1.00  9.88        C
ATOM   2397   C    GLY A 169      15.777  10.485  27.780  1.00 11.11        C
ATOM   2398   O    GLY A 169      15.483  11.623  28.208  1.00 10.34        O
ATOM   2399   N    ASN A 170      14.882   9.492  27.676  1.00 11.54        N
ATOM   2401   CA   ASN A 170      13.483   9.636  28.090  1.00 11.53        C
ATOM   2403   CB   ASN A 170      12.579   9.382  26.872  1.00 11.78        C
ATOM   2406   CG   ASN A 170      12.911  10.285  25.682  1.00 13.65        C
```

```
ATOM   2407  OD1 ASN A 170     13.358  11.427  25.856  1.00 14.26          O
ATOM   2408  ND2 ASN A 170     12.666   9.791  24.465  1.00 10.92          N
ATOM   2411  C   ASN A 170     13.116   8.658  29.184  1.00 12.69          C
ATOM   2412  O   ASN A 170     12.046   8.036  29.123  1.00 12.66          O
ATOM   2413  N   SER A 171     13.989   8.483  30.170  1.00 12.61          N
ATOM   2415  CA  SER A 171     13.754   7.487  31.223  1.00 13.48          C
ATOM   2417  CB  SER A 171     15.025   6.692  31.423  1.00 14.78          C
ATOM   2420  OG  SER A 171     15.277   5.967  30.233  1.00 13.11          O
ATOM   2422  C   SER A 171     13.308   8.147  32.538  1.00 13.22          C
ATOM   2423  O   SER A 171     13.429   7.526  33.583  1.00 14.40          O
ATOM   2424  N   GLY A 172     12.811   9.371  32.496  1.00 14.32          N
ATOM   2426  CA  GLY A 172     12.428  10.098  33.710  1.00 14.13          C
ATOM   2429  C   GLY A 172     11.127   9.606  34.292  1.00 15.66          C
ATOM   2430  O   GLY A 172     10.473   8.814  33.614  1.00 15.22          O
ATOM   2431  N   SER A 173     10.681  10.134  35.424  1.00 14.94          N
ATOM   2433  CA  SER A 173     11.269  11.278  36.134  1.00 16.57          C
ATOM   2435  CB  SER A 173     10.144  12.174  36.639  1.00 17.91          C
ATOM   2438  OG  SER A 173      9.384  11.435  37.607  1.00 18.04          O
ATOM   2440  C   SER A 173     12.196  10.908  37.265  1.00 16.47          C
ATOM   2441  O   SER A 173     12.751  11.790  37.970  1.00 15.55          O
ATOM   2442  N   GLY A 174     12.476   9.615  37.359  1.00 15.06          N
ATOM   2444  CA  GLY A 174     13.318   9.075  38.400  1.00 16.35          C
ATOM   2447  C   GLY A 174     14.715   9.629  38.233  1.00 17.64          C
ATOM   2448  O   GLY A 174     15.159   9.906  37.086  1.00 17.46          O
ATOM   2449  N   SER A 175     15.404   9.827  39.351  1.00 17.08          N
ATOM   2451  CA  SER A 175     16.752  10.404  39.336  1.00 18.22          C
ATOM   2453  CB  SER A 175     17.129  10.794  40.759  1.00 19.80          C
ATOM   2456  OG  SER A 175     16.121  11.654  41.308  1.00 21.20          O
ATOM   2458  C   SER A 175     17.783   9.457  38.777  1.00 16.85          C
ATOM   2459  O   SER A 175     17.638   8.238  38.884  1.00 15.37          O
ATOM   2460  N   ASN A 176     18.838  10.010  38.168  1.00 16.57          N
ATOM   2462  CA  ASN A 176     19.966   9.230  37.675  1.00 15.32          C
ATOM   2464  CB  ASN A 176     20.679   8.475  38.817  1.00 17.33          C
ATOM   2467  CG  ASN A 176     22.174   8.352  38.565  1.00 20.64          C
ATOM   2468  OD1 ASN A 176     22.676   9.003  37.649  1.00 19.65          O
ATOM   2469  ND2 ASN A 176     22.881   7.508  39.336  1.00 23.68          N
ATOM   2472  C   ASN A 176     19.634   8.250  36.592  1.00 15.22          C
ATOM   2473  O   ASN A 176     20.208   7.146  36.528  1.00 16.86          O
ATOM   2474  N   THR A 177     18.718   8.639  35.723  1.00 14.53          N
ATOM   2476  CA  THR A 177     18.299   7.815  34.612  1.00 14.30          C
ATOM   2478  CB  THR A 177     16.768   7.831  34.488  1.00 13.61          C
ATOM   2480  OG1 THR A 177     16.255   9.161  34.632  1.00 12.76          O
ATOM   2482  CG2 THR A 177     16.053   7.001  35.629  1.00 14.89          C
ATOM   2486  C   THR A 177     18.907   8.265  33.267  1.00 13.62          C
ATOM   2487  O   THR A 177     18.555   7.704  32.213  1.00 14.34          O
ATOM   2488  N   ILE A 178     19.736   9.305  33.324  1.00 12.33          N
ATOM   2490  CA  ILE A 178     20.436   9.809  32.125  1.00 10.85          C
ATOM   2492  CB  ILE A 178     21.473  10.886  32.543  1.00 11.28          C
ATOM   2494  CG1 ILE A 178     22.118  11.576  31.337  1.00 11.95          C
ATOM   2497  CD1 ILE A 178     22.981  12.833  31.722  1.00 12.82          C
ATOM   2501  CG2 ILE A 178     22.550  10.300  33.406  1.00 11.10          C
ATOM   2505  C   ILE A 178     21.057   8.663  31.350  1.00 10.78          C
ATOM   2506  O   ILE A 178     21.582   7.715  31.950  1.00 10.96          O
ATOM   2507  N   GLY A 179     20.973   8.706  30.032  1.00  9.07          N
ATOM   2509  CA  GLY A 179     21.658   7.732  29.200  1.00 10.43          C
ATOM   2512  C   GLY A 179     22.842   8.334  28.433  1.00 10.17          C
ATOM   2513  O   GLY A 179     23.302   9.423  28.716  1.00  9.05          O
ATOM   2514  N   PHE A 180     23.302   7.579  27.474  1.00 10.42          N
ATOM   2516  CA  PHE A 180     24.566   7.766  26.771  1.00  9.90          C
ATOM   2518  CB  PHE A 180     25.597   6.710  27.248  1.00 11.20          C
ATOM   2521  CG  PHE A 180     25.926   6.868  28.691  1.00 10.40          C
ATOM   2522  CD1 PHE A 180     25.089   6.304  29.673  1.00 12.43          C
ATOM   2524  CE1 PHE A 180     25.346   6.539  31.013  1.00 13.80          C
```

```
ATOM   2526  CZ   PHE A 180      26.377   7.353  31.379  1.00 14.77           C
ATOM   2528  CE2  PHE A 180      27.195   7.936  30.428  1.00 14.15           C
ATOM   2530  CD2  PHE A 180      26.951   7.710  29.086  1.00 12.50           C
ATOM   2532  C    PHE A 180      24.307   7.663  25.268  1.00 10.49           C
ATOM   2533  O    PHE A 180      23.545   6.804  24.833  1.00 11.55           O
ATOM   2534  N    PRO A 181      25.023   8.448  24.458  1.00  9.50           N
ATOM   2535  CA   PRO A 181      26.196   9.246  24.890  1.00  9.13           C
ATOM   2537  CB   PRO A 181      26.937   9.496  23.524  1.00  9.24           C
ATOM   2540  CG   PRO A 181      25.855   9.591  22.649  1.00 11.31           C
ATOM   2543  CD   PRO A 181      24.874   8.496  23.002  1.00 10.03           C
ATOM   2546  C    PRO A 181      25.983  10.570  25.610  1.00 10.79           C
ATOM   2547  O    PRO A 181      26.959  11.251  25.986  1.00  9.76           O
ATOM   2548  N    GLY A 182      24.743  11.019  25.752  1.00 10.07           N
ATOM   2550  CA   GLY A 182      24.480  12.260  26.450  1.00 10.91           C
ATOM   2553  C    GLY A 182      25.260  12.460  27.748  1.00 10.65           C
ATOM   2554  O    GLY A 182      25.843  13.532  27.983  1.00 10.24           O
ATOM   2555  N    GLY A 183      25.246  11.400  28.570  1.00 10.23           N
ATOM   2557  CA   GLY A 183      25.860  11.370  29.888  1.00 11.34           C
ATOM   2560  C    GLY A 183      27.370  11.393  29.929  1.00 11.75           C
ATOM   2561  O    GLY A 183      27.957  11.394  31.025  1.00 10.33           O
ATOM   2562  N    LEU A 184      28.007  11.351  28.761  1.00 11.62           N
ATOM   2564  CA   LEU A 184      29.474  11.436  28.718  1.00 11.77           C
ATOM   2566  CB   LEU A 184      30.022  11.038  27.369  1.00 10.76           C
ATOM   2569  CG   LEU A 184      29.612   9.640  26.946  1.00 13.28           C
ATOM   2571  CD1  LEU A 184      29.958   9.447  25.484  1.00 13.01           C
ATOM   2575  CD2  LEU A 184      30.306   8.563  27.839  1.00 15.87           C
ATOM   2579  C    LEU A 184      29.966  12.827  29.034  1.00 11.00           C
ATOM   2580  O    LEU A 184      29.321  13.827  28.682  1.00 10.77           O
ATOM   2581  N    VAL A 185      31.180  12.922  29.584  1.00 11.17           N
ATOM   2583  CA   VAL A 185      31.673  14.225  29.979  1.00 10.77           C
ATOM   2585  CB   VAL A 185      32.994  14.115  30.811  1.00 13.00           C
ATOM   2587  CG1  VAL A 185      33.978  13.569  30.013  1.00 16.83           C
ATOM   2591  CG2  VAL A 185      33.500  15.504  31.144  1.00 16.03           C
ATOM   2595  C    VAL A 185      31.868  15.172  28.842  1.00  9.95           C
ATOM   2596  O    VAL A 185      31.683  16.370  28.972  1.00 10.97           O
ATOM   2597  N    ASN A 186      32.181  14.614  27.684  1.00 10.43           N
ATOM   2599  CA   ASN A 186      32.483  15.376  26.517  1.00 11.79           C
ATOM   2601  CB   ASN A 186      33.763  14.865  25.836  1.00 13.55           C
ATOM   2604  CG   ASN A 186      35.029  15.152  26.660  1.00 14.82           C
ATOM   2605  OD1  ASN A 186      35.093  16.110  27.370  1.00 19.19           O
ATOM   2606  ND2  ASN A 186      36.021  14.326  26.517  1.00 21.64           N
ATOM   2609  C    ASN A 186      31.305  15.525  25.536  1.00 11.85           C
ATOM   2610  O    ASN A 186      31.485  15.915  24.384  1.00 11.39           O
ATOM   2611  N    ALA A 187      30.108  15.138  25.977  1.00 10.51           N
ATOM   2613  CA   ALA A 187      28.904  15.382  25.179  1.00 11.08           C
ATOM   2615  CB   ALA A 187      28.189  14.150  24.848  1.00 11.19           C
ATOM   2619  C    ALA A 187      27.984  16.317  25.975  1.00 10.66           C
ATOM   2620  O    ALA A 187      27.878  16.147  27.186  1.00 11.05           O
ATOM   2621  N    VAL A 188      27.318  17.248  25.288  1.00  9.26           N
ATOM   2623  CA   VAL A 188      26.326  18.148  25.895  1.00  9.21           C
ATOM   2625  CB   VAL A 188      26.120  19.418  25.046  1.00  9.41           C
ATOM   2627  CG1  VAL A 188      25.035  20.249  25.661  1.00  7.57           C
ATOM   2631  CG2  VAL A 188      27.448  20.164  24.893  1.00 10.01           C
ATOM   2635  C    VAL A 188      24.996  17.346  25.984  1.00  9.78           C
ATOM   2636  O    VAL A 188      24.349  17.137  24.959  1.00 10.45           O
ATOM   2637  N    ALA A 189      24.572  16.989  27.200  1.00  8.67           N
ATOM   2639  CA   ALA A 189      23.325  16.236  27.430  1.00  9.35           C
ATOM   2641  CB   ALA A 189      23.379  15.510  28.763  1.00 10.27           C
ATOM   2645  C    ALA A 189      22.197  17.214  27.451  1.00  8.43           C
ATOM   2646  O    ALA A 189      22.179  18.183  28.238  1.00  9.35           O
ATOM   2647  N    VAL A 190      21.182  16.948  26.651  1.00  9.14           N
ATOM   2649  CA   VAL A 190      20.084  17.882  26.554  1.00  8.32           C
ATOM   2651  CB   VAL A 190      19.843  18.296  25.119  1.00  8.58           C
```

```
ATOM   2653  CG1 VAL A 190     18.731  19.309  25.052  1.00 11.50          C
ATOM   2657  CG2 VAL A 190     21.084  18.873  24.482  1.00  9.20          C
ATOM   2661  C   VAL A 190     18.791  17.317  27.087  1.00  9.51          C
ATOM   2662  O   VAL A 190     18.340  16.256  26.625  1.00  9.51          O
ATOM   2663  N   ALA A 191     18.236  17.973  28.093  1.00  9.50          N
ATOM   2665  CA  ALA A 191     16.934  17.559  28.685  1.00 10.18          C
ATOM   2667  CB  ALA A 191     16.868  18.057  30.134  1.00  8.69          C
ATOM   2671  C   ALA A 191     15.800  18.184  27.900  1.00 10.24          C
ATOM   2672  O   ALA A 191     16.007  19.182  27.249  1.00 10.28          O
ATOM   2673  N   ALA A 192     14.570  17.659  28.021  1.00 11.22          N
ATOM   2675  CA  ALA A 192     13.428  18.171  27.272  1.00 11.79          C
ATOM   2677  CB  ALA A 192     12.593  17.012  26.728  1.00 13.73          C
ATOM   2681  C   ALA A 192     12.499  19.027  28.134  1.00 11.54          C
ATOM   2682  O   ALA A 192     12.048  18.549  29.179  1.00 11.30          O
ATOM   2683  N   LEU A 193     12.222  20.238  27.673  1.00 11.58          N
ATOM   2685  CA  LEU A 193     11.194  21.103  28.258  1.00 11.55          C
ATOM   2687  CB  LEU A 193     11.519  22.561  28.037  1.00 12.06          C
ATOM   2690  CG  LEU A 193     12.844  23.095  28.613  1.00 10.40          C
ATOM   2692  CD1 LEU A 193     13.137  24.484  28.211  1.00  9.32          C
ATOM   2696  CD2 LEU A 193     12.752  22.903  30.081  1.00 13.29          C
ATOM   2700  C   LEU A 193      9.852  20.802  27.577  1.00 14.05          C
ATOM   2701  O   LEU A 193      9.814  20.460  26.414  1.00 13.73          O
ATOM   2702  N   GLU A 194      8.755  21.004  28.305  1.00 13.56          N
ATOM   2704  CA  GLU A 194      7.422  21.049  27.647  1.00 13.63          C
ATOM   2706  CB  GLU A 194      6.472  20.125  28.359  1.00 12.74          C
ATOM   2709  CG  GLU A 194      6.320  20.410  29.837  1.00 16.62          C
ATOM   2712  CD  GLU A 194      5.490  19.404  30.603  1.00 19.10          C
ATOM   2713  OE1 GLU A 194      5.288  18.280  30.118  1.00 20.53          O
ATOM   2714  OE2 GLU A 194      5.153  19.744  31.765  1.00 17.20          O
ATOM   2715  C   GLU A 194      6.934  22.460  27.699  1.00 14.48          C
ATOM   2716  O   GLU A 194      7.502  23.275  28.431  1.00 13.32          O
ATOM   2717  N   ASN A 195      5.862  22.778  26.954  1.00 14.74          N
ATOM   2719  CA  ASN A 195      5.416  24.169  26.857  1.00 15.00          C
ATOM   2721  CB  ASN A 195      4.773  24.467  25.479  1.00 16.14          C
ATOM   2724  CG  ASN A 195      4.612  25.970  25.198  1.00 16.46          C
ATOM   2725  OD1 ASN A 195      5.236  26.803  25.855  1.00 14.84          O
ATOM   2726  ND2 ASN A 195      3.775  26.318  24.208  1.00 13.91          N
ATOM   2729  C   ASN A 195      4.439  24.440  27.945  1.00 16.03          C
ATOM   2730  O   ASN A 195      3.256  24.692  27.662  1.00 15.98          O
ATOM   2731  N   VAL A 196      4.904  24.381  29.170  1.00 16.13          N
ATOM   2733  CA  VAL A 196      4.106  24.623  30.344  1.00 16.89          C
ATOM   2735  CB  VAL A 196      3.739  23.348  31.019  1.00 18.38          C
ATOM   2737  CG1 VAL A 196      3.058  23.613  32.326  1.00 20.19          C
ATOM   2741  CG2 VAL A 196      2.922  22.415  30.070  1.00 18.32          C
ATOM   2745  C   VAL A 196      4.991  25.380  31.307  1.00 17.79          C
ATOM   2746  O   VAL A 196      6.215  25.147  31.344  1.00 17.15          O
ATOM   2747  N   GLN A 197      4.410  26.305  32.055  1.00 17.34          N
ATOM   2749  CA  GLN A 197      5.171  27.048  33.060  1.00 16.33          C
ATOM   2751  CB  GLN A 197      4.838  28.518  33.012  1.00 16.74          C
ATOM   2754  CG  GLN A 197      4.987  29.169  31.720  1.00 16.89          C
ATOM   2757  CD  GLN A 197      6.455  29.357  31.343  1.00 18.48          C
ATOM   2758  OE1 GLN A 197      7.216  30.009  32.096  1.00 14.66          O
ATOM   2759  NE2 GLN A 197      6.850  28.769  30.223  1.00 15.37          N
ATOM   2762  C   GLN A 197      4.907  26.538  34.467  1.00 17.79          C
ATOM   2763  O   GLN A 197      3.778  26.114  34.825  1.00 18.50          O
ATOM   2764  N   GLN A 198      5.977  26.409  35.232  1.00 17.07          N
ATOM   2766  CA  GLN A 198      5.879  25.977  36.627  1.00 17.39          C
ATOM   2768  CB  GLN A 198      5.865  24.485  36.778  1.00 17.84          C
ATOM   2771  CG  GLN A 198      5.744  24.058  38.164  1.00 18.38          C
ATOM   2774  CD  GLN A 198      5.797  22.558  38.413  1.00 25.24          C
ATOM   2775  OE1 GLN A 198      6.612  21.813  37.815  1.00 26.03          O
ATOM   2776  NE2 GLN A 198      4.927  22.090  39.323  1.00 29.00          N
ATOM   2779  C   GLN A 198      6.998  26.623  37.362  1.00 17.51          C
```

```
ATOM   2780  O    GLN A 198       8.156  26.681  36.904  1.00 16.23           O
ATOM   2781  N    ASN A 199       6.655  27.147  38.520  1.00 18.31           N
ATOM   2783  CA   ASN A 199       7.612  27.890  39.321  1.00 19.42           C
ATOM   2785  CB   ASN A 199       8.676  26.953  39.915  1.00 19.53           C
ATOM   2788  CG   ASN A 199       8.107  25.949  40.861  1.00 24.01           C
ATOM   2789  OD1  ASN A 199       7.226  26.254  41.691  1.00 23.71           O
ATOM   2790  ND2  ASN A 199       8.598  24.738  40.769  1.00 25.09           N
ATOM   2793  C    ASN A 199       8.285  29.018  38.592  1.00 20.51           C
ATOM   2794  O    ASN A 199       9.491  29.356  38.863  1.00 20.07           O
ATOM   2795  N    GLY A 200       7.533  29.653  37.712  1.00 19.65           N
ATOM   2797  CA   GLY A 200       8.005  30.820  36.991  1.00 20.16           C
ATOM   2800  C    GLY A 200       8.883  30.609  35.774  1.00 19.26           C
ATOM   2801  O    GLY A 200       9.347  31.577  35.177  1.00 18.58           O
ATOM   2802  N    THR A 201       9.091  29.348  35.384  1.00 17.29           N
ATOM   2804  CA   THR A 201       9.876  29.045  34.196  1.00 16.82           C
ATOM   2806  CB   THR A 201      11.327  28.563  34.596  1.00 16.41           C
ATOM   2808  OG1  THR A 201      11.309  27.246  35.174  1.00 17.63           O
ATOM   2810  CG2  THR A 201      11.954  29.437  35.622  1.00 17.27           C
ATOM   2814  C    THR A 201       9.248  27.935  33.389  1.00 15.50           C
ATOM   2815  O    THR A 201       8.267  27.356  33.817  1.00 14.83           O
ATOM   2816  N    TYR A 202       9.845  27.608  32.230  1.00 15.66           N
ATOM   2818  CA   TYR A 202       9.469  26.407  31.571  1.00 14.51           C
ATOM   2820  CB   TYR A 202      10.308  26.183  30.300  1.00 15.38           C
ATOM   2823  CG   TYR A 202       9.853  27.053  29.177  1.00 12.00           C
ATOM   2824  CD1  TYR A 202       8.775  26.682  28.359  1.00 12.96           C
ATOM   2826  CE1  TYR A 202       8.344  27.510  27.364  1.00 13.55           C
ATOM   2828  CZ   TYR A 202       8.995  28.682  27.157  1.00 12.70           C
ATOM   2829  OH   TYR A 202       8.586  29.559  26.172  1.00 14.68           O
ATOM   2831  CE2  TYR A 202      10.027  29.063  27.961  1.00 14.77           C
ATOM   2833  CD2  TYR A 202      10.441  28.245  28.957  1.00 13.26           C
ATOM   2835  C    TYR A 202       9.637  25.229  32.488  1.00 14.45           C
ATOM   2836  O    TYR A 202      10.442  25.231  33.415  1.00 14.03           O
ATOM   2837  N    ARG A 203       8.894  24.168  32.206  1.00 14.53           N
ATOM   2839  CA   ARG A 203       8.939  22.971  32.988  1.00 13.25           C
ATOM   2841  CB   ARG A 203       7.454  22.543  33.262  1.00 14.52           C
ATOM   2844  CG   ARG A 203       7.315  21.347  34.102  1.00 14.19           C
ATOM   2847  CD   ARG A 203       5.795  21.083  34.523  1.00 15.99           C
ATOM   2850  NE   ARG A 203       5.730  20.106  35.572  1.00 17.45           N
ATOM   2852  CZ   ARG A 203       5.729  18.806  35.402  1.00 17.33           C
ATOM   2853  NH1  ARG A 203       5.762  18.306  34.191  1.00 15.77           N
ATOM   2856  NH2  ARG A 203       5.741  17.996  36.447  1.00 18.53           N
ATOM   2859  C    ARG A 203       9.595  21.805  32.251  1.00 12.75           C
ATOM   2860  O    ARG A 203       9.285  21.570  31.076  1.00 12.82           O
ATOM   2861  N    VAL A 204      10.505  21.090  32.907  1.00 12.18           N
ATOM   2863  CA   VAL A 204      11.043  19.877  32.378  1.00 11.32           C
ATOM   2865  CB   VAL A 204      12.141  19.338  33.296  1.00 11.73           C
ATOM   2867  CG1  VAL A 204      12.765  18.035  32.735  1.00 11.27           C
ATOM   2871  CG2  VAL A 204      13.229  20.373  33.437  1.00 12.71           C
ATOM   2875  C    VAL A 204       9.925  18.831  32.221  1.00 12.28           C
ATOM   2876  O    VAL A 204       9.172  18.549  33.185  1.00 12.08           O
ATOM   2877  N    ALA A 205       9.855  18.166  31.063  1.00 12.11           N
ATOM   2879  CA   ALA A 205       8.882  17.061  30.935  1.00 12.63           C
ATOM   2881  CB   ALA A 205       8.725  16.612  29.473  1.00 14.20           C
ATOM   2885  C    ALA A 205       9.205  15.894  31.835  1.00 14.44           C
ATOM   2886  O    ALA A 205      10.338  15.546  32.067  1.00 12.72           O
ATOM   2887  N    ASP A 206       8.160  15.251  32.353  1.00 12.46           N
ATOM   2889  CA   ASP A 206       8.351  14.138  33.226  1.00 14.42           C
ATOM   2891  CB   ASP A 206       7.015  13.579  33.660  1.00 15.15           C
ATOM   2894  CG   ASP A 206       6.273  14.456  34.620  1.00 20.28           C
ATOM   2895  OD1  ASP A 206       6.717  15.552  35.015  1.00 16.78           O
ATOM   2896  OD2  ASP A 206       5.164  14.025  35.032  1.00 21.17           O
ATOM   2897  C    ASP A 206       9.161  13.020  32.556  1.00 13.89           C
ATOM   2898  O    ASP A 206       9.920  12.348  33.229  1.00 15.04           O
```

```
ATOM   2899   N    PHE A 207      9.016  12.821  31.246  1.00  11.78           N
ATOM   2901   CA   PHE A 207      9.710  11.721  30.612  1.00  13.96           C
ATOM   2903   CB   PHE A 207      9.163  11.360  29.213  1.00  13.87           C
ATOM   2906   CG   PHE A 207      9.290  12.439  28.191  1.00  14.05           C
ATOM   2907   CD1  PHE A 207     10.521  12.704  27.630  1.00  13.46           C
ATOM   2909   CE1  PHE A 207     10.677  13.677  26.709  1.00  14.99           C
ATOM   2911   CZ   PHE A 207      9.577  14.463  26.305  1.00  12.88           C
ATOM   2913   CE2  PHE A 207      8.325  14.173  26.841  1.00  15.57           C
ATOM   2915   CD2  PHE A 207      8.199  13.191  27.787  1.00  14.48           C
ATOM   2917   C    PHE A 207     11.220  11.917  30.546  1.00  12.81           C
ATOM   2918   O    PHE A 207     11.950  10.945  30.339  1.00  12.43           O
ATOM   2919   N    SER A 208     11.670  13.163  30.626  1.00  13.24           N
ATOM   2921   CA   SER A 208     13.099  13.459  30.389  1.00  12.69           C
ATOM   2923   CB   SER A 208     13.277  14.980  30.333  1.00  13.69           C
ATOM   2926   OG   SER A 208     14.593  15.399  30.016  1.00  11.00           O
ATOM   2928   C    SER A 208     13.997  12.799  31.432  1.00  11.85           C
ATOM   2929   O    SER A 208     13.726  12.841  32.612  1.00  12.56           O
ATOM   2930   N    SER A 209     15.095  12.168  31.001  1.00  11.34           N
ATOM   2932   CA   SER A 209     15.961  11.503  31.941  1.00  12.26           C
ATOM   2934   CB   SER A 209     17.003  10.655  31.240  1.00  10.68           C
ATOM   2937   OG   SER A 209     16.442   9.566  30.515  1.00  11.25           O
ATOM   2939   C    SER A 209     16.666  12.506  32.852  1.00  11.42           C
ATOM   2940   O    SER A 209     17.108  13.568  32.420  1.00  12.21           O
ATOM   2941   N    ARG A 210     16.797  12.128  34.107  1.00  13.04           N
ATOM   2943   CA   ARG A 210     17.480  12.973  35.089  1.00  12.02           C
ATOM   2945   CB   ARG A 210     16.783  12.925  36.439  1.00  11.62           C
ATOM   2948   CG   ARG A 210     15.644  13.914  36.659  1.00  10.95           C
ATOM   2951   CD   ARG A 210     14.531  13.936  35.593  1.00  12.61           C
ATOM   2954   NE   ARG A 210     13.496  14.936  35.948  1.00  13.59           N
ATOM   2956   CZ   ARG A 210     12.450  15.250  35.214  1.00  14.23           C
ATOM   2957   NH1  ARG A 210     12.267  14.655  34.035  1.00  14.26           N
ATOM   2960   NH2  ARG A 210     11.550  16.168  35.652  1.00  13.18           N
ATOM   2963   C    ARG A 210     18.906  12.544  35.328  1.00  11.40           C
ATOM   2964   O    ARG A 210     19.201  11.401  35.281  1.00  12.42           O
ATOM   2965   N    GLY A 211     19.756  13.520  35.625  1.00  13.31           N
ATOM   2967   CA   GLY A 211     21.140  13.289  35.977  1.00  11.79           C
ATOM   2970   C    GLY A 211     21.263  12.757  37.395  1.00  13.79           C
ATOM   2971   O    GLY A 211     20.286  12.467  38.054  1.00  13.33           O
ATOM   2972   N    ASN A 212     22.508  12.644  37.831  1.00  12.68           N
ATOM   2974   CA   ASN A 212     22.852  12.132  39.139  1.00  13.86           C
ATOM   2976   CB   ASN A 212     24.300  11.647  39.062  1.00  14.05           C
ATOM   2979   CG   ASN A 212     24.801  11.076  40.380  1.00  15.77           C
ATOM   2980   OD1  ASN A 212     24.034  10.961  41.330  1.00  20.22           O
ATOM   2981   ND2  ASN A 212     26.057  10.638  40.402  1.00  21.14           N
ATOM   2984   C    ASN A 212     22.711  13.289  40.154  1.00  13.72           C
ATOM   2985   O    ASN A 212     23.466  14.254  40.141  1.00  13.40           O
ATOM   2986   N    PRO A 213     21.803  13.173  41.121  1.00  14.98           N
ATOM   2987   CA   PRO A 213     21.672  14.248  42.125  1.00  16.29           C
ATOM   2989   CB   PRO A 213     20.586  13.722  43.065  1.00  17.58           C
ATOM   2992   CG   PRO A 213     19.803  12.803  42.224  1.00  16.96           C
ATOM   2995   CD   PRO A 213     20.863  12.072  41.386  1.00  15.73           C
ATOM   2998   C    PRO A 213     22.966  14.577  42.864  1.00  16.46           C
ATOM   2999   O    PRO A 213     23.194  15.766  43.089  1.00  18.16           O
ATOM   3000   N    ALA A 214     23.809  13.573  43.084  1.00  17.81           N
ATOM   3002   CA   ALA A 214     25.058  13.706  43.843  1.00  18.79           C
ATOM   3004   CB   ALA A 214     25.746  12.336  44.004  1.00  19.37           C
ATOM   3008   C    ALA A 214     26.022  14.653  43.188  1.00  18.92           C
ATOM   3009   O    ALA A 214     26.872  15.225  43.890  1.00  17.86           O
ATOM   3010   N    THR A 215     25.899  14.879  41.869  1.00  17.03           N
ATOM   3012   CA   THR A 215     26.868  15.751  41.205  1.00  16.66           C
ATOM   3014   CB   THR A 215     27.741  14.942  40.212  1.00  18.38           C
ATOM   3016   OG1  THR A 215     26.907  14.218  39.271  1.00  15.43           O
ATOM   3018   CG2  THR A 215     28.532  13.928  40.970  1.00  18.05           C
```

```
ATOM   3022  C    THR A 215     26.278  16.952  40.479  1.00  16.40           C
ATOM   3023  O    THR A 215     26.955  17.636  39.745  1.00  16.41           O
ATOM   3024  N    ALA A 216     25.035  17.244  40.773  1.00  14.91           N
ATOM   3026  CA   ALA A 216     24.408  18.446  40.249  1.00  16.14           C
ATOM   3028  CB   ALA A 216     22.944  18.182  39.938  1.00  16.49           C
ATOM   3032  C    ALA A 216     24.479  19.548  41.271  1.00  17.73           C
ATOM   3033  O    ALA A 216     24.240  19.279  42.445  1.00  21.27           O
ATOM   3034  N    GLY A 217     24.699  20.763  40.840  1.00  16.83           N
ATOM   3036  CA   GLY A 217     24.701  21.920  41.725  1.00  17.35           C
ATOM   3039  C    GLY A 217     25.994  22.704  41.603  1.00  18.14           C
ATOM   3040  O    GLY A 217     26.068  23.906  41.983  1.00  19.91           O
ATOM   3041  N    ASP A 218     27.007  22.103  41.001  1.00  17.40           N
ATOM   3043  CA   ASP A 218     28.294  22.767  40.941  1.00  16.68           C
ATOM   3045  CB   ASP A 218     29.346  21.732  41.259  1.00  17.36           C
ATOM   3048  CG   ASP A 218     29.393  20.585  40.244  1.00  20.24           C
ATOM   3049  OD1  ASP A 218     28.520  20.437  39.333  1.00  17.05           O
ATOM   3050  OD2  ASP A 218     30.286  19.734  40.356  1.00  20.78           O
ATOM   3051  C    ASP A 218     28.648  23.526  39.664  1.00  15.65           C
ATOM   3052  O    ASP A 218     29.638  24.231  39.613  1.00  15.75           O
ATOM   3053  N    TYR A 219     27.756  23.522  38.689  1.00  15.41           N
ATOM   3055  CA   TYR A 219     28.003  24.099  37.380  1.00  13.99           C
ATOM   3057  CB   TYR A 219     27.987  25.607  37.463  1.00  14.94           C
ATOM   3060  CG   TYR A 219     26.611  26.197  37.674  1.00  15.83           C
ATOM   3061  CD1  TYR A 219     25.642  26.086  36.702  1.00  12.98           C
ATOM   3063  CE1  TYR A 219     24.385  26.648  36.871  1.00  12.57           C
ATOM   3065  CZ   TYR A 219     24.125  27.301  38.052  1.00  18.74           C
ATOM   3066  OH   TYR A 219     22.947  27.921  38.286  1.00  18.11           O
ATOM   3068  CE2  TYR A 219     25.090  27.438  39.021  1.00  19.74           C
ATOM   3070  CD2  TYR A 219     26.309  26.869  38.843  1.00  19.78           C
ATOM   3072  C    TYR A 219     29.346  23.584  36.756  1.00  14.17           C
ATOM   3073  O    TYR A 219     29.978  24.283  35.984  1.00  14.67           O
ATOM   3074  N    ILE A 220     29.676  22.330  37.031  1.00  13.56           N
ATOM   3076  CA   ILE A 220     30.775  21.624  36.414  1.00  15.58           C
ATOM   3078  CB   ILE A 220     31.961  21.438  37.355  1.00  15.45           C
ATOM   3080  CG1  ILE A 220     32.502  22.809  37.761  1.00  20.96           C
ATOM   3083  CD1  ILE A 220     33.397  22.711  39.015  1.00  23.15           C
ATOM   3087  CG2  ILE A 220     33.087  20.625  36.671  1.00  18.47           C
ATOM   3091  C    ILE A 220     30.227  20.278  35.956  1.00  13.00           C
ATOM   3092  O    ILE A 220     29.633  19.505  36.691  1.00  12.94           O
ATOM   3093  N    ILE A 221     30.482  19.995  34.684  1.00  15.43           N
ATOM   3095  CA   ILE A 221     29.934  18.782  34.088  1.00  13.93           C
ATOM   3097  CB   ILE A 221     29.793  18.960  32.541  1.00  12.79           C
ATOM   3099  CG1  ILE A 221     28.733  19.979  32.164  1.00  12.82           C
ATOM   3102  CD1  ILE A 221     27.361  19.711  32.673  1.00  12.70           C
ATOM   3106  CG2  ILE A 221     29.513  17.651  31.889  1.00  14.76           C
ATOM   3110  C    ILE A 221     30.836  17.584  34.406  1.00  14.63           C
ATOM   3111  O    ILE A 221     32.059  17.583  34.006  1.00  14.50           O
ATOM   3112  N    GLN A 222     30.246  16.602  35.097  1.00  13.31           N
ATOM   3114  CA   GLN A 222     30.786  15.267  35.285  1.00  14.28           C
ATOM   3116  CB   GLN A 222     30.772  14.858  36.759  1.00  16.33           C
ATOM   3119  CG   GLN A 222     31.775  15.669  37.602  1.00  18.83           C
ATOM   3122  CD   GLN A 222     31.204  16.936  38.223  1.00  21.01           C
ATOM   3123  OE1  GLN A 222     29.998  17.058  38.469  1.00  22.83           O
ATOM   3124  NE2  GLN A 222     32.089  17.874  38.517  1.00  23.39           N
ATOM   3127  C    GLN A 222     29.935  14.289  34.460  1.00  14.17           C
ATOM   3128  O    GLN A 222     28.921  14.664  33.874  1.00  12.09           O
ATOM   3129  N    GLU A 223     30.438  13.079  34.313  1.00  13.56           N
ATOM   3131  CA   GLU A 223     29.646  12.052  33.675  1.00  14.32           C
ATOM   3133  CB   GLU A 223     30.455  10.767  33.682  1.00  14.62           C
ATOM   3136  CG   GLU A 223     29.787   9.618  32.984  1.00  15.34           C
ATOM   3139  CD   GLU A 223     30.759   8.474  32.706  1.00  16.41           C
ATOM   3140  OE1  GLU A 223     31.400   8.407  31.648  1.00  19.83           O
ATOM   3141  OE2  GLU A 223     30.808   7.645  33.564  1.00  19.76           O
```

```
ATOM   3142  C    GLU A 223    28.315  11.911  34.451  1.00  12.77         C
ATOM   3143  O    GLU A 223    28.294  11.981  35.679  1.00  13.97         O
ATOM   3144  N    ARG A 224    27.210  11.785  33.700  1.00  11.67         N
ATOM   3146  CA   ARG A 224    25.834  11.585  34.178  1.00  11.39         C
ATOM   3148  CB   ARG A 224    25.730  10.549  35.305  1.00  12.52         C
ATOM   3151  CG   ARG A 224    26.269   9.156  34.960  1.00  16.20         C
ATOM   3154  CD   ARG A 224    25.988   8.116  36.088  1.00  19.39         C
ATOM   3157  NE   ARG A 224    24.589   7.737  36.052  1.00  19.97         N
ATOM   3159  CZ   ARG A 224    24.123   6.785  35.271  1.00  23.20         C
ATOM   3160  NH1  ARG A 224    24.939   6.070  34.516  1.00  22.67         N
ATOM   3163  NH2  ARG A 224    22.835   6.530  35.234  1.00  20.20         N
ATOM   3166  C    ARG A 224    25.199  12.934  34.600  1.00  11.27         C
ATOM   3167  O    ARG A 224    24.137  12.954  35.247  1.00  12.76         O
ATOM   3168  N    ASP A 225    25.779  14.065  34.167  1.00  10.64         N
ATOM   3170  CA   ASP A 225    25.141  15.322  34.440  1.00  11.57         C
ATOM   3172  CB   ASP A 225    26.137  16.421  34.763  1.00  12.18         C
ATOM   3175  CG   ASP A 225    26.783  16.290  36.115  1.00  11.47         C
ATOM   3176  OD1  ASP A 225    26.396  15.431  36.930  1.00  12.63         O
ATOM   3177  OD2  ASP A 225    27.738  17.024  36.366  1.00  12.98         O
ATOM   3178  C    ASP A 225    24.386  15.833  33.187  1.00  11.72         C
ATOM   3179  O    ASP A 225    24.880  15.761  32.028  1.00  10.65         O
ATOM   3180  N    ILE A 226    23.195  16.366  33.428  1.00  10.89         N
ATOM   3182  CA   ILE A 226    22.449  17.089  32.399  1.00  10.95         C
ATOM   3184  CB   ILE A 226    20.988  17.379  32.826  1.00  10.97         C
ATOM   3186  CG1  ILE A 226    20.296  16.066  33.224  1.00   8.65         C
ATOM   3189  CD1  ILE A 226    20.064  15.192  32.004  1.00  12.39         C
ATOM   3193  CG2  ILE A 226    20.260  18.217  31.828  1.00  12.31         C
ATOM   3197  C    ILE A 226    23.117  18.428  32.195  1.00   8.74         C
ATOM   3198  O    ILE A 226    23.561  19.076  33.123  1.00  10.81         O
ATOM   3199  N    GLU A 227    23.254  18.849  30.971  1.00   9.24         N
ATOM   3201  CA   GLU A 227    23.902  20.150  30.715  1.00   9.53         C
ATOM   3203  CB   GLU A 227    24.905  19.979  29.553  1.00   8.94         C
ATOM   3206  CG   GLU A 227    25.869  21.131  29.345  1.00   9.79         C
ATOM   3209  CD   GLU A 227    27.217  20.634  28.789  1.00  11.40         C
ATOM   3210  OE1  GLU A 227    27.527  19.431  28.857  1.00   8.56         O
ATOM   3211  OE2  GLU A 227    27.953  21.441  28.255  1.00  11.05         O
ATOM   3212  C    GLU A 227    22.936  21.331  30.450  1.00   9.10         C
ATOM   3213  O    GLU A 227    23.053  22.402  31.074  1.00  10.35         O
ATOM   3214  N    VAL A 228    21.984  21.152  29.553  1.00   8.61         N
ATOM   3216  CA   VAL A 228    21.094  22.222  29.176  1.00   7.54         C
ATOM   3218  CB   VAL A 228    21.567  23.010  27.946  1.00   5.96         C
ATOM   3220  CG1  VAL A 228    22.834  23.726  28.263  1.00   8.28         C
ATOM   3224  CG2  VAL A 228    21.680  22.127  26.740  1.00   8.08         C
ATOM   3228  C    VAL A 228    19.727  21.575  28.904  1.00   7.83         C
ATOM   3229  O    VAL A 228    19.663  20.362  28.747  1.00   9.43         O
ATOM   3230  N    SER A 229    18.716  22.426  28.809  1.00  10.13         N
ATOM   3232  CA   SER A 229    17.369  22.024  28.520  1.00   9.88         C
ATOM   3234  CB   SER A 229    16.457  22.371  29.674  1.00  10.86         C
ATOM   3237  OG   SER A 229    16.811  21.670  30.827  1.00  11.90         O
ATOM   3239  C    SER A 229    16.865  22.806  27.305  1.00  11.07         C
ATOM   3240  O    SER A 229    17.290  23.949  27.065  1.00  11.59         O
ATOM   3241  N    ALA A 230    15.964  22.167  26.536  1.00  10.22         N
ATOM   3243  CA   ALA A 230    15.337  22.824  25.379  1.00   9.98         C
ATOM   3245  CB   ALA A 230    16.245  22.786  24.202  1.00  11.98         C
ATOM   3249  C    ALA A 230    14.001  22.181  25.011  1.00  10.40         C
ATOM   3250  O    ALA A 230    13.639  21.143  25.539  1.00  11.74         O
ATOM   3251  N    PRO A 231    13.241  22.873  24.162  1.00  12.52         N
ATOM   3252  CA   PRO A 231    11.915  22.369  23.794  1.00  11.45         C
ATOM   3254  CB   PRO A 231    11.440  23.308  22.676  1.00  12.82         C
ATOM   3257  CG   PRO A 231    12.076  24.609  23.005  1.00  12.47         C
ATOM   3260  CD   PRO A 231    13.518  24.184  23.540  1.00  12.90         C
ATOM   3263  C    PRO A 231    11.967  20.969  23.241  1.00  10.90         C
ATOM   3264  O    PRO A 231    12.689  20.713  22.238  1.00  11.39         O
```

```
ATOM   3265  N   GLY A 232      11.194  20.071  23.863  1.00 11.69           N
ATOM   3267  CA  GLY A 232      11.218  18.675  23.478  1.00 12.12           C
ATOM   3270  C   GLY A 232       9.857  17.972  23.431  1.00 14.63           C
ATOM   3271  O   GLY A 232       9.814  16.852  22.976  1.00 17.38           O
ATOM   3272  N   ALA A 233       8.775  18.641  23.807  1.00 13.80           N
ATOM   3274  CA  ALA A 233       7.441  17.975  23.841  1.00 15.69           C
ATOM   3276  CB  ALA A 233       6.812  18.002  25.228  1.00 15.50           C
ATOM   3280  C   ALA A 233       6.565  18.649  22.794  1.00 15.70           C
ATOM   3281  O   ALA A 233       6.479  19.892  22.725  1.00 15.71           O
ATOM   3282  N   SER A 234       6.008  17.837  21.901  1.00 15.70           N
ATOM   3284  CA  SER A 234       5.123  18.323  20.820  1.00 17.34           C
ATOM   3286  CB  SER A 234       3.816  18.866  21.396  1.00 19.34           C
ATOM   3289  OG  SER A 234       3.151  17.862  22.071  1.00 24.09           O
ATOM   3291  C   SER A 234       5.746  19.302  19.869  1.00 16.08           C
ATOM   3292  O   SER A 234       5.311  20.419  19.727  1.00 16.37           O
ATOM   3293  N   VAL A 235       6.816  18.859  19.244  1.00 13.70           N
ATOM   3295  CA  VAL A 235       7.597  19.607  18.309  1.00 13.30           C
ATOM   3297  CB  VAL A 235       9.124  19.358  18.536  1.00 11.29           C
ATOM   3299  CG1 VAL A 235       9.948  19.994  17.533  1.00 11.79           C
ATOM   3303  CG2 VAL A 235       9.475  19.925  19.919  1.00 13.78           C
ATOM   3307  C   VAL A 235       7.284  19.242  16.876  1.00 14.46           C
ATOM   3308  O   VAL A 235       7.529  18.141  16.413  1.00 15.04           O
ATOM   3309  N   GLU A 236       6.773  20.208  16.151  1.00 15.81           N
ATOM   3311  CA  GLU A 236       6.539  20.075  14.717  1.00 15.96           C
ATOM   3313  CB  GLU A 236       5.419  21.059  14.323  1.00 16.03           C
ATOM   3316  CG  GLU A 236       5.033  21.028  12.863  1.00 18.99           C
ATOM   3319  CD  GLU A 236       3.833  21.939  12.549  1.00 22.94           C
ATOM   3320  OE1 GLU A 236       3.422  22.715  13.457  1.00 20.60           O
ATOM   3321  OE2 GLU A 236       3.255  21.772  11.420  1.00 20.10           O
ATOM   3322  C   GLU A 236       7.751  20.349  13.881  1.00 16.01           C
ATOM   3323  O   GLU A 236       8.534  21.272  14.139  1.00 17.39           O
ATOM   3324  N   SER A 237       8.023  19.462  12.905  1.00 14.14           N
ATOM   3326  CA  SER A 237       9.105  19.655  12.025  1.00 14.47           C
ATOM   3328  CB  SER A 237      10.410  19.109  12.632  1.00 11.88           C
ATOM   3331  OG  SER A 237      11.513  19.504  11.921  1.00 11.55           O
ATOM   3333  C   SER A 237       8.819  18.900  10.715  1.00 14.97           C
ATOM   3334  O   SER A 237       7.699  18.345  10.543  1.00 16.60           O
ATOM   3335  N   THR A 238       9.838  18.892   9.886  1.00 15.64           N
ATOM   3337  CA  THR A 238       9.851  18.230   8.581  1.00 17.00           C
ATOM   3339  CB  THR A 238      11.090  18.559   7.844  1.00 18.10           C
ATOM   3341  OG1 THR A 238      12.267  18.454   8.677  1.00 16.55           O
ATOM   3343  CG2 THR A 238      11.152  19.996   7.339  1.00 16.33           C
ATOM   3347  C   THR A 238       9.752  16.703   8.759  1.00 19.28           C
ATOM   3348  O   THR A 238      10.213  16.169   9.768  1.00 18.16           O
ATOM   3349  N   TRP A 239       9.203  16.032   7.739  1.00 19.48           N
ATOM   3351  CA  TRP A 239       8.933  14.587   7.765  1.00 18.83           C
ATOM   3353  CB  TRP A 239       7.503  14.322   8.122  1.00 19.25           C
ATOM   3356  CG  TRP A 239       7.182  12.954   8.642  1.00 19.71           C
ATOM   3357  CD1 TRP A 239       6.343  12.009   8.085  1.00 23.51           C
ATOM   3359  NE1 TRP A 239       6.263  10.907   8.899  1.00 22.49           N
ATOM   3361  CE2 TRP A 239       7.081  11.114   9.985  1.00 21.32           C
ATOM   3362  CD2 TRP A 239       7.651  12.400   9.853  1.00 20.74           C
ATOM   3363  CE3 TRP A 239       8.529  12.864  10.851  1.00 18.32           C
ATOM   3365  CZ3 TRP A 239       8.751  12.055  11.962  1.00 21.08           C
ATOM   3367  CH2 TRP A 239       8.166  10.788  12.061  1.00 20.53           C
ATOM   3369  CZ2 TRP A 239       7.349  10.289  11.079  1.00 22.21           C
ATOM   3371  C   TRP A 239       9.325  13.931   6.461  1.00 17.68           C
ATOM   3372  O   TRP A 239       9.417  14.577   5.423  1.00 19.48           O
ATOM   3373  N   TYR A 240       9.679  12.642   6.550  1.00 17.80           N
ATOM   3375  CA  TYR A 240      10.332  11.947   5.485  1.00 17.76           C
ATOM   3377  CB  TYR A 240      10.862  10.568   5.938  1.00 20.08           C
ATOM   3380  CG  TYR A 240       9.864   9.469   6.036  1.00 17.92           C
ATOM   3381  CD1 TYR A 240       8.997   9.367   7.097  1.00 20.96           C
```

```
ATOM   3383   CE1  TYR A 240     8.113   8.325   7.179  1.00 22.25          C
ATOM   3385   CZ   TYR A 240     8.150   7.317   6.171  1.00 27.67          C
ATOM   3386   OH   TYR A 240     7.269   6.247   6.207  1.00 28.63          O
ATOM   3388   CE2  TYR A 240     9.034   7.397   5.159  1.00 25.36          C
ATOM   3390   CD2  TYR A 240     9.884   8.442   5.084  1.00 24.19          C
ATOM   3392   C    TYR A 240     9.453  11.746   4.253  1.00 19.14          C
ATOM   3393   O    TYR A 240     9.992  11.532   3.199  1.00 19.72          O
ATOM   3394   N    THR A 241     8.172  11.873   4.401  1.00 21.32          N
ATOM   3396   CA   THR A 241     7.309  11.777   3.214  1.00 24.31          C
ATOM   3398   CB   THR A 241     6.015  11.129   3.549  1.00 24.08          C
ATOM   3400   OG1  THR A 241     5.443  11.711   4.720  1.00 25.38          O
ATOM   3402   CG2  THR A 241     6.238   9.670   3.924  1.00 28.14          C
ATOM   3406   C    THR A 241     7.020  13.113   2.587  1.00 26.01          C
ATOM   3407   O    THR A 241     6.175  13.200   1.682  1.00 27.85          O
ATOM   3408   N    GLY A 242     7.684  14.159   3.041  1.00 26.13          N
ATOM   3410   CA   GLY A 242     7.445  15.480   2.484  1.00 26.65          C
ATOM   3413   C    GLY A 242     6.544  16.381   3.303  1.00 25.41          C
ATOM   3414   O    GLY A 242     6.524  17.583   3.068  1.00 29.27          O
ATOM   3415   N    GLY A 243     5.786  15.891   4.246  1.00 23.67          N
ATOM   3417   CA   GLY A 243     5.002  16.882   4.959  1.00 23.78          C
ATOM   3420   C    GLY A 243     5.719  17.300   6.239  1.00 21.57          C
ATOM   3421   O    GLY A 243     6.944  17.532   6.218  1.00 20.45          O
ATOM   3422   N    TYR A 244     4.939  17.380   7.296  1.00 22.18          N
ATOM   3424   CA   TYR A 244     5.404  17.745   8.651  1.00 21.26          C
ATOM   3426   CB   TYR A 244     4.991  19.201   8.963  1.00 20.92          C
ATOM   3429   CG   TYR A 244     5.467  20.078   7.877  1.00 20.48          C
ATOM   3430   CD1  TYR A 244     4.696  20.312   6.749  1.00 20.98          C
ATOM   3432   CE1  TYR A 244     5.167  21.082   5.733  1.00 21.20          C
ATOM   3434   CZ   TYR A 244     6.396  21.604   5.785  1.00 20.81          C
ATOM   3435   OH   TYR A 244     6.852  22.332   4.745  1.00 23.64          O
ATOM   3437   CE2  TYR A 244     7.201  21.392   6.929  1.00 17.10          C
ATOM   3439   CD2  TYR A 244     6.714  20.660   7.920  1.00 14.54          C
ATOM   3441   C    TYR A 244     4.789  16.816   9.621  1.00 21.28          C
ATOM   3442   O    TYR A 244     3.778  16.224   9.353  1.00 23.43          O
ATOM   3443   N    ASN A 245     5.381  16.692  10.800  1.00 19.64          N
ATOM   3445   CA   ASN A 245     4.866  15.855  11.825  1.00 18.70          C
ATOM   3447   CB   ASN A 245     5.322  14.418  11.590  1.00 18.51          C
ATOM   3450   CG   ASN A 245     4.644  13.438  12.450  1.00 18.67          C
ATOM   3451   OD1  ASN A 245     3.509  13.611  12.925  1.00 21.26          O
ATOM   3452   ND2  ASN A 245     5.350  12.323  12.689  1.00 22.00          N
ATOM   3455   C    ASN A 245     5.304  16.401  13.154  1.00 16.91          C
ATOM   3456   O    ASN A 245     6.289  17.194  13.212  1.00 17.80          O
ATOM   3457   N    THR A 246     4.533  16.035  14.141  1.00 17.22          N
ATOM   3459   CA   THR A 246     4.696  16.445  15.504  1.00 17.23          C
ATOM   3461   CB   THR A 246     3.454  17.242  15.971  1.00 17.67          C
ATOM   3463   OG1  THR A 246     3.368  18.458  15.223  1.00 18.24          O
ATOM   3465   CG2  THR A 246     3.534  17.710  17.400  1.00 20.37          C
ATOM   3469   C    THR A 246     4.993  15.244  16.374  1.00 17.33          C
ATOM   3470   O    THR A 246     4.123  14.398  16.664  1.00 16.60          O
ATOM   3471   N    ILE A 247     6.221  15.237  16.902  1.00 16.92          N
ATOM   3473   CA   ILE A 247     6.655  14.201  17.813  1.00 16.77          C
ATOM   3475   CB   ILE A 247     7.536  13.106  17.091  1.00 16.85          C
ATOM   3477   CG1  ILE A 247     8.722  13.699  16.372  1.00 16.22          C
ATOM   3480   CD1  ILE A 247     9.721  12.674  15.892  1.00 14.19          C
ATOM   3484   CG2  ILE A 247     6.620  12.272  16.155  1.00 18.78          C
ATOM   3488   C    ILE A 247     7.394  14.839  19.045  1.00 14.93          C
ATOM   3489   O    ILE A 247     7.628  16.050  19.098  1.00 15.83          O
ATOM   3490   N    SER A 248     7.659  14.020  20.030  1.00 14.46          N
ATOM   3492   CA   SER A 248     8.268  14.443  21.285  1.00 14.38          C
ATOM   3494   CB   SER A 248     7.262  14.230  22.426  1.00 14.68          C
ATOM   3497   OG   SER A 248     6.080  14.988  22.271  1.00 16.96          O
ATOM   3499   C    SER A 248     9.470  13.594  21.672  1.00 13.40          C
ATOM   3500   O    SER A 248     9.626  12.448  21.227  1.00 12.06          O
```

```
ATOM   3501  N    GLY A 249      10.306  14.151  22.557  1.00  11.90           N
ATOM   3503  CA   GLY A 249      11.402  13.420  23.140  1.00  12.25           C
ATOM   3506  C    GLY A 249      12.615  14.294  23.393  1.00  11.45           C
ATOM   3507  O    GLY A 249      12.672  15.399  22.898  1.00  12.12           O
ATOM   3508  N    THR A 250      13.607  13.759  24.103  1.00  10.99           N
ATOM   3510  CA   THR A 250      14.898  14.476  24.200  1.00   9.84           C
ATOM   3512  CB   THR A 250      15.835  13.937  25.257  1.00   9.57           C
ATOM   3514  OG1  THR A 250      15.885  12.543  25.195  1.00  10.83           O
ATOM   3516  CG2  THR A 250      15.260  14.258  26.612  1.00  11.12           C
ATOM   3520  C    THR A 250      15.506  14.531  22.800  1.00   9.75           C
ATOM   3521  O    THR A 250      16.312  15.393  22.517  1.00  11.31           O
ATOM   3522  N    SER A 251      15.101  13.616  21.938  1.00   9.71           N
ATOM   3524  CA   SER A 251      15.396  13.711  20.513  1.00  10.29           C
ATOM   3526  CB   SER A 251      14.647  12.660  19.691  1.00  10.44           C
ATOM   3529  OG   SER A 251      15.278  11.374  19.720  1.00  11.64           O
ATOM   3531  C    SER A 251      15.132  15.058  19.850  1.00  10.60           C
ATOM   3532  O    SER A 251      15.871  15.475  18.968  1.00  12.05           O
ATOM   3533  N    MET A 252      14.023  15.698  20.246  1.00  11.35           N
ATOM   3535  CA   MET A 252      13.598  16.937  19.649  1.00  12.05           C
ATOM   3537  CB   MET A 252      12.081  17.000  19.803  1.00  10.74           C
ATOM   3540  CG   MET A 252      11.275  16.163  18.811  1.00  16.05           C
ATOM   3543  SD   MET A 252      11.445  14.393  18.855  1.00  12.67           S
ATOM   3544  CE   MET A 252      12.360  14.105  17.508  1.00  14.70           C
ATOM   3548  C    MET A 252      14.248  18.146  20.363  1.00  11.00           C
ATOM   3549  O    MET A 252      14.372  19.200  19.805  1.00  10.97           O
ATOM   3550  N    ALA A 253      14.615  17.978  21.628  1.00  11.44           N
ATOM   3552  CA   ALA A 253      15.300  19.023  22.361  1.00   9.30           C
ATOM   3554  CB   ALA A 253      15.282  18.677  23.852  1.00   8.63           C
ATOM   3558  C    ALA A 253      16.733  19.234  21.842  1.00   9.29           C
ATOM   3559  O    ALA A 253      17.173  20.367  21.599  1.00  10.72           O
ATOM   3560  N    THR A 254      17.388  18.107  21.578  1.00   9.20           N
ATOM   3562  CA   THR A 254      18.776  18.047  21.127  1.00   9.63           C
ATOM   3564  CB   THR A 254      19.131  16.592  20.838  1.00  11.67           C
ATOM   3566  OG1  THR A 254      19.001  15.823  22.036  1.00   9.34           O
ATOM   3568  CG2  THR A 254      20.504  16.467  20.434  1.00   9.85           C
ATOM   3572  C    THR A 254      19.041  18.957  19.927  1.00   9.01           C
ATOM   3573  O    THR A 254      19.932  19.782  19.958  1.00   9.02           O
ATOM   3574  N    PRO A 255      18.255  18.848  18.856  1.00  10.43           N
ATOM   3575  CA   PRO A 255      18.491  19.725  17.711  1.00   9.72           C
ATOM   3577  CB   PRO A 255      17.607  19.149  16.621  1.00  11.04           C
ATOM   3580  CG   PRO A 255      16.511  18.430  17.368  1.00  11.23           C
ATOM   3583  CD   PRO A 255      17.253  17.817  18.535  1.00  11.38           C
ATOM   3586  C    PRO A 255      18.195  21.185  17.941  1.00  10.12           C
ATOM   3587  O    PRO A 255      18.724  22.018  17.171  1.00   9.83           O
ATOM   3588  N    HIS A 256      17.398  21.535  18.943  1.00  10.53           N
ATOM   3590  CA   HIS A 256      17.233  22.947  19.219  1.00  10.79           C
ATOM   3592  CB   HIS A 256      16.192  23.258  20.298  1.00  11.66           C
ATOM   3595  CG   HIS A 256      14.748  23.136  19.820  1.00  12.48           C
ATOM   3596  ND1  HIS A 256      14.086  21.930  19.732  1.00  11.32           N
ATOM   3598  CE1  HIS A 256      12.849  22.144  19.296  1.00  14.50           C
ATOM   3600  NE2  HIS A 256      12.709  23.434  19.040  1.00  12.79           N
ATOM   3602  CD2  HIS A 256      13.889  24.074  19.348  1.00  12.20           C
ATOM   3604  C    HIS A 256      18.572  23.451  19.658  1.00  11.12           C
ATOM   3605  O    HIS A 256      18.977  24.554  19.302  1.00  12.50           O
ATOM   3606  N    VAL A 257      19.264  22.660  20.474  1.00  10.13           N
ATOM   3608  CA   VAL A 257      20.558  23.078  20.969  1.00   9.71           C
ATOM   3610  CB   VAL A 257      20.966  22.264  22.219  1.00  10.12           C
ATOM   3612  CG1  VAL A 257      22.407  22.589  22.653  1.00  10.84           C
ATOM   3616  CG2  VAL A 257      19.951  22.451  23.324  1.00  11.41           C
ATOM   3620  C    VAL A 257      21.632  22.993  19.871  1.00  10.99           C
ATOM   3621  O    VAL A 257      22.483  23.871  19.782  1.00  11.55           O
ATOM   3622  N    ALA A 258      21.664  21.933  19.070  1.00  10.95           N
ATOM   3624  CA   ALA A 258      22.625  21.857  17.985  1.00  10.24           C
```

```
ATOM   3626  CB  ALA A 258    22.459  20.568  17.233  1.00  9.96          C
ATOM   3630  C   ALA A 258    22.446  23.027  17.061  1.00 11.33          C
ATOM   3631  O   ALA A 258    23.426  23.621  16.598  1.00 10.81          O
ATOM   3632  N   GLY A 259    21.191  23.351  16.799  1.00 11.66          N
ATOM   3634  CA  GLY A 259    20.929  24.467  15.916  1.00 12.69          C
ATOM   3637  C   GLY A 259    21.378  25.792  16.529  1.00 11.32          C
ATOM   3638  O   GLY A 259    21.928  26.653  15.830  1.00 12.25          O
ATOM   3639  N   LEU A 260    21.044  25.991  17.781  1.00 10.90          N
ATOM   3641  CA  LEU A 260    21.453  27.191  18.479  1.00 11.40          C
ATOM   3643  CB  LEU A 260    20.945  27.240  19.877  1.00 10.96          C
ATOM   3646  CG  LEU A 260    21.372  28.483  20.678  1.00 11.84          C
ATOM   3648  CD1 LEU A 260    20.781  29.683  20.099  1.00 16.22          C
ATOM   3652  CD2 LEU A 260    20.948  28.349  22.174  1.00 12.84          C
ATOM   3656  C   LEU A 260    22.984  27.291  18.477  1.00 12.53          C
ATOM   3657  O   LEU A 260    23.558  28.384  18.205  1.00 11.28          O
ATOM   3658  N   ALA A 261    23.644  26.153  18.745  1.00 11.39          N
ATOM   3660  CA  ALA A 261    25.093  26.133  18.661  1.00 11.97          C
ATOM   3662  CB  ALA A 261    25.604  24.716  18.889  1.00 13.65          C
ATOM   3666  C   ALA A 261    25.607  26.638  17.292  1.00 12.95          C
ATOM   3667  O   ALA A 261    26.563  27.413  17.203  1.00 12.12          O
ATOM   3668  N   ALA A 262    24.998  26.144  16.229  1.00 11.02          N
ATOM   3670  CA  ALA A 262    25.435  26.520  14.869  1.00 12.24          C
ATOM   3672  CB  ALA A 262    24.731  25.669  13.828  1.00 12.25          C
ATOM   3676  C   ALA A 262    25.200  28.002  14.619  1.00 13.52          C
ATOM   3677  O   ALA A 262    26.045  28.684  13.996  1.00 13.55          O
ATOM   3678  N   LYS A 263    24.091  28.505  15.135  1.00 13.42          N
ATOM   3680  CA  LYS A 263    23.730  29.913  15.021  1.00 13.54          C
ATOM   3682  CB  LYS A 263    22.319  30.164  15.511  1.00 14.20          C
ATOM   3685  CG  LYS A 263    21.797  31.603  15.356  1.00 13.78          C
ATOM   3688  CD  LYS A 263    20.412  31.725  15.984  1.00 15.37          C
ATOM   3691  CE  LYS A 263    19.936  33.158  16.011  1.00 17.70          C
ATOM   3694  NZ  LYS A 263    19.671  33.740  14.618  1.00 14.92          N
ATOM   3698  C   LYS A 263    24.754  30.774  15.736  1.00 12.81          C
ATOM   3699  O   LYS A 263    25.225  31.781  15.148  1.00 13.25          O
ATOM   3700  N   ILE A 264    25.133  30.393  16.961  1.00 12.96          N
ATOM   3702  CA  ILE A 264    26.107  31.136  17.723  1.00 12.47          C
ATOM   3704  CB  ILE A 264    26.209  30.579  19.150  1.00 12.83          C
ATOM   3706  CG1 ILE A 264    24.895  30.799  19.927  1.00 12.96          C
ATOM   3709  CD1 ILE A 264    24.829  30.017  21.259  1.00 13.94          C
ATOM   3713  CG2 ILE A 264    27.382  31.146  19.925  1.00 12.54          C
ATOM   3717  C   ILE A 264    27.478  31.081  17.018  1.00 12.78          C
ATOM   3718  O   ILE A 264    28.147  32.110  16.835  1.00 12.72          O
ATOM   3719  N   TRP A 265    27.843  29.890  16.527  1.00 12.74          N
ATOM   3721  CA  TRP A 265    29.194  29.711  15.984  1.00 13.37          C
ATOM   3723  CB  TRP A 265    29.445  28.252  15.665  1.00 13.60          C
ATOM   3726  CG  TRP A 265    30.859  27.836  15.758  1.00 12.37          C
ATOM   3727  CD1 TRP A 265    31.987  28.624  15.964  1.00 12.89          C
ATOM   3729  NE1 TRP A 265    33.101  27.823  16.063  1.00 14.49          N
ATOM   3731  CE2 TRP A 265    32.705  26.514  15.998  1.00 16.16          C
ATOM   3732  CD2 TRP A 265    31.313  26.493  15.796  1.00 12.95          C
ATOM   3733  CE3 TRP A 265    30.679  25.254  15.657  1.00 14.36          C
ATOM   3735  CZ3 TRP A 265    31.472  24.110  15.707  1.00 12.68          C
ATOM   3737  CH2 TRP A 265    32.809  24.180  15.941  1.00 12.39          C
ATOM   3739  CZ2 TRP A 265    33.462  25.359  16.069  1.00 15.97          C
ATOM   3741  C   TRP A 265    29.378  30.560  14.732  1.00 13.71          C
ATOM   3742  O   TRP A 265    30.423  31.117  14.530  1.00 17.12          O
ATOM   3743  N   SER A 266    28.330  30.599  13.922  1.00 15.25          N
ATOM   3745  CA  SER A 266    28.307  31.345  12.676  1.00 14.75          C
ATOM   3747  CB  SER A 266    27.000  31.130  11.945  1.00 15.47          C
ATOM   3750  OG  SER A 266    26.987  31.917  10.741  1.00 18.19          O
ATOM   3752  C   SER A 266    28.498  32.835  12.962  1.00 16.77          C
ATOM   3753  O   SER A 266    29.218  33.537  12.227  1.00 16.07          O
ATOM   3754  N   ALA A 267    27.903  33.307  14.053  1.00 15.63          N
```

```
ATOM   3756  CA   ALA A 267    28.046  34.711  14.450  1.00  17.65           C
ATOM   3758  CB   ALA A 267    26.911  35.093  15.318  1.00  19.01           C
ATOM   3762  C    ALA A 267    29.405  35.056  15.092  1.00  19.17           C
ATOM   3763  O    ALA A 267    29.744  36.249  15.277  1.00  18.66           O
ATOM   3764  N    ASN A 268    30.188  34.053  15.465  1.00  17.88           N
ATOM   3766  CA   ASN A 268    31.557  34.318  15.886  1.00  20.05           C
ATOM   3768  CB   ASN A 268    31.688  34.603  17.324  1.00  20.47           C
ATOM   3771  CG   ASN A 268    33.120  35.029  17.701  1.00  23.58           C
ATOM   3772  OD1  ASN A 268    34.017  35.076  16.860  1.00  24.59           O
ATOM   3773  ND2  ASN A 268    33.327  35.286  18.961  1.00  27.93           N
ATOM   3776  C    ASN A 268    32.437  33.156  15.520  1.00  18.98           C
ATOM   3777  O    ASN A 268    32.651  32.264  16.321  1.00  17.09           O
ATOM   3778  N    THR A 269    32.915  33.169  14.289  1.00  19.21           N
ATOM   3780  CA   THR A 269    33.696  32.076  13.772  1.00  19.25           C
ATOM   3782  CB   THR A 269    33.838  32.153  12.207  1.00  21.27           C
ATOM   3784  OG1  THR A 269    34.379  33.443  11.889  1.00  21.88           O
ATOM   3786  CG2  THR A 269    32.479  32.127  11.565  1.00  23.50           C
ATOM   3790  C    THR A 269    35.055  32.025  14.380  1.00  19.11           C
ATOM   3791  O    THR A 269    35.761  31.117  14.072  1.00  19.77           O
ATOM   3792  N    SER A 270    35.435  32.929  15.297  1.00  19.17           N
ATOM   3794  CA   SER A 270    36.732  32.774  15.936  1.00  19.54           C
ATOM   3796  CB  ASER A 270    37.231  34.105  16.454  0.50  19.48           C
ATOM   3797  CB  BSER A 270    37.264  34.116  16.400  0.50  19.66           C
ATOM   3802  OG  ASER A 270    36.235  34.749  17.225  0.50  21.34           O
ATOM   3803  OG  BSER A 270    37.628  34.922  15.280  0.50  22.88           O
ATOM   3806  C    SER A 270    36.679  31.795  17.100  1.00  17.42           C
ATOM   3807  O    SER A 270    37.688  31.382  17.637  1.00  19.69           O
ATOM   3808  N    LEU A 271    35.493  31.392  17.484  1.00  15.84           N
ATOM   3810  CA   LEU A 271    35.364  30.456  18.595  1.00  13.46           C
ATOM   3812  CB   LEU A 271    33.910  30.345  19.026  1.00  14.65           C
ATOM   3815  CG   LEU A 271    33.146  31.575  19.466  1.00  15.68           C
ATOM   3817  CD1  LEU A 271    31.659  31.196  19.782  1.00  18.69           C
ATOM   3821  CD2  LEU A 271    33.807  32.216  20.685  1.00  21.20           C
ATOM   3825  C    LEU A 271    35.806  29.028  18.220  1.00  12.04           C
ATOM   3826  O    LEU A 271    35.573  28.546  17.091  1.00  11.86           O
ATOM   3827  N    SER A 272    36.323  28.335  19.220  1.00  12.57           N
ATOM   3829  CA   SER A 272    36.545  26.919  19.145  1.00  11.98           C
ATOM   3831  CB   SER A 272    37.710  26.489  20.040  1.00  12.49           C
ATOM   3834  OG   SER A 272    37.433  26.778  21.421  1.00  12.53           O
ATOM   3836  C    SER A 272    35.275  26.244  19.663  1.00  11.47           C
ATOM   3837  O    SER A 272    34.372  26.889  20.269  1.00   9.92           O
ATOM   3838  N    HIS A 273    35.225  24.937  19.454  1.00  11.48           N
ATOM   3840  CA   HIS A 273    34.074  24.187  19.920  1.00  11.75           C
ATOM   3842  CB   HIS A 273    34.187  22.750  19.445  1.00  13.59           C
ATOM   3845  CG   HIS A 273    35.160  21.921  20.197  1.00  13.01           C
ATOM   3846  ND1  HIS A 273    36.456  22.312  20.379  1.00  11.73           N
ATOM   3848  CE1  HIS A 273    37.072  21.402  21.124  1.00  13.52           C
ATOM   3850  NE2  HIS A 273    36.243  20.394  21.343  1.00  12.58           N
ATOM   3852  CD2  HIS A 273    35.039  20.708  20.787  1.00  14.38           C
ATOM   3854  C    HIS A 273    33.887  24.268  21.427  1.00  11.79           C
ATOM   3855  O    HIS A 273    32.723  24.252  21.930  1.00  10.83           O
ATOM   3856  N    SER A 274    34.975  24.276  22.191  1.00  10.50           N
ATOM   3858  CA   SER A 274    34.813  24.319  23.643  1.00  10.49           C
ATOM   3860  CB   SER A 274    36.035  23.803  24.397  1.00  13.18           C
ATOM   3863  OG   SER A 274    37.161  24.639  24.166  1.00  12.23           O
ATOM   3865  C    SER A 274    34.478  25.702  24.113  1.00  12.50           C
ATOM   3866  O    SER A 274    33.783  25.849  25.123  1.00  11.08           O
ATOM   3867  N    GLN A 275    34.919  26.739  23.418  1.00  11.42           N
ATOM   3869  CA   GLN A 275    34.425  28.072  23.784  1.00  11.21           C
ATOM   3871  CB   GLN A 275    35.132  29.188  22.999  1.00  11.23           C
ATOM   3874  CG   GLN A 275    36.608  29.464  23.375  1.00  13.15           C
ATOM   3877  CD   GLN A 275    37.192  30.473  22.413  1.00  13.10           C
ATOM   3878  OE1  GLN A 275    37.323  30.182  21.228  1.00  15.35           O
```

```
ATOM   3879  NE2 GLN A 275     37.555  31.677  22.923  1.00 11.91          N
ATOM   3882  C   GLN A 275     32.898  28.177  23.457  1.00 11.10          C
ATOM   3883  O   GLN A 275     32.150  28.776  24.217  1.00 10.23          O
ATOM   3884  N   LEU A 276     32.492  27.556  22.340  1.00 10.08          N
ATOM   3886  CA  LEU A 276     31.067  27.521  21.932  1.00 10.49          C
ATOM   3888  CB  LEU A 276     30.909  26.866  20.582  1.00 10.65          C
ATOM   3891  CG  LEU A 276     29.466  26.606  20.164  1.00  8.36          C
ATOM   3893  CD1 LEU A 276     28.715  27.856  20.038  1.00  9.01          C
ATOM   3897  CD2 LEU A 276     29.489  25.869  18.849  1.00 11.63          C
ATOM   3901  C   LEU A 276     30.272  26.790  23.041  1.00 11.06          C
ATOM   3902  O   LEU A 276     29.226  27.252  23.494  1.00 10.87          O
ATOM   3903  N   ARG A 277     30.783  25.671  23.524  1.00  9.13          N
ATOM   3905  CA  ARG A 277     30.104  24.934  24.560  1.00 11.95          C
ATOM   3907  CB  ARG A 277     30.915  23.671  24.913  1.00 10.95          C
ATOM   3910  CG  ARG A 277     30.335  22.793  26.018  1.00 13.22          C
ATOM   3913  CD  ARG A 277     31.103  21.490  26.229  1.00 13.28          C
ATOM   3916  NE  ARG A 277     30.415  20.601  27.149  1.00 13.25          N
ATOM   3918  CZ  ARG A 277     30.892  19.416  27.518  1.00 14.34          C
ATOM   3919  NH1 ARG A 277     32.089  19.014  27.110  1.00 14.38          N
ATOM   3922  NH2 ARG A 277     30.190  18.660  28.345  1.00 13.28          N
ATOM   3925  C   ARG A 277     29.974  25.722  25.858  1.00 12.12          C
ATOM   3926  O   ARG A 277     28.949  25.722  26.538  1.00 12.16          O
ATOM   3927  N   THR A 278     31.023  26.469  26.175  1.00 12.13          N
ATOM   3929  CA  THR A 278     31.049  27.295  27.359  1.00 11.62          C
ATOM   3931  CB  THR A 278     32.461  27.918  27.485  1.00 12.51          C
ATOM   3933  OG1 THR A 278     33.379  26.892  27.846  1.00 15.78          O
ATOM   3935  CG2 THR A 278     32.551  28.829  28.624  1.00 17.34          C
ATOM   3939  C   THR A 278     29.990  28.388  27.287  1.00 10.34          C
ATOM   3940  O   THR A 278     29.372  28.715  28.294  1.00 11.22          O
ATOM   3941  N   GLU A 279     29.775  28.943  26.101  1.00 10.24          N
ATOM   3943  CA  GLU A 279     28.804  30.034  25.920  1.00 10.36          C
ATOM   3945  CB  GLU A 279     29.090  30.744  24.607  1.00 13.04          C
ATOM   3948  CG  GLU A 279     28.155  31.799  24.172  1.00 13.67          C
ATOM   3951  CD  GLU A 279     27.827  32.923  25.148  1.00 11.41          C
ATOM   3952  OE1 GLU A 279     28.413  33.084  26.245  1.00 12.14          O
ATOM   3953  OE2 GLU A 279     26.928  33.691  24.766  1.00 13.94          O
ATOM   3954  C   GLU A 279     27.387  29.439  25.978  1.00 10.50          C
ATOM   3955  O   GLU A 279     26.477  30.093  26.462  1.00 10.16          O
ATOM   3956  N   LEU A 280     27.198  28.205  25.499  1.00 10.73          N
ATOM   3958  CA  LEU A 280     25.898  27.507  25.652  1.00 10.31          C
ATOM   3960  CB  LEU A 280     25.846  26.124  24.973  1.00 12.52          C
ATOM   3963  CG  LEU A 280     25.772  26.107  23.451  1.00 13.85          C
ATOM   3965  CD1 LEU A 280     25.964  24.631  22.911  1.00 17.63          C
ATOM   3969  CD2 LEU A 280     24.435  26.669  22.957  1.00 15.10          C
ATOM   3973  C   LEU A 280     25.613  27.411  27.150  1.00 10.89          C
ATOM   3974  O   LEU A 280     24.492  27.626  27.606  1.00 12.00          O
ATOM   3975  N   GLN A 281     26.630  27.040  27.907  1.00  9.57          N
ATOM   3977  CA  GLN A 281     26.479  26.931  29.322  1.00 11.52          C
ATOM   3979  CB  GLN A 281     27.755  26.360  29.925  1.00 11.03          C
ATOM   3982  CG  GLN A 281     28.028  24.891  29.487  1.00 12.22          C
ATOM   3985  CD  GLN A 281     29.376  24.377  29.981  1.00 14.98          C
ATOM   3986  OE1 GLN A 281     30.115  25.172  30.555  1.00 15.61          O
ATOM   3987  NE2 GLN A 281     29.731  23.103  29.697  1.00  9.95          N
ATOM   3990  C   GLN A 281     26.161  28.313  29.924  1.00 10.71          C
ATOM   3991  O   GLN A 281     25.309  28.409  30.815  1.00 11.77          O
ATOM   3992  N   ASN A 282     26.883  29.367  29.484  1.00 10.78          N
ATOM   3994  CA  ASN A 282     26.722  30.715  30.090  1.00 10.24          C
ATOM   3996  CB  ASN A 282     27.683  31.745  29.504  1.00 10.08          C
ATOM   3999  CG  ASN A 282     29.136  31.482  29.812  1.00 13.02          C
ATOM   4000  OD1 ASN A 282     29.471  30.830  30.809  1.00 14.49          O
ATOM   4001  ND2 ASN A 282     30.003  32.000  28.973  1.00 12.29          N
ATOM   4004  C   ASN A 282     25.275  31.172  29.788  1.00 11.08          C
ATOM   4005  O   ASN A 282     24.588  31.667  30.681  1.00 12.10          O
```

```
ATOM   4006  N    ARG A 283    24.829  30.902  28.575  1.00   9.30        N
ATOM   4008  CA   ARG A 283    23.484  31.298  28.160  1.00  10.89        C
ATOM   4010  CB   ARG A 283    23.305  31.055  26.697  1.00  10.96        C
ATOM   4013  CG   ARG A 283    23.894  32.191  25.842  1.00  13.15        C
ATOM   4016  CD   ARG A 283    23.768  31.880  24.383  1.00  11.91        C
ATOM   4019  NE   ARG A 283    24.469  32.831  23.466  1.00  12.89        N
ATOM   4021  CZ   ARG A 283    23.985  33.290  22.311  1.00  15.41        C
ATOM   4022  NH1  ARG A 283    22.780  33.006  21.903  1.00  15.22        N
ATOM   4025  NH2  ARG A 283    24.722  34.053  21.528  1.00  14.46        N
ATOM   4028  C    ARG A 283    22.450  30.528  28.963  1.00  10.28        C
ATOM   4029  O    ARG A 283    21.390  31.063  29.298  1.00  11.49        O
ATOM   4030  N    ALA A 284    22.741  29.263  29.220  1.00  10.27        N
ATOM   4032  CA   ALA A 284    21.789  28.438  29.957  1.00  10.07        C
ATOM   4034  CB   ALA A 284    22.358  27.011  30.158  1.00  11.06        C
ATOM   4038  C    ALA A 284    21.493  29.093  31.306  1.00  10.53        C
ATOM   4039  O    ALA A 284    20.349  29.096  31.809  1.00  11.23        O
ATOM   4040  N    LYS A 285    22.539  29.620  31.899  1.00  10.42        N
ATOM   4042  CA   LYS A 285    22.446  30.158  33.238  1.00  10.99        C
ATOM   4044  CB   LYS A 285    23.846  30.372  33.821  1.00  11.62        C
ATOM   4047  CG   LYS A 285    24.664  29.120  34.054  1.00  12.18        C
ATOM   4050  CD   LYS A 285    26.057  29.529  34.477  1.00  16.21        C
ATOM   4053  CE   LYS A 285    27.062  28.492  34.451  1.00  15.01        C
ATOM   4056  NZ   LYS A 285    28.349  29.067  35.077  1.00  17.35        N
ATOM   4060  C    LYS A 285    21.566  31.386  33.281  1.00  11.59        C
ATOM   4061  O    LYS A 285    21.120  31.753  34.363  1.00  12.70        O
ATOM   4062  N    VAL A 286    21.419  32.094  32.168  1.00  10.88        N
ATOM   4064  CA   VAL A 286    20.604  33.255  32.110  1.00  12.69        C
ATOM   4066  CB   VAL A 286    20.719  33.961  30.788  1.00  12.21        C
ATOM   4068  CG1  VAL A 286    19.838  35.220  30.746  1.00  15.12        C
ATOM   4072  CG2  VAL A 286    22.221  34.402  30.612  1.00  13.05        C
ATOM   4076  C    VAL A 286    19.153  32.850  32.362  1.00  13.03        C
ATOM   4077  O    VAL A 286    18.399  33.651  32.960  1.00  13.04        O
ATOM   4078  N    TYR A 287    18.788  31.649  31.885  1.00  11.44        N
ATOM   4080  CA   TYR A 287    17.427  31.124  32.057  1.00  12.02        C
ATOM   4082  CB   TYR A 287    16.740  30.949  30.718  1.00  12.84        C
ATOM   4085  CG   TYR A 287    16.587  32.235  29.976  1.00  12.74        C
ATOM   4086  CD1  TYR A 287    15.615  33.157  30.344  1.00  14.60        C
ATOM   4088  CE1  TYR A 287    15.490  34.343  29.748  1.00  14.77        C
ATOM   4090  CZ   TYR A 287    16.343  34.709  28.753  1.00  15.62        C
ATOM   4091  OH   TYR A 287    16.139  35.938  28.183  1.00  18.73        O
ATOM   4093  CE2  TYR A 287    17.372  33.852  28.377  1.00  13.12        C
ATOM   4095  CD2  TYR A 287    17.521  32.646  28.995  1.00  11.74        C
ATOM   4097  C    TYR A 287    17.427  29.860  32.905  1.00  12.13        C
ATOM   4098  O    TYR A 287    17.303  28.740  32.452  1.00  13.99        O
ATOM   4099  N    ASP A 288    17.631  30.057  34.208  1.00  12.75        N
ATOM   4101  CA   ASP A 288    17.553  29.009  35.170  1.00  11.58        C
ATOM   4103  CB   ASP A 288    17.721  29.636  36.551  1.00  11.65        C
ATOM   4106  CG   ASP A 288    17.912  28.612  37.687  1.00  15.60        C
ATOM   4107  OD1  ASP A 288    18.065  27.373  37.473  1.00  17.35        O
ATOM   4108  OD2  ASP A 288    17.934  29.001  38.898  1.00  15.75        O
ATOM   4109  C    ASP A 288    16.174  28.337  35.056  1.00  12.45        C
ATOM   4110  O    ASP A 288    15.186  29.032  34.950  1.00  13.43        O
ATOM   4111  N    ILE A 289    16.112  27.026  34.986  1.00  10.72        N
ATOM   4113  CA   ILE A 289    14.812  26.271  34.969  1.00  12.80        C
ATOM   4115  CB   ILE A 289    14.860  25.168  33.912  1.00  12.78        C
ATOM   4117  CG1  ILE A 289    15.042  25.785  32.535  1.00  13.67        C
ATOM   4120  CD1  ILE A 289    13.745  26.474  31.867  1.00  13.36        C
ATOM   4124  CG2  ILE A 289    13.659  24.220  33.989  1.00  14.37        C
ATOM   4128  C    ILE A 289    14.517  25.742  36.352  1.00  13.79        C
ATOM   4129  O    ILE A 289    15.376  25.140  37.045  1.00  12.95        O
ATOM   4130  N    LYS A 290    13.299  26.006  36.825  1.00  14.91        N
ATOM   4132  CA   LYS A 290    12.904  25.675  38.185  1.00  16.59        C
ATOM   4134  CB   LYS A 290    12.600  26.955  38.930  1.00  17.67        C
```

```
ATOM   4137  CG  LYS A 290    13.878  27.786  39.143  1.00 21.86           C
ATOM   4140  CD  LYS A 290    13.627  28.893  40.134  1.00 28.07           C
ATOM   4143  CE  LYS A 290    13.554  30.215  39.470  1.00 34.10           C
ATOM   4146  NZ  LYS A 290    13.272  31.317  40.512  1.00 39.60           N
ATOM   4150  C   LYS A 290    11.648  24.807  38.235  1.00 16.71           C
ATOM   4151  O   LYS A 290    11.200  24.444  39.330  1.00 18.64           O
ATOM   4152  N   GLY A 291    11.119  24.507  37.071  1.00 15.39           N
ATOM   4154  CA  GLY A 291     9.895  23.740  36.907  1.00 14.24           C
ATOM   4157  C   GLY A 291    10.134  22.294  36.570  1.00 14.85           C
ATOM   4158  O   GLY A 291    10.997  21.990  35.707  1.00 15.05           O
ATOM   4159  N   GLY A 292     9.376  21.387  37.185  1.00 15.54           N
ATOM   4161  CA  GLY A 292     9.524  19.984  36.896  1.00 14.12           C
ATOM   4164  C   GLY A 292    10.116  19.187  38.030  1.00 15.07           C
ATOM   4165  O   GLY A 292    10.717  19.734  38.959  1.00 15.42           O
ATOM   4166  N   ILE A 293     9.932  17.878  37.980  1.00 14.74           N
ATOM   4168  CA  ILE A 293    10.363  17.018  39.044  1.00 15.41           C
ATOM   4170  CB  ILE A 293     9.881  15.599  38.776  1.00 15.32           C
ATOM   4172  CG1 ILE A 293     8.342  15.549  38.894  1.00 18.82           C
ATOM   4175  CD1 ILE A 293     7.731  14.260  38.471  1.00 21.20           C
ATOM   4179  CG2 ILE A 293    10.526  14.610  39.740  1.00 15.00           C
ATOM   4183  C   ILE A 293    11.885  17.052  39.176  1.00 16.26           C
ATOM   4184  O   ILE A 293    12.586  16.688  38.214  1.00 14.62           O
ATOM   4185  N   GLY A 294    12.367  17.439  40.346  1.00 14.72           N
ATOM   4187  CA  GLY A 294    13.810  17.581  40.559  1.00 15.36           C
ATOM   4190  C   GLY A 294    14.449  18.897  40.107  1.00 13.63           C
ATOM   4191  O   GLY A 294    15.660  19.095  40.315  1.00 15.06           O
ATOM   4192  N   ALA A 295    13.688  19.803  39.516  1.00 14.00           N
ATOM   4194  CA  ALA A 295    14.220  21.064  39.058  1.00 13.92           C
ATOM   4196  CB  ALA A 295    13.381  21.661  37.869  1.00 14.66           C
ATOM   4200  C   ALA A 295    14.226  21.990  40.236  1.00 16.73           C
ATOM   4201  O   ALA A 295    13.451  21.811  41.178  1.00 16.12           O
ATOM   4202  N   GLY A 296    15.094  22.986  40.227  1.00 16.79           N
ATOM   4204  CA  GLY A 296    15.114  23.941  41.340  1.00 18.72           C
ATOM   4207  C   GLY A 296    16.052  25.058  41.052  1.00 17.66           C
ATOM   4208  O   GLY A 296    16.601  25.160  39.963  1.00 16.21           O
ATOM   4209  N   THR A 297    16.240  25.979  41.992  1.00 17.88           N
ATOM   4211  CA  THR A 297    17.144  27.068  41.712  1.00 17.28           C
ATOM   4213  CB  THR A 297    17.117  27.981  42.941  1.00 19.91           C
ATOM   4215  OG1 THR A 297    15.763  28.455  43.102  1.00 19.89           O
ATOM   4217  CG2 THR A 297    17.981  29.177  42.689  1.00 19.92           C
ATOM   4221  C   THR A 297    18.563  26.600  41.472  1.00 17.13           C
ATOM   4222  O   THR A 297    19.104  25.832  42.248  1.00 18.37           O
ATOM   4223  N   GLY A 298    19.193  27.074  40.410  1.00 14.91           N
ATOM   4225  CA  GLY A 298    20.569  26.702  40.153  1.00 15.10           C
ATOM   4228  C   GLY A 298    20.684  25.492  39.273  1.00 14.01           C
ATOM   4229  O   GLY A 298    19.717  25.039  38.656  1.00 12.62           O
ATOM   4230  N   ASP A 299    21.882  24.932  39.243  1.00 13.64           N
ATOM   4232  CA  ASP A 299    22.189  23.741  38.460  1.00 13.68           C
ATOM   4234  CB  ASP A 299    23.689  23.623  38.447  1.00 14.28           C
ATOM   4237  CG  ASP A 299    24.229  22.342  37.843  1.00 15.32           C
ATOM   4238  OD1 ASP A 299    23.578  21.787  36.970  1.00 13.87           O
ATOM   4239  OD2 ASP A 299    25.330  21.885  38.238  1.00 13.70           O
ATOM   4240  C   ASP A 299    21.507  22.602  39.092  1.00 14.87           C
ATOM   4241  O   ASP A 299    21.657  22.381  40.306  1.00 16.32           O
ATOM   4242  N   ASP A 300    20.664  21.901  38.348  1.00 12.69           N
ATOM   4244  CA  ASP A 300    19.921  20.812  38.953  1.00 12.30           C
ATOM   4246  CB  ASP A 300    18.489  21.282  39.369  1.00 12.84           C
ATOM   4249  CG  ASP A 300    17.615  21.597  38.192  1.00 11.72           C
ATOM   4250  OD1 ASP A 300    17.330  20.687  37.428  1.00 13.65           O
ATOM   4251  OD2 ASP A 300    17.079  22.691  38.018  1.00 14.33           O
ATOM   4252  C   ASP A 300    19.928  19.603  38.011  1.00 13.91           C
ATOM   4253  O   ASP A 300    20.278  19.699  36.822  1.00 13.63           O
ATOM   4254  N   TYR A 301    19.514  18.462  38.539  1.00 13.49           N
```

```
ATOM   4256   CA    TYR A 301      19.627  17.220  37.803  1.00  12.93        C
ATOM   4258   CB    TYR A 301      19.868  16.046  38.777  1.00  11.22        C
ATOM   4261   CG    TYR A 301      18.804  15.846  39.823  1.00  13.88        C
ATOM   4262   CD1   TYR A 301      17.648  15.225  39.523  1.00  14.91        C
ATOM   4264   CE1   TYR A 301      16.621  15.079  40.531  1.00  17.24        C
ATOM   4266   CZ    TYR A 301      16.809  15.580  41.788  1.00  19.26        C
ATOM   4267   OH    TYR A 301      15.803  15.394  42.776  1.00  20.79        O
ATOM   4269   CE2   TYR A 301      17.977  16.242  42.081  1.00  16.95        C
ATOM   4271   CD2   TYR A 301      18.948  16.384  41.095  1.00  13.89        C
ATOM   4273   C     TYR A 301      18.421  16.951  36.875  1.00  11.64        C
ATOM   4274   O     TYR A 301      18.419  15.954  36.154  1.00  11.11        O
ATOM   4275   N     ALA A 302      17.435  17.846  36.832  1.00  11.29        N
ATOM   4277   CA    ALA A 302      16.389  17.756  35.829  1.00  10.84        C
ATOM   4279   CB    ALA A 302      15.006  18.227  36.391  1.00  12.99        C
ATOM   4283   C     ALA A 302      16.684  18.574  34.581  1.00  10.79        C
ATOM   4284   O     ALA A 302      16.424  18.146  33.444  1.00  11.72        O
ATOM   4285   N     SER A 303      17.194  19.774  34.790  1.00  10.48        N
ATOM   4287   CA    SER A 303      17.364  20.706  33.697  1.00   9.41        C
ATOM   4289   CB    SER A 303      16.512  21.934  33.969  1.00   9.70        C
ATOM   4292   OG    SER A 303      16.992  22.660  35.130  1.00  12.05        O
ATOM   4294   C     SER A 303      18.819  21.172  33.468  1.00  10.16        C
ATOM   4295   O     SER A 303      19.049  21.949  32.566  1.00  10.53        O
ATOM   4296   N     GLY A 304      19.742  20.739  34.303  1.00   8.73        N
ATOM   4298   CA    GLY A 304      21.132  21.139  34.201  1.00  10.18        C
ATOM   4301   C     GLY A 304      21.273  22.616  34.479  1.00  10.17        C
ATOM   4302   O     GLY A 304      20.710  23.138  35.422  1.00  10.02        O
ATOM   4303   N     PHE A 305      22.102  23.286  33.664  1.00  11.73        N
ATOM   4305   CA    PHE A 305      22.452  24.644  33.880  1.00  10.68        C
ATOM   4307   CB    PHE A 305      23.661  25.048  33.036  1.00  10.84        C
ATOM   4310   CG    PHE A 305      24.977  24.418  33.419  1.00  11.70        C
ATOM   4311   CD1   PHE A 305      25.070  23.280  34.195  1.00  12.20        C
ATOM   4313   CE1   PHE A 305      26.316  22.689  34.511  1.00  12.74        C
ATOM   4315   CZ    PHE A 305      27.499  23.265  33.973  1.00  11.93        C
ATOM   4317   CE2   PHE A 305      27.380  24.372  33.184  1.00  11.62        C
ATOM   4319   CD2   PHE A 305      26.157  24.941  32.891  1.00  12.26        C
ATOM   4321   C     PHE A 305      21.349  25.614  33.554  1.00  11.33        C
ATOM   4322   O     PHE A 305      21.368  26.748  34.022  1.00  11.39        O
ATOM   4323   N     GLY A 306      20.364  25.161  32.811  1.00  10.40        N
ATOM   4325   CA    GLY A 306      19.276  26.018  32.414  1.00  10.88        C
ATOM   4328   C     GLY A 306      18.926  25.834  30.963  1.00  11.20        C
ATOM   4329   O     GLY A 306      19.208  24.812  30.318  1.00   9.40        O
ATOM   4330   N     TYR A 307      18.291  26.863  30.443  1.00  10.29        N
ATOM   4332   CA    TYR A 307      17.803  26.905  29.077  1.00  11.10        C
ATOM   4334   CB    TYR A 307      16.311  27.194  29.162  1.00  10.85        C
ATOM   4337   CG    TYR A 307      15.557  27.309  27.870  1.00  10.79        C
ATOM   4338   CD1   TYR A 307      16.087  26.885  26.660  1.00  10.35        C
ATOM   4340   CE1   TYR A 307      15.374  27.001  25.479  1.00  10.97        C
ATOM   4342   CZ    TYR A 307      14.086  27.458  25.503  1.00  13.85        C
ATOM   4343   OH    TYR A 307      13.388  27.607  24.308  1.00  13.18        O
ATOM   4345   CE2   TYR A 307      13.531  27.912  26.705  1.00  13.38        C
ATOM   4347   CD2   TYR A 307      14.260  27.849  27.860  1.00  11.68        C
ATOM   4349   C     TYR A 307      18.479  27.939  28.217  1.00  12.67        C
ATOM   4350   O     TYR A 307      18.140  29.113  28.271  1.00  13.67        O
ATOM   4351   N     PRO A 308      19.440  27.555  27.389  1.00  11.31        N
ATOM   4352   CA    PRO A 308      20.118  28.547  26.531  1.00  11.31        C
ATOM   4354   CB    PRO A 308      21.315  27.780  25.971  1.00  10.82        C
ATOM   4357   CG    PRO A 308      20.941  26.293  26.070  1.00  11.80        C
ATOM   4360   CD    PRO A 308      19.833  26.179  27.077  1.00  12.92        C
ATOM   4363   C     PRO A 308      19.245  29.003  25.361  1.00  11.34        C
ATOM   4364   O     PRO A 308      18.604  28.159  24.689  1.00  10.74        O
ATOM   4365   N     ARG A 309      19.314  30.266  25.051  1.00  12.86        N
ATOM   4367   CA    ARG A 309      18.427  30.864  24.076  1.00  13.91        C
ATOM   4369   CB    ARG A 309      17.252  31.527  24.844  1.00  13.02        C
```

```
ATOM  4372  CG   ARG A 309   16.469  30.638  25.781  1.00  13.54      C
ATOM  4375  CD   ARG A 309   15.375  31.251  26.661  1.00  14.50      C
ATOM  4378  NE   ARG A 309   14.099  31.449  25.978  1.00  15.52      N
ATOM  4380  CZ   ARG A 309   13.022  31.993  26.525  1.00  16.18      C
ATOM  4381  NH1  ARG A 309   13.055  32.423  27.777  1.00  12.53      N
ATOM  4384  NH2  ARG A 309   11.891  32.006  25.832  1.00  15.18      N
ATOM  4387  C    ARG A 309   19.139  31.870  23.213  1.00  14.57      C
ATOM  4388  O    ARG A 309   20.288  32.256  23.468  1.00  13.36      O
ATOM  4389  N    VAL A 310   18.455  32.367  22.180  1.00  13.70      N
ATOM  4391  CA   VAL A 310   19.053  33.390  21.325  1.00  15.05      C
ATOM  4393  CB   VAL A 310   18.134  33.596  20.101  1.00  14.71      C
ATOM  4395  CG1  VAL A 310   18.580  34.793  19.258  1.00  18.54      C
ATOM  4399  CG2  VAL A 310   18.121  32.368  19.265  1.00  14.81      C
ATOM  4403  C    VAL A 310   19.272  34.671  22.112  1.00  16.45      C
ATOM  4404  O    VAL A 310   20.251  35.382  21.905  1.00  15.49      O
ATOM  4405  N    LYS A 311   18.316  34.979  22.979  1.00  17.77      N
ATOM  4407  CA   LYS A 311   18.415  36.106  23.940  1.00  18.52      C
ATOM  4409  CB   LYS A 311   17.942  37.380  23.244  1.00  18.11      C
ATOM  4412  CG   LYS A 311   16.677  37.137  22.440  1.00  23.20      C
ATOM  4415  CD   LYS A 311   15.574  38.112  22.630  1.00  33.02      C
ATOM  4418  CE   LYS A 311   15.667  39.252  21.732  1.00  34.63      C
ATOM  4421  NZ   LYS A 311   14.289  39.716  21.277  1.00  36.21      N
ATOM  4425  C    LYS A 311   17.532  35.897  25.178  1.00  17.62      C
ATOM  4426  O    LYS A 311   16.770  34.912  25.199  1.00  16.50      O
ATOM  4427  OXT  LYS A 311   17.534  36.644  26.172  1.00  19.57      O
ATOM  4428  CA   CA  C 312   28.232  18.547  38.069  1.00  13.89      CA
ATOM  4429  CA   CA  C 313   17.608  25.091  37.856  1.00  18.60      CA
ATOM  4430  CA   CA  C 314   27.338  14.925  29.013  0.60  10.25      CA
ATOM  4431  N    ALA B 318    2.727   2.475  36.156  1.00  30.60      N
ATOM  4433  CA   ALA B 318    2.319   3.152  34.902  1.00  28.10      C
ATOM  4435  CB   ALA B 318    1.428   4.295  35.200  1.00  27.75      C
ATOM  4439  C    ALA B 318    3.422   3.533  33.900  1.00  24.85      C
ATOM  4440  O    ALA B 318    3.103   3.596  32.739  1.00  27.46      O
ATOM  4443  N    THR B 319    4.625   3.965  34.273  1.00  24.93      N
ATOM  4445  CA   THR B 319    5.658   4.224  33.231  1.00  23.26      C
ATOM  4447  CB   THR B 319    6.154   5.690  33.296  1.00  24.03      C
ATOM  4449  OG1  THR B 319    6.811   5.953  34.535  1.00  27.37      O
ATOM  4451  CG2  THR B 319    4.960   6.670  33.258  1.00  27.35      C
ATOM  4455  C    THR B 319    6.926   3.305  33.235  1.00  22.75      C
ATOM  4456  O    THR B 319    7.820   3.498  32.406  1.00  21.26      O
ATOM  4457  N    GLU B 320    7.027   2.401  34.205  1.00  21.07      N
ATOM  4459  CA   GLU B 320    8.177   1.559  34.324  1.00  21.49      C
ATOM  4461  CB   GLU B 320    9.328   2.280  35.014  1.00  21.67      C
ATOM  4464  CG   GLU B 320    8.980   2.681  36.413  1.00  25.74      C
ATOM  4467  CD   GLU B 320   10.174   3.130  37.222  1.00  34.05      C
ATOM  4468  OE1  GLU B 320   10.962   3.910  36.698  1.00  36.87      O
ATOM  4469  OE2  GLU B 320   10.295   2.711  38.394  1.00  39.11      O
ATOM  4470  C    GLU B 320    7.818   0.308  35.082  1.00  20.20      C
ATOM  4471  O    GLU B 320    6.914   0.330  35.945  1.00  20.85      O
ATOM  4472  N    TRP B 321    8.526  -0.774  34.748  1.00  18.06      N
ATOM  4474  CA   TRP B 321    8.271  -2.092  35.310  1.00  17.54      C
ATOM  4476  CB   TRP B 321    7.595  -2.961  34.277  1.00  15.93      C
ATOM  4479  CG   TRP B 321    6.265  -2.537  33.906  1.00  18.41      C
ATOM  4480  CD1  TRP B 321    5.089  -3.007  34.445  1.00  16.53      C
ATOM  4482  NE1  TRP B 321    4.017  -2.406  33.836  1.00  19.73      N
ATOM  4484  CE2  TRP B 321    4.470  -1.523  32.886  1.00  15.64      C
ATOM  4485  CD2  TRP B 321    5.889  -1.576  32.903  1.00  15.37      C
ATOM  4486  CE3  TRP B 321    6.596  -0.772  31.992  1.00  14.76      C
ATOM  4488  CZ3  TRP B 321    5.852   0.097  31.140  1.00  17.06      C
ATOM  4490  CH2  TRP B 321    4.428   0.089  31.165  1.00  17.68      C
ATOM  4492  CZ2  TRP B 321    3.757  -0.693  32.047  1.00  18.99      C
ATOM  4494  C    TRP B 321    9.570  -2.716  35.728  1.00  16.82      C
ATOM  4495  O    TRP B 321   10.068  -3.654  35.131  1.00  16.09      O
```

```
ATOM   4496  N   PRO B 322    10.186  -2.151  36.755  1.00  18.33        N
ATOM   4497  CA  PRO B 322    11.493  -2.628  37.202  1.00  19.01        C
ATOM   4499  CB  PRO B 322    11.829  -1.725  38.392  1.00  19.99        C
ATOM   4502  CG  PRO B 322    10.556  -1.019  38.744  1.00  19.55        C
ATOM   4505  CD  PRO B 322     9.651  -1.061  37.575  1.00  19.07        C
ATOM   4508  C   PRO B 322    11.484  -4.065  37.641  1.00  19.86        C
ATOM   4509  O   PRO B 322    12.495  -4.748  37.546  1.00  20.25        O
ATOM   4510  N   GLU B 323    10.334  -4.557  38.050  1.00  20.04        N
ATOM   4512  CA  GLU B 323    10.262  -5.906  38.546  1.00  20.93        C
ATOM   4514  CB  GLU B 323     8.960  -6.041  39.331  1.00  23.24        C
ATOM   4517  CG  GLU B 323     7.708  -5.954  38.441  1.00  26.81        C
ATOM   4520  CD  GLU B 323     7.184  -4.530  38.089  1.00  27.51        C
ATOM   4521  OE1 GLU B 323     7.879  -3.511  38.275  1.00  20.70        O
ATOM   4522  OE2 GLU B 323     5.996  -4.461  37.641  1.00  30.50        O
ATOM   4523  C   GLU B 323    10.325  -6.934  37.407  1.00  18.55        C
ATOM   4524  O   GLU B 323    10.521  -8.111  37.642  1.00  16.50        O
ATOM   4525  N   LEU B 324    10.256  -6.485  36.172  1.00  16.08        N
ATOM   4527  CA  LEU B 324    10.357  -7.398  35.050  1.00  15.61        C
ATOM   4529  CB  LEU B 324     9.626  -6.849  33.846  1.00  15.54        C
ATOM   4532  CG  LEU B 324     8.113  -6.779  34.039  1.00  16.64        C
ATOM   4534  CD1 LEU B 324     7.437  -6.061  32.923  1.00  19.42        C
ATOM   4538  CD2 LEU B 324     7.648  -8.212  34.152  1.00  19.22        C
ATOM   4542  C   LEU B 324    11.815  -7.755  34.643  1.00  14.32        C
ATOM   4543  O   LEU B 324    12.017  -8.619  33.836  1.00  14.01        O
ATOM   4544  N   VAL B 325    12.792  -7.073  35.189  1.00  15.66        N
ATOM   4546  CA  VAL B 325    14.180  -7.370  34.816  1.00  15.56        C
ATOM   4548  CB  VAL B 325    15.184  -6.410  35.456  1.00  15.71        C
ATOM   4550  CG1 VAL B 325    16.629  -6.860  35.094  1.00  16.62        C
ATOM   4554  CG2 VAL B 325    14.953  -4.946  34.988  1.00  14.67        C
ATOM   4558  C   VAL B 325    14.478  -8.817  35.197  1.00  15.70        C
ATOM   4559  O   VAL B 325    14.181  -9.219  36.316  1.00  15.01        O
ATOM   4560  N   GLY B 326    14.985  -9.609  34.247  1.00  16.26        N
ATOM   4562  CA  GLY B 326    15.302 -11.008  34.494  1.00  15.67        C
ATOM   4565  C   GLY B 326    14.166 -11.958  34.140  1.00  16.74        C
ATOM   4566  O   GLY B 326    14.358 -13.159  34.108  1.00  16.04        O
ATOM   4567  N   LYS B 327    12.957 -11.432  33.950  1.00  16.30        N
ATOM   4569  CA  LYS B 327    11.848 -12.266  33.510  1.00  17.25        C
ATOM   4571  CB  LYS B 327    10.514 -11.632  33.958  1.00  17.70        C
ATOM   4574  CG  LYS B 327    10.573 -11.307  35.417  1.00  22.70        C
ATOM   4577  CD  LYS B 327     9.217 -11.378  36.140  1.00  30.58        C
ATOM   4580  CE  LYS B 327     9.404 -11.302  37.674  1.00  31.61        C
ATOM   4583  NZ  LYS B 327    10.674 -11.962  38.027  1.00  33.63        N
ATOM   4587  C   LYS B 327    11.837 -12.497  32.021  1.00  16.69        C
ATOM   4588  O   LYS B 327    12.479 -11.805  31.222  1.00  15.76        O
ATOM   4589  N   SER B 328    11.055 -13.485  31.601  1.00  18.24        N
ATOM   4591  CA  SER B 328    10.972 -13.809  30.188  1.00  17.59        C
ATOM   4593  CB  SER B 328    10.280 -15.154  29.994  1.00  19.45        C
ATOM   4596  OG  SER B 328     8.887 -15.026  30.192  1.00  18.39        O
ATOM   4598  C   SER B 328    10.206 -12.757  29.431  1.00  18.63        C
ATOM   4599  O   SER B 328     9.375 -12.020  30.003  1.00  17.45        O
ATOM   4600  N   VAL B 329    10.494 -12.653  28.140  1.00  18.81        N
ATOM   4602  CA  VAL B 329     9.771 -11.690  27.299  1.00  20.52        C
ATOM   4604  CB  VAL B 329    10.317 -11.663  25.852  1.00  21.15        C
ATOM   4606  CG1 VAL B 329     9.817 -12.809  25.060  1.00  22.84        C
ATOM   4610  CG2 VAL B 329     9.914 -10.411  25.159  1.00  25.35        C
ATOM   4614  C   VAL B 329     8.276 -11.962  27.279  1.00  20.54        C
ATOM   4615  O   VAL B 329     7.460 -11.049  27.237  1.00  19.76        O
ATOM   4616  N   GLU B 330     7.906 -13.228  27.332  1.00  21.77        N
ATOM   4618  CA  GLU B 330     6.504 -13.544  27.379  1.00  22.29        C
ATOM   4620  CB  GLU B 330     6.331 -15.048  27.153  1.00  24.60        C
ATOM   4623  CG  GLU B 330     6.850 -15.472  25.754  1.00  27.68        C
ATOM   4626  CD  GLU B 330     8.341 -15.854  25.674  1.00  35.11        C
ATOM   4627  OE1 GLU B 330     9.141 -15.647  26.617  1.00  28.88        O
```

70

```
ATOM   4628  OE2 GLU B 330      8.722 -16.426  24.632  1.00 41.94           O
ATOM   4629  C   GLU B 330      5.809 -13.084  28.642  1.00 22.11           C
ATOM   4630  O   GLU B 330      4.676 -12.586  28.584  1.00 21.27           O
ATOM   4631  N   GLU B 331      6.452 -13.239  29.790  1.00 21.03           N
ATOM   4633  CA  GLU B 331      5.904 -12.751  31.043  1.00 21.48           C
ATOM   4635  CB  GLU B 331      6.730 -13.205  32.262  1.00 22.76           C
ATOM   4638  CG  GLU B 331      5.844 -13.545  33.454  1.00 31.85           C
ATOM   4641  CD  GLU B 331      6.490 -13.408  34.816  1.00 38.17           C
ATOM   4642  OE1 GLU B 331      7.584 -14.024  35.014  1.00 46.35           O
ATOM   4643  OE2 GLU B 331      5.886 -12.712  35.693  1.00 37.37           O
ATOM   4644  C   GLU B 331      5.838 -11.243  31.046  1.00 19.51           C
ATOM   4645  O   GLU B 331      4.892 -10.668  31.537  1.00 18.89           O
ATOM   4646  N   ALA B 332      6.882 -10.609  30.514  1.00 18.18           N
ATOM   4648  CA  ALA B 332      6.951  -9.187  30.538  1.00 15.95           C
ATOM   4650  CB  ALA B 332      8.254  -8.709  29.931  1.00 16.83           C
ATOM   4654  C   ALA B 332      5.801  -8.640  29.757  1.00 15.42           C
ATOM   4655  O   ALA B 332      5.163  -7.697  30.182  1.00 14.99           O
ATOM   4656  N   LYS B 333      5.580  -9.189  28.589  1.00 15.39           N
ATOM   4658  CA  LYS B 333      4.489  -8.693  27.748  1.00 17.34           C
ATOM   4660  CB  LYS B 333      4.458  -9.450  26.441  1.00 17.67           C
ATOM   4663  CG  LYS B 333      5.438  -9.004  25.401  1.00 21.32           C
ATOM   4666  CD  LYS B 333      5.200  -9.807  24.128  1.00 23.42           C
ATOM   4669  CE  LYS B 333      6.357  -9.619  23.113  1.00 28.93           C
ATOM   4672  NZ  LYS B 333      6.005 -10.290  21.820  1.00 28.19           N
ATOM   4676  C   LYS B 333      3.127  -8.789  28.452  1.00 17.99           C
ATOM   4677  O   LYS B 333      2.338  -7.868  28.410  1.00 15.95           O
ATOM   4678  N   LYS B 334      2.858  -9.904  29.125  1.00 19.30           N
ATOM   4680  CA  LYS B 334      1.595 -10.046  29.842  1.00 19.86           C
ATOM   4682  CB  LYS B 334      1.417 -11.432  30.481  1.00 21.24           C
ATOM   4685  CG  LYS B 334      0.949 -12.476  29.568  1.00 28.52           C
ATOM   4688  CD  LYS B 334      0.499 -13.819  30.317  1.00 33.48           C
ATOM   4691  CE  LYS B 334      0.650 -15.022  29.319  1.00 37.14           C
ATOM   4694  NZ  LYS B 334      0.870 -14.604  27.850  1.00 36.97           N
ATOM   4698  C   LYS B 334      1.457  -8.983  30.890  1.00 18.97           C
ATOM   4699  O   LYS B 334      0.386  -8.343  31.021  1.00 16.86           O
ATOM   4700  N   VAL B 335      2.522  -8.754  31.658  1.00 16.84           N
ATOM   4702  CA  VAL B 335      2.480  -7.783  32.726  1.00 16.65           C
ATOM   4704  CB  VAL B 335      3.743  -7.823  33.591  1.00 15.76           C
ATOM   4706  CG1 VAL B 335      3.817  -6.655  34.612  1.00 19.24           C
ATOM   4710  CG2 VAL B 335      3.789  -9.108  34.341  1.00 17.38           C
ATOM   4714  C   VAL B 335      2.288  -6.382  32.203  1.00 15.76           C
ATOM   4715  O   VAL B 335      1.494  -5.615  32.763  1.00 16.18           O
ATOM   4716  N   ILE B 336      3.033  -6.023  31.167  1.00 15.08           N
ATOM   4718  CA  ILE B 336      2.903  -4.703  30.635  1.00 14.79           C
ATOM   4720  CB  ILE B 336      3.967  -4.441  29.592  1.00 14.82           C
ATOM   4722  CG1 ILE B 336      5.290  -4.318  30.367  1.00 16.31           C
ATOM   4725  CD1 ILE B 336      6.479  -4.602  29.579  1.00 19.31           C
ATOM   4729  CG2 ILE B 336      3.543  -3.266  28.733  1.00 16.34           C
ATOM   4733  C   ILE B 336      1.508  -4.472  30.074  1.00 15.10           C
ATOM   4734  O   ILE B 336      0.914  -3.437  30.347  1.00 16.57           O
ATOM   4735  N   LEU B 337      0.956  -5.456  29.390  1.00 15.14           N
ATOM   4737  CA  LEU B 337     -0.343  -5.235  28.769  1.00 15.94           C
ATOM   4739  CB  LEU B 337     -0.645  -6.290  27.727  1.00 15.77           C
ATOM   4742  CG  LEU B 337      0.121  -6.195  26.404  1.00 13.58           C
ATOM   4744  CD1 LEU B 337      0.021  -7.498  25.632  1.00 15.44           C
ATOM   4748  CD2 LEU B 337     -0.350  -5.049  25.553  1.00 17.85           C
ATOM   4752  C   LEU B 337     -1.450  -5.146  29.810  1.00 17.57           C
ATOM   4753  O   LEU B 337     -2.511  -4.531  29.544  1.00 17.36           O
ATOM   4754  N   GLN B 338     -1.210  -5.702  30.985  1.00 18.09           N
ATOM   4756  CA  GLN B 338     -2.195  -5.610  32.047  1.00 21.31           C
ATOM   4758  CB  GLN B 338     -1.830  -6.557  33.205  1.00 21.97           C
ATOM   4761  CG  GLN B 338     -1.842  -8.050  32.775  1.00 26.01           C
ATOM   4764  CD  GLN B 338     -1.562  -9.127  33.893  1.00 29.25           C
```

```
ATOM  4765  OE1 GLN B 338   -0.720  -8.942  34.831  1.00 34.90      O
ATOM  4766  NE2 GLN B 338   -2.211 -10.262  33.738  1.00 29.07      N
ATOM  4769  C   GLN B 338   -2.398  -4.143  32.469  1.00 22.67      C
ATOM  4770  O   GLN B 338   -3.530  -3.694  32.741  1.00 22.11      O
ATOM  4771  N   ASP B 339   -1.318  -3.366  32.465  1.00 21.30      N
ATOM  4773  CA  ASP B 339   -1.377  -1.936  32.761  1.00 21.98      C
ATOM  4775  CB  ASP B 339   -0.047  -1.478  33.381  1.00 22.85      C
ATOM  4778  CG  ASP B 339    0.213  -2.106  34.710  1.00 26.72      C
ATOM  4779  OD1 ASP B 339   -0.740  -2.105  35.518  1.00 33.25      O
ATOM  4780  OD2 ASP B 339    1.269  -2.680  35.026  1.00 26.47      O
ATOM  4781  C   ASP B 339   -1.644  -1.066  31.555  1.00 22.06      C
ATOM  4782  O   ASP B 339   -2.247   0.004  31.667  1.00 23.60      O
ATOM  4783  N   LYS B 340   -1.218  -1.515  30.387  1.00 19.19      N
ATOM  4785  CA  LYS B 340   -1.226  -0.722  29.218  1.00 18.98      C
ATOM  4787  CB  LYS B 340    0.186  -0.070  29.118  1.00 18.88      C
ATOM  4790  CG  LYS B 340    0.345   0.901  28.024  1.00 18.59      C
ATOM  4793  CD  LYS B 340    1.805   1.491  28.014  1.00 19.17      C
ATOM  4796  CE  LYS B 340    1.999   2.462  26.848  1.00 20.90      C
ATOM  4799  NZ  LYS B 340    1.137   3.683  27.041  1.00 23.19      N
ATOM  4803  C   LYS B 340   -1.611  -1.550  28.035  1.00 18.75      C
ATOM  4804  O   LYS B 340   -0.822  -1.920  27.224  1.00 18.54      O
ATOM  4805  N   PRO B 341   -2.906  -1.822  27.892  1.00 17.67      N
ATOM  4806  CA  PRO B 341   -3.391  -2.768  26.911  1.00 17.68      C
ATOM  4808  CB  PRO B 341   -4.926  -2.753  27.108  1.00 18.59      C
ATOM  4811  CG  PRO B 341   -5.155  -1.955  28.331  1.00 19.20      C
ATOM  4814  CD  PRO B 341   -3.919  -1.347  28.825  1.00 20.06      C
ATOM  4817  C   PRO B 341   -3.101  -2.444  25.491  1.00 15.31      C
ATOM  4818  O   PRO B 341   -3.022  -3.289  24.614  1.00 19.02      O
ATOM  4819  N   GLU B 342   -2.986  -1.143  25.254  1.00 17.33      N
ATOM  4821  CA  GLU B 342   -2.745  -0.687  23.906  1.00 17.70      C
ATOM  4823  CB  GLU B 342   -3.615   0.545  23.555  1.00 20.27      C
ATOM  4826  CG  GLU B 342   -5.028   0.042  23.191  1.00 21.84      C
ATOM  4829  CD  GLU B 342   -6.029   1.135  22.803  1.00 30.03      C
ATOM  4830  OE1 GLU B 342   -5.646   2.108  22.127  1.00 34.62      O
ATOM  4831  OE2 GLU B 342   -7.217   0.982  23.131  1.00 29.42      O
ATOM  4832  C   GLU B 342   -1.232  -0.491  23.581  1.00 17.99      C
ATOM  4833  O   GLU B 342   -0.912  -0.071  22.473  1.00 17.45      O
ATOM  4834  N   ALA B 343   -0.348  -0.902  24.474  1.00 18.01      N
ATOM  4836  CA  ALA B 343    1.075  -0.825  24.187  1.00 17.75      C
ATOM  4838  CB  ALA B 343    1.886  -1.320  25.374  1.00 17.76      C
ATOM  4842  C   ALA B 343    1.501  -1.509  22.934  1.00 18.41      C
ATOM  4843  O   ALA B 343    1.018  -2.612  22.539  1.00 16.39      O
ATOM  4844  N   GLN B 344    2.398  -0.808  22.223  1.00 16.93      N
ATOM  4846  CA  GLN B 344    3.007  -1.326  21.061  1.00 16.94      C
ATOM  4848  CB  GLN B 344    3.197  -0.264  19.951  1.00 18.67      C
ATOM  4851  CG  GLN B 344    1.915   0.367  19.450  1.00 22.45      C
ATOM  4854  CD  GLN B 344    1.041  -0.668  18.836  1.00 23.74      C
ATOM  4855  OE1 GLN B 344    1.336  -1.176  17.737  1.00 28.25      O
ATOM  4856  NE2 GLN B 344    0.022  -1.076  19.570  1.00 24.17      N
ATOM  4859  C   GLN B 344    4.371  -1.804  21.578  1.00 16.61      C
ATOM  4860  O   GLN B 344    5.276  -0.987  21.833  1.00 15.55      O
ATOM  4861  N   ILE B 345    4.503  -3.109  21.745  1.00 14.81      N
ATOM  4863  CA  ILE B 345    5.747  -3.697  22.338  1.00 15.57      C
ATOM  4865  CB  ILE B 345    5.426  -4.864  23.229  1.00 14.52      C
ATOM  4867  CG1 ILE B 345    4.420  -4.437  24.307  1.00 16.03      C
ATOM  4870  CD1 ILE B 345    4.237  -5.385  25.452  1.00 21.70      C
ATOM  4874  CG2 ILE B 345    6.683  -5.445  23.832  1.00 18.60      C
ATOM  4878  C   ILE B 345    6.713  -4.108  21.261  1.00 15.60      C
ATOM  4879  O   ILE B 345    6.317  -4.759  20.335  1.00 14.53      O
ATOM  4880  N   ILE B 346    7.947  -3.618  21.338  1.00 15.09      N
ATOM  4882  CA  ILE B 346    9.006  -3.853  20.373  1.00 17.65      C
ATOM  4884  CB  ILE B 346    9.607  -2.448  19.887  1.00 18.73      C
ATOM  4886  CG1 ILE B 346    8.486  -1.515  19.488  1.00 25.44      C
```

72

```
ATOM   4889   CD1  ILE  B  346      7.656   -2.110   18.400   1.00   27.09          C
ATOM   4893   CG2  ILE  B  346     10.538   -2.639   18.736   1.00   24.66          C
ATOM   4897   C    ILE  B  346     10.126   -4.549   21.136   1.00   15.44          C
ATOM   4898   O    ILE  B  346     10.515   -4.048   22.174   1.00   15.03          O
ATOM   4899   N    VAL  B  347     10.621   -5.685   20.637   1.00   15.30          N
ATOM   4901   CA   VAL  B  347     11.701   -6.425   21.310   1.00   14.26          C
ATOM   4903   CB   VAL  B  347     11.363   -7.916   21.411   1.00   13.95          C
ATOM   4905   CG1  VAL  B  347     12.550   -8.744   21.949   1.00   15.52          C
ATOM   4909   CG2  VAL  B  347     10.056   -8.103   22.171   1.00   16.01          C
ATOM   4913   C    VAL  B  347     12.980   -6.253   20.518   1.00   13.34          C
ATOM   4914   O    VAL  B  347     12.999   -6.387   19.275   1.00   13.71          O
ATOM   4915   N    LEU  B  348     14.025   -5.824   21.225   1.00   13.58          N
ATOM   4917   CA   LEU  B  348     15.334   -5.595   20.654   1.00   14.50          C
ATOM   4919   CB   LEU  B  348     15.629   -4.101   20.619   1.00   14.73          C
ATOM   4922   CG   LEU  B  348     14.624   -3.255   19.836   1.00   18.66          C
ATOM   4924   CD1  LEU  B  348     14.885   -1.758   20.172   1.00   20.34          C
ATOM   4928   CD2  LEU  B  348     14.781   -3.515   18.370   1.00   21.48          C
ATOM   4932   C    LEU  B  348     16.420   -6.246   21.500   1.00   13.69          C
ATOM   4933   O    LEU  B  348     16.298   -6.400   22.715   1.00   12.97          O
ATOM   4934   N    PRO  B  349     17.533   -6.626   20.864   1.00   13.80          N
ATOM   4935   CA   PRO  B  349     18.630   -7.173   21.635   1.00   12.71          C
ATOM   4937   CB   PRO  B  349     19.686   -7.528   20.564   1.00   14.22          C
ATOM   4940   CG   PRO  B  349     18.940   -7.614   19.288   1.00   17.12          C
ATOM   4943   CD   PRO  B  349     17.802   -6.586   19.422   1.00   13.86          C
ATOM   4946   C    PRO  B  349     19.238   -6.154   22.603   1.00   12.67          C
ATOM   4947   O    PRO  B  349     19.358   -4.987   22.270   1.00   10.43          O
ATOM   4948   N    VAL  B  350     19.679   -6.629   23.756   1.00   10.88          N
ATOM   4950   CA   VAL  B  350     20.463   -5.844   24.676   1.00   11.84          C
ATOM   4952   CB   VAL  B  350     20.967   -6.740   25.861   1.00   12.85          C
ATOM   4954   CG1  VAL  B  350     21.918   -7.822   25.385   1.00   14.03          C
ATOM   4958   CG2  VAL  B  350     21.614   -5.822   26.921   1.00   13.28          C
ATOM   4962   C    VAL  B  350     21.627   -5.189   23.892   1.00   10.88          C
ATOM   4963   O    VAL  B  350     22.262   -5.815   23.000   1.00   10.87          O
ATOM   4964   N    GLY  B  351     21.864   -3.926   24.205   1.00   11.86          N
ATOM   4966   CA   GLY  B  351     22.882   -3.099   23.581   1.00   11.51          C
ATOM   4969   C    GLY  B  351     22.512   -2.331   22.313   1.00   12.47          C
ATOM   4970   O    GLY  B  351     23.335   -1.618   21.744   1.00   10.98          O
ATOM   4971   N    THR  B  352     21.315   -2.561   21.822   1.00   11.64          N
ATOM   4973   CA   THR  B  352     20.841   -1.861   20.642   1.00   11.92          C
ATOM   4975   CB   THR  B  352     19.508   -2.419   20.225   1.00   13.71          C
ATOM   4977   OG1  THR  B  352     19.641   -3.812   19.860   1.00   10.89          O
ATOM   4979   CG2  THR  B  352     18.993   -1.703   18.981   1.00   12.02          C
ATOM   4983   C    THR  B  352     20.720   -0.383   20.920   1.00   11.24          C
ATOM   4984   O    THR  B  352     20.235    0.015   21.976   1.00   12.17          O
ATOM   4985   N    ILE  B  353     21.218    0.435   20.012   1.00   11.34          N
ATOM   4987   CA   ILE  B  353     21.125    1.893   20.166   1.00   11.58          C
ATOM   4989   CB   ILE  B  353     22.322    2.605   19.504   1.00   13.21          C
ATOM   4991   CG1  ILE  B  353     23.642    2.014   19.992   1.00   11.55          C
ATOM   4994   CD1  ILE  B  353     23.795    1.980   21.496   1.00   15.61          C
ATOM   4998   CG2  ILE  B  353     22.294    4.098   19.802   1.00   13.36          C
ATOM   5002   C    ILE  B  353     19.828    2.332   19.509   1.00   11.44          C
ATOM   5003   O    ILE  B  353     19.485    1.827   18.417   1.00   11.02          O
ATOM   5004   N    VAL  B  354     19.136    3.268   20.180   1.00   10.73          N
ATOM   5006   CA   VAL  B  354     17.785    3.663   19.740   1.00   11.42          C
ATOM   5008   CB   VAL  B  354     16.693    3.043   20.663   1.00   11.30          C
ATOM   5010   CG1  VAL  B  354     16.741    1.521   20.568   1.00   11.88          C
ATOM   5014   CG2  VAL  B  354     16.873    3.542   22.103   1.00   12.49          C
ATOM   5018   C    VAL  B  354     17.558    5.152   19.702   1.00   10.76          C
ATOM   5019   O    VAL  B  354     18.289    5.918   20.294   1.00   11.15          O
ATOM   5020   N    THR  B  355     16.607    5.599   18.894   1.00   10.94          N
ATOM   5022   CA   THR  B  355     16.207    7.001   18.884   1.00   12.16          C
ATOM   5024   CB   THR  B  355     15.004    7.267   17.939   1.00   13.72          C
ATOM   5026   OG1  THR  B  355     13.885    6.481   18.413   1.00   16.24          O
```

```
ATOM   5028   CG2  THR  B  355     15.313    6.840   16.561   1.00  14.33          C
ATOM   5032   C    THR  B  355     15.684    7.378   20.254   1.00  12.37          C
ATOM   5033   O    THR  B  355     15.263    6.554   21.050   1.00  11.43          O
ATOM   5034   N    MET  B  356     15.787    8.659   20.540   1.00  12.52          N
ATOM   5036   CA   MET  B  356     15.258    9.222   21.775   1.00  11.53          C
ATOM   5038   CB   MET  B  356     16.311   10.075   22.468   1.00  11.42          C
ATOM   5041   CG   MET  B  356     17.427    9.202   23.035   1.00  12.95          C
ATOM   5044   SD   MET  B  356     16.825    8.040   24.219   1.00  15.08          S
ATOM   5045   CE   MET  B  356     17.963    6.799   24.107   1.00  20.89          C
ATOM   5049   C    MET  B  356     13.952    9.961   21.568   1.00  13.71          C
ATOM   5050   O    MET  B  356     13.675   11.024   22.215   1.00  12.74          O
ATOM   5051   N    GLU  B  357     13.148    9.417   20.659   1.00  14.50          N
ATOM   5053   CA   GLU  B  357     11.765    9.869   20.543   1.00  14.30          C
ATOM   5055   CB   GLU  B  357     11.135    9.357   19.210   1.00  15.52          C
ATOM   5058   CG   GLU  B  357      9.674    9.735   19.153   1.00  15.25          C
ATOM   5061   CD   GLU  B  357      8.947    9.187   17.960   1.00  18.46          C
ATOM   5062   OE1  GLU  B  357      7.768    9.559   17.802   1.00  19.97          O
ATOM   5063   OE2  GLU  B  357      9.543    8.377   17.237   1.00  20.59          O
ATOM   5064   C    GLU  B  357     11.001    9.282   21.703   1.00  14.50          C
ATOM   5065   O    GLU  B  357     11.233    8.123   22.030   1.00  14.99          O
ATOM   5066   N    TYR  B  358     10.129   10.074   22.335   1.00  13.93          N
ATOM   5068   CA   TYR  B  358      9.207    9.612   23.363   1.00  14.38          C
ATOM   5070   CB   TYR  B  358      9.026   10.601   24.499   1.00  14.43          C
ATOM   5073   CG   TYR  B  358      8.057   10.136   25.563   1.00  12.84          C
ATOM   5074   CD1  TYR  B  358      8.436    9.165   26.487   1.00  14.52          C
ATOM   5076   CE1  TYR  B  358      7.559    8.706   27.463   1.00  19.65          C
ATOM   5078   CZ   TYR  B  358      6.266    9.240   27.504   1.00  19.95          C
ATOM   5079   OH   TYR  B  358      5.321    8.903   28.447   1.00  26.07          O
ATOM   5081   CE2  TYR  B  358      5.873   10.184   26.607   1.00  16.96          C
ATOM   5083   CD2  TYR  B  358      6.767   10.650   25.643   1.00  18.09          C
ATOM   5085   C    TYR  B  358      7.867    9.326   22.712   1.00  15.23          C
ATOM   5086   O    TYR  B  358      7.142   10.261   22.340   1.00  14.02          O
ATOM   5087   N    ARG  B  359      7.551    8.048   22.606   1.00  15.15          N
ATOM   5089   CA   ARG  B  359      6.294    7.557   22.003   1.00  16.88          C
ATOM   5091   CB   ARG  B  359      6.615    6.465   20.998   1.00  18.79          C
ATOM   5094   CG   ARG  B  359      6.824    6.884   19.703   1.00  22.92          C
ATOM   5097   CD   ARG  B  359      6.435    5.794   18.692   1.00  25.80          C
ATOM   5100   NE   ARG  B  359      7.237    6.083   17.556   1.00  25.82          N
ATOM   5102   CZ   ARG  B  359      7.515    5.241   16.593   1.00  26.84          C
ATOM   5103   NH1  ARG  B  359      7.000    3.996   16.593   1.00  26.53          N
ATOM   5106   NH2  ARG  B  359      8.297    5.668   15.627   1.00  28.90          N
ATOM   5109   C    ARG  B  359      5.449    6.946   23.095   1.00  17.14          C
ATOM   5110   O    ARG  B  359      5.767    5.861   23.625   1.00  15.98          O
ATOM   5111   N    ILE  B  360      4.401    7.656   23.504   1.00  16.16          N
ATOM   5113   CA   ILE  B  360      3.633    7.262   24.670   1.00  17.53          C
ATOM   5115   CB   ILE  B  360      2.575    8.354   24.998   1.00  17.66          C
ATOM   5117   CG1  ILE  B  360      1.895    8.067   26.333   1.00  24.45          C
ATOM   5120   CD1  ILE  B  360      1.711    9.278   27.140   1.00  28.72          C
ATOM   5124   CG2  ILE  B  360      1.506    8.417   23.930   1.00  18.76          C
ATOM   5128   C    ILE  B  360      2.947    5.899   24.595   1.00  16.17          C
ATOM   5129   O    ILE  B  360      2.649    5.307   25.629   1.00  19.59          O
ATOM   5130   N    ASP  B  361      2.692    5.431   23.402   1.00  19.19          N
ATOM   5132   CA   ASP  B  361      2.049    4.131   23.290   1.00  20.04          C
ATOM   5134   CB   ASP  B  361      1.070    4.141   22.156   1.00  22.33          C
ATOM   5137   CG   ASP  B  361     -0.210    4.932   22.509   1.00  27.91          C
ATOM   5138   OD1  ASP  B  361     -0.701    4.823   23.693   1.00  33.70          O
ATOM   5139   OD2  ASP  B  361     -0.734    5.711   21.687   1.00  38.62          O
ATOM   5140   C    ASP  B  361      3.025    2.996   23.111   1.00  19.38          C
ATOM   5141   O    ASP  B  361      2.580    1.872   22.958   1.00  18.79          O
ATOM   5142   N    ARG  B  362      4.342    3.291   23.099   1.00  15.34          N
ATOM   5144   CA   ARG  B  362      5.342    2.241   22.870   1.00  15.87          C
ATOM   5146   CB   ARG  B  362      6.480    2.793   21.992   1.00  13.90          C
ATOM   5149   CG   ARG  B  362      7.657    1.839   21.867   1.00  16.63          C
```

```
ATOM    5152   CD   ARG B 362      8.570    2.234   20.721  1.00  16.00          C
ATOM    5155   NE   ARG B 362      9.204    3.494   21.085  1.00  15.40          N
ATOM    5157   CZ   ARG B 362      9.808    4.315   20.234  1.00  14.22          C
ATOM    5158   NH1  ARG B 362      9.893    4.014   18.939  1.00  16.86          N
ATOM    5161   NH2  ARG B 362     10.295    5.449   20.686  1.00  14.53          N
ATOM    5164   C    ARG B 362      5.970    1.737   24.163  1.00  14.75          C
ATOM    5165   O    ARG B 362      6.130    2.504   25.117  1.00  13.39          O
ATOM    5166   N    VAL B 363      6.285    0.467   24.199  1.00  13.88          N
ATOM    5168   CA   VAL B 363      7.159   -0.094   25.241  1.00  14.50          C
ATOM    5170   CB   VAL B 363      6.448   -0.910   26.349  1.00  14.42          C
ATOM    5172   CG1  VAL B 363      7.477   -1.351   27.372  1.00  17.68          C
ATOM    5176   CG2  VAL B 363      5.401   -0.069   27.015  1.00  16.62          C
ATOM    5180   C    VAL B 363      8.230   -0.943   24.576  1.00  14.33          C
ATOM    5181   O    VAL B 363      7.928   -1.989   23.984  1.00  14.74          O
ATOM    5182   N    ARG B 364      9.483   -0.510   24.651  1.00  13.57          N
ATOM    5184   CA   ARG B 364     10.570   -1.349   24.131  1.00  13.59          C
ATOM    5186   CB   ARG B 364     11.753   -0.458   23.713  1.00  12.88          C
ATOM    5189   CG   ARG B 364     11.565    0.380   22.482  1.00  14.21          C
ATOM    5192   CD   ARG B 364     12.671    1.411   22.216  1.00  18.75          C
ATOM    5195   NE   ARG B 364     12.606    2.066   20.950  1.00  16.44          N
ATOM    5197   CZ   ARG B 364     13.123    3.278   20.638  1.00  18.34          C
ATOM    5198   NH1  ARG B 364     13.591    4.073   21.531  1.00  21.95          N
ATOM    5201   NH2  ARG B 364     13.073    3.670   19.412  1.00  23.97          N
ATOM    5204   C    ARG B 364     11.046   -2.339   25.177  1.00  14.09          C
ATOM    5205   O    ARG B 364     11.154   -1.994   26.361  1.00  16.64          O
ATOM    5206   N    LEU B 365     11.340   -3.548   24.735  1.00  13.84          N
ATOM    5208   CA   LEU B 365     11.904   -4.519   25.617  1.00  12.64          C
ATOM    5210   CB   LEU B 365     11.017   -5.737   25.667  1.00  14.17          C
ATOM    5213   CG   LEU B 365      9.653   -5.571   26.305  1.00  14.30          C
ATOM    5215   CD1  LEU B 365      8.907   -6.860   26.267  1.00  17.46          C
ATOM    5219   CD2  LEU B 365      9.813   -5.054   27.714  1.00  18.97          C
ATOM    5223   C    LEU B 365     13.235   -4.929   25.065  1.00  13.48          C
ATOM    5224   O    LEU B 365     13.307   -5.386   23.919  1.00  13.99          O
ATOM    5225   N    PHE B 366     14.271   -4.775   25.887  1.00  12.70          N
ATOM    5227   CA   PHE B 366     15.619   -5.176   25.533  1.00  12.04          C
ATOM    5229   CB   PHE B 366     16.651   -4.148   25.971  1.00  12.03          C
ATOM    5232   CG   PHE B 366     16.476   -2.809   25.291  1.00  13.71          C
ATOM    5233   CD1  PHE B 366     15.591   -1.889   25.820  1.00  13.42          C
ATOM    5235   CE1  PHE B 366     15.373   -0.702   25.229  1.00  16.78          C
ATOM    5237   CZ   PHE B 366     16.025   -0.376   24.078  1.00  16.52          C
ATOM    5239   CE2  PHE B 366     16.899   -1.322   23.444  1.00  15.87          C
ATOM    5241   CD2  PHE B 366     17.121   -2.554   24.083  1.00  15.60          C
ATOM    5243   C    PHE B 366     15.875   -6.502   26.202  1.00  13.03          C
ATOM    5244   O    PHE B 366     15.700   -6.682   27.407  1.00  13.72          O
ATOM    5245   N    VAL B 367     16.319   -7.448   25.407  1.00  13.72          N
ATOM    5247   CA   VAL B 367     16.457   -8.824   25.906  1.00  14.35          C
ATOM    5249   CB   VAL B 367     15.408   -9.719   25.263  1.00  15.42          C
ATOM    5251   CG1  VAL B 367     13.988   -9.325   25.626  1.00  17.97          C
ATOM    5255   CG2  VAL B 367     15.608   -9.853   23.736  1.00  13.87          C
ATOM    5259   C    VAL B 367     17.829   -9.418   25.654  1.00  14.47          C
ATOM    5260   O    VAL B 367     18.508   -9.099   24.686  1.00  13.82          O
ATOM    5261   N    ASP B 368     18.186  -10.385   26.500  1.00  15.21          N
ATOM    5263   CA   ASP B 368     19.421  -11.145   26.337  1.00  14.26          C
ATOM    5265   CB   ASP B 368     20.020  -11.526   27.668  1.00  12.83          C
ATOM    5268   CG   ASP B 368     19.168  -12.492   28.461  1.00  13.78          C
ATOM    5269   OD1  ASP B 368     18.383  -13.277   27.864  1.00  15.03          O
ATOM    5270   OD2  ASP B 368     19.284  -12.487   29.687  1.00  14.11          O
ATOM    5271   C    ASP B 368     19.197  -12.347   25.438  1.00  15.37          C
ATOM    5272   O    ASP B 368     18.090  -12.572   24.940  1.00  13.77          O
ATOM    5273   N    LYS B 369     20.201  -13.219   25.295  1.00  15.53          N
ATOM    5275   CA   LYS B 369     20.096  -14.274   24.320  1.00  16.17          C
ATOM    5277   CB   LYS B 369     21.492  -14.913   24.031  1.00  18.57          C
ATOM    5280   CG   LYS B 369     22.335  -14.040   23.132  1.00  23.43          C
```

```
ATOM   5283   CD   LYS B 369      21.692 -13.930  21.723  1.00 33.33          C
ATOM   5286   CE   LYS B 369      21.825 -15.245  20.863  1.00 37.24          C
ATOM   5289   NZ   LYS B 369      20.834 -15.304  19.703  1.00 39.15          N
ATOM   5293   C    LYS B 369      19.120 -15.342  24.746  1.00 17.09          C
ATOM   5294   O    LYS B 369      18.736 -16.166  23.914  1.00 16.83          O
ATOM   5295   N    LEU B 370      18.744 -15.352  26.029  1.00 15.51          N
ATOM   5297   CA   LEU B 370      17.739 -16.298  26.509  1.00 15.40          C
ATOM   5299   CB   LEU B 370      18.053 -16.814  27.899  1.00 15.53          C
ATOM   5302   CG   LEU B 370      19.358 -17.657  28.054  1.00 17.89          C
ATOM   5304   CD1  LEU B 370      19.624 -17.980  29.490  1.00 16.28          C
ATOM   5308   CD2  LEU B 370      19.237 -18.944  27.314  1.00 17.77          C
ATOM   5312   C    LEU B 370      16.346 -15.711  26.481  1.00 17.02          C
ATOM   5313   O    LEU B 370      15.426 -16.340  27.005  1.00 16.78          O
ATOM   5314   N    ASP B 371      16.191 -14.538  25.884  1.00 15.62          N
ATOM   5316   CA   ASP B 371      14.909 -13.828  25.822  1.00 17.82          C
ATOM   5318   CB   ASP B 371      13.844 -14.610  25.070  1.00 16.70          C
ATOM   5321   CG   ASP B 371      13.943 -14.455  23.561  1.00 26.05          C
ATOM   5322   OD1  ASP B 371      14.579 -13.492  23.026  1.00 26.78          O
ATOM   5323   OD2  ASP B 371      13.333 -15.252  22.835  1.00 35.44          O
ATOM   5324   C    ASP B 371      14.392 -13.390  27.197  1.00 16.31          C
ATOM   5325   O    ASP B 371      13.179 -13.251  27.432  1.00 15.98          O
ATOM   5326   N    ASN B 372      15.334 -13.152  28.115  1.00 14.12          N
ATOM   5328   CA   ASN B 372      15.057 -12.509  29.380  1.00 13.45          C
ATOM   5330   CB   ASN B 372      15.846 -13.137  30.518  1.00 14.25          C
ATOM   5333   CG   ASN B 372      15.359 -14.530  30.868  1.00 17.78          C
ATOM   5334   OD1  ASN B 372      14.170 -14.830  30.726  1.00 15.50          O
ATOM   5335   ND2  ASN B 372      16.280 -15.394  31.268  1.00 15.27          N
ATOM   5338   C    ASN B 372      15.328 -11.025  29.328  1.00 13.56          C
ATOM   5339   O    ASN B 372      16.185 -10.535  28.526  1.00 12.51          O
ATOM   5340   N    ILE B 373      14.521 -10.271  30.078  1.00 12.00          N
ATOM   5342   CA   ILE B 373      14.595  -8.793  30.098  1.00 12.80          C
ATOM   5344   CB   ILE B 373      13.409  -8.177  30.868  1.00 12.48          C
ATOM   5346   CG1  ILE B 373      12.115  -8.624  30.263  1.00 14.38          C
ATOM   5349   CD1  ILE B 373      12.021  -8.386  28.913  1.00 13.59          C
ATOM   5353   CG2  ILE B 373      13.467  -6.685  30.862  1.00 13.18          C
ATOM   5357   C    ILE B 373      15.888  -8.319  30.716  1.00 12.70          C
ATOM   5358   O    ILE B 373      16.217  -8.667  31.872  1.00 13.25          O
ATOM   5359   N    ALA B 374      16.619  -7.490  29.940  1.00 12.49          N
ATOM   5361   CA   ALA B 374      17.962  -7.065  30.334  1.00 11.98          C
ATOM   5363   CB   ALA B 374      18.894  -6.974  29.099  1.00 11.19          C
ATOM   5367   C    ALA B 374      18.005  -5.723  31.031  1.00 12.88          C
ATOM   5368   O    ALA B 374      19.017  -5.394  31.640  1.00 13.61          O
ATOM   5369   N    GLU B 375      16.957  -4.922  30.954  1.00 12.63          N
ATOM   5371   CA   GLU B 375      16.927  -3.628  31.592  1.00 11.71          C
ATOM   5373   CB   GLU B 375      17.669  -2.571  30.740  1.00 13.04          C
ATOM   5376   CG   GLU B 375      17.020  -2.247  29.443  1.00 13.36          C
ATOM   5379   CD   GLU B 375      17.854  -1.238  28.611  1.00 16.47          C
ATOM   5380   OE1  GLU B 375      18.961  -1.591  28.279  1.00 19.52          O
ATOM   5381   OE2  GLU B 375      17.355  -0.152  28.242  1.00 18.89          O
ATOM   5382   C    GLU B 375      15.464  -3.195  31.858  1.00 12.84          C
ATOM   5383   O    GLU B 375      14.563  -3.750  31.276  1.00 12.27          O
ATOM   5384   N    VAL B 376      15.277  -2.235  32.747  1.00 12.90          N
ATOM   5386   CA   VAL B 376      13.918  -1.872  33.175  1.00 13.25          C
ATOM   5388   CB   VAL B 376      13.941  -0.719  34.173  1.00 13.63          C
ATOM   5390   CG1  VAL B 376      12.515  -0.240  34.514  1.00 15.72          C
ATOM   5394   CG2  VAL B 376      14.681  -1.129  35.424  1.00 19.49          C
ATOM   5398   C    VAL B 376      13.110  -1.441  32.018  1.00 12.36          C
ATOM   5399   O    VAL B 376      13.458  -0.494  31.358  1.00 12.35          O
ATOM   5400   N    PRO B 377      12.016  -2.117  31.700  1.00 13.27          N
ATOM   5401   CA   PRO B 377      11.169  -1.588  30.649  1.00 14.34          C
ATOM   5403   CB   PRO B 377      10.141  -2.697  30.437  1.00 15.95          C
ATOM   5406   CG   PRO B 377      10.777  -3.950  31.039  1.00 13.76          C
ATOM   5409   CD   PRO B 377      11.585  -3.453  32.170  1.00 13.52          C
```

```
ATOM   5412   C    PRO B  377   10.472   -0.302   31.046   1.00  14.46        C
ATOM   5413   O    PRO B  377   10.078   -0.158   32.190   1.00  14.50        O
ATOM   5414   N    ARG B  378   10.291    0.603   30.087   1.00  14.99        N
ATOM   5416   CA   ARG B  378    9.687    1.912   30.298   1.00  14.79        C
ATOM   5418   CB   ARG B  378   10.756    3.004   30.386   1.00  14.41        C
ATOM   5421   CG   ARG B  378   11.717    2.809   31.542   1.00  16.49        C
ATOM   5424   CD   ARG B  378   12.848    3.892   31.600   1.00  17.97        C
ATOM   5427   NE   ARG B  378   13.805    3.569   32.642   1.00  15.14        N
ATOM   5429   CZ   ARG B  378   13.602    3.673   33.949   1.00  19.35        C
ATOM   5430   NH1  ARG B  378   12.452    4.173   34.445   1.00  21.30        N
ATOM   5433   NH2  ARG B  378   14.548    3.250   34.752   1.00  20.05        N
ATOM   5436   C    ARG B  378    8.797    2.263   29.129   1.00  14.34        C
ATOM   5437   O    ARG B  378    8.974    1.810   28.013   1.00  14.31        O
ATOM   5438   N    VAL B  379    7.801    3.052   29.411   1.00  13.60        N
ATOM   5440   CA   VAL B  379    7.028    3.650   28.358   1.00  14.99        C
ATOM   5442   CB   VAL B  379    5.847    4.399   28.967   1.00  16.05        C
ATOM   5444   CG1  VAL B  379    5.148    5.163   27.928   1.00  17.97        C
ATOM   5448   CG2  VAL B  379    4.863    3.410   29.606   1.00  19.07        C
ATOM   5452   C    VAL B  379    7.899    4.629   27.551   1.00  13.40        C
ATOM   5453   O    VAL B  379    8.683    5.397   28.127   1.00  13.64        O
ATOM   5454   N    GLY B  380    7.742    4.666   26.241   1.00  13.02        N
ATOM   5456   CA   GLY B  380    8.480    5.637   25.459   1.00  14.94        C
ATOM   5459   C    GLY B  380    8.968    5.181   24.107   1.00  13.89        C
ATOM   5460   O    GLY B  380    8.939    3.974   23.839   1.00  13.82        O
ATOM   5461   OXT  GLY B  380    9.391    6.068   23.366   1.00  15.44        O
ATOM   5462   O    HOH W    1   26.337   16.956   29.710   1.00   8.65        O
ATOM   5465   O    HOH W    2    9.939    2.105   25.538   1.00  12.99        O
ATOM   5468   O    HOH W    3   22.328   10.101   24.400   1.00  12.55        O
ATOM   5471   O    HOH W    4   30.572   18.292   23.118   1.00   9.33        O
ATOM   5474   O    HOH W    5    8.147   23.150   18.782   1.00  14.00        O
ATOM   5477   O    HOH W    6   11.956   29.794   31.575   1.00  15.66        O
ATOM   5480   O    HOH W    7   36.742   23.674   17.265   1.00  14.06        O
ATOM   5483   O    HOH W    8   26.462   19.745   37.226   1.00  12.03        O
ATOM   5486   O    HOH W    9   23.101    0.721   12.656   1.00  12.19        O
ATOM   5489   O    HOH W   10   20.065   -2.650   26.156   1.00  20.32        O
ATOM   5492   O    HOH W   11   18.435   36.223   15.049   1.00  20.55        O
ATOM   5495   O    HOH W   12   18.961  -14.287   31.415   1.00  12.28        O
ATOM   5498   O    HOH W   13   13.655   -3.630   28.503   1.00  11.25        O
ATOM   5501   O    HOH W   14    6.772   28.494   24.564   1.00  16.02        O
ATOM   5504   O    HOH W   15   25.827   -0.949   23.096   1.00  13.13        O
ATOM   5507   O    HOH W   16   10.548   23.630   17.206   1.00  15.05        O
ATOM   5510   O    HOH W   17   21.366   -0.008   27.896   1.00  14.00        O
ATOM   5513   O    HOH W   18    6.571   29.390   22.100   1.00  18.38        O
ATOM   5516   O    HOH W   19   25.418    4.779   24.010   1.00  11.22        O
ATOM   5519   O    HOH W   20   15.446   15.813   32.417   1.00  13.39        O
ATOM   5522   O    HOH W   21    5.625   22.360   17.718   1.00  14.91        O
ATOM   5525   O    HOH W   22   27.953    5.617   25.060   1.00  12.69        O
ATOM   5528   O    HOH W   23   13.200   17.441   16.128   1.00  12.98        O
ATOM   5531   O    HOH W   24   42.359   19.143   18.719   1.00  14.71        O
ATOM   5534   O    HOH W   25   24.537   -2.025   19.216   1.00  12.97        O
ATOM   5537   O    HOH W   26   27.926   25.732    6.249   1.00  17.25        O
ATOM   5540   O    HOH W   27   39.025   23.474   22.653   1.00  12.82        O
ATOM   5543   O    HOH W   28   23.815   15.465   37.744   1.00  13.10        O
ATOM   5546   O    HOH W   29   18.367   -9.817   33.092   1.00  17.61        O
ATOM   5549   O    HOH W   30   20.380   12.554   15.652   1.00  10.66        O
ATOM   5552   O    HOH W   31   18.651    1.596   26.271   1.00  14.85        O
ATOM   5555   O    HOH W   32   35.209    6.007   10.838   1.00  15.93        O
ATOM   5558   O    HOH W   33   18.465   24.874   35.632   1.00  11.70        O
ATOM   5561   O    HOH W   34   20.815   27.470   36.539   1.00  14.90        O
ATOM   5564   O    HOH W   35   20.733   10.911    9.565   1.00  14.64        O
ATOM   5567   O    HOH W   36    4.788   27.744   28.587   1.00  16.40        O
ATOM   5570   O    HOH W   37    8.972   17.007   35.207   1.00  15.88        O
ATOM   5573   O    HOH W   38   33.433   11.882   27.279   1.00  15.71        O
```

```
ATOM    5576  O    HOH  W   39    11.974    0.247   27.662   1.00  14.69          O
ATOM    5579  O    HOH  W   40    11.026   22.081   14.883   1.00  14.23          O
ATOM    5582  O    HOH  W   41    26.884   -0.716   26.776   1.00  14.35          O
ATOM    5585  O    HOH  W   42    41.266   13.179   18.993   1.00  17.71          O
ATOM    5588  O    HOH  W   43    27.981   -2.861   17.044   1.00  20.05          O
ATOM    5591  O    HOH  W   44     2.212   -4.670   21.196   1.00  17.41          O
ATOM    5594  O    HOH  W   45     5.416   16.195   31.844   1.00  15.02          O
ATOM    5597  O    HOH  W   46    20.229   32.354   26.988   1.00  15.42          O
ATOM    5600  O    HOH  W   47    27.214   14.437   31.277   1.00  10.24          O
ATOM    5603  O    HOH  W   48    24.332   32.917   12.832   1.00  16.80          O
ATOM    5606  O    HOH  W   49     9.986    5.426   33.075   1.00  16.06          O
ATOM    5609  O    HOH  W   50    21.134   30.372   36.728   1.00  15.50          O
ATOM    5612  O    HOH  W   51     4.815   20.991   24.941   1.00  17.09          O
ATOM    5615  O    HOH  W   52    39.195    8.284   21.866   1.00  17.72          O
ATOM    5618  O    HOH  W   53    24.661    0.260   25.378   1.00  13.02          O
ATOM    5621  O    HOH  W   54     6.599   11.219   19.732   1.00  15.01          O
ATOM    5624  O    HOH  W   55    -1.402   -3.742   22.540   1.00  17.94          O
ATOM    5627  O    HOH  W   56    23.967   19.287   36.011   1.00  12.16          O
ATOM    5630  O    HOH  W   57    32.403   10.495   30.395   1.00  18.72          O
ATOM    5633  O    HOH  W   58    30.411   20.433    3.925   1.00  19.29          O
ATOM    5636  O    HOH  W   59    14.110    5.986   23.754   1.00  21.44          O
ATOM    5639  O    HOH  W   60     9.502   31.255   31.238   1.00  19.33          O
ATOM    5642  O    HOH  W   61    17.881    7.531   29.614   1.00  18.16          O
ATOM    5645  O    HOH  W   62    35.920   21.100   27.416   1.00  18.14          O
ATOM    5648  O    HOH  W   63    21.184   17.585   43.689   1.00  19.63          O
ATOM    5651  O    HOH  W   64    11.422    6.357   17.386   1.00  17.47          O
ATOM    5654  O    HOH  W   65     7.568   24.486    1.717   1.00  26.99          O
ATOM    5657  O    HOH  W   66    11.835   23.396   12.551   1.00  17.33          O
ATOM    5660  O    HOH  W   67    15.674   -0.267   13.102   1.00  28.14          O
ATOM    5663  O    HOH  W   68    35.246    4.205   18.695   1.00  18.92          O
ATOM    5666  O    HOH  W   69    12.071    7.219   35.891   1.00  17.95          O
ATOM    5669  O    HOH  W   70    33.151    1.122   14.747   1.00  19.80          O
ATOM    5672  O    HOH  W   71    22.406   17.015   36.075   1.00  12.42          O
ATOM    5675  O    HOH  W   72    20.744    5.444   32.336   1.00  24.35          O
ATOM    5678  O    HOH  W   73    20.988   -0.601   11.337   1.00  17.02          O
ATOM    5681  O    HOH  W   74    32.168   21.857   32.986   1.00  16.30          O
ATOM    5684  O    HOH  W   75    28.340   34.388   18.531   1.00  14.67          O
ATOM    5687  O    HOH  W   76    27.395   34.930   22.269   1.00  15.72          O
ATOM    5690  O    HOH  W   77    -2.881    1.257   27.203   1.00  24.36          O
ATOM    5693  O    HOH  W   78     1.320   22.974   26.514   1.00  16.10          O
ATOM    5696  O    HOH  W   79    20.014    2.049    4.590   1.00  18.49          O
ATOM    5699  O    HOH  W   80    20.131  -10.521   22.803   1.00  16.67          O
ATOM    5702  O    HOH  W   81     2.801  -12.938   26.738   1.00  21.33          O
ATOM    5705  O    HOH  W   82    13.524   14.422   42.122   1.00  22.70          O
ATOM    5708  O    HOH  W   83    41.288   13.787    8.384   1.00  21.34          O
ATOM    5711  O    HOH  W   84    33.383   31.044   25.382   1.00  20.83          O
ATOM    5714  O    HOH  W   85    39.402   29.928   19.487   1.00  19.25          O
ATOM    5717  O    HOH  W   86    14.181   -0.702   28.733   1.00  20.17          O
ATOM    5720  O    HOH  W   87    11.217    5.903   27.361   1.00  18.74          O
ATOM    5723  O    HOH  W   88    28.627   31.304   33.345   1.00  18.56          O
ATOM    5726  O    HOH  W   89    25.546   35.322   19.001   1.00  21.03          O
ATOM    5729  O    HOH  W   90    17.693   -0.865   33.818   1.00  23.45          O
ATOM    5732  O    HOH  W   91    16.853    2.781   26.050   1.00  20.34          O
ATOM    5735  O    HOH  W   92    34.612   21.168   14.066   1.00  15.33          O
ATOM    5738  O    HOH  W   93    19.619   19.119    0.936   1.00  29.35          O
ATOM    5741  O    HOH  W   94    20.721   21.727   42.621   1.00  20.03          O
ATOM    5744  O    HOH  W   95    17.040   19.477   42.629   1.00  20.78          O
ATOM    5747  O    HOH  W   96    20.111   -0.122   24.735   1.00  19.14          O
ATOM    5750  O    HOH  W   97     3.609   25.498   17.596   1.00  22.36          O
ATOM    5753  O    HOH  W   98    22.201   34.610   13.464   1.00  19.68          O
ATOM    5756  O    HOH  W   99     1.306   27.285   34.611   1.00  28.89          O
ATOM    5759  O    HOH  W  100     1.618   26.901   31.789   1.00  16.56          O
ATOM    5762  O    HOH  W  101    34.765    7.143   28.884   1.00  25.03          O
```

```
ATOM   5765  O   HOH W 102    39.459    5.374   23.644  1.00 21.05           O
ATOM   5768  O   HOH W 103     7.666    6.005   10.390  1.00 23.68           O
ATOM   5771  O   HOH W 104    25.629    6.850   39.327  1.00 25.93           O
ATOM   5774  O   HOH W 105     4.516   10.592   22.714  1.00 16.32           O
ATOM   5777  O   HOH W 106    23.065   -8.147   22.078  1.00 22.91           O
ATOM   5780  O   HOH W 107    15.215    5.229    8.027  1.00 18.64           O
ATOM   5783  O   HOH W 108     9.120   -7.084   18.432  1.00 24.86           O
ATOM   5786  O   HOH W 109     9.059   32.376   26.670  1.00 22.20           O
ATOM   5789  O   HOH W 110    28.414   12.755    0.995  1.00 20.29           O
ATOM   5792  O   HOH W 111    14.310   19.153   -3.544  1.00 25.42           O
ATOM   5795  O   HOH W 112    18.007  -11.145   20.744  1.00 25.31           O
ATOM   5798  O   HOH W 113    38.102   21.625   11.695  1.00 21.89           O
ATOM   5801  O   HOH W 114     6.120   13.736   30.142  1.00 24.46           O
ATOM   5804  O   HOH W 115    15.906  -18.386   31.627  1.00 22.15           O
ATOM   5807  O   HOH W 116    22.389   33.907   19.142  1.00 22.92           O
ATOM   5810  O   HOH W 117    32.200   35.724   21.334  1.00 26.32           O
ATOM   5813  O   HOH W 118     2.190   17.510    6.678  1.00 25.22           O
ATOM   5816  O   HOH W 119     1.118    4.704   29.359  1.00 25.72           O
ATOM   5819  O   HOH W 120     9.611    1.556   17.312  1.00 23.94           O
ATOM   5822  O   HOH W 121     3.629   23.370   16.136  1.00 22.93           O
ATOM   5825  O   HOH W 122    32.907   25.232    8.564  1.00 22.34           O
ATOM   5828  O   HOH W 123    -1.806   -9.230   29.885  1.00 26.14           O
ATOM   5831  O   HOH W 124    32.230   21.567   30.291  1.00 23.29           O
ATOM   5834  O   HOH W 125    37.450   14.678   29.329  1.00 25.24           O
ATOM   5837  O   HOH W 126    15.339   36.760   18.883  1.00 20.61           O
ATOM   5840  O   HOH W 127    10.235    7.567   31.063  1.00 21.51           O
ATOM   5843  O   HOH W 128    24.897   37.442   19.549  1.00 24.86           O
ATOM   5846  O   HOH W 129    17.030  -13.244   22.390  1.00 21.48           O
ATOM   5849  O   HOH W 130     9.040    8.412   14.673  1.00 27.72           O
ATOM   5852  O   HOH W 131    14.720   31.661   34.320  1.00 22.71           O
ATOM   5855  O   HOH W 132    19.535    8.925    0.482  1.00 21.18           O
ATOM   5858  O   HOH W 133    12.077    0.962   18.542  1.00 22.23           O
ATOM   5861  O   HOH W 134     3.441   12.279   24.403  1.00 21.97           O
ATOM   5864  O   HOH W 135    13.235   21.344   -4.722  1.00 25.46           O
ATOM   5867  O   HOH W 136     4.989   14.655   27.582  1.00 28.75           O
ATOM   5870  O   HOH W 137    -0.388   24.768   31.623  1.00 23.62           O
ATOM   5873  O   HOH W 138     9.733   10.653   39.989  1.00 28.29           O
ATOM   5876  O   HOH W 139     5.022    2.800   18.295  1.00 34.14           O
ATOM   5879  O   HOH W 140    26.430   -3.864   19.091  1.00 26.57           O
ATOM   5882  O   HOH W 141    33.127   12.459   35.197  1.00 24.55           O
ATOM   5885  O   HOH W 142     4.529   14.653   24.602  1.00 27.79           O
ATOM   5888  O   HOH W 143    34.889    3.006   29.679  1.00 26.45           O
ATOM   5891  O   HOH W 144    26.472   27.781    5.265  1.00 24.30           O
ATOM   5894  O   HOH W 145     9.844    1.973   10.352  1.00 30.48           O
ATOM   5897  O   HOH W 146    23.113   35.988   15.667  1.00 24.26           O
ATOM   5900  O   HOH W 147     3.506   18.934   26.329  1.00 23.36           O
ATOM   5903  O   HOH W 148    41.932   12.669   10.942  1.00 26.60           O
ATOM   5906  O   HOH W 149    40.619   12.671   22.354  1.00 26.08           O
ATOM   5909  O   HOH W 150    33.062   38.217   15.479  1.00 37.76           O
ATOM   5912  O   HOH W 151     3.554   31.071   10.635  1.00 26.96           O
ATOM   5915  O   HOH W 152    14.084    8.928   41.767  1.00 28.97           O
ATOM   5918  O   HOH W 153    29.827    1.222    9.591  1.00 30.85           O
ATOM   5921  O   HOH W 154    23.088   -1.722   26.918  1.00 27.80           O
ATOM   5924  O   HOH W 155     8.435    8.580   36.298  1.00 27.72           O
ATOM   5927  O   HOH W 156    42.926   15.621    8.285  1.00 29.85           O
ATOM   5930  O   HOH W 157     6.654   11.279   36.797  1.00 28.44           O
ATOM   5933  O   HOH W 158    15.300    6.642   27.237  1.00 23.08           O
ATOM   5936  O   HOH W 159    14.085  -11.817   20.799  1.00 27.86           O
ATOM   5939  O   HOH W 160    -1.521   -6.774   36.408  1.00 31.93           O
ATOM   5942  O   HOH W 161    15.519    1.708   31.201  1.00 27.02           O
ATOM   5945  O   HOH W 162     0.621   -5.366   35.349  1.00 28.46           O
ATOM   5948  O   HOH W 163    18.036  -12.751   33.648  1.00 22.09           O
ATOM   5951  O   HOH W 164    32.843   18.669   30.344  1.00 28.48           O
```

```
ATOM   5954  O   HOH W 165    27.765  11.874  38.295  1.00 18.10           O
ATOM   5957  O   HOH W 166     1.781  17.606  13.084  1.00 28.85           O
ATOM   5960  O   HOH W 167    20.211   0.158   6.249  1.00 29.98           O
ATOM   5963  O   HOH W 168     2.759  19.112  32.488  1.00 24.07           O
ATOM   5966  O   HOH W 169    33.968  18.793  32.524  1.00 23.54           O
ATOM   5969  O   HOH W 170    -1.571 -13.592  26.165  1.00 34.45           O
ATOM   5972  O   HOH W 171    39.370  28.627  17.071  1.00 26.29           O
ATOM   5975  O   HOH W 172    17.376  32.794  35.436  1.00 23.98           O
ATOM   5978  O   HOH W 173     9.391   6.761  35.029  1.00 21.74           O
ATOM   5981  O   HOH W 174    16.352  11.687  43.877  1.00 31.51           O
ATOM   5984  O   HOH W 175    36.018   4.292  25.853  1.00 21.19           O
ATOM   5987  O   HOH W 176    24.899  -2.518  10.289  1.00 21.66           O
ATOM   5990  O   HOH W 177    -1.286   2.934  25.647  1.00 31.88           O
ATOM   5993  O   HOH W 178    13.449 -16.540  28.665  1.00 26.26           O
ATOM   5996  O   HOH W 179    13.301  -0.613  16.357  1.00 27.05           O
ATOM   5999  O   HOH W 180    24.842  17.585  44.930  1.00 26.85           O
ATOM   6002  O   HOH W 181     5.856  18.874  39.008  1.00 29.19           O
ATOM   6005  O   HOH W 182    -1.630   2.543  31.697  1.00 33.70           O
ATOM   6008  O   HOH W 183    38.130  17.164   1.491  1.00 33.90           O
ATOM   6011  O   HOH W 184    38.533  33.710  21.252  1.00 23.89           O
ATOM   6014  O   HOH W 185     8.687  18.331   1.042  1.00 28.46           O
ATOM   6017  O   HOH W 186    13.162   5.211  37.558  1.00 29.88           O
ATOM   6020  O   HOH W 187    13.148 -16.170  33.001  1.00 24.01           O
ATOM   6023  O   HOH W 188    17.877  11.059   1.344  1.00 30.31           O
ATOM   6026  O   HOH W 189     1.036 -11.099  25.828  1.00 27.54           O
ATOM   6029  O   HOH W 190    19.608  24.676   5.693  1.00 30.78           O
ATOM   6032  O   HOH W 191    19.946  19.409  42.111  1.00 26.14           O
ATOM   6035  O   HOH W 192     3.476  10.629  29.248  1.00 33.62           O
ATOM   6038  O   HOH W 193    30.257  27.659  31.979  1.00 37.42           O
ATOM   6041  O   HOH W 194    16.442  32.431   6.611  1.00 24.66           O
ATOM   6044  O   HOH W 195    34.073  12.318   2.203  1.00 35.07           O
ATOM   6047  O   HOH W 196     4.395  17.038  28.248  1.00 26.36           O
ATOM   6050  O   HOH W 197    33.318   3.825  31.828  1.00 23.35           O
ATOM   6053  O   HOH W 198    18.983  33.274  37.816  1.00 19.94           O
ATOM   6056  O   HOH W 199    13.726  12.394  40.361  1.00 26.01           O
ATOM   6059  O   HOH W 200    12.010   7.108   2.281  1.00 28.49           O
ATOM   6062  O   HOH W 201    17.870   4.803  31.837  1.00 28.97           O
ATOM   6065  O   HOH W 202    27.323  19.119  43.497  1.00 31.89           O
ATOM   6068  O   HOH W 203    24.085  33.024   8.818  1.00 27.59           O
ATOM   6071  O   HOH W 204    19.302  -7.180  37.095  1.00 25.01           O
ATOM   6074  O   HOH W 205    34.921   3.097  15.744  1.00 35.90           O
ATOM   6077  O   HOH W 206    22.046  36.365  19.883  1.00 33.71           O
ATOM   6080  O   HOH W 207     4.178  27.080  39.663  1.00 28.68           O
ATOM   6083  O   HOH W 208    21.450  25.719   2.335  1.00 29.50           O
ATOM   6086  O   HOH W 209     7.625  37.543  22.457  1.00 27.39           O
ATOM   6089  O   HOH W 210    27.905   0.363  30.805  1.00 27.13           O
ATOM   6092  O   HOH W 211    29.963  26.817  34.574  1.00 28.33           O
ATOM   6095  O   HOH W 212    37.812  29.413  15.021  1.00 35.13           O
ATOM   6098  O   HOH W 213    31.600   5.299  33.905  1.00 30.36           O
ATOM   6101  O   HOH W 214     0.934   2.893  30.953  1.00 26.27           O
ATOM   6104  O   HOH W 215    15.151 -19.091  27.503  1.00 36.44           O
ATOM   6107  O   HOH W 216    31.891  29.580  32.140  1.00 23.31           O
ATOM   6110  O   HOH W 217    13.828  -7.788  38.638  1.00 33.17           O
ATOM   6113  O   HOH W 218    37.026   8.221   8.178  1.00 43.15           O
ATOM   6116  O   HOH W 219    12.026  -5.374  16.938  1.00 29.86           O
ATOM   6119  O   HOH W 220    -1.767  -3.163  19.781  1.00 23.61           O
ATOM   6122  O   HOH W 221     5.748   3.990  37.161  1.00 34.03           O
ATOM   6125  O   HOH W 222    15.126  10.026   2.394  1.00 29.52           O
ATOM   6128  O   HOH W 223    28.930  25.732   2.063  1.00 32.92           O
ATOM   6131  O   HOH W 224    17.834  38.165  18.660  1.00 32.25           O
ATOM   6134  O   HOH W 225    15.576  -9.633  19.956  1.00 29.35           O
ATOM   6137  O   HOH W 226    21.532  33.500  36.344  1.00 30.91           O
ATOM   6140  O   HOH W 227    37.166  25.308  14.969  1.00 30.80           O
```

```
ATOM   6143  O   HOH W 228     4.201  13.978  20.632  1.00 29.14          O
ATOM   6146  O   HOH W 229    -8.921   0.073  20.951  1.00 29.35          O
ATOM   6149  O   HOH W 230    30.930  14.280   0.673  1.00 42.54          O
ATOM   6152  O   HOH W 231     0.993 -10.363  23.294  1.00 35.64          O
ATOM   6155  O   HOH W 232    19.283  -9.456  35.875  1.00 21.72          O
ATOM   6158  O   HOH W 233    29.715  33.139   9.438  1.00 28.66          O
ATOM   6161  O   HOH W 234     2.904  -7.322  21.953  1.00 29.37          O
ATOM   6164  O   HOH W 235    -0.395  23.877  34.029  1.00 37.81          O
ATOM   6167  O   HOH W 236    15.054  -3.907  38.561  1.00 26.91          O
ATOM   6170  O   HOH W 237    25.729  34.682  11.457  1.00 29.23          O
ATOM   6173  O   HOH W 238     9.385  33.323  24.057  1.00 36.55          O
ATOM   6176  O   HOH W 239    24.093  -5.021  21.077  1.00 26.10          O
ATOM   6179  O   HOH W 240    34.767  17.185  37.911  1.00 29.03          O
ATOM   6182  O   HOH W 241    18.069  25.299  24.326  1.00 23.95          O
ATOM   6185  O   HOH W 242    25.539  23.840  -0.133  1.00 27.32          O
ATOM   6188  O   HOH W 243    -8.581  -0.882  24.380  1.00 32.46          O
ATOM   6191  O   HOH W 244    37.140  34.955  20.025  1.00 39.20          O
ATOM   6194  O   HOH W 245    25.828  -6.464  17.951  1.00 35.90          O
ATOM   6197  O   HOH W 246    20.526   5.042   2.568  1.00 23.17          O
ATOM   6200  O   HOH W 247    16.909  37.789  30.355  1.00 24.49          O
ATOM   6203  O   HOH W 248     4.170 -13.753  24.179  1.00 34.75          O
ATOM   6206  O   HOH W 249     4.757  29.554  36.890  1.00 27.07          O
ATOM   6209  O   HOH W 250    14.985  25.383  44.611  1.00 36.67          O
ATOM   6212  O   HOH W 251    21.002  34.942  26.743  1.00 24.40          O
ATOM   6215  O   HOH W 252    35.187  37.614  16.171  1.00 41.70          O
ATOM   6218  O   HOH W 253     9.429  35.849  24.299  1.00 29.47          O
ATOM   6221  O   HOH W 254    22.360  -8.508  14.886  1.00 39.42          O
ATOM   6224  O   HOH W 255    27.125  28.229   2.829  1.00 36.93          O
ATOM   6227  O   HOH W 256     7.686   9.225  33.089  1.00 35.16          O
ATOM   6230  O   HOH W 257     4.744   8.641   8.479  1.00 31.36          O
ATOM   6233  O   HOH W 258    43.322  15.064  19.229  1.00 34.65          O
ATOM   6236  O   HOH W 259    12.158  34.202  31.572  1.00 22.62          O
ATOM   6239  O   HOH W 260    40.415  22.262  16.091  1.00 26.05          O
ATOM   6242  O   HOH W 261     7.689  33.643  34.608  1.00 27.52          O
ATOM   6245  O   HOH W 262    -2.516 -11.500  29.608  1.00 27.56          O
ATOM   6248  O   HOH W 263    23.197  30.603  38.577  1.00 30.81          O
ATOM   6251  O   HOH W 264     1.669  -4.135  18.399  1.00 34.72          O
ATOM   6254  O   HOH W 265    31.682  18.313   2.510  1.00 27.38          O
ATOM   6257  O   HOH W 266    21.515  33.588  40.094  1.00 30.03          O
ATOM   6260  O   HOH W 267    16.458  13.271  -0.901  1.00 38.54          O
ATOM   6263  O   HOH W 268    40.177  32.128  16.843  1.00 41.26          O
ATOM   6266  O   HOH W 269    12.143  -2.734  15.885  1.00 27.02          O
ATOM   6269  O   HOH W 270    27.486  -3.196  12.318  1.00 30.88          O
ATOM   6272  O   HOH W 271    15.668   6.307  39.384  1.00 31.07          O
ATOM   6275  O   HOH W 272     7.819   6.715  30.569  1.00 22.48          O
ATOM   6278  O   HOH W 273    29.983  17.529  41.917  1.00 33.57          O
ATOM   6281  O   HOH W 274     2.674   6.648  20.688  1.00 34.77          O
ATOM   6284  O   HOH W 275    16.983   2.502  33.738  1.00 29.85          O
ATOM   6287  O   HOH W 276    18.800  36.320  34.162  1.00 30.58          O
ATOM   6290  O   HOH W 277    12.363  24.605  -1.596  1.00 34.55          O
ATOM   6293  O   HOH W 278    14.702  17.110  -5.593  1.00 27.29          O
ATOM   6296  O   HOH W 279    40.591  12.389   6.204  1.00 33.96          O
ATOM   6299  O   HOH W 280    31.608  16.687   1.057  1.00 38.88          O
ATOM   6302  O   HOH W 281    23.897 -11.952  20.222  1.00 34.68          O
ATOM   6305  O   HOH W 282    11.219  39.478  21.517  1.00 33.54          O
ATOM   6308  O   HOH W 283     2.552  16.703  24.563  1.00 35.86          O
ATOM   6311  O   HOH W 284    27.258   9.495  42.694  1.00 30.97          O
ATOM   6314  O   HOH W 285     5.535   8.881  16.549  1.00 28.65          O
ATOM   6317  O   HOH W 286     2.189  24.192  36.099  1.00 31.47          O
ATOM   6320  O   HOH W 287    19.058  -1.798  12.329  1.00 34.23          O
ATOM   6323  O   HOH W 288    10.635  34.933  33.408  1.00 30.86          O
ATOM   6326  O   HOH W 289     4.333  -0.576  36.893  1.00 44.65          O
ATOM   6329  O   HOH W 290    25.069  21.389  -1.195  1.00 37.85          O
```

```
ATOM  6332  O   HOH W 291    28.073   7.826  39.103  1.00 28.63        O
ATOM  6335  O   HOH W 292    14.225  35.260  33.101  1.00 43.65        O
ATOM  6338  O   HOH W 293    18.965  -2.916  15.137  1.00 38.32        O
ATOM  6341  O   HOH W 294    40.370  19.000  13.716  1.00 33.50        O
ATOM  6344  O   HOH W 295     6.261  32.033  33.639  1.00 30.04        O
ATOM  6347  O   HOH W 296    13.696  37.943  17.160  1.00 36.07        O
ATOM  6350  O   HOH W 297     2.518  21.734  35.950  1.00 40.41        O
ATOM  6353  O   HOH W 298    31.821  26.389  38.109  1.00 33.84        O
ATOM  6356  O   HOH W 299    -1.406   2.015  20.824  1.00 32.14        O
ATOM  6359  O   HOH W 300    27.841   5.928  34.623  1.00 31.37        O
ATOM  6362  O   HOH W 301    33.128  25.346  29.949  1.00 26.11        O
ATOM  6365  O   HOH W 302    16.952  35.715  12.956  1.00 35.08        O
ATOM  6368  O   HOH W 303    19.607  39.276  19.928  1.00 43.31        O
ATOM  6371  O   HOH W 304    31.667  24.286  33.803  1.00 31.06        O
ATOM  6374  O   HOH W 305     9.682  34.631  35.657  1.00 36.70        O
ATOM  6377  O   HOH W 306    24.913  37.958  16.991  1.00 33.31        O
ATOM  6380  O   HOH W 307    27.526  -1.443   8.577  1.00 31.08        O
ATOM  6383  O   HOH W 308    34.923  14.237  35.665  1.00 33.29        O
ATOM  6386  O   HOH W 309    23.480   3.819  33.037  1.00 28.70        O
ATOM  6389  O   HOH W 310    39.917  30.468  13.351  1.00 43.99        O
ATOM  6392  O   HOH W 311    20.005  30.742  39.713  1.00 31.34        O
ATOM  6395  O   HOH W 312     1.762  19.900  10.728  1.00 33.53        O
ATOM  6398  O   HOH W 313    21.282  35.887  11.600  1.00 34.66        O
ATOM  6401  O   HOH W 314    22.512  -2.583   9.740  1.00 30.42        O
ATOM  6404  O   HOH W 315    19.079   3.993  34.105  1.00 40.61        O
ATOM  6407  O   HOH W 316     2.068  20.663  16.198  1.00 29.05        O
ATOM  6410  O   HOH W 317     2.691   6.046  36.126  1.00 41.50        O
ATOM  6413  O   HOH W 318    34.645   5.648   8.339  1.00 37.23        O
ATOM  6416  O   HOH W 319    23.607   9.100  43.132  1.00 28.98        O
ATOM  6419  O   HOH W 320    32.041  25.470   4.056  1.00 37.21        O
ATOM  6422  O   HOH W 321    20.362   8.689  26.048  1.00 25.83        O
ATOM  6425  O   HOH W 322    11.708   6.948  24.652  1.00 11.83        O
ATOM  6428  O   HOH W 323    27.069   1.261   5.299  1.00 22.09        O
ATOM  6431  O   HOH W 324    23.654  25.543  41.612  1.00 22.43        O
ATOM  6434  O   HOH W 325    23.776  33.886  17.441  1.00 26.34        O
ATOM  6437  O   HOH W 326    34.498  18.045  27.924  1.00 26.83        O
ATOM  6440  O   HOH W 327    34.129   9.041  28.001  1.00 29.40        O
ATOM  6443  O   HOH W 328    22.398  -4.833  18.306  1.00 31.97        O
ATOM  6446  O   HOH W 329    32.650  36.061  13.569  1.00 32.16        O
ATOM  6449  O   HOH W 330    18.875   6.699  27.476  1.00 31.00        O
ATOM  6452  O   HOH W 331    43.627  18.936   5.210  1.00 33.15        O
ATOM  6455  O   HOH W 332    13.390  23.630  -3.991  1.00 34.31        O
ATOM  6458  O   HOH W 333    -0.102   6.438  34.964  1.00 48.55        O
ATOM  6461  O   HOH W 334    -0.118   6.687  37.269  1.00 35.34        O
ATOM  6464  O   HOH W 335    37.771   7.069  11.352  1.00 32.60        O
ATOM  6467  O   HOH W 336    31.257  24.829  41.652  1.00 34.05        O
ATOM  6470  O   HOH W 337    10.129  21.939  40.735  1.00 34.24        O
ATOM  6473  O   HOH W 338     6.286  34.026  36.723  1.00 34.35        O
ATOM  6476  O   HOH W 339    10.691  33.573  28.674  1.00 35.22        O
ATOM  6479  O   HOH W 340    12.399   2.285  25.575  1.00 35.31        O
ATOM  6482  O   HOH W 341    34.307   9.058   2.642  1.00 42.37        O
ATOM  6485  O   HOH W 342    15.597   2.822  28.675  1.00 34.26        O
ATOM  6488  O   HOH W 343    11.007  37.789  24.517  1.00 39.01        O
ATOM  6491  O   HOH W 344     6.436 -13.362  22.903  1.00 41.47        O
ATOM  6494  O   HOH W 345    19.680  17.126  45.857  1.00 37.81        O
ATOM  6497  O   HOH W 346    10.533  36.113  27.412  1.00 34.50        O
ATOM  6500  O   HOH W 347    41.809  19.887  16.869  1.00 37.61        O
ATOM  6503  O   HOH W 348    21.611  15.053  -0.998  1.00 45.55        O
ATOM  6506  O   HOH W 349    22.337  -9.031  17.861  1.00 49.31        O
ATOM  6509  O   HOH W 350     9.303  25.984  -1.560  1.00 39.69        O
ATOM  6512  O   HOH W 351    13.153   3.923  27.389  1.00 35.74        O
ATOM  6515  O   HOH W 352    20.365   4.572  37.533  1.00 43.93        O
ATOM  6518  O   HOH W 353    -2.246  27.322  10.724  1.00 42.74        O
```

```
ATOM   6521  O   HOH W 354     9.435  33.000   4.908  1.00 38.59           O
ATOM   6524  O   HOH W 355    15.473  17.843  44.371  1.00 40.00           O
ATOM   6527  O   HOH W 356    13.009  31.258  32.197  1.00 21.10           O
ATOM   6530  O   HOH W 357    36.838   8.242   3.607  1.00 35.04           O
ATOM   6533  O   HOH W 358    30.674  -0.077  10.899  1.00 34.69           O
ATOM   6536  O   HOH W 359    31.146  37.498  19.980  1.00 36.95           O
ATOM   6539  O   HOH W 360    16.880  -0.664   8.461  1.00 39.07           O
ATOM   6542  O   HOH W 361    40.707   6.845  20.023  1.00 32.40           O
ATOM   6545  O   HOH W 362    19.502  25.524   0.606  1.00 41.49           O
ATOM   6548  O   HOH W 363    27.574  22.139  44.653  1.00 52.17           O
ATOM   6551  O   HOH W 364    11.308  11.015  41.537  1.00 41.08           O
ATOM   6554  O   HOH W 365     9.385 -15.147  33.423  1.00 33.24           O
ATOM   6557  O   HOH W 366     8.340  36.413   6.953  1.00 46.38           O
ATOM   6560  O   HOH W 367     1.749  16.224  29.518  1.00 42.28           O
ATOM   6563  O   HOH W 368    21.762  29.936  41.718  1.00 39.52           O
ATOM   6566  O   HOH W 369     5.916   6.084   8.101  1.00 42.08           O
ATOM   6569  O   HOH W 370    22.021  34.841  24.376  1.00 41.54           O
ATOM   6572  O   HOH W 371    21.487 -18.393  21.594  1.00 38.90           O
ATOM   6575  O   HOH W 372    39.073   5.237   9.752  1.00 40.75           O
ATOM   6578  O   HOH W 373    23.013  -4.111   6.967  1.00 39.88           O
ATOM   6581  O   HOH W 374    14.536  36.281  12.246  1.00 37.61           O
ATOM   6584  O   HOH W 375    29.859  34.515  20.996  1.00 28.10           O
ATOM   6587  O   HOH W 376    28.570  38.564  14.823  1.00 51.95           O
ATOM   6590  O   HOH W 377    33.330  21.383   4.315  1.00 36.55           O
ATOM   6593  O   HOH W 378    44.550  14.418  11.401  1.00 45.79           O
ATOM   6596  O   HOH W 379    20.051  17.411  -1.528  1.00 37.86           O
ATOM   6599  O   HOH W 380     0.588  25.706  15.094  1.00 42.46           O
ATOM   6602  O   HOH W 381     4.339  -3.887  18.462  1.00 42.68           O
ATOM   6605  O   HOH W 382    15.219  38.779  25.987  1.00 45.72           O
ATOM   6608  O   HOH W 383    26.263  -1.807   0.657  1.00 35.43           O
ATOM   6611  O   HOH W 384    43.222   8.889  13.058  1.00 39.12           O
ATOM   6614  O   HOH W 385     3.804  15.072  37.146  1.00 35.83           O
ATOM   6617  O   HOH W 386    13.685  22.152  44.161  1.00 46.34           O
ATOM   6620  O   HOH W 387    39.745   6.714  14.451  1.00 43.87           O
ATOM   6623  O   HOH W 388     4.160  10.246  14.729  1.00 40.70           O
ATOM   6626  O   HOH W 389     8.951   3.749   5.381  1.00 35.43           O
ATOM   6629  O   HOH W 390    12.861 -18.451  24.960  1.00 37.73           O
ATOM   6632  O   HOH W 391     2.829  15.459  20.671  1.00 39.80           O
ATOM   6635  O   HOH W 392    13.542   1.619  37.956  1.00 42.36           O
ATOM   6638  O   HOH W 393    16.727  31.361  39.312  1.00 38.23           O
ATOM   6641  O   HOH W 394    36.063   3.510  12.324  1.00 41.15           O
ATOM   6644  O   HOH W 395     5.800   1.387  38.322  1.00 38.68           O
ATOM   6647  O   HOH W 396    12.445  36.715  28.596  1.00 36.10           O
ATOM   6650  O   HOH W 397     2.782  12.760  27.641  1.00 47.29           O
ATOM   6653  O   HOH W 398    -1.700  -3.625  37.395  1.00 36.77           O
ATOM   6656  O   HOH W 399    41.093  10.367  21.318  1.00 46.59           O
ATOM   6659  O   HOH W 400    21.734  -5.069   9.760  1.00 48.11           O
ATOM   6662  O   HOH W 401    14.290   3.253  24.475  1.00 36.49           O
ATOM   6665  O   HOH W 402    22.729   2.974  -0.954  1.00 40.03           O
ATOM   6668  O   HOH W 403     4.597  11.469   0.380  1.00 42.88           O
ATOM   6671  O   HOH W 404    17.898   4.552  -0.927  1.00 39.85           O
```

**Claims**

1. A TY145 like subtilase variant which is at least 63% homologous to the sequence of SEQ ID NO:1, preferably at least 65%, at least 70%, at least 74%, at least 80%, at least 83%, at least 90%, at least 91%, at least 92%, at least 93%, at least 94%, at least 95%, at least 96%, at least 97%, at least 98% or at least 99% homologous to the sequence of SEQ ID NO:1, which variant comprises an alteration in one or more positions located at a distance of not more than 10Å to one of the ion-binding sites of TY145, wherein the positions, as specified in SEQ ID NO:1, located at a distance of not more than 10Å to:

   a) the Weak ion-binding site are: 154, 155, 158, 164, 165, 166, 167, 168, 178, 179, 180, 181, 182, 183, 184, 185, 186, 187, 188, 189, 190, 191, 211, 220, 221, 222, 223, 224, 225, 226, 227, 228, 277, 281 and 305,
   b) the Near ion-binding site are: 185, 211, 212, 213, 214, 215, 216, 217, 218, 219, 220, 221, 222, 223, 224, 225, 226, 227, 277, 281, 299, 300, 301, 304, 305,
   c) the Far ion-binding site are: 193, 198, 199, 201, 202, 204, 216, 217, 219, 226, 227, 228, 229, 284, 285, 286, 287, 288, 289, 290, 291, 292, 293, 294, 295, 296, 297, 298, 299, 300, 301, 302, 303, 304, 305, 306 and 307.

2. The TY145 like subtilase variant according to claim 1 wherein the alterations are one or more of the substitutions I220S,T,D,E; T215S,D,E; G298A,S,T,D,E; G296A,S,T,D,E; V185T,D,E; I221N,D,T,E; V185P and 1293P.

3. The TY145 like subtilase variant according to claim 1, in which one or more ion-binding sites have been removed, wherein said variant comprises one or both of the alterations

a) deletion of the region K290-D300, or at least one deletion of one amino acid residue in the region K290-D300 of SEQ ID NO:1 and subsequent insertion of one or more amino acid residues, preferably insertion between 1289 and Y301 of the sequence GDS (SEQ ID NO: 49) or DST (SEQ ID NO: 50), and preferably further comprising the substitution S303Y,

b) deletion of the region N212-R224, or at least one deletion of one amino acid residue in the region N212-R224 of SEQ ID NO:1 and subsequent insertion of one or more amino acid residues, preferably insertion of a proline residue or an alanine residue between G211 and D225.

4. A TY145 like subtilase variant comprising the introduction of a ion-binding site corresponding to the Strong ion-binding site of the Subtilisin family subtilases, wherein said variant has a deletion of the region H83-G90, or at least one deletion of one amino acid residue in the region H83-G90, of SEQ ID NO:1 and subsequent insertion of one or more amino acid residues, preferably insertion of the sequence LNNSIG (SEQ ID NO: 48) between residues A82 and V91.

5. A Subtilisin family subtilase variant in which the Strong ion-binding site has been removed, wherein said variant comprises deletion of the region L75-G80 (BPN' numbering), or at least one deletion of one amino acid residue in the region L75-G80 (BPN' numbering), or a corresponding region in another Subtilisin family subtilase, and subsequent insertion of one or more amino acid residues, preferably insertion of the sequence GGSNG (SEQ ID NO: 51) of positions 84-88 of TY145 (SEQ ID NO:1) between A74 and V81, and preferably further comprising one or both of the substitutions L80Y and Q2A,N.

6. A TY145 like subtilase variant which is at least 63% homologous to the sequence of SEQ ID NO:1, preferably at least 65%, at least 70%, at least 74%, at least 80%, at least 83%, at least 90%, at least 91%, at least 92%, at least 93%, at least 94%, at least 95%, at least 96%, at least 97%, at least 98% or at least 99% homologous to the sequence of SEQ ID NO:1, said variant comprises one or more alterations in one or more of the positions contained in the following highly mobile regions:

84, 85, 86, 87 and 88,
108, 109, 110, 111, 112, 113, 114, 115, 116 and 117,
141, 142, 143, 144, 145 and 146,
150, 151 and 152,
169, 170 and 171,
200 and 201,
211,212,213,214,215,216,217,218,219 and 220,
242 and 243,
268, 269 and 270.

7. The TY145 like subtilase variant according to claim 6, comprising the alterations D115P and/or T269P.

8. A TY145 like subtilase variant which is at least 63% homologous to the sequence of SEQ ID NO:1, preferably at least 65%, at least 70%, at least 74%, at least 80%, at least 83%, at least 90%, at least 91%, at least 92%, at least 93%, at least 94%, at least 95%, at least 96%, at least 97%, at least 98% or at least 99% homologous to the sequence of SEQ ID NO:1, said variant comprises one or more alterations in one or more of the positions contained in the following mobile regions:

1, 2, 3, 4, 5, 6 and 7,
17, 18, 19, 20, 21, 22 and 23,
38, 39, 40, 41, 42, 43, 44, 45, 46, 47, 48, 49 and 50,
57, 58, 59, 60, 61, 62, 63, 64, 65, 66, 67, 68 and 69,
84, 85, 86, 87, 88, 89, 90, 91 and 92,
107, 108, 109 and 110,
239, 240, 241, 242 and 243
265 and 266,

wherein said alterations preferably are introduced in one or both of the regions 57-69 and 84-92.

9. The TY145 like subtilase variant according to claim 8, comprising the substitution D116H,K,R and /or Q18P.

10. A TY145 like subtilase variant which is at least 63% homologous to the sequence of SEO ID NO:1, preferably at least 65%, at least 70%, at least 74%, at least 80%, at least 83%, at least 90%, at least 91%, at least 92%, at least 93%, at least 94%, at least 95%, at least 96%, at least 97%, at least 98% or at least 99% homologous to the sequence of SEQ ID NO:1, said variant comprising an alteration in one or more of the positions contained in the following regions:

   16, 17, 18, 19, 20, 21 and 22,
   40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72 and 73,
   118, 119, 120, 121, 122, 123, 124, 125, 126, 127, 128, 129, 130 and 131,
   140, 141, 142, 143, 144, 145, 146, 147, 148, 149, 150, 151, 152, 153, 154, 155, 156, 157,158, 159, 160 and 161,
   275, 276, 277, 278, 279, 280, 281, 282, 283, 284, 285, 286, 287, 288, 289, 290, 291, 292, 293 and 294,

   wherein such alterations preferably are made in one or both the regions 40-73 and 140-161, preferably in the sub-regions 65-73 and 140-150.

11. A TY145 like subtilase variant which is at least 63% homologous to the sequence of SEQ ID NO:1, preferably at least 65%, at least 70%, at least 74%, at least 80%, at least 83%, at least 90%, at least 91%, at least 92%, at least 93%, at least 94%, at least 95%, at least 96%, at least 97%, at least 98% or at least 99% homologous to the sequence of SEQ ID NO:1, said variant comprises an alteration in one or more of the following positions: 35,36,70,72,106,109,110,111,112,113,114,117,139,140,141,142,143,144,145, 147,150,167,168,169,170,171,172,173,174,177,180,207,239,247,148,149,150,151 and 252 of SEQ ID NO:1.

12. A TY145 like subtilase variant which is at least 63% homologous to the sequence of SEQ ID NO:1, preferably at least 65%, at least 70%, at least 74%, at least 80%, at least 83%, at least 90%, at least 91 %, at least 92%, at least 93%, at least 94%, at least 95%, at least 96%, at least 97%, at least 98% or at least 99% homologous to the sequence of SEQ ID NO:1, said variant comprises an alteration in one or more of the following positions: V31, V38, T79, V80, L81, V188, T254, wherein preferred variants comprise one or more of V311, T79S and V80A.

13. The TY145 like subtilase variant according to any of the previous claims, comprising one or more disulfide bridges introduced by one or more of the following modifications: G26C+A95C; A167C+T254C; R203C+G292C; V228C+A284C, wherein the positions corresponds to the positions in SEQ ID NO:1

14. The TY145 like subtilase variant according to any of the previous claims, comprising an alteration of an Asn-Gly sequence by deletion or substitution of at least one of the Asn or Gly residues, preferably the Asn residue.

15. The variant of claim 14 comprising substitution of the Asn and/or Gly residue with an amino acid residue selected from the group consisting of A, Q, S, P, T and Y.

16. The variant of claim 15, wherein the substitution is performed in one or more of the following positions:

   B. sphaericus: 198-199, 240-241
   TY145: 87-88, 109-110, 199-200
   TA41: 83-84, 198-199
   TA39: 88-89, 198-199

17. The TY145 like subtilase variant according to any of the previous claims, comprising an alteration of a tyrosine residue by deletion or substitution, preferably to phenylalanine.

18. The variant of claim 17, wherein the substitution is performed in one or more of the following positions:

   B. sphaericus: 14, 91, 102, 112, 155, 157, 172, 179, 201, 206, 211, 218, 235, 239, 243, 292, 300,
   TY145: 15, 39, 92, 103, 113, 156, 158, 202, 219, 240, 244, 287, 301, 307,
   TA41: 15, 91, 102, 112, 155, 157, 179,201,218,235,243,
   TA39: 15, 61, 91, 102, 112, 155, 157, 173, 179, 201, 211, 218, 235, 243, 267, 281, 284, 292, 293,296

19. A TY145 like subtilase variant comprising an alteration of a methionine residue residue by deletion or substitution, preferably to a serine or alanine residue.

20. The variant of claim 19, wherein the substitution is performed in one or more of the following positions:

> B. sphaericus: 138, 251,
> TY145: 139, 252,
> TA41: 1, 138,251,
> TA39: 1, 138, 251,

21. A BPN' like subtilase variant comprising one or more of the alterations V28I,A,L; I35V,A,L; T71S; I72A,G,V; A73L, G; M175V,A; and T224S,A, wherein preferred variants of Savinase comprises one or more of the substitutions V28I, I35V, T71S, I72A, A73L, M175V and T224S (BPN' numbering), especially variants comprising the combinations V28I+I35V, V28I+T71S, V28I+I72A, V28I+A73L, V28I+M175V, I35V+T71S, I35V+I72A, I35V+A73L, I35V+A73L, I35V+M175V, T71S +I72A, T71S +A73L, T71S +A73L, T71S +M175V, 172A +A73L, I72A +A73L, I72A +M175V, A73L +M175V.

22. A BPN' like subtilase variant comprising one or more of the following alterations:

> a) deletion of residues PSPSATLEQAVN (SEQ ID NO: 23) (positions 129-140) in Savinase (BPN' numbering) and subsequent insertion of residues SAKDSLIASAVD (SEQ ID NO: 22) (positions 144-155) from TY145 between S128 and S141 in Savinase,
> b) deletion of residues SGNSGAGSISYPARYA (SEQ ID NO: 25) (positions 153-172) in Savinase (BPN' numbering) and subsequent insertion of residues AGNSGSGSNTIGFPGGLV (SEQ ID NO: 24)(positions 168-185) from TY145 between A152 and N173 in Savinase,
> c) deletion of residues VNVQSTYPGSTYASLN (SEQ ID NO: 27) (positions 203-218) in Savinase (BPN' numbering) and subsequent insertion of residues ASVESTWYTGGYNTIS (SEQ ID NO: 26) (positions 233-248) from TY145 between G202 and G219 in Savinase.

23. A BPN' like subtilase variant comprising one or more of the following alterations:

> a) deletion of residues LSLGSPS (SEQ ID NO: 29) (positions 124-130) in Savinase (BPN' numbering) and subsequent insertion of residues MSLGSSG (SEQ ID NO: 28) (positions 138-144) from TA39 subtilase between N123 and P131 in Savinase,
> b) deletion of residues LSLGSPSPSATL (SEQ ID NO: 31) (positions 124-135) in Savinase variant V104S (BPN' numbering) and subsequent insertion of residues MSLGSSGESSLI (SEQ ID NO: 30) (positions 138-149) from TA39 subtilase between N123 and E136 in Savinase variant V104S.

24. A BPN' like subtilase variant comprising one or more of the following alterations:

> a) deletion of residues VQAPAAHN (SEQ ID NO: 33) (positions 11-18) in Savinase (BPN' numbering) and subsequent insertion of residues NNSSITQT (SEQ ID NO: 32) (positions 16-23) from TA39 subtilase between R10 and R19 in Savinase,
> b) deletion of residues VPG*EPST (SEQ ID NO: 35) (positions 51-58) in Savinase (BPN' numbering) and subsequent insertion of residues TVGTTYTN (SEQ ID NO: 34 positions 56-63 from TA39 subtilase between F50 and Q59 in Savinase,
> c) deletion of residues GN (positions 61-62) in Savinase (BPN' numbering) and subsequent insertion of residues RQ (positions 69-70) from TA39 subtilase between D60 and G63 in Savinase.
> d) deletion of residues PSPSATL (SEQ ID NO: 37) (positions 129-135) in Savinase (BPN' numbering) and subsequent insertion of residues SGESSLI (SEQ ID NO: 36) (positions 143-149) from TA39 subtilase between S128 and E136 in Savinase.
> e) deletion of residues YPGSTYASL (SEQ ID NO: 39) (positions 209-217) in Savinase (BPN' numbering) and subsequent insertion of residues WFDGGYATI (SEQ ID NO: 38) (positions 238-246) from TA39 subtilase between T208 and N218 in Savinase.

25. A BPN' like subtilase variant comprising one or more of the following alterations:

> a) deletion of residues VPG*EPST (SEQ ID NO: 41) (positions 51-58) in Savinase (BPN' numbering) and subsequent insertion of residues TVGTNFTD (SEQ ID NO: 40) (positions 56-63) from TA41 subtilase between F50 and Q59 in Savinase,

b) deletion of residues ALNNSI (SEQ ID NO: 43) (positions 74-79) in Savinase (BPN' numbering) and subsequent insertion of residues NGGTGS (SEQ ID NO: 42) (positions 83-88) from TA41 subtilase between A73 and G80 in Savinase.

c) deletion of residues ASGSGSV (SEQ ID NO: 45) (positions 98-104) in Savinase (BPN' numbering) and subsequent insertion of residues DDGSGYA (SEQ ID NO: 44) (positions 107-113) from TA41 subtilase between G97 and S105 in Savinase.

d) deletion of residues KQKNPSW (SEQ ID NO: 47) (positions 235-241) in Savinase (BPN' numbering) and subsequent insertion of residues WAQSPAA (SEQ ID NO: 46) (positions 264-270) from TA41 subtilase between V234 and S242 in Savinase.

26. An isolated nucleic acid sequence comprising a nucleic acid sequence, which encodes for the subtilase or subtilase variant defined or produced in any of the preceding claims.

27. An isolated nucleic acid sequence according to claim 26, wherein the nucleic acid sequence is selected form the group consisting of:

a) a nucleic acid sequence having at least 40% homology with the nucleic acid sequence shown in SEQ ID NO: 20 or SEQ ID NO:21, and
b) a nucleic acid sequence which hybridizes under low stringency conditions, preferably under medium stringency conditions, in particular under high stringency conditions, with
c) a complementary strand of the nucleic acid sequence shown in SEQ ID NO:20 or SEQ ID NO:21, or
d) a subsequence of any of a), b) or c) of at least 100 nucleotides.

28. An isolated nucleic acid sequence according to claim 27, wherein the nucleic acid sequence has at least 45%, at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, or at least 99% homology with the nucleic acid sequence shown in SEQ ID NO:20 or SEQ ID NO:21.

29. An isolated nucleic acid construct comprising a nucleic acid sequence as defined in any of claims 26-28, operably linked to one or more control sequences capable of directing the expression of the polypeptide in a suitable expression host.

30. A recombinant host cell comprising the nucleic acid construct of claim 29.

31. A method for producing the subtilase or subtilase variant defined or produced in any of claims 1 to 25, the method comprising:

a) cultivating the recombinant host cell of claim 30 under conditions conducive to the production of the subtilase variant; and
b) recovering the variant.

32. A detergent composition comprising a subtilase or subtilase variant defined or produced in any of claims 1 to 25.

33. Use of a subtilase or subtilase variant defined or produced in any of claims 1 to 25 in cleaning or washing applications.

```
*RASQQIPWG  IKAIYNNDTL  TSTTGGSGIN  IAVLDTGVNT  SHPDLVNNVE   49  B.sphaericus
AVPSTQTPWG  IKSIYNDQSI  TKTTGGSGIK  VAVLDTGVYT  SHLDLAGSAE   50  TY145
 AASQSTPWG  IKAIYNNSNL  TSTSGGAGIN  IAVLDTGVNT  NHPDLSNNVE   49  TA41
 AASQSTPWG  IKAIYNNSSI  TQTSGGGGIN  IAVLDTGVNT  NHPDLRNNVE   49  TA39
           .           .           .           .
***AQSVPWG  ISRVQAPAAH  NRGLTGSGVK  VAVLDTGIS*  THPDL**NIR   44  Savinase


QCKDFTGATT  PINNSCTDRN  GHGTHVAGTA  LADGGSDQAG  IYGVAPDADL   99
QCKDFTQSNP  LVDGSCTDRQ  GHGTHVAGTV  LAHGGSNGQG  VYGVAPQAKL  100
QCKDFTVGTN  FTDNSCTDRQ  GHGTHVAGSA  LANGGT*GSG  VYGVAPEADL   98
QCKDFTVGTT  YTNNSCTDRQ  GHGTHVAGSA  LADGGT*GNG  VYGVAPDADL   98
.           .           .           .           .
GGASFVPGEP  ****STQDGN  GHGTHVAGTI  AALNN**SIG  VLGVAPSAEL   88


WAYKVLLDSG  SGYSDDIAAA  IRHAADQATA  TGTKTIISMS  LGSSANNSLI  149
WAYKVLGDNG  SGYSDDIAAA  IRHVADEASR  TGSKVVINMS  LGSSAKDSLI  150
WAYKVLGDDG  SGYADDIAEA  IRHAGDQATA  LNTKVVINMS  LGSSGESSLI  148
WAYKVLGDDG  SGYADDIAAA  IRHAGDQATA  LNTKVVINMS  LGSSGESSLI  148
 .          .           .           .           .
YAVKVLGASG  SGSVSSIAQG  LEWAGNNG**  ***MHVANLS  LGSPSPSATL  133


SSAVNYAYSK  GVLIVAAAGN  SGYSQGTIGY  PGALPNAIAV  AALENVQQNG  199
ASAVDYAYGK  GVLIVAAAGN  SGSGSNTIGF  PGGLVNAVAV  AALENVQQNG  200
TNAVDYAYDK  GVLIIAAAGN  SGPKPGSIGY  PGALVNAVAV  AALENTIQNG  198
TNAVNYSYNK  GVLIIAAAGN  SGPYQGSIGY  PGALVNAVAV  AALENKVENG  198
 .          .           .          .           .
EQAVNSATSR  GVLVVAASGN  SGA**GSISY  PARYANAMAV  GATDQN****  177


TYRVADYSSR  GYISTAGDYV  IQEGDIEISA  PGSSVYSTWY  NGGYNTISGT  249
TYRVADFSSR  GNPATAGDYI  IQERDIEVSA  PGASVESTWY  TGGYNTISGT  250
TYRVADFSSR  GHKRTAGDYV  IQKGDVEISA  PGAAVYSTWF  DGGYATISGT  248
TYRVADFSSR  GYSWTDGDYA  IQKGDVEISA  PGAAIYSTWF  DGGYATISGT  248
          .           .           .           .
*NNRASFSQY  GA********  ****GLDIVA  PGVNVQSTYP  GSTYASLNGT  214


SMATPHVSGL  AAKIWAENPS  LSNTQLRSNL  QERAKSVDIK  GGYGAAIGDD  299
SMATPHVAGL  AAKIWSANTS  LSHSQLRTEL  QNRAKVYDIK  GGIGAGTGDD  300
SMASPHAAGL  AAKIWAQSPA  ASNVDVRGEL  QTRASVNDIL  SGNSAGSGDD  298
SMASPHAAGL  AAKIWAQYPS  ASNVDVRGEL  QYRAYENDIL  SGYYAGYGDD  298
.           .           .          .           .
SMATPHVAGA  AALVKQKNPS  WSNVQIRNHL  KNTA**TSLG  *******ST   254


YASGFGFARV  Q                                              310
YASGFGYPRV  K                                              311
IASGFGFAKV  Q                                              309
FASGFGFATV  Q                                              309
.           .
NLYGSGLVNA  EAATR                                          269
```

**Fig. 1**

```
No:  1                     10                20                30
a)   A-Q-S-V-P-Y-G-V-S-Q-I-K-A-P-A-L-H-S-Q-G-Y-T-G-S-N-V-K-V-A-V-
b)   A-Q-S-V-P-W-G-I-S-R-V-Q-A-P-A-A-H-N-R-G-L-T-G-S-G-V-K-V-A-V-

No:            36   40               50        56   60
a)   I-D-S-G-I-D-S-S-H-P-D-L-K-V-A-G-G-A-S-M-V-P-S-E-T-N-P-F-Q-D-
b)   L-D-T-G-I-*-S-T-H-P-D-L-N-I-R-G-G-A-S-F-V-P-G-E-P-*-S-T-Q-D-

No:                 70             80             90
a)   N-N-S-H-G-T-H-V-A-G-T-V-A-A-L-N-N-S-I-G-V-L-G-V-A-P-S-A-S-L-
b)   G-N-G-H-G-T-H-V-A-G-T-I-A-A-L-N-N-S-I-G-V-L-G-V-A-P-S-A-E-L-

No:                 100            110            120
a)   Y-A-V-K-V-L-G-A-D-G-S-G-Q-Y-S-W-I-I-N-G-I-E-W-A-I-A-N-N-M-D-
b)   Y-A-V-K-V-L-G-A-S-G-S-G-S-V-S-S-I-A-Q-G-L-E-W-A-G-N-N-G-M-H-

No:                 130            140            150
a)   V-I-N-M-S-L-G-G-P-S-G-S-A-A-L-K-A-A-V-D-K-A-V-A-S-G-V-V-V-V-
b)   V-A-N-L-S-L-G-S-P-S-P-S-A-T-L-E-Q-A-V-N-S-A-T-S-R-G-V-L-V-V-

No:                 160            170            180
a)   A-A-A-G-N-E-G-T-S-G-S-S-S-T-V-G-Y-P-G-K-Y-P-S-V-I-A-V-G-A-V-
b)   A-A-S-G-N-S-G-A-*-G-S-I-S-*-*-*-Y-P-A-R-Y-A-N-A-M-A-V-G-A-T-

No:                 190            200            210
a)   D-S-S-N-Q-R-A-S-F-S-S-V-G-P-E-L-D-V-M-A-P-G-V-S-I-Q-S-T-L-P-
b)   D-Q-N-N-N-R-A-S-F-S-Q-Y-G-A-G-L-D-I-V-A-P-G-V-N-V-Q-S-T-Y-P-

No:                 220            230            240
a)   G-N-K-Y-G-A-Y-N-G-T-S-M-A-S-P-H-V-A-G-A-A-A-L-I-L-S-K-H-P-N-
b)   G-S-T-Y-A-S-L-N-G-T-S-M-A-T-P-H-V-A-G-A-A-A-L-V-K-Q-K-N-P-S-

No:                 250            260            270
a)   W-T-N-T-Q-V-R-S-S-L-E-N-T-T-T-K-L-G-D-S-F-Y-Y-G-K-G-L-I-N-V-
b)   W-S-N-V-Q-I-R-N-H-L-K-N-T-A-T-S-L-G-S-T-N-L-Y-G-S-G-L-V-N-A-

No:            275
a)   Q-A-A-A-Q
b)   E-A-A-T-R


a) = BPN'

b) = Savinase
```

## Fig. 2

**Fig. 3**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 9217577 A
- WO 0071691 A
- WO 9634946 A
- US 4288627 A
- US 4599311 A
- US 4745051 A
- EP 397485 A
- US 5155037 A
- US 5162222 A
- WO 9117243 A
- US 4882279 A
- US 4879231 A
- EP 272277 A
- EP 230023 A
- US 5077214 A
- WO 8901029 A
- WO 8901028 A
- US 4775624 A
- US 4879236 A
- WO 8906279 A
- WO 8906270 A
- WO 9425583 A
- WO 9219729 A
- WO 9820115 A
- WO 9820116 A
- WO 9834946 A
- EP 258068 A
- EP 305216 A
- WO 9613580 A
- EP 218272 A
- EP 331376 A
- GB 1372034 A
- WO 9506720 A
- WO 9627002 A
- WO 9612012 A
- JP 64744992 B
- WO 9116422 A
- WO 9205249 A
- WO 9401541 A
- EP 407225 A
- EP 260105 A
- WO 9535381 A
- WO 9600292 A
- WO 9530744 A
- WO 9425578 A
- WO 9514783 A
- WO 9522615 A
- WO 9704079 A
- WO 9707202 A
- GB 1296839 A
- WO 9402597 A
- WO 9418314 A
- WO 9623873 A
- WO 9743424 A
- US 4435307 A
- US 5648263 A
- US 5691178 A
- US 5776757 A
- WO 8909259 A
- EP 0495257 A
- EP 0531372 A
- WO 9611262 A
- WO 9629397 A
- WO 9808940 A
- WO 9407998 A
- EP 0531315 A
- US 5457046 A
- US 5686593 A
- US 5763254 A
- WO 9524471 A
- WO 9812307 A
- DK 9800299 W
- WO 9324618 A
- WO 9510602 A
- WO 9815257 A
- US 4106991 A
- US 4661452 A
- GB 1483591 A
- EP 238216 A
- WO 9219709 A
- WO 9219708 A
- WO 0242740 A

**Non-patent literature cited in the description**

- **SIEZEN RJ ; LEUNISSEN JAM.** *Protein Science,* 1997, vol. 6, 501-523
- **DAVAIL S et al.** *The Journal of Biological Chemistry,* 1994, vol. 269 (26), 17448-17453
- **NARINX E et al.** *Protein Engineering,* 1997, vol. 10 (11), 1271-1279
- **MIYAZAKI K et al.** *J Mol Biol,* 2000, vol. 297, 1015-1026

- **WINTRODE TL et al.** *Journal of Biological Chemistry,* 2000, vol. 275 (41), 31635-31640
- **SIEZEN et al.** *Protein Engng.,* 1991, vol. 4, 719-737
- **SIEZEN et al.** *Protein Science,* 1997, vol. 6, 501-523
- **SIEZEN et al.** *Protein Engng,* 1991, vol. 4, 719-737
- **S.B. NEEDLEMAN ; C.D. WUNSCH.** *Journal of Molecular Biology,* 1970, vol. 48, 443-445
- **DAVAIL S et al.** *J. Biol. Chem.,* 1994, vol. 269, 17448-17453
- **FEINBERG, A. P. ; VOGELSTEIN, B.** *Anal. Biochem.,* 1983, vol. 132, 6-13
- **R.J. SIEZEN ; J.A.M LEUNISSEN.** *Protein science,* 1997, vol. 6 (3), 501-523
- **STOUT, G.K. ; JENSEN, L.H.** X-Ray Structure Determination. John Wiley & Sons, Inc, 1989
- **KABSCH ; SANDER.** *Biopolymers,* 1983, vol. 22, 2577-2637
- **SAMBROOK et al.** Molecular cloning: A laboratory manual. Cold Spring Harbor lab, 1989
- Current protocols in Molecular Biology. John Wiley and Sons, 1995
- Molecular Biological Methods for Bacillus. John Wiley and Sons, 1990
- DNA Cloning: A Practical Approach. 1985, vol. I and II
- Oligonucleotide Synthesis. 1984
- Nucleic Acid Hybridization. 1985
- Transcription And Translation. 1984
- Animal Cell Culture. 1986
- Immobilized Cells And Enzymes. IRL Press, 1986
- **B. PERBAL.** A Practical Guide To Molecular Cloning. 1984
- **L.J. JENSEN et al.** *Nucleic Acid Research,* 1998, vol. 26, 697-702
- **LEUNG et al.** *Technique,* 1989, vol. 1, 11-15
- **FOWLER et al.** *Molec. Gen. Genet,* 1974, vol. 133, 179-191
- **TOMANDL, D. et al.** *Journal of Computer-Aided Molecular Design,* 1997, vol. 11, 29-38
- **JENSEN, LJ ; ANDERSEN, KV ; SVENDSEN, A ; KRETZSCHMAR, T.** *Nucleic Acids Research,* 1998, vol. 26, 697-702
- **EHRLICH.** *Proceedings of the National Academy of Sciences USA,* 1978, vol. 75, 1433
- **VILLA-KAMAROFF et al.** *Proceedings of the National Academy of Sciences USA,* 1978, vol. 75, 3727-3731
- Useful proteins from recombinant bacteria. *Scientific American,* 1980, vol. 242, 74-94
- **MCKNIGHT et al.** *The EMBO J.,* 1985, vol. 4, 2093-2099
- **HITZEMAN et al.** *J. Biol. Chem.,* 1980, vol. 255, 12073-12080
- *J. Mol. Appl. Gen.,* 1982, vol. 1, 419-434
- **YOUNG et al.** Genetic Engineering of Microorganisms for Chemicals. Plenum Press, 1982
- **RUSSELL et al.** *Nature,* 1983, vol. 304, 652-654
- **ROMANOS et al.** *Yeast,* 1992, vol. 8, 423-488
- **SUBRAMANI et al.** *Mol. Cell Biol.,* 1981, vol. 1, 854-864
- **PALMITER et al.** *Science,* 1983, vol. 222, 809-814
- **VASUVEDAN et al.** *FEBS Lett.,* 1992, vol. 311, 7-11
- **J.M. VLAK et al.** *J. Gen. Virology,* 1988, vol. 69, 765-776
- **GLEESON et al.** *J. Gen. Microbiol,* 1986, vol. 132, 3459-3465
- Soc. App. Bacteriol. Symposium Series No. 9. 1980
- Biochemistry and Genetics of Yeast. 1987
- The Yeasts. 1987
- The Molecular Biology of the Yeast Saccharomyces. 1981
- Guide to Yeast Genetics and Molecular Biology, Methods in Enzymology. Academic Press, Inc, vol. 194, 182-187
- **ITO et al.** *Journal of Bacteriology,* 1983, vol. 153, 163
- **HINNEN et al.** *Proceedings of the National Academy of Sciences USA,* 1978, vol. 75, 1920
- **KAUFMAN ; SHARP.** *J. Mol. Biol.,* 1982, vol. 159, 601-621
- **SOUTHERN ; BERG.** *J. Mol. Appl. Genet.,* 1982, vol. 1, 327-341
- **LOYTER et al.** *Proc. Natl. Acad. Sci. USA,* 1982, vol. 79, 422-426
- **WIGLER et al.** *Cell,* 1978, vol. 14, 725
- **CORSARO ; PEARSON.** *Somatic Cell Genetics,* 1981, vol. 7, 603
- **AUSUBEL et al.** Current Protocols in Molecular Biology. John Wiley and Sons, Inc, 1987
- **HAWLEY-NELSON et al.** *Focus,* 1993, vol. 15, 73
- **CICCARONE et al.** *Focus,* 1993, vol. 15, 80
- **GRAHAM ; VAN DER EB.** *Virology,* 1973, vol. 52, 456
- **NEUMANN et al.** *EMBO J.,* 1982, vol. 1, 841-845
- More Gene Manipulations in Fungi. Academic Press, 1991
- Protein Purification. VCH Publishers, 1989
- **DARTOIS et al.** *Biochemica et Biophysica Acta,* 1993, vol. 1131, 253-360
- **SAMBROOK et al.** Molecular Cloning: A Laboratory Manual. Cold Spring Harbor, 1989

SEQUENCE LISTING

<110>  Novozymes A/S

<120>  Subtilases

<130>  10203

<160>  51

<170>  PatentIn version 3.4

<210>  1
<211>  311
<212>  PRT
<213>  Bacillus sp. TY145


<220>
<221>  PEPTIDE
<222>  (1)..(311)
<223>  Protease TY145

<400>  1

Ala Val Pro Ser Thr Gln Thr Pro Trp Gly Ile Lys Ser Ile Tyr Asn
1               5                   10                  15


Asp Gln Ser Ile Thr Lys Thr Thr Gly Gly Ser Gly Ile Lys Val Ala
            20                  25                  30


Val Leu Asp Thr Gly Val Tyr Thr Ser His Leu Asp Leu Ala Gly Ser
        35                  40                  45


Ala Glu Gln Cys Lys Asp Phe Thr Gln Ser Asn Pro Leu Val Asp Gly
    50                  55                  60


Ser Cys Thr Asp Arg Gln Gly His Gly Thr His Val Ala Gly Thr Val
65                  70                  75                  80


Leu Ala His Gly Gly Ser Asn Gly Gln Gly Val Tyr Gly Val Ala Pro
                85                  90                  95


Gln Ala Lys Leu Trp Ala Tyr Lys Val Leu Gly Asp Asn Gly Ser Gly
            100                 105                 110


Tyr Ser Asp Asp Ile Ala Ala Ala Ile Arg His Val Ala Asp Glu Ala
            115                 120                 125


Ser Arg Thr Gly Ser Lys Val Val Ile Asn Met Ser Leu Gly Ser Ser
        130                 135                 140


Ala Lys Asp Ser Leu Ile Ala Ser Ala Val Asp Tyr Ala Tyr Gly Lys
145                 150                 155                 160


Gly Val Leu Ile Val Ala Ala Ala Gly Asn Ser Gly Ser Gly Ser Asn
                165                 170                 175

Thr Ile Gly Phe Pro Gly Gly Leu Val Asn Ala Val Ala Val Ala Ala
            180                 185                 190

Leu Glu Asn Val Gln Gln Asn Gly Thr Tyr Arg Val Ala Asp Phe Ser
        195                 200                 205

Ser Arg Gly Asn Pro Ala Thr Ala Gly Asp Tyr Ile Ile Gln Glu Arg
    210                 215                 220

Asp Ile Glu Val Ser Ala Pro Gly Ala Ser Val Glu Ser Thr Trp Tyr
225                 230                 235                 240

Thr Gly Gly Tyr Asn Thr Ile Ser Gly Thr Ser Met Ala Thr Pro His
                245                 250                 255

Val Ala Gly Leu Ala Ala Lys Ile Trp Ser Ala Asn Thr Ser Leu Ser
            260                 265                 270

His Ser Gln Leu Arg Thr Glu Leu Gln Asn Arg Ala Lys Val Tyr Asp
        275                 280                 285

Ile Lys Gly Gly Ile Gly Ala Gly Thr Gly Asp Asp Tyr Ala Ser Gly
        290                 295                 300

Phe Gly Tyr Pro Arg Val Lys
305                 310


<210>   2
<211>   420
<212>   PRT
<213>   Bacillus sp. TA39


<220>
<221>   PEPTIDE
<222>   (1)..(420)
<223>   Protease BSTA39

<400>   2

Met Lys Arg Ser Gly Lys Ile Phe Thr Thr Ala Met Leu Ala Val Thr
1               5                   10                  15

Leu Met Met Pro Ala Met Gly Val Ser Ala Asn Glu Gly Asn Ala Ala
            20                  25                  30

Ala Glu Gly Asn Glu Lys Phe Arg Val Leu Val Asp Ser Val Asp Gln
        35                  40                  45

Lys Asn Leu Lys Asn Ala Lys Gln Gln Tyr Gly Val His Trp Asp Phe
    50                  55                  60

Ala Gly Glu Gly Phe Thr Thr Asp Met Asn Glu Lys Gln Phe Asn Ala
65                  70                  75                  80

Leu Lys Lys Asn Lys Asn Leu Thr Val Glu Lys Val Pro Glu Leu Glu
                    85                  90              95

Ile Ala Thr Ala Thr Asp Lys Pro Glu Ala Leu Tyr Asn Ala Met Ala
            100                 105             110

Ala Ser Gln Ser Thr Pro Trp Gly Ile Lys Ala Ile Tyr Asn Asn Ser
        115             120             125

Ser Ile Thr Gln Thr Ser Gly Gly Gly Ile Asn Ile Ala Val Leu
    130             135             140

Asp Thr Gly Val Asn Thr Asn His Pro Asp Leu Arg Asn Asn Val Glu
145             150             155             160

Gln Cys Lys Asp Phe Thr Val Gly Thr Thr Tyr Thr Asn Asn Ser Cys
            165             170             175

Thr Asp Arg Gln Gly His Gly Thr His Val Ala Gly Ser Ala Leu Ala
            180             185             190

Asp Gly Gly Thr Gly Asn Gly Val Tyr Gly Val Ala Pro Asp Ala Asp
        195             200             205

Leu Trp Ala Tyr Lys Val Leu Gly Asp Asp Gly Ser Gly Tyr Ala Asp
    210             215             220

Asp Ile Ala Ala Ala Ile Arg His Ala Gly Asp Gln Ala Thr Ala Leu
225             230             235             240

Asn Thr Lys Val Val Ile Asn Met Ser Leu Gly Ser Ser Gly Glu Ser
            245             250             255

Ser Leu Ile Thr Asn Ala Val Asn Tyr Ser Tyr Asn Lys Gly Val Leu
            260             265             270

Ile Ile Ala Ala Ala Gly Asn Ser Gly Pro Tyr Gln Gly Ser Ile Gly
        275             280             285

Tyr Pro Gly Ala Leu Val Asn Ala Val Ala Val Ala Ala Leu Glu Asn
    290             295             300

Lys Val Glu Asn Gly Thr Tyr Arg Val Ala Asp Phe Ser Ser Arg Gly
305             310             315             320

Tyr Ser Trp Thr Asp Gly Asp Tyr Ala Ile Gln Lys Gly Asp Val Glu
            325             330             335

Ile Ser Ala Pro Gly Ala Ala Ile Tyr Ser Thr Trp Phe Asp Gly Gly
            340             345             350

Tyr Ala Thr Ile Ser Gly Thr Ser Met Ala Ser Pro His Ala Ala Gly

```
                355                    360                    365

        Leu Ala Ala Lys Ile Trp Ala Gln Tyr Pro Ser Ala Ser Asn Val Asp
            370                 375             380

        Val Arg Gly Glu Leu Gln Tyr Arg Ala Tyr Glu Asn Asp Ile Leu Ser
        385                 390             395                 400

        Gly Tyr Tyr Ala Gly Tyr Gly Asp Asp Phe Ala Ser Gly Phe Gly Phe
                        405             410                 415

        Ala Thr Val Gln
                    420


        <210>  3
        <211>  419
        <212>  PRT
        <213>  Bacillus sp. TA41


        <220>
        <221>  PEPTIDE
        <222>  (1)..(419)
        <223>  Protease BSTA41

        <400>  3

        Met Lys Arg Ser Gly Lys Ile Phe Thr Thr Ala Met Leu Ala Val Thr
        1               5               10              15

        Leu Met Met Pro Ala Ile Gly Val Ser Ala Asn Arg Gly Asn Ala Ala
                    20              25              30

        Asp Gly Asn Glu Lys Phe Arg Val Leu Val Asp Ser Ala Asn Gln Asn
                35              40              45

        Asn Leu Lys Asn Val Lys Glu Gln Tyr Gly Val His Trp Asp Phe Ala
            50              55              60

        Gly Glu Gly Phe Thr Thr Asn Met Asn Glu Lys Gln Phe Asn Ala Leu
        65              70              75              80

        Gln Asn Asn Lys Asn Leu Thr Val Glu Lys Val Pro Glu Leu Glu Ile
                    85              90              95

        Ala Thr Ala Thr Asn Lys Pro Glu Ala Leu Tyr Asn Ala Met Ala Ala
                    100             105             110

        Ser Gln Ser Thr Pro Trp Gly Ile Lys Ala Ile Tyr Asn Asn Ser Asn
                115             120             125

        Leu Thr Ser Thr Ser Gly Gly Ala Gly Ile Asn Ile Ala Val Leu Asp
            130             135             140

        Thr Gly Val Asn Thr Asn His Pro Asp Leu Ser Asn Asn Val Glu Gln
```

```
            145                  150                  155                  160

      Cys Lys Asp Phe Thr Val Gly Thr Asn Phe Thr Asp Asn Ser Cys Thr
                      165                  170                  175

      Asp Arg Gln Gly His Gly Thr His Val Ala Gly Ser Ala Leu Ala Asn
                  180                  185                  190

      Gly Gly Thr Gly Ser Gly Val Tyr Gly Val Ala Pro Glu Ala Asp Leu
                  195                  200                  205

      Trp Ala Tyr Lys Val Leu Gly Asp Asp Gly Ser Gly Tyr Ala Asp Asp
          210                  215                  220

      Ile Ala Glu Ala Ile Arg His Ala Gly Asp Gln Ala Thr Ala Leu Asn
      225                  230                  235                  240

      Thr Lys Val Val Ile Asn Met Ser Leu Gly Ser Ser Gly Glu Ser Ser
                      245                  250                  255

      Leu Ile Thr Asn Ala Val Asp Tyr Ala Tyr Asp Lys Gly Val Leu Ile
                  260                  265                  270

      Ile Ala Ala Ala Gly Asn Ser Gly Pro Lys Pro Gly Ser Ile Gly Tyr
                  275                  280                  285

      Pro Gly Ala Leu Val Asn Ala Val Ala Val Ala Ala Leu Glu Asn Thr
          290                  295                  300

      Ile Gln Asn Gly Thr Tyr Arg Val Ala Asp Phe Ser Ser Arg Gly His
      305                  310                  315                  320

      Lys Arg Thr Ala Gly Asp Tyr Val Ile Gln Lys Gly Asp Val Glu Ile
                      325                  330                  335

      Ser Ala Pro Gly Ala Ala Val Tyr Ser Thr Trp Phe Asp Gly Gly Tyr
                  340                  345                  350

      Ala Thr Ile Ser Gly Thr Ser Met Ala Ser Pro His Ala Ala Gly Leu
              355                  360                  365

      Ala Ala Lys Ile Trp Ala Gln Ser Pro Ala Ala Ser Asn Val Asp Val
          370                  375                  380

      Arg Gly Glu Leu Gln Thr Arg Ala Ser Val Asn Asp Ile Leu Ser Gly
      385                  390                  395                  400

      Asn Ser Ala Gly Ser Gly Asp Asp Ile Ala Ser Gly Phe Gly Phe Ala
                      405                  410                  415

      Lys Val Gln
```

<210> 4
<211> 310
<212> PRT
<213> Bacillus sphaericus


<220>
<221> PEPTIDE
<222> (1)..(310)
<223> Protease Sphericase

<400> 4

Arg Ala Ser Gln Gln Ile Pro Trp Gly Ile Lys Ala Ile Tyr Asn Asn
1               5               10              15


Asp Thr Leu Thr Ser Thr Thr Gly Gly Ser Gly Ile Asn Ile Ala Val
            20              25              30


Leu Asp Thr Gly Val Asn Thr Ser His Pro Asp Leu Val Asn Asn Val
        35              40              45


Glu Gln Cys Lys Asp Phe Thr Gly Ala Thr Thr Pro Ile Asn Asn Ser
    50              55              60


Cys Thr Asp Arg Asn Gly His Gly Thr His Val Ala Gly Thr Ala Leu
65              70              75              80


Ala Asp Gly Gly Ser Asp Gln Ala Gly Ile Tyr Gly Val Ala Pro Asp
                85              90              95


Ala Asp Leu Trp Ala Tyr Lys Val Leu Leu Asp Ser Gly Ser Gly Tyr
            100             105             110


Ser Asp Asp Ile Ala Ala Ala Ile Arg His Ala Ala Asp Gln Ala Thr
        115             120             125


Ala Thr Gly Thr Lys Thr Ile Ile Ser Met Ser Leu Gly Ser Ser Ala
    130             135             140


Asn Asn Ser Leu Ile Ser Ser Ala Val Asn Tyr Ala Tyr Ser Lys Gly
145             150             155             160


Val Leu Ile Val Ala Ala Ala Gly Asn Ser Gly Tyr Ser Gln Gly Thr
                165             170             175


Ile Gly Tyr Pro Gly Ala Leu Pro Asn Ala Ile Ala Val Ala Ala Leu
            180             185             190


Glu Asn Val Gln Gln Asn Gly Thr Tyr Arg Val Ala Asp Tyr Ser Ser
        195             200             205


Arg Gly Tyr Ile Ser Thr Ala Gly Asp Tyr Val Ile Gln Glu Gly Asp
        210             215             220

```
Ile Glu Ile Ser Ala Pro Gly Ser Ser Val Tyr Ser Thr Trp Tyr Asn
225             230             235             240

Gly Gly Tyr Asn Thr Ile Ser Gly Thr Ser Met Ala Thr Pro His Val
            245             250             255

Ser Gly Leu Ala Ala Lys Ile Trp Ala Glu Asn Pro Ser Leu Ser Asn
            260             265             270

Thr Gln Leu Arg Ser Asn Leu Gln Glu Arg Ala Lys Ser Val Asp Ile
        275             280             285

Lys Gly Gly Tyr Gly Ala Ala Ile Gly Asp Asp Tyr Ala Ser Gly Phe
    290             295             300

Gly Phe Ala Arg Val Gln
305             310
```

```
<210>  5
<211>  275
<212>  PRT
<213>  Bacillus amyloliquefaciens


<220>
<221>  PEPTIDE
<222>  (1)..(275)
<223>  Protease BPN'

<400>  5
```

```
Ala Gln Ser Val Pro Tyr Gly Val Ser Gln Ile Lys Ala Pro Ala Leu
1               5               10              15

His Ser Gln Gly Tyr Thr Gly Ser Asn Val Lys Val Ala Val Ile Asp
            20              25              30

Ser Gly Ile Asp Ser Ser His Pro Asp Leu Lys Val Ala Gly Gly Ala
            35              40              45

Ser Met Val Pro Ser Glu Thr Asn Pro Phe Gln Asp Asn Asn Ser His
    50              55              60

Gly Thr His Val Ala Gly Thr Val Ala Ala Leu Asn Asn Ser Ile Gly
65              70              75              80

Val Leu Gly Val Ala Pro Ser Ala Ser Leu Tyr Ala Val Lys Val Leu
            85              90              95

Gly Ala Asp Gly Ser Gly Gln Tyr Ser Trp Ile Ile Asn Gly Ile Glu
            100             105             110

Trp Ala Ile Ala Asn Asn Met Asp Val Ile Asn Met Ser Leu Gly Gly
            115             120             125
```

```
Pro Ser Gly Ser Ala Ala Leu Lys Ala Ala Val Asp Lys Ala Val Ala
    130             135             140

Ser Gly Val Val Val Val Ala Ala Ala Gly Asn Glu Gly Thr Ser Gly
145             150             155             160

Ser Ser Ser Thr Val Gly Tyr Pro Gly Lys Tyr Pro Ser Val Ile Ala
                165             170             175

Val Gly Ala Val Asp Ser Ser Asn Gln Arg Ala Ser Phe Ser Ser Val
            180             185             190

Gly Pro Glu Leu Asp Val Met Ala Pro Gly Val Ser Ile Gln Ser Thr
        195             200             205

Leu Pro Gly Asn Lys Tyr Gly Ala Tyr Asn Gly Thr Ser Met Ala Ser
    210             215             220

Pro His Val Ala Gly Ala Ala Ala Leu Ile Leu Ser Lys His Pro Asn
225             230             235             240

Trp Thr Asn Thr Gln Val Arg Ser Ser Leu Glu Asn Thr Thr Thr Lys
                245             250             255

Leu Gly Asp Ser Phe Tyr Tyr Gly Lys Gly Leu Ile Asn Val Gln Ala
                260             265             270

Ala Ala Gln
        275
```

```
<210>   6
<211>   269
<212>   PRT
<213>   Bacillus lentus


<220>
<221>   PEPTIDE
<222>   (1)..(269)
<223>   Protease Savinase or Subtilisin 309

<400>   6
```

```
Ala Gln Ser Val Pro Trp Gly Ile Ser Arg Val Gln Ala Pro Ala Ala
1               5               10              15

His Asn Arg Gly Leu Thr Gly Ser Gly Val Lys Val Ala Val Leu Asp
            20              25              30

Thr Gly Ile Ser Thr His Pro Asp Leu Asn Ile Arg Gly Gly Ala Ser
        35              40              45

Phe Val Pro Gly Glu Pro Ser Thr Gln Asp Gly Asn Gly His Gly Thr
    50              55              60
```

His Val Ala Gly Thr Ile Ala Ala Leu Asn Asn Ser Ile Gly Val Leu
65                  70                  75                  80

Gly Val Ala Pro Ser Ala Glu Leu Tyr Ala Val Lys Val Leu Gly Ala
                85                  90                  95

Ser Gly Ser Gly Ser Val Ser Ser Ile Ala Gln Gly Leu Glu Trp Ala
                100                 105                 110

Gly Asn Asn Gly Met His Val Ala Asn Leu Ser Leu Gly Ser Pro Ser
            115                 120                 125

Pro Ser Ala Thr Leu Glu Gln Ala Val Asn Ser Ala Thr Ser Arg Gly
        130                 135                 140

Val Leu Val Val Ala Ala Ser Gly Asn Ser Gly Ala Gly Ser Ile Ser
145                 150                 155                 160

Tyr Pro Ala Arg Tyr Ala Asn Ala Met Ala Val Gly Ala Thr Asp Gln
                165                 170                 175

Asn Asn Asn Arg Ala Ser Phe Ser Gln Tyr Gly Ala Gly Leu Asp Ile
            180                 185                 190

Val Ala Pro Gly Val Asn Val Gln Ser Thr Tyr Pro Gly Ser Thr Tyr
        195                 200                 205

Ala Ser Leu Asn Gly Thr Ser Met Ala Thr Pro His Val Ala Gly Ala
        210                 215                 220

Ala Ala Leu Val Lys Gln Lys Asn Pro Ser Trp Ser Asn Val Gln Ile
225                 230                 235                 240

Arg Asn His Leu Lys Asn Thr Ala Thr Ser Leu Gly Ser Thr Asn Leu
                245                 250                 255

Tyr Gly Ser Gly Leu Val Asn Ala Glu Ala Ala Thr Arg
            260                 265

<210>   7
<211>   60
<212>   DNA
<213>   Artificial Sequence

<220>
<223>   synthetic oligopeptide

<220>
<221>   misc_feature
<222>   (1)..(60)
<223>   primer 28-35-CN

<400>   7

tagatctgga tgagtggawv yccctgtatc gaggacagcw rbttttacac cagaacctgt        60

<210>    8
<211>    18
<212>    DNA
<213>    Artificial Sequence

<220>
<223>    synthetic oligopeptide

<220>
<221>    misc_feature
<222>    (1)..(18)
<223>    primer 28-35-NC

<400>    8
tccactcatc cagatcta                                                       18

<210>    9
<211>    45
<212>    DNA
<213>    Artificial Sequence

<220>
<223>    synthetic oligopeptide

<220>
<221>    misc_feature
<222>    (1)..(45)
<223>    primer 71-72-73-CN (I)

<400>    9
aatcgaattg tttaaagcag cwvyygwccc ggccacatgc gtgcc                         45

<210>    10
<211>    45
<212>    DNA
<213>    Artificial Sequence

<220>
<223>    synthetic oligopeptide

<220>
<221>    misc_feature
<222>    (1)..(45)
<223>    primer 71-72-73-CN (II)

<400>    10
aatcgaattg tttaaagcaa gwvyygwccc ggccacatgc gtgcc                         45

<210>    11
<211>    45
<212>    DNA
<213>    Artificial Sequence

<220>
<223>    synthetic oligopeptide

<220>
<221>    misc_feature
<222>    (1)..(45)

```
<223>   primer 71-72-73-CN (III)

<400>   11
aatcgaattg tttaaagcgc cwvyygwccc ggccacatgc gtgcc                    45


<210>   12
<211>   18
<212>   DNA
<213>   Artificial Sequence

<220>
<223>   synthetic oligopeptide


<220>
<221>   misc_feature
<222>   (1)..(18)
<223>   primer 71-72-73-NC

<400>   12
gctttaaaca attcgatt                                                  18


<210>   13
<211>   24
<212>   DNA
<213>   Artificial Sequence

<220>
<223>   synthetic oligopeptide


<220>
<221>   misc_feature
<222>   (1)..(24)
<223>   primer 139

<400>   13
gattaacgcg ttgccgcttc tgcg                                           24


<210>   14
<211>   39
<212>   DNA
<213>   Artificial Sequence

<220>
<223>   synthetic oligopeptide


<220>
<221>   misc_feature
<222>   (1)..(39)
<223>   primer 175-CN (I)

<400>   14
atcagtagct ccgactgcca ytgcgttcgc atagcgcgc                           39


<210>   15
<211>   39
<212>   DNA
<213>   Artificial Sequence

<220>
<223>   synthetic oligopeptide
```

```
<220>
<221>  misc_feature
<222>  (1)..(39)
<223>  primer 175-CN (II)

<400>  15
atcagtagct ccgactgccg ctgcgttcgc atagcgcgc                              39


<210>  16
<211>  18
<212>  DNA
<213>  Artificial Sequence

<220>
<223>  synthetic oligopeptide


<220>
<221>  misc_feature
<222>  (1)..(18)
<223>  primer 175-NC

<400>  16
gcagtcggag ctactgat                                                     18


<210>  17
<211>  39
<212>  DNA
<213>  Artificial Sequence

<220>
<223>  synthetic oligopeptide


<220>
<221>  misc_feature
<222>  (1)..(39)
<223>  primer 224-CN

<400>  17
cgcacctgca acatgaggcg hagccatcga tgtaccgtt                              39


<210>  18
<211>  18
<212>  DNA
<213>  Artificial Sequence

<220>
<223>  synthetic oligopeptide


<220>
<221>  misc_feature
<222>  (1)..(18)
<223>  primer 224-NC

<400>  18
cctcatgttg caggtgcg                                                     18


<210>  19
<211>  22
<212>  DNA
<213>  Artificial Sequence

<220>
```

```
<223>   synthetic oligopeptide


<220>
<221>   misc_feature
<222>   (1)..(22)
<223>   primer 317-CN

<400>   19
tggcgcaatc ggtaccatgg gg                                              22


<210>   20
<211>   936
<212>   DNA
<213>   Bacillus sp. TY145

<400>   20
gcggtaccaa gtacacaaac cccttggggc ataaagtcaa tttataatga tcaatcaatt      60

acaaaaacaa ctggaggcag cggaattaag gtagctgttt tagatacagg ggtttataca     120

agccatttag atttagctgg ttctgccgag caatgcaagg attttaccca atctaatcct     180

ttagtagatg gttcatgcac cgatcgccaa gggcatggta cacatgttgc cggaactgta     240

ttggcgcatg gaggcagtaa tggacaaggc gtttacgggg tggctccgca agcgaaacta     300

tgggcatata aagtattagg agataacggc agcggatact ctgatgatat tgcagcagct     360

atcagacatg tagctgatga agcttcacgt acaggttcca aagtagtaat taatatgtcg     420

ctaggttcat ctgccaagga ttcattgatt gctagtgcag tagattatgc atatggaaaa     480

ggtgtattaa tcgttgctgc ggctggtaat agtgggtcag gcagcaatac aatcggcttt     540

cctggcgggc ttgtaaatgc agtggcagta gcggcattgg agaatgttca gcaaaatgga     600

acttatcgag tagctgattt ctcatctaga gggaatccgg caactgctgg agattatatc     660

attcaagagc gtgatattga agtttcagct ccgggagcaa gtgtagagtc tacatggtac     720

actggcggtt ataatacgat cagcggtaca tcaatggcta cacctcatgt agctgggtta     780

gctgctaaaa tctggtcagc gaatacttca ttaagtcata gccaactgcg cacagaattg     840

caaaatcgcg ctaaagtata tgatattaaa ggtggtatcg gagccggaac aggtgacgat     900

tatgcatcag ggttcggata tccaagagta aaataa                              936


<210>   21
<211>   1143
<212>   DNA
<213>   Bacillus lentus

<400>   21
atgaagaaac cgttggggaa aattgtcgca agcaccgcac tactcatttc tgttgctttt     60

agttcatcga tcgcatcggc tgctgaagaa gcaaaagaaa aatatttaat tggctttaat     120

gagcaggaag ctgtcagtga gtttgtagaa caagtagagg caaatgacga ggtcgccatt     180

ctctctgagg aagaggaagt cgaaattgaa ttgcttcatg aatttgaaac gattcctgtt     240

ttatccgttg agttaagccc agaagatgtg gacgcgcttg aactcgatcc agcgatttct     300

tatattgaag aggatgcaga agtaacgaca atggcgcaat cggtaccatg gggaattagc     360
```

```
cgtgtgcaag  ccccagctgc  ccataaccgt  ggattgacag  gttctggtgt  aaaagttgct    420

gtcctcgata  cagggatatc  cactcatcca  gatctaaata  ttcgtggtgg  cgcaagcttt    480

gtaccagggg  aaccgtcgac  tcaagatggg  aatgggcatg  gcacgcatgt  ggccgggacg    540

atcgctgctt  taaacaattc  gattggcgtt  cttggcgtag  cgccgagcgc  tgagctatac    600

gctgttaaag  tcctaggggc  gagcggttca  ggttcggtca  gctcgattgc  ccaaggattg    660

gaatgggcag  ggaacaatgg  catgcacgtt  gctaatttga  gtttaggaag  cccttcgcca    720

agtgccacac  tcgagcaagc  tgttaatagc  gcgacttcta  gaggcgttct  tgttgtagcg    780

gcatctggga  attcaggtgc  aggctcaatc  agctatccgg  cgcgctatgc  gaacgcaatg    840

gcagtcggag  ctactgatca  aaacaacaac  cgcgctagct  tttcacagta  tggcgcaggc    900

cttgacattg  tcgcacccgg  ggtaaacgtg  cagagcacat  acccaggttc  aacatatgcc    960

agcttaaacg  gtacatcgat  ggctactcct  catgttgcag  gtgcggccgc  ccttgttaaa   1020

caaaagaacc  catcttggtc  taatgtacaa  attcgaaatc  atctaaagaa  tacggcaact   1080

agtttaggaa  gcacgaactt  gtatggaagc  ggacttgtta  acgcagaagc  ggcaacgcgt   1140

taa                                                                     1143
```

```
<210>  22
<211>  12
<212>  PRT
<213>  Artificial sequence

<220>
<223>  Highly mobile region of Savinase

<400>  22

Ser Ala Lys Asp Ser Leu Ile Ala Ser Ala Val Asp
1               5                   10


<210>  23
<211>  12
<212>  PRT
<213>  Artificial sequence

<220>
<223>  Highly mobile region of Savinase

<400>  23

Pro Ser Pro Ser Ala Thr Leu Glu Gln Ala Val Asn
1               5                   10


<210>  24
<211>  18
<212>  PRT
<213>  Artificial sequence

<220>
<223>  Highly mobile region of Savinase

<400>  24

Ala Gly Asn Ser Gly Ser Gly Ser Asn Thr Ile Gly Phe Pro Gly Gly
1               5                   10                  15
```

Leu Val


<210> 25
<211> 16
<212> PRT
<213> Artificial sequence

<220>
<223> Highly mobile region of Savinase

<400> 25

Ser Gly Asn Ser Gly Ala Gly Ser Ile Ser Tyr Pro Ala Arg Tyr Ala
1               5                   10                  15


<210> 26
<211> 16
<212> PRT
<213> Artificial sequence

<220>
<223> Highly mobile region of Savinase

<400> 26

Ala Ser Val Glu Ser Thr Trp Tyr Thr Gly Gly Tyr Asn Thr Ile Ser
1               5                   10                  15


<210> 27
<211> 16
<212> PRT
<213> Artificial sequence

<220>
<223> Highly mobile region of Savinase

<400> 27

Val Asn Val Gln Ser Thr Tyr Pro Gly Ser Thr Tyr Ala Ser Leu Asn
1               5                   10                  15


<210> 28
<211> 7
<212> PRT
<213> Artificial sequence

<220>
<223> Highly mobile region of Savinase

<400> 28

Met Ser Leu Gly Ser Ser Gly
1               5


<210> 29
<211> 7
<212> PRT
<213> Artificial sequence

<220>
<223> Highly mobile region of Savinase

<400> 29

Leu Ser Leu Gly Ser Pro Ser
1               5


<210> 30
<211> 12
<212> PRT
<213> Artificial sequence

<220>
<223> Highly mobile region of Savinase

<400> 30

Met Ser Leu Gly Ser Ser Gly Glu Ser Ser Leu Ile
1               5                   10


<210> 31
<211> 12
<212> PRT
<213> Artificial sequence

<220>
<223> Highly mobile region of Savinase

<400> 31

Leu Ser Leu Gly Ser Pro Ser Pro Ser Ala Thr Leu
1               5                   10


<210> 32
<211> 8
<212> PRT
<213> Artificial sequence

<220>
<223> Highly mobile region of Savinase

<400> 32

Asn Asn Ser Ser Ile Thr Gln Thr
1               5


<210> 33
<211> 8
<212> PRT
<213> Artificial sequence

<220>
<223> Highly mobile region of Savinase

<400> 33

Val Gln Ala Pro Ala Ala His Asn
1               5


<210> 34
<211> 8
<212> PRT
<213> Artificial sequence

<220>

<223>    Highly mobile region of Savinase

<400>    34

Thr Val Gly Thr Thr Tyr Thr Asn
1                   5


<210>    35
<211>    7
<212>    PRT
<213>    Artificial sequence

<220>
<223>    Highly mobile region of Savinase

<400>    35

Val Pro Gly Glu Pro Ser Thr
1                   5


<210>    36
<211>    7
<212>    PRT
<213>    Artificial sequence

<220>
<223>    Highly mobile region of Savinase

<400>    36

Ser Gly Glu Ser Ser Leu Ile
1                   5


<210>    37
<211>    7
<212>    PRT
<213>    Artificial sequence

<220>
<223>    Highly mobile region of Savinase

<400>    37

Pro Ser Pro Ser Ala Thr Leu
1                   5


<210>    38
<211>    9
<212>    PRT
<213>    Artificial sequence

<220>
<223>    Highly mobile region of Savinase

<400>    38

Trp Phe Asp Gly Gly Tyr Ala Thr Ile
1                   5


<210>    39
<211>    9
<212>    PRT
<213>    Artificial sequence

```
<220>
<223>  Highly mobile region of Savinase

<400>  39

Tyr Pro Gly Ser Thr Tyr Ala Ser Leu
1               5


<210>  40
<211>  8
<212>  PRT
<213>  Artificial sequence

<220>
<223>  Highly mobile region of Savinase

<400>  40

Thr Val Gly Thr Asn Phe Thr Asp
1               5


<210>  41
<211>  7
<212>  PRT
<213>  Artificial sequence

<220>
<223>  Highly mobile region of Savinase

<400>  41

Val Pro Gly Glu Pro Ser Thr
1               5


<210>  42
<211>  6
<212>  PRT
<213>  Artificial sequence

<220>
<223>  Highly mobile region of Savinase

<400>  42

Asn Gly Gly Thr Gly Ser
1               5


<210>  43
<211>  6
<212>  PRT
<213>  Artificial sequence

<220>
<223>  Highly mobile region of Savinase

<400>  43

Ala Leu Asn Asn Ser Ile
1               5


<210>  44
<211>  7
<212>  PRT
<213>  Artificial sequence
```

```
<220>
<223>   Highly mobile region of Savinase

<400>   44

Asp Asp Gly Ser Gly Tyr Ala
1               5


<210>   45
<211>   7
<212>   PRT
<213>   Artificial sequence

<220>
<223>   Highly mobile region of Savinase

<400>   45

Ala Ser Gly Ser Gly Ser Val
1               5


<210>   46
<211>   7
<212>   PRT
<213>   Artificial sequence

<220>
<223>   Highly mobile region of Savinase

<400>   46

Trp Ala Gln Ser Pro Ala Ala
1               5


<210>   47
<211>   7
<212>   PRT
<213>   Artificial sequence

<220>
<223>   Highly mobile region of Savinase

<400>   47

Lys Gln Lys Asn Pro Ser Trp
1               5


<210>   48
<211>   6
<212>   PRT
<213>   Artificial sequence

<220>
<223>   Ion binding site

<400>   48

Leu Asn Asn Ser Ile Gly
1               5


<210>   49
<211>   3
<212>   PRT
```

```
<213>   Artificial sequence

<220>
<223>   Ion binding site

<400>   49

Gly Asp Ser
1


<210>   50
<211>   3
<212>   PRT
<213>   Artificial sequence

<220>
<223>   Ion binding site

<400>   50

Asp Ser Thr
1


<210>   51
<211>   5
<212>   PRT
<213>   Artificial sequence

<220>
<223>   Ion binding site

<400>   51

Gly Gly Ser Asn Gly
1               5
```